# FASCICULE DE BREVET EUROPEEN

(11) **EP 3 630 759 B1**
(45) Date de publication et mention de la délivrance du brevet: **01.05.2024**
(21) Numéro de dépôt: 18726802.4
(22) Date de dépôt: 23.05.2018
(51) Int. Cl.: C07D 471/04, C07D 239/42, A61K 31/519, A61P 35/00

(54) **COMPOSÉS INHIBITEURS DES CANAUX IONIQUES POUR LE TRAITEMENT DU CANCER**
IONENKANALINHIBITORVERBINDUNGEN ZUR KREBSBEHANDLUNG
COMPOUNDS USEFUL AS ION CHANNEL INHIBITORS FOR THE TREATMENT OF CANCER

(30) Priorité: 23.05.2017 FR 1754564
(43) Date de publication de la demande: 08.04.2020
(73) Titulaire: Centre National de la Recherche Scientifique, 75016 Paris (FR); Université d'Orléans, 45100 Orléans (FR); Université de Tours, 37020 Tours (FR); Institut National de la Santé et de la Recherche Médicale (INSERM), 75013 Paris 13 (FR); Chu, Nantes, 44000 Nantes (FR)
(72) Inventeur: ROUTIER, Sylvain, 45510 Tigy (FR); BURON, Frédéric, 45100 Orleans (FR); RODRIGUES, Nuno, 48000 Mende (FR); FOURRIERE-GRANDCLAUDE, Gaëlle, 77500 Chelles (FR); VANDIER, Christophe, 37520 La Riche (FR); CHANTOME, Aurélie, 37000 Tours (FR); POTIER-CARTEREAU, Marie, 37520 La Riche (FR); GUEGUINOU, Maxime, 37000 Tours (FR); MARIONNEAU-LAMBOT, Séverine, 44470 Thouare Sur Loire (FR)
(74) Mandataire: Novagraaf Technologies
(86) Numéro de dépôt international: PCT/EP2018/063543
(87) Numéro de publication internationale: WO 2018/215557

(56) Documents cités:
- EP-A1- 0 795 556
- EP-A1- 1 018 514
- WO-A1-97/18212
- WO-A1-2006/097441
- WO-A1-2010/093419
- WO-A1-2011/135259
- WO-A1-2012/125668
- WO-A2-2014/145512
- WO-A2-2015/027222
- CN-A- 103 664 903
- US-A- 5 654 307
- US-A1- 2017 071 944
- US-B1- 6 395 733
- MICHELLYS PIERRE-YVES ET AL: "Design and synthesis of novel selective anaplastic lymphoma kinase inhibitors", BIOORGANIC & MEDICINAL CHEMISTRY LETTERS, PERGAMON, AMSTERDAM, NL, vol. 26, no. 3, 17 novembre 2015 (2015-11-17), pages 1090-1096, XP029391884, ISSN: 0960-894X, DOI: 10.1016/J.BMCL.2015.11.049
- POTIER M ET AL: "Identification of SK3 channel as a new mediator of breast cancer cell migration", MOLECULAR CANCER THERAPEUTICS, AMERICAN ASSOCIATION FOR CANCER RESEARCH, US, vol. 5, no. 11, 1 novembre 2006 (2006-11-01), pages 2946-2953, XP002428454, ISSN: 1535-7163, DOI: 10.1158/1535-7163.MCT-06-0194
- Susan Lepri ET AL: "Structure?metabolism relationships in human- AOX: Chemical insights from a large database of aza-aromatic and amide compounds", Proceedings of the National Academy of Sciences, vol. 114, no. 16, 3 April 2017 (2017-04-03), pages E3178-E3187, XP055689694, ISSN: 0027-8424, DOI: 10.1073/pnas.1618881114 & Lepri Susan ET AL: "Supplementary Information Appendix Structure Metabolism Relationships In hAOX Enzyme. Chemical Insights Into A Large Database Of Aza-Aromatic And Amide Compounds Table S3. Susceptibility of quinoline-and isoquinoline-based compounds to hAOX. Table S4. Susceptibility of quinoxaline-and its isomers-ba", , 3 April 2017 (2017-04-03), XP55892688, Retrieved from the Internet: URL:https://doi.org/10.1073/pnas.161888111 4 [retrieved on 2022-02-16]
- Wu Chien-Huang ET AL: "Discovery of Novel Stem Cell Mobilizers That Target the CXCR4 Receptor", ChemMedChem Communications, vol. 7, no. 2, 20 December 2011 (2011-12-20), pages 209-212, XP055892465, DE ISSN: 1860-7179, DOI: 10.1002/cmdc.201100525
- Paul R. Gilson ET AL: "Optimization of 2-Anilino 4-Amino Substituted Quinazolines into Potent Antimalarial Agents with Oral in Vivo Activity", Journal of Medicinal Chemistry, vol. 60, no. 3, 26 January 2017 (2017-01-26), pages 1171-1188, XP055393734, US ISSN: 0022-2623, DOI: 10.1021/acs.jmedchem.6b01673

## Description

La présente invention a pour objet de nouveaux composés, notamment de type pyridopyrimidine, présentant des propriétés d'inhibition des canaux ioniques, notamment SK3. Elle a également pour objet l'utilisation desdits composés pour le traitement des cancers.

Les thérapeutiques majeures des présentations précoces des cancers sont la chirurgie et/ou la radiothérapie, et en adjuvant l'hormonothérapie et pour le cancer du sein, la chimiothérapie. L'apparition de métastases viscérales après un cancer du sein ou de la prostate correspond généralement au passage à une étape palliative. La durée ainsi que la qualité de la survie du patient dépendent de la sensibilité de la maladie aux traitements anticancéreux. Leur prévention ainsi que leur traitement impliquent de nouvelles approches. Actuellement, diverses cibles potentielles de voies biologiques sont à l'étude pour atteindre ce but.

Chez la femme, le cancer du sein représente la cause la plus importante de décès par cancer dans le monde. Le cancer du sein possède un tropisme osseux marqué et une dépendance aux hormones stéroïdes, facteurs favorisant la croissance tumorale, la survie cellulaire et l'invasion cellulaire.

Lorsqu'un cancer métastase, le pronostic vital est souvent engagé. Lors de la croissance tumorale (tumeur primaire), des cellules cancéreuses se détachent de la tumeur primitive et migrent, soit par voie lymphatique (les ganglions sont reliés entre eux par de fins canaux, l'ensemble constituant le système lymphatique), soit par voie sanguine. Cette aptitude d'un cancer à métastaser est très certainement déterminée par un ensemble de facteurs biologiques qui constituent aujourd'hui autant de pistes de recherche pour bloquer ce pouvoir métastatique d'un cancer.

Actuellement, il n'existe pas de thérapie ciblée contre la migration ou l'invasion des cellules cancéreuses, ces propriétés permettant pourtant aux cellules tumorales de sortir de la tumeur primitive, de pénétrer dans la circulation lymphatique et sanguine, et de se développer à distance dans des tissus non tumoraux de l'organisme entier. La recherche dans ce domaine anti-métastatique est peu développée ou n'a pour l'instant pas été en mesure d'apporter une réponse adéquate. C'est pourquoi l'identification tant de nouvelles cibles anti-métastatiques que de nouveaux médicaments à visée « anti-métastatique » sont une priorité et doivent s'intensifier.

Le document US5654307 concerne de nouveaux inhibiteurs d'aminopyridopyrimidine 4-substituée et d'aminopyridopyrimidine 4-substituée de la famille des récepteurs du facteur de croissance épidermique de la tyrosine kinase, et est cité ici comme arrière plan technologique.

Le document WO2014145512 est également connu de l'arrière plan technologique, les composés divulgués dans ce document ne comportant pas de motif tétrahydronaphtalène contrairement aux composés ciblés selon l'invention qui sont des inhibiteurs des canaux ioniques, et notamment du canal SK3.

Les canaux et échangeurs ioniques sont des protéines transmembranaires dont l'activité est étroitement liée à l'état des membranes cellulaires.

Parmi les nombreux canaux ioniques, le canal SK3, un canal de la famille des canaux potassiques de petite conductance activé par le calcium et dont l'expression est régulée par les oestrogènes, favorise la migration de cellules cancéreuses.

Par ailleurs, l'inhibition du canal SK3, dans des cellules cancéreuses épithéliales du sein, inhibait le développement de métastases osseuses. D'autre part, les cellules épithéliales cancéreuses prostatiques expriment la protéine SK3 contrairement aux cellules épithéliales non cancéreuses. Par conséquent, cette découverte se place dans un champ d'investigation émergent et indique que le canal SK3 pourrait être une nouvelle cible thérapeutique contre toute forme de cancer ayant tendance à métastaser, surtout vers les tissus osseux.

La présente invention a pour but de fournir de nouveaux inhibiteurs des canaux ioniques, et notamment du canal SK3.

La présente invention a également pour but de fournir de nouveaux inhibiteurs des canaux ioniques SK3 et/ou SK2.

La présente invention a pour but en outre de fournir des composés capables d'induire un effet anti-métastatique.

Un composé de formule générale (I) suivante, est présenté à titre illustratif : 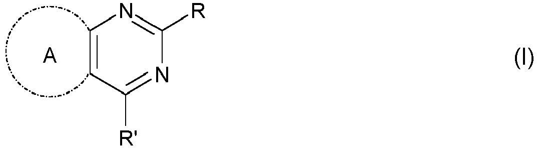
. A₁ représentant un radical -NH- ou un radical -NH-CH₂- ;
. Cy₁ représentant :
   *soit un groupe phényle, éventuellement substitué,
   *soit un cycle aliphatique comprenant au moins 5 atomes de carbone, éventuellement substitué, et éventuellement fusionné à un cycle (hétéro)aryle comprenant de 2 à 6 atomes de carbone,
   *soit un cycle hétéroaliphatique comprenant au moins 4 atomes de carbone, éventuellement substitué, et éventuellement fusionné à un cycle (hétéro)aryle comprenant de 2 à 6 atomes de carbone ;
   lesdits cycles (hétéro)aliphatiques pouvant être substitués par au moins un substituant choisi parmi les groupes Rₐ, ORₐ, OCH₂OCH₃, C(=O)Rₐ, C(=O)ORₐ, NRₐR_{b} ou F, Rₐ et R_{b} étant tels que définis ci-dessus,
Cy₁ étant un groupe phényle substitué ou un cycle (hétéro)aliphatique, substitué ou non, lorsque R est -NH-Cy₁ et R' est H ;
- A représente un cycle fusionné (hétéro)aromatique comprenant de 5 à 10, de préférence de 5 à 7 atomes, notamment comprenant de 1 à 6 atomes de carbone et éventuellement de 1 à 3 hétéroatomes notamment choisis parmi N, O et S,
   A étant éventuellement substitué par au moins un substituant choisi parmi : les atomes d'halogène, les groupes cyano, NO₂, (C₁-C₆)alkyles, (hétéro)alkyles, (hétéro)cycloalkyles, alcènes, alcynes, carbonyles ou amides ;
   ainsi que ses sels pharmaceutiquement acceptables,
   ledit composé de formule (I) étant sous forme de stéréoisomère pur ou sous forme de mélange d'énantiomères et/ou de diastéréoisomères, y compris de mélanges racémiques,
   pour son utilisation pour le traitement des cancers.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels peuvent être préparés avec des acides pharmaceutiquement acceptables, mais les sels d'autres acides utiles, par exemple, pour la purification ou l'isolement des composés de formule (I) font également partie de l'invention.

Selon l'invention, le terme "(hétéro)cycloalkyle" englobe à la fois les termes "cycloalkyle" et "hétérocycloalkyle", ces termes étant tels que définis ci-après.

Selon l'invention, le terme "(hétéro)alkyle" englobe à la fois les termes "alkyle" et "hétéroalkyle", ces termes étant tels que définis ci-après.

Selon l'invention, le terme "(hétéro)aryle" englobe à la fois les termes "aryle" et "hétéroaryle", ces termes étant tels que définis ci-après.

Selon l'invention, le terme "(hétéro)aromatique" englobe à la fois les termes "aromatique" et "hétéroaromatique", ces termes étant tels que définis ci-après.

Selon l'invention, le terme "(hétéro)aliphatique" englobe à la fois les termes "aliphatique" et "hétéroaliphatique", ces termes étant tels que définis ci-après.

Dans le cadre de la présente invention, on entend par groupe (Cₜ-C_{z}) un groupe comprenant une chaîne carbonée pouvant avoir de t à z atomes de carbone, par exemple C₁-C₆ une chaîne carbonée qui peut avoir de 1 à 6 atomes de carbone.

Dans le cadre de la présente invention, le terme "atome d'halogène" désigne les atomes fluor, chlore, brome ou iode.

Selon l'invention, le terme "alkyle" désigne des groupes aliphatiques hydrocarbonés, saturés, linéaires ou ramifiés comprenant, sauf mention contraire, de 1 à 6 atomes de carbone. A titre d'exemples, on peut citer les groupes méthyle, éthyle, n-propyle, isopropyle, butyle, isobutyle, tertbutyle ou pentyle.

Selon l'invention, le terme "hétéroalkyle" désigne des groupes aliphatiques hydrocarbonés, saturés, linéaires ou ramifiés comprenant, sauf mention contraire, de 1 à 6 atomes de carbone, et comprenant au moins un hétéroatome. A titre d'exemples, on peut citer les groupes (C₁-C₆)alcoxy, ou encore les groupes amines, ces groupes pouvant en outre être substitués. Parmi ces groupes, on peut citer des groupes -O-A₂-O-A'₂-NH₂ ou -NH-A₂-NH₂, les radicaux A₂ et A'₂ représentant des radicaux alkylènes comprenant de 1 à 4 atomes de carbone.

Selon l'invention, le terme "cycloalkyle" désigne des groupes carbonés cycliques comprenant, sauf mention contraire, de 3 à 6 atomes de carbone. A titre d'exemples, on peut citer les groupes cyclopropyle, cyclobutyle, cyclopentyle ou cyclohexyle.

Selon l'invention, les groupes aryles sont des groupes aromatiques cycliques comprenant entre 6 et 10 atomes de carbone. A titre d'exemples de groupes aryles, on peut citer les groupes phényle ou naphtyle.

Selon l'invention, le terme "hétéroaryle" désigne un groupe monocyclique ou bicyclique aromatique de 5 à 10 chaînons contenant de 1 à 4 hétéroatomes choisis parmi O, S ou N.

A titre d'exemples, on peut citer les groupes imidazolyle, thiazolyle, oxazolyle, furanyle, thiophényle, pyrazolyle, oxadiazolyle, tétrazolyle, pyridinyle, pyrazinyle, pyrimidinyle, pyridazinyle, indolyle, benzofuranyle, benzothiophényle, benzoxazolyle, benzimidazolyle, indazolyle, benzothiazolyle, isobenzothiazolyle, benzotriazolyle, quinoléinyle, isoquinoléinyle.

A titre d'hétéroaryle comprenant 5 à 6 atomes, dont 1 à 4 atomes d'azote, on peut notamment citer les groupes représentatifs suivants : pyrrolyle, pyrazolyle, 1,2,3-triazolyle, 1,2,4-triazolyle, tétrazolyle et 1,2,3-triazinyle.

On peut également citer, à titre d'hétéroaryle, le thiophényle, l'oxazolyle, le furazanyle, le 1,2,4-thiadiazolyle, le naphthyridinyle, le quinoxalinyle, le phtalazinyle, l'imidazo[1,2-a]pyridine, l'imidazo[2,1-b]thiazolyle, le cinnolinyle, le benzofurazanyle, l'azaindolyle, le benzimidazolyle, le benzothiophényle, le thiénopyridyle, le thiénopyrimidinyle, le pyrrolopyridyle, l'imidazopyridyle, le benzoazaindole, le 1,2,4-triazinyle, l'indolizinyle, l'isoxazolyle, l'isoquinolinyle, l'isothiazolyle, le purinyle, le quinazolinyle, le quinolinyle, l'isoquinolyle, le 1,3,4-thiadiazolyle, le thiazolyle, l'isothiazolyle, le carbazolyle, ainsi que les groupes correspondants issus de leur fusion ou de la fusion avec le noyau phényle.

Selon l'invention le terme "hétérocycloalkyle" désigne un groupe monocyclique ou bicyclique saturé ou partiellement insaturé de 4 à 10 chaînons, comprenant de un à trois hétéroatomes choisis parmi O, S ou N, le groupe hétérocycloalkyle pouvant être rattaché au reste de la molécule par un atome de carbone ou par un hétéroatome.

A titre d'hétérocycloalkyle saturé comprenant de 5 à 6 atomes, on peut citer l'oxétanyle, le tétrahydrofuranyle, le dioxolanyle, le pyrrolidinyle, l'azépinyle, l'oxazépinyle, le pyrazolidinyle, l'imidazolidinyle, le tétrahydrothiophényle, le dithiolanyle, le thiazolidinyle, le tétrahydropyranyle, le tétrahydropyridinyle, le dioxanyle, le morpholinyle, le pipéridinyle, le pipérazinyle, le tétrahydrothiopyranyle, le dithianyle, le thiomorpholinyle, ou l'isoxazolidinyle.

Les radicaux "alkyle", "cycloalkyle", "aryle", "hétéroaryle" et "hétérocycloalkyle" susmentionnés peuvent être substitués par un ou plusieurs substituants. Parmi ces substituants, on peut citer les groupes suivants : amino, hydroxy, thiol, oxo, halogène, alkyle, alcoxy, alkylthio, alkylamino, aryloxy, arylalcoxy, cyano, trifluorométhyle, carboxy ou carboxyalkyle.

Lorsqu'un radical alkyle est substitué par un groupe aryle, on parle de radical "arylalkyle" ou "aralkyle". Les radicaux "arylalkyles" ou "aralkyles" sont des radicaux aryl-alkyl-, les groupes aryles et alkyles étant tels que définis ci-dessus. Parmi les radicaux arylalkyles, on peut notamment citer le radical benzyle ou phénéthyle.

Selon l'invention, le terme "alkylthio" désigne un groupe -S-alkyle, le groupe alkyle étant tel que défini ci-dessus.

Selon l'invention, le terme "alkylamino" désigne un groupe -NH-alkyle ou N(alkyle)₂, le groupe alkyle étant tel que défini ci-dessus.

Selon l'invention, le terme "aryloxy" désigne un groupe -O-aryle, le groupe aryle étant tel que défini ci-dessus.

Selon l'invention, le terme "arylalcoxy" désigne un groupe aryl-alcoxy-, les groupes aryles et alcoxy étant tels que définis ci-dessus.

Selon l'invention, le terme "carboxyalkyle" désigne un groupe HOOC-alkyl-, le groupe alkyle étant tel que défini ci-dessus. Comme exemple de groupes carboxyalkyles, on peut citer notamment le carboxyméthyle ou le carboxyéthyle.

Selon l'invention, le terme "carboxyle" désigne un groupe C(=O)OH et le terme "oxo" ou "carbonyle" désigne un groupe C(=O).

Selon l'invention, le terme "alcène" désigne un groupe aliphatique hydrocarboné acyclique, linéaire ou ramifié, comprenant une double liaison carbone-carbone et répondant à la formule CₙH₂ₙ. Selon l'invention, les alcènes comprenant, sauf mention contraire, de 2 à 6 atomes de carbone. Selon la présente invention, le radical "alcényle" désigne un radical correspondant au groupe alcène susmentionné auquel on a enlevé un atome d'hydrogène.

Selon l'invention, le terme "alcyne" désigne un groupe aliphatique hydrocarboné acyclique, linéaire ou ramifié, comprenant une triple liaison carbone-carbone et répondant à la formule CₙH₂ₙ₋₂. Selon l'invention, les alcènes comprenant, sauf mention contraire, de 2 à 6 atomes de carbone. Selon la présente invention, le radical "alcynyle" désigne un radical correspondant au groupe alcyne susmentionné auquel on a enlevé un atome d'hydrogène. Selon l'invention, les radicaux alcynyles peuvent être substitués, par exemple par un groupe alkyle ou un groupe alkylamino.

Selon l'invention, le terme "alcyne" désigne un groupe aliphatique hydrocarboné acyclique, linéaire ou ramifié, comprenant une triple liaison carbone-carbone et répondant à la formule CₙH₂ₙ₋₂. Selon l'invention, les alcènes comprenant, sauf mention contraire, de 2 à 6 atomes de carbone. Selon la présente invention, le radical "alcynyle" désigne un radical correspondant au groupe alcyne susmentionné auquel on a enlevé un atome d'hydrogène. Selon l'invention, les radicaux alcynyles peuvent être substitués, par exemple par un groupe alkyle ou un groupe alkylamino.

Selon l'invention, le terme "carbonyle" désigne à la fois les cétones et les aldéhydes, mais également les acides carboxyliques et dérivés.

Selon l'invention, le terme "amide" désigne un groupe dérivé d'un acide carboxylique de type R-C(=O)-NR'R".

Selon l'invention, la fusion entre le cycle A et l'hétérocycle des composés de formule (I) peut être effectué par l'intermédiaire d'un hétéroatome, par exemple par un atome d'azote.

Selon un mode de réalisation, lorsque, dans la formule (I) ne relevant pas de l'invention, A est substitué par au moins un substituant, celui-ci est choisi par exemple parmi les atomes d'halogène, les groupes cyano, NO₂, (C₁-C₆)alkyles, (hétéro)alkyles, (hétéro)cycloalkyles, les radicaux alcényles, alcynyles, ou encore les composés carbonylés (c'est-à-dire comprenant un groupe C(=O)) ou par un radical amido de type -C(=O)-NR'R", R' et R" représentant des groupes (C₁-C₆)alkyles par exemple.

Une famille autre de composés ne relevant pas de l'invention est constituée par des composés de formule (II) suivante : 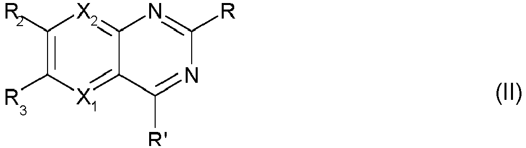 dans laquelle :
- R et R' sont tels que définis ci-dessus dans la formule (I),
- soit X₁ représente un atome d'azote et X₂ représente un groupe C(R₄),
- soit X₁ représente un groupe C(R₅) et X₂ représente un atome d'azote,
- soit X₁ et X₂ représentent un atome d'azote,
- R₂, R₃, R₄ et R₅ représentent, indépendamment les uns des autres, H ou un substituant choisi dans le groupe constitué des atomes d'halogène, des groupes (C₁-C₆)(cyclo)alkyles, des groupes NRₐR_{b} et des groupes ORₐ, Rₐ et R_{b}, indépendamment l'un de l'autre, représentant H ou un groupe (C₁-C₆)alkyle.

Selon un mode de réalisation, dans la formule (II), X₁ représente un atome d'azote et X₂ représente un groupe C(R₄).

Selon un mode de réalisation, dans la formule (II), X₁ représente un groupe C(R₅) et X₂ représente un atome d'azote.

Selon un mode de réalisation, dans la formule (II), X₁ représente un atome d'azote, X₂ représente un groupe C(R₄), R représente un groupe R₁ et R' représente un groupe -A₁-Cy₁.

Selon un mode de réalisation, dans la formule (II), X₁ représente un groupe C(R₅), X₂ représente un atome d'azote, R représente un groupe R₁ et R' représente un groupe -A₁-Cy₁.

Selon un mode de réalisation, dans la formule (II), X₁ représente un groupe C(R₅), X₂ représente un atome d'azote, R représente un groupe R₁ et R' représente un groupe -A₁-Cy₁.

Selon un mode de réalisation, dans la formule (II), X₁ représente un atome d'azote, X₂ représente un groupe C(R₄), R représente un groupe R₁ et R' représente un groupe -A₁-Cy₁.

Une famille de composés ne relevant pas de l'invention est constituée par des composés de formule (III) suivante : 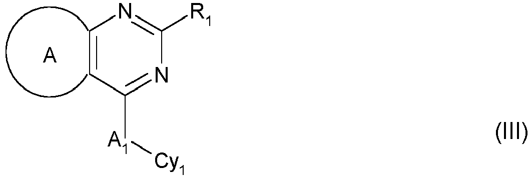 dans laquelle A, R₁, A₁ et Cy₁ sont tels que définis ci-dessus dans la formule (I).

Un composé de formule (III) correspond à un composé de formule (I) dans laquelle R représente un groupe R₁ et R' représente un groupe -A₁-Cy₁.

De préférence, dans la formule (III), A représente un cycle fusionné hétéroaromatique comprenant de 5 à 7 atomes, comprenant de 1 à 6 atomes de carbone et au moins un atome d'azote.

De préférence, dans la formule (III), Cy₁ représente un cycle aliphatique ou un cycle hétéroaliphatique tels que définis ci-dessus dans la formule (I), lesdits cycles aliphatique ou hétéroaliphatique étant éventuellement fusionnés à un cycle hétéroaryle tel que défini ci-dessus.

Une autre famille de composés ne relevant pas de l'invention est constituée par des composés de formule (III-1) suivante : 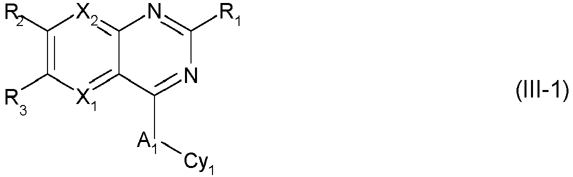 dans laquelle :
- R₁, A₁ et Cy₁ sont tels que définis ci-dessus dans la formule (I),
- soit X₁ représente un atome d'azote et X₂ représente un groupe C(R₄),
- soit X₁ représente un groupe C(R₅) et X₂ représente un atome d'azote,
- soit X₁ et X₂ représentent un atome d'azote,
- R₂, R₃, R₄ et R₅, représentent, indépendamment les uns des autres, H ou un substituant choisi dans le groupe constitué des atomes d'halogène, des groupes (C₁-C₆)(cyclo)alkyles, des groupes NRₐR_{b} et des groupes ORₐ, Rₐ et R_{b}, indépendamment l'un de l'autre, représentant H ou un groupe (C₁-C₆)alkyle.

Un composé de formule (III-1) correspond à un composé de formule (III) dans laquelle A représente un cycle fusionné hétéroaromatique, éventuellement substitué (par R₂ et/ou R₃), comprenant 6 atomes, dont 4 ou 5 atomes de carbone et 1 ou 2 atome(s) d'azote, R représente un groupe R₁ et R' représente un groupe -A₁-Cy₁.

Une sous-famille de composés est constituée par des composés de formule (III-1) telle que définie ci-dessus, dans laquelle soit X₁ représente un atome d'azote et X₂ représente un groupe C(R₄), soit X₁ représente un groupe C(R₅) et X₂ représente un atome d'azote.

Une famille de composés ne relevant pas de l'invention est constituée par des composés de formule (IV) suivante : 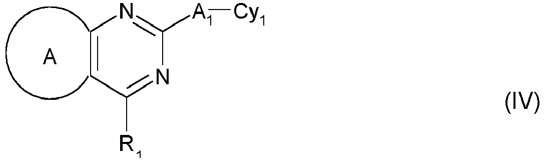 dans laquelle A, R₁, A₁ et Cy₁ sont tels que définis dans la revendication 1.

Un composé de formule (IV) correspond à un composé de formule (I) dans laquelle R représente un groupe -A₁-Cy₁ et R' représente un groupe R₁.

De préférence, dans la formule (IV), A représente un cycle fusionné hétéroaromatique comprenant de 5 à 7 atomes, comprenant de 1 à 6 atomes de carbone et au moins un atome d'azote.

Une autre famille de composés ne relevant pas de l'invention est constituée par des composés de formule (IV-1) suivante : 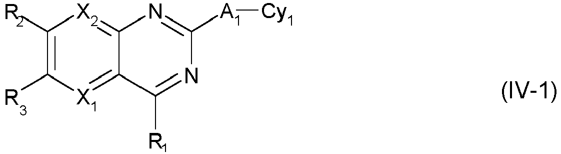 dans laquelle :
- R₁, A₁ et Cy₁ sont tels que définis dans la formule (I),
- soit X₁ représente un atome d'azote et X₂ représente un groupe C(R₄),
- soit X₁ représente un groupe C(R₅) et X₂ représente un atome d'azote,
- R₂, R₃, R₄ et R₅, représentent, indépendamment les uns des autres, H ou un substituant choisi dans le groupe constitué des atomes d'halogène, des groupes (C₁-C₆)(cyclo)alkyles, des groupes NRₐR_{b} et des groupes ORₐ, Rₐ et R_{b}, indépendamment l'un de l'autre, représentant H ou un groupe (C₁-C₆)alkyle.

Un composé de formule (IV-1) correspond à un composé de formule (IV) dans laquelle A représente un cycle fusionné hétéroaromatique, éventuellement substitué (par R₂ et/ou R₃), comprenant 6 atomes, dont 4 ou 5 atomes de carbone et 1 ou 2 atome(s) d'azote, R représente un groupe R₁ et R' représente un groupe -A₁-Cy₁.

Une sous-famille de composés est constituée par des composés de formule (IV-1) telle que définie ci-dessus, dans laquelle soit X₁ représente un atome d'azote et X₂ représente un groupe C(R₄), soit X₁ représente un groupe C(R₅) et X₂ représente un atome d'azote.

Selon un mode de réalisation, dans les formules (II), (III-1) et (IV-1) susmentionnées, R₃ est H.

Selon un mode de réalisation, dans les formules (II), (III-1) et (IV-1) susmentionnées, R₂ et R₃ sont H.

Une autre famille de composés ne relevant pas de l'invention est constituée par des composés de formule (III-2) suivante : 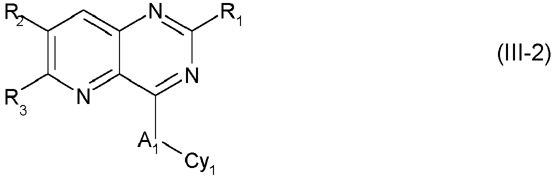 dans laquelle :
- R₁, A₁ et Cy₁ sont tels que définis ci-dessus dans la formule (I),
- R₂ et R₃ représentent, indépendamment les uns des autres, H ou un substituant choisi dans le groupe constitué des atomes d'halogène, des groupes (C₁-C₆)(cyclo)alkyles, des groupes NRₐR_{b} et des groupes ORₐ, Rₐ et R_{b}, indépendamment l'un de l'autre, représentant H ou un groupe (C₁-C₆)alkyle.

De préférence, dans la formule (III-2), R₃ est H.

De préférence, dans la formule (III-2), R₂ et R₃ sont H.

Une autre famille de composés ne relevant pas de l'invention est constituée par des composés de formule (III-3) suivante : 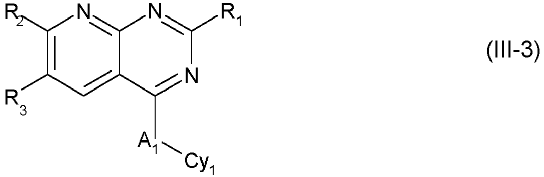 dans laquelle :
- R₁, A₁ et Cy₁ sont tels que définis ci-dessus dans la formule (I),
- R₂ et R₃ représentent, indépendamment les uns des autres, H ou un substituant choisi dans le groupe constitué des atomes d'halogène, des groupes (C₁-C₆)(cyclo)alkyles, des groupes NRₐR_{b} et des groupes ORₐ, Rₐ et R_{b}, indépendamment l'un de l'autre, représentant H ou un groupe (C₁-C₆)alkyle.

De préférence, dans la formule (III-3), R₃ est H.

De préférence, dans la formule (III-3), R₂ et R₃ sont H.

Une autre famille de composés ne relevant pas de l'invention est constituée par des composés de formule (IV-2) suivante : 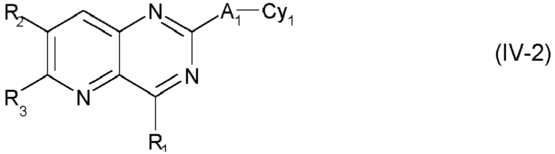 dans laquelle :
- R₁, A₁ et Cy₁ sont tels que définis ci-dessus dans la formule (I),
- R₂ et R₃ représentent, indépendamment les uns des autres, H ou un substituant choisi dans le groupe constitué des atomes d'halogène, des groupes (C₁-C₆)(cyclo)alkyles, des groupes NRₐR_{b} et des groupes ORₐ, Rₐ et R_{b}, indépendamment l'un de l'autre, représentant H ou un groupe (C₁-C₆)alkyle.

De préférence, dans la formule (IV-2), R₃ est H.

De préférence, dans la formule (IV-2), R₂ et R₃ sont H.

Une autre famille de composés ne relevant pas de l'invention est constituée par des composés de formule (IV-3) suivante : 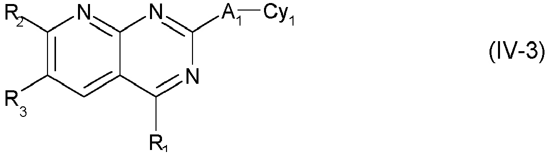 dans laquelle :
- R₁, A₁ et Cy₁ sont tels que définis ci-dessus dans la formule (I),
- R₂ et R₃ représentent, indépendamment les uns des autres, H ou un substituant choisi dans le groupe constitué des atomes d'halogène, des groupes (C₁-C₆)(cyclo)alkyles, des groupes NRₐR_{b} et des groupes ORₐ, Rₐ et R_{b}, indépendamment l'un de l'autre, représentant H ou un groupe (C₁-C₆)alkyle.

De préférence, dans la formule (IV-3), R₃ est H.

De préférence, dans la formule (IV-3), R₂ et R₃ sont H.

La présente invention concerne les composés de formule (III-4) suivante : 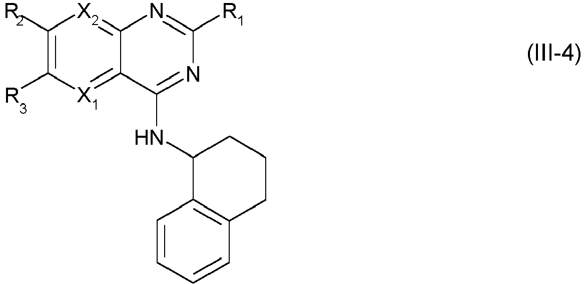 dans laquelle :
- X₁, X₂ et R₁ sont tels que définis ci-dessus dans la formule (I),
- R₂ et R₃ représentent, indépendamment les uns des autres, H ou un substituant choisi dans le groupe constitué des atomes d'halogène, des groupes (C₁-C₆)(cyclo)alkyles, des groupes NRₐR_{b} et des groupes ORₐ, Rₐ et R_{b}, indépendamment l'un de l'autre, représentant H ou un groupe (C₁-C₆)alkyle.

De préférence, dans la formule (III-4), R₃ est H.

De préférence, dans la formule (III-4), R₂ et R₃ sont H.

De préférence, dans la formule (III-4), X₁ représente un atome d'azote et X₂ représente un groupe CH.

De préférence, dans la formule (III-4), X₁ représente un groupe CH et X₂ représente un atome d'azote.

Une autre famille de composés ne rentrant pas dans le cadre de l'invention est constituée par des composés de formule (IV-4) suivante : 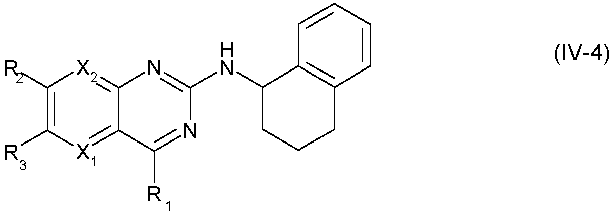 dans laquelle :
- X₁, X₂ et R₁ sont tels que définis ci-dessus dans la formule (I),
- R₂ et R₃ représentent, indépendamment les uns des autres, H ou un substituant choisi dans le groupe constitué des atomes d'halogène, des groupes (C₁-C₆)(cyclo)alkyles, des groupes NRₐR_{b} et des groupes ORₐ, Rₐ et R_{b}, indépendamment l'un de l'autre, représentant H ou un groupe (C₁-C₆)alkyle.

De préférence, dans la formule (IV-4), R₃ est H.

De préférence, dans la formule (IV-4), R₂ et R₃ sont H.

De préférence, dans la formule (IV-4), X₁ représente un atome d'azote et X₂ représente un groupe CH.

De préférence, dans la formule (IV-4), X₁ représente un groupe CH et X₂ représente un atome d'azote.

Parmi les composés préférés utilisés selon l'invention, on peut citer les composés suivants :

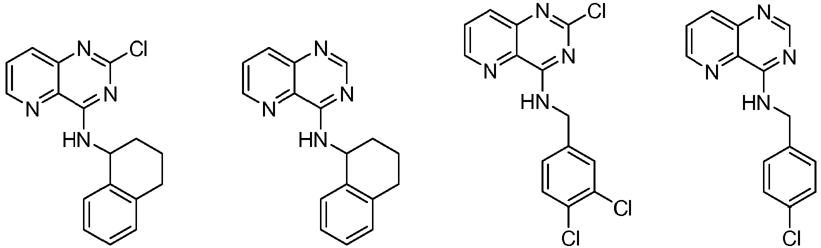

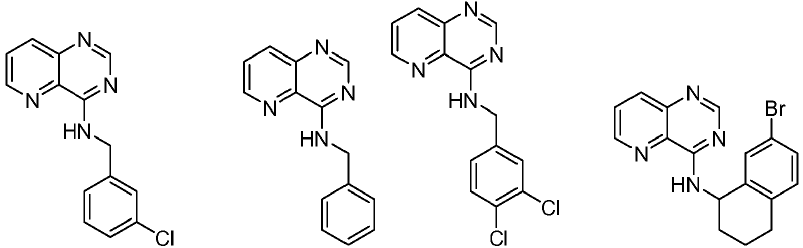

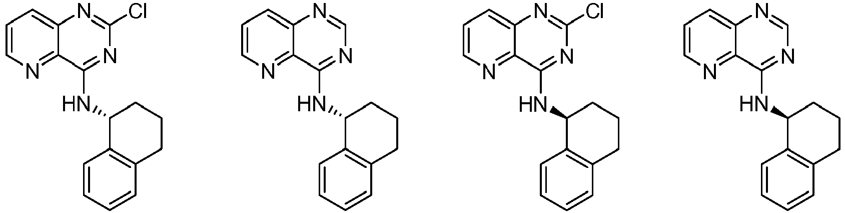

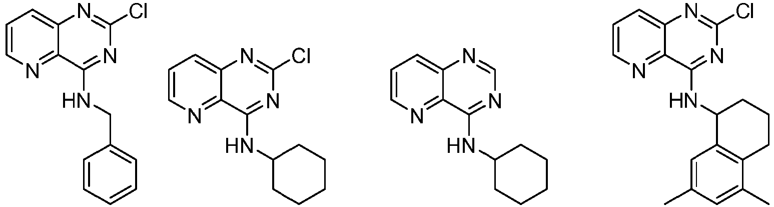

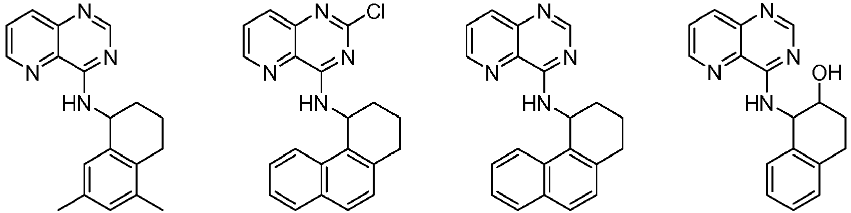

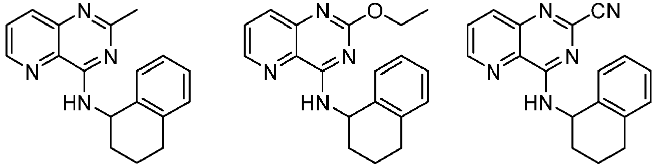

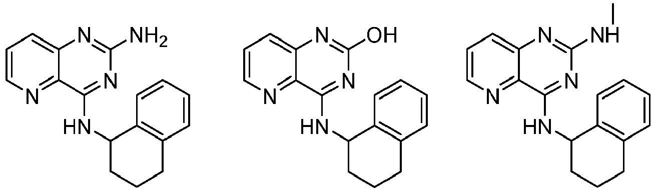

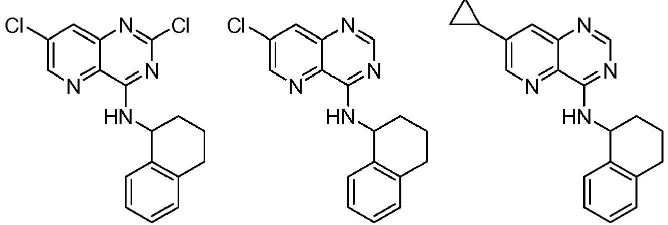

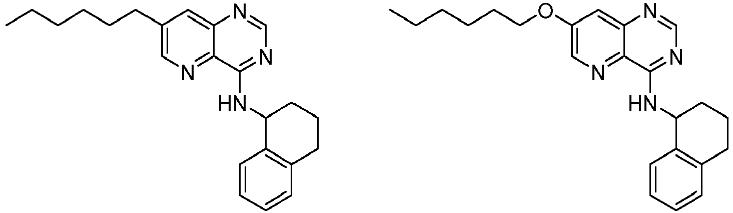

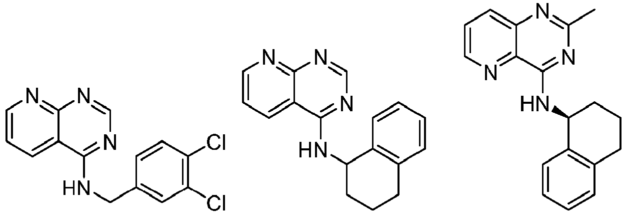

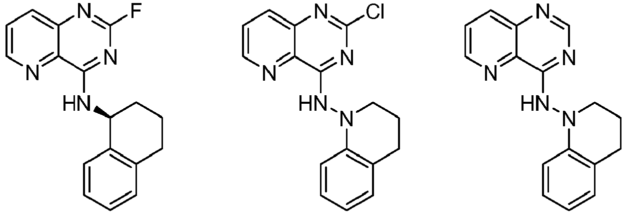

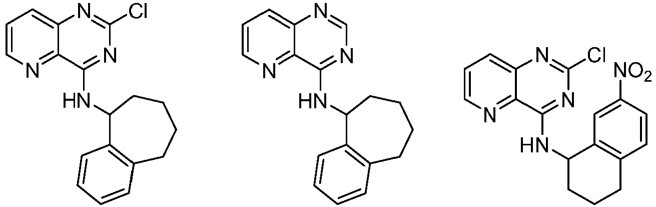

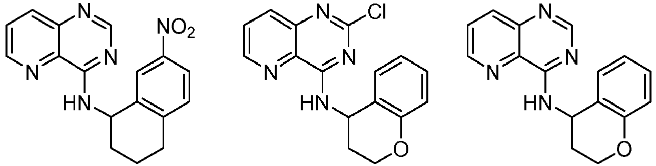

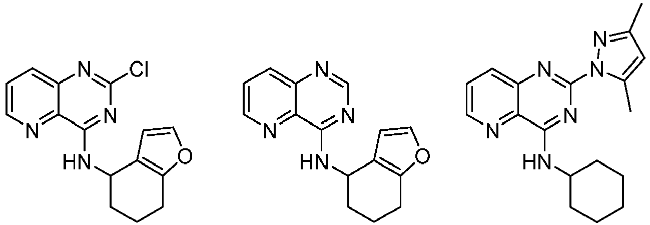

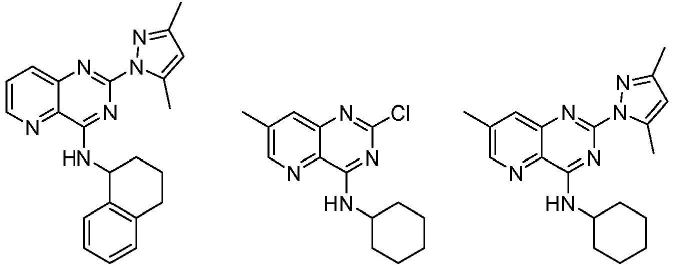

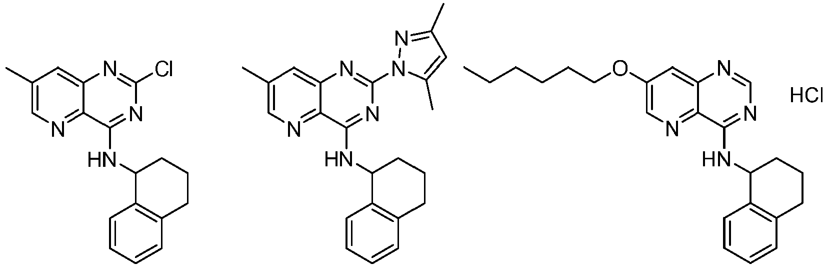

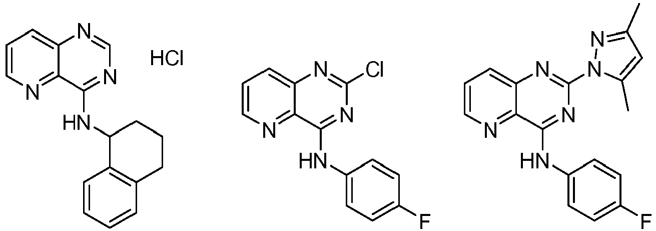

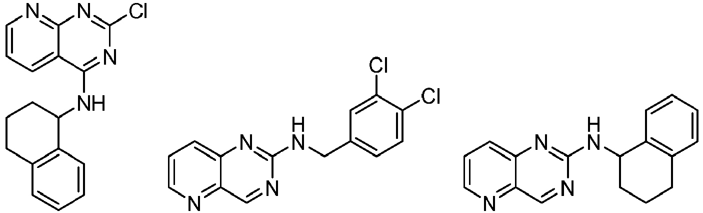

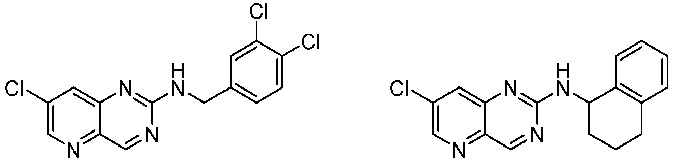

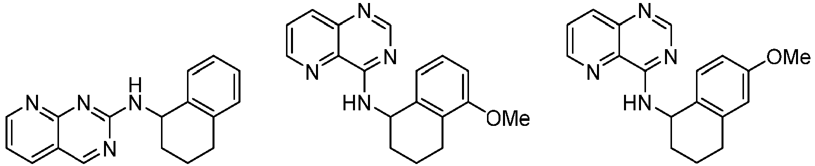

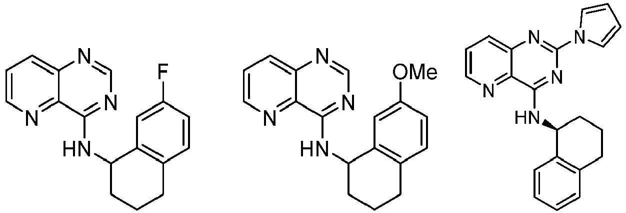

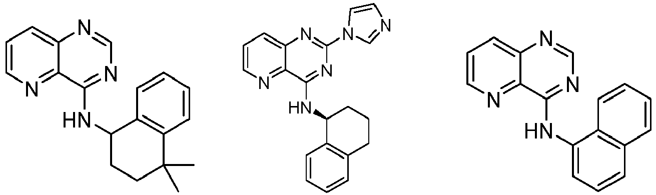

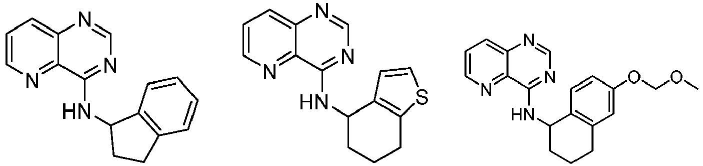

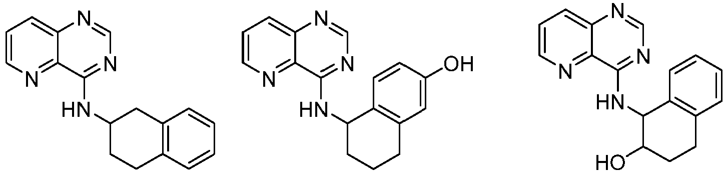

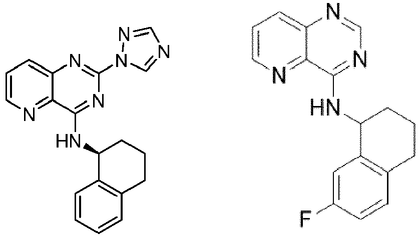

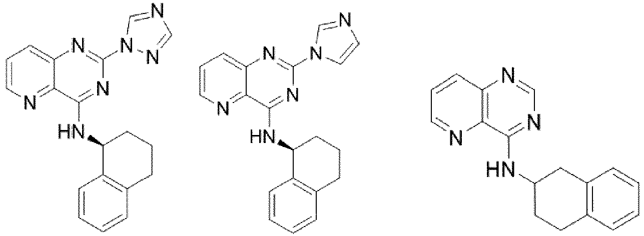

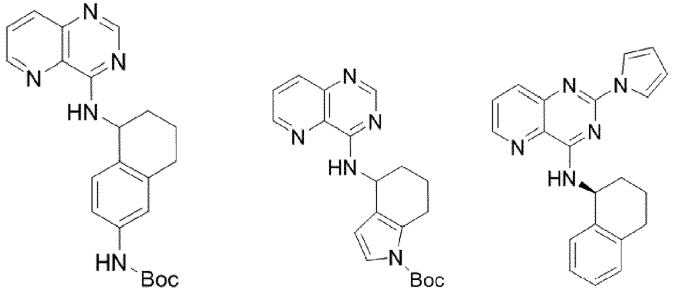

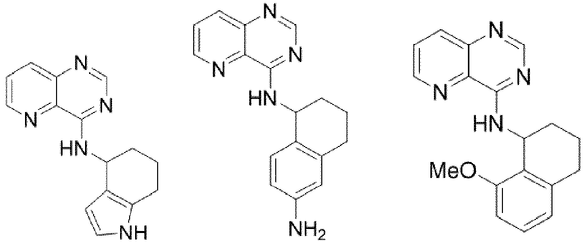

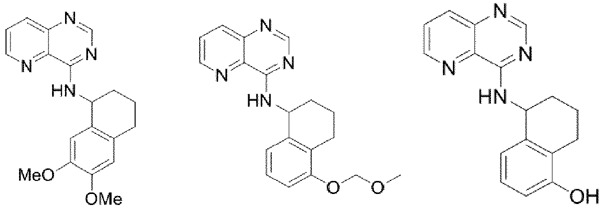

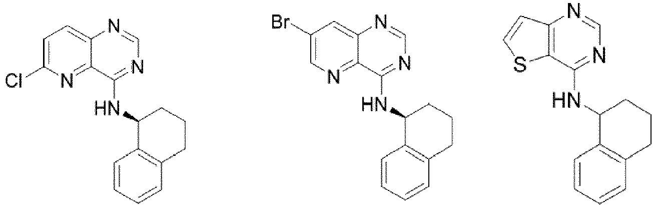

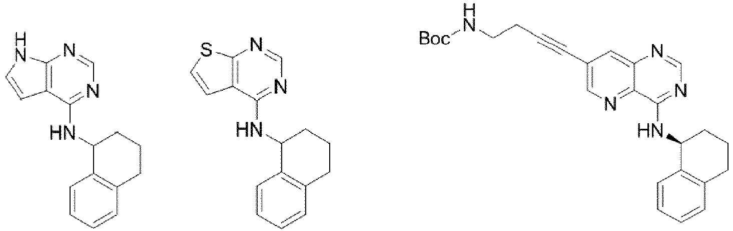

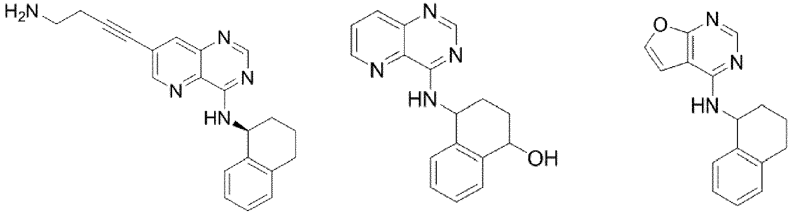

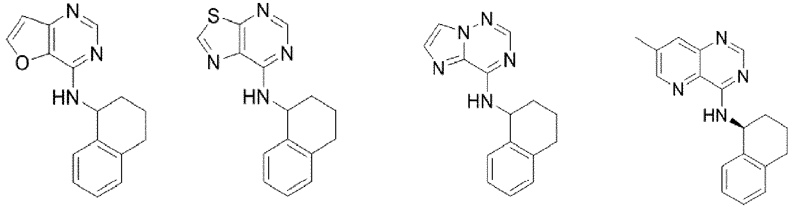

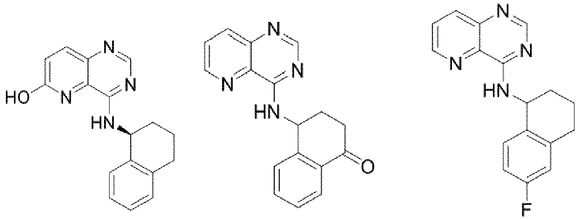

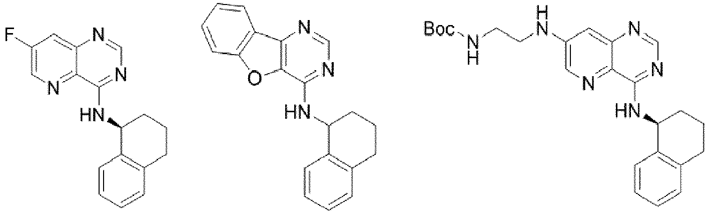

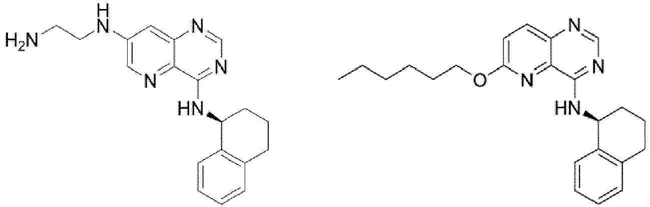

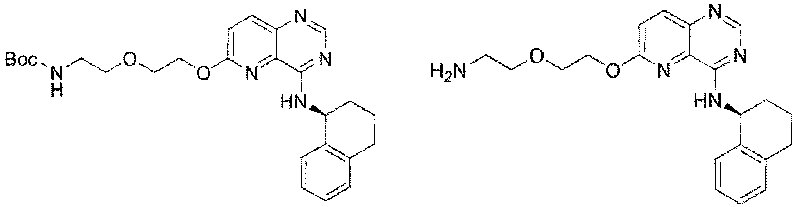

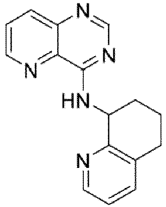

Peut être également cité à titre illustratif un composé de formule (V) : 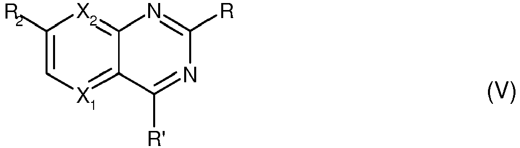 dans laquelle :
- R et R' sont tels que définis ci-dessus dans la formule (I),
- soit X₁ représente un atome d'azote et X₂ représente un groupe C(R₄),
- soit X₁ représente un groupe C(R₅) et X₂ représente un atome d'azote,
- R₂, R₄ et R₅, représentent, indépendamment les uns des autres, H ou un substituant choisi dans le groupe constitué des atomes d'halogène, des groupes (C₁-C₆)(cyclo)alkyles, des groupes NRₐR_{b} et des groupes ORₐ, Rₐ et R_{b}, indépendamment l'un de l'autre, représentant H ou un groupe (C₁-C₆)alkyle

ainsi que ses sels pharmaceutiquement acceptables,
ledit composé de formule (I) étant sous forme de stéréoisomère pur ou sous forme de mélange d'énantiomères et/ou de diastéréoisomères, y compris de mélanges racémiques,
à l'exception des composés suivants :

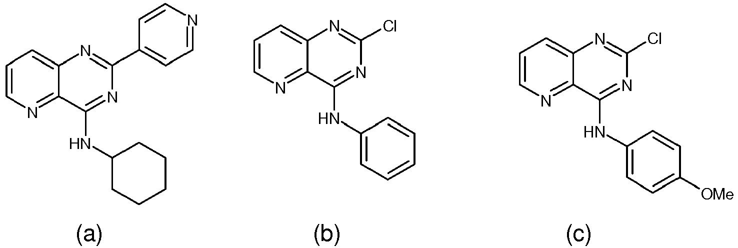

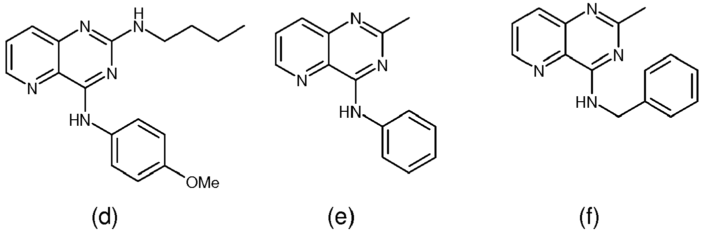

De préférence, dans la formule (V), R₂ est H.

De préférence, dans la formule (V), X₁ représente un atome d'azote et X₂ représente un groupe CH.

De préférence, dans la formule (V), X₁ représente un groupe CH et X₂ représente un atome d'azote.

Selon un mode de réalisation, des composés ne relevant pas de l'invention répondent à la formule générale (VI) suivante : 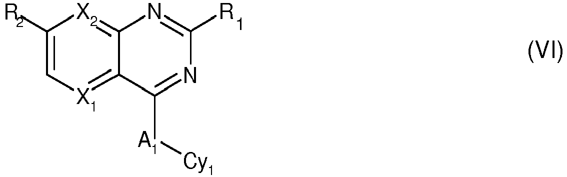 dans laquelle :
- R₁, A₁ et Cy₁ sont tels que définis ci-dessus dans la formule (I),
- soit X₁ représente un atome d'azote et X₂ représente un groupe C(R₄),
- soit X₁ représente un groupe C(R₅) et X₂ représente un atome d'azote,
- R₂, R₄ et R₅, représentent, indépendamment les uns des autres, H ou un substituant choisi dans le groupe constitué des atomes d'halogène, des groupes (C₁-C₆)(cyclo)alkyles, des groupes NRₐR_{b} et des groupes ORₐ, Rₐ et R_{b}, indépendamment l'un de l'autre, représentant H ou un groupe (C₁-C₆)alkyle,
à l'exception des composés (a), (b), (c), (d), (e) et (f) susmentionnés.

De préférence, dans la formule (VI), R₂ est H.

De préférence, dans la formule (VI), X₁ représente un atome d'azote et X₂ représente un groupe CH.

De préférence, dans la formule (VI), X₁ représente un groupe CH et X₂ représente un atome d'azote.

Selon un mode de réalisation, des composés de relevant pas de l'invention répondent à la formule générale (VII) suivante : 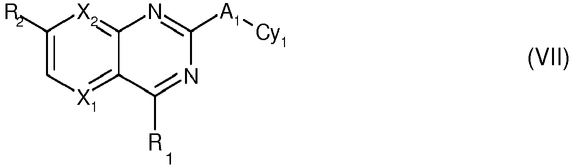 dans laquelle :
- R₁, A₁ et Cy₁ sont tels que définis ci-dessus dans la formule (I),
- soit X₁ représente un atome d'azote et X₂ représente un groupe C(R₄),
- soit X₁ représente un groupe C(R₅) et X₂ représente un atome d'azote,
- R₂, R₄ et R₅, représentent, indépendamment les uns des autres, H ou un substituant choisi dans le groupe constitué des atomes d'halogène, des groupes (C₁-C₆)(cyclo)alkyles, des groupes NRₐR_{b} et des groupes ORₐ, Rₐ et R_{b}, indépendamment l'un de l'autre, représentant H ou un groupe (C₁-C₆)alkyle.

De préférence, dans la formule (VII), R₂ est H.

De préférence, dans la formule (VII), X₁ représente un atome d'azote et X₂ représente un groupe CH.

De préférence, dans la formule (VII), X₁ représente un groupe CH et X₂ représente un atome d'azote.

Selon un mode de réalisation, les composés de l'invention répondent à la formule générale (VI-1) suivante : 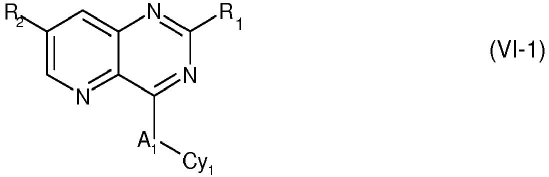 dans laquelle :
- R₁, A₁ et Cy₁ sont tels que définis ci-dessus dans la formule (I),
- R₂ représente H ou un substituant choisi dans le groupe constitué des atomes d'halogène, des groupes (C₁-C₆)(cyclo)alkyles, des groupes NRₐR_{b} et des groupes ORₐ, Rₐ et R_{b}, indépendamment l'un de l'autre, représentant H ou un groupe (C₁-C₆)alkyle,
à l'exception des composés (a), (b), (c), (d), (e) et (f) susmentionnés.

De préférence, dans la formule (VI-1), R₂ est H.

Selon un mode de réalisation, des composés ne relevant pas de l'invention répondent à la formule générale (VI-2) suivante : 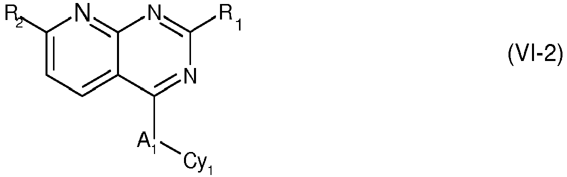 dans laquelle :
- R₁, A₁ et Cy₁ sont tels que définis ci-dessus dans la formule (I),
- R₂ représente H ou un substituant choisi dans le groupe constitué des atomes d'halogène, des groupes (C₁-C₆)(cyclo)alkyles, des groupes NRₐR_{b} et des groupes ORₐ, Rₐ et R_{b}, indépendamment l'un de l'autre, représentant H ou un groupe (C₁-C₆)alkyle.

De préférence, dans la formule (VI-2), R₂ est H.

Selon un mode de réalisation, des composés ne relevant pas de l'invention répondent à la formule générale (VII-1) suivante : 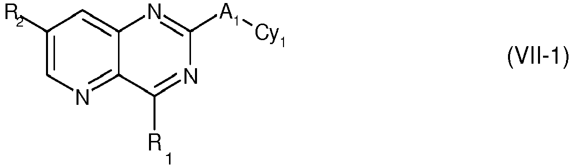 dans laquelle :
- R₁, A₁ et Cy₁ sont tels que définis ci-dessus dans la formule (I),
- R₂ représente H ou un substituant choisi dans le groupe constitué des atomes d'halogène, des groupes (C₁-C₆)(cyclo)alkyles, des groupes NRₐR_{b} et des groupes ORₐ, Rₐ et R_{b}, indépendamment l'un de l'autre, représentant H ou un groupe (C₁-C₆)alkyle.

De préférence, dans la formule (VII-1), R₂ est H.

Selon un mode de réalisation, des composés ne relevant pas de l'invention répondent à la formule générale (VII-2) suivante : 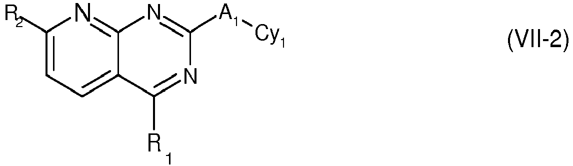 dans laquelle :
- R₁, A₁ et Cy₁ sont tels que définis ci-dessus dans la formule (I),
- R₂ représente H ou un substituant choisi dans le groupe constitué des atomes d'halogène, des groupes (C₁-C₆)(cyclo)alkyles, des groupes NRₐR_{b} et des groupes ORₐ, Rₐ et R_{b}, indépendamment l'un de l'autre, représentant H ou un groupe (C₁-C₆)alkyle.

De préférence, dans la formule (VII-2), R₂ est H.

Les composés selon l'invention peuvent être utilisés pour la préparation de médicaments, en particulier de médicaments inhibiteurs des canaux ioniques, notamment de SK3 et/ou SK2.

La présente invention concerne donc également un composé de formule (VI-1) telle que définie ci-dessus, pour son utilisation en tant qu'inhibiteur des canaux ioniques, notamment de SK3 et/ou SK2.

Ainsi, selon un autre de ses aspects, l'invention a pour objet des médicaments qui comprennent un composé de formule (VI-1) ou un sel d'addition de ce dernier à un acide pharmaceutiquement acceptable.

Ces médicaments trouvent leur emploi en thérapeutique, notamment dans le traitement du cancer.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, ainsi qu'au moins un excipient pharmaceutiquement acceptable.

Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (VI-1) ci-dessus, ou son sel, peut être administré sous forme unitaire d'administration, en mélange avec des excipients pharmaceutiques classiques, aux animaux et aux êtres humains pour le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

Selon la pratique habituelle, le dosage approprié à chaque patient est déterminé par le médecin selon le mode d'administration, le poids et la réponse dudit patient.

La présente invention concerne un composé de formule (VI-1) selon la présente invention pour son utilisation pour le traitement du cancer.

La présente invention concerne un composé de formule (VI-1) telle que définie ci-dessus, ou un sel d'addition de ce composé à un acide pharmaceutiquement acceptable, pour son utilisation à titre de médicament.

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables.

### EXEMPLES

### PRÉPARATION DE COMPOSÉS SELON L'INVENTION

Les composés selon l'invention sont préparés selon les schémas 1 à 5 ci-dessous :

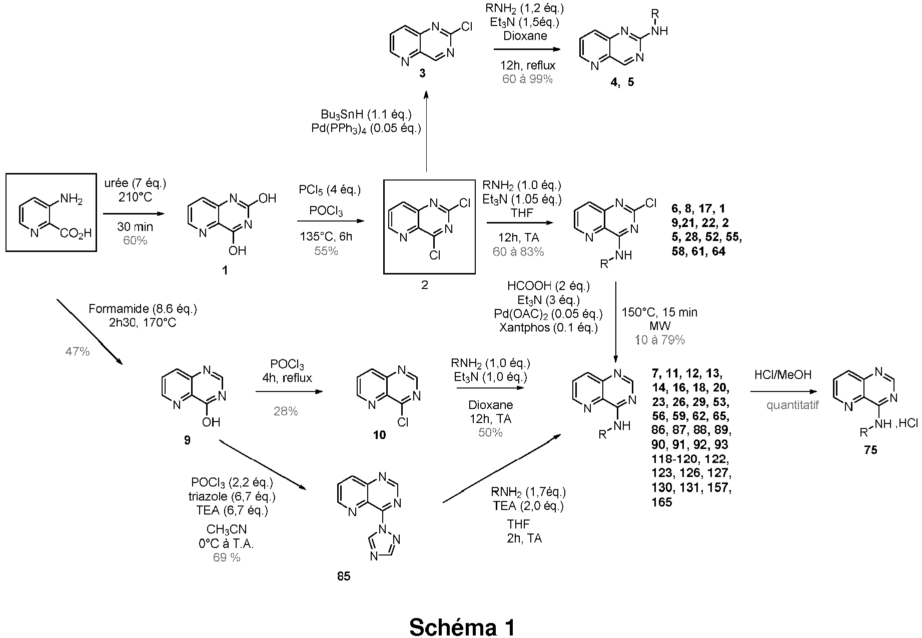

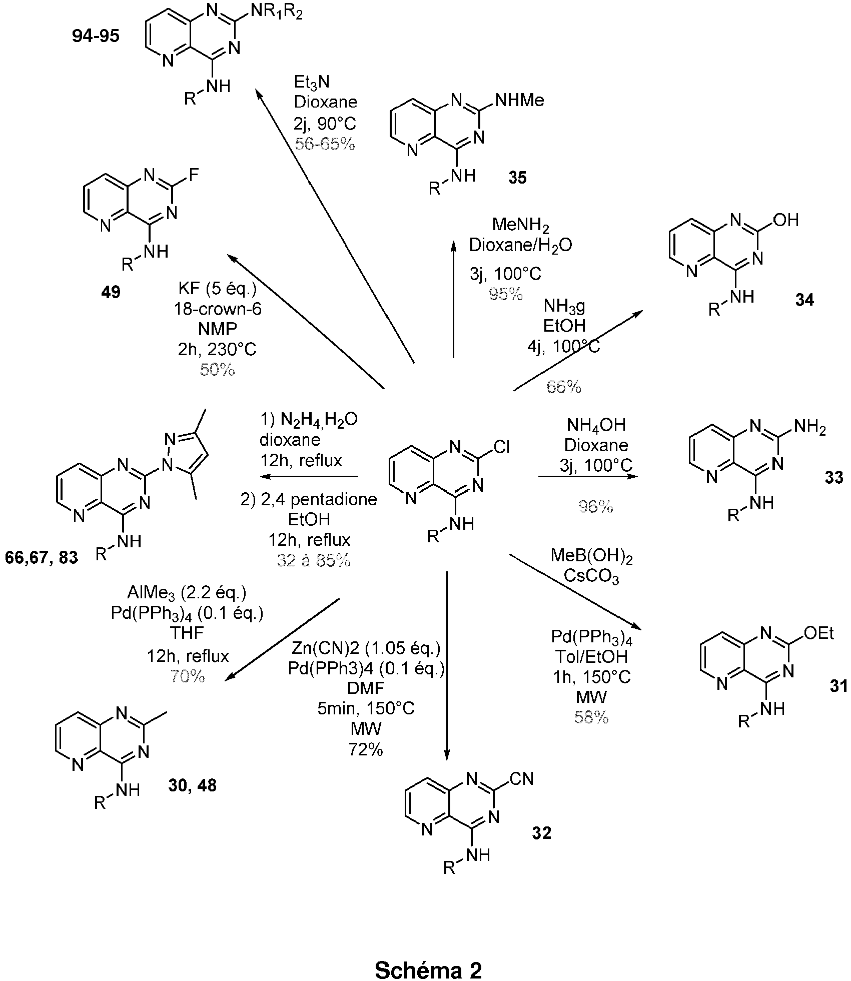

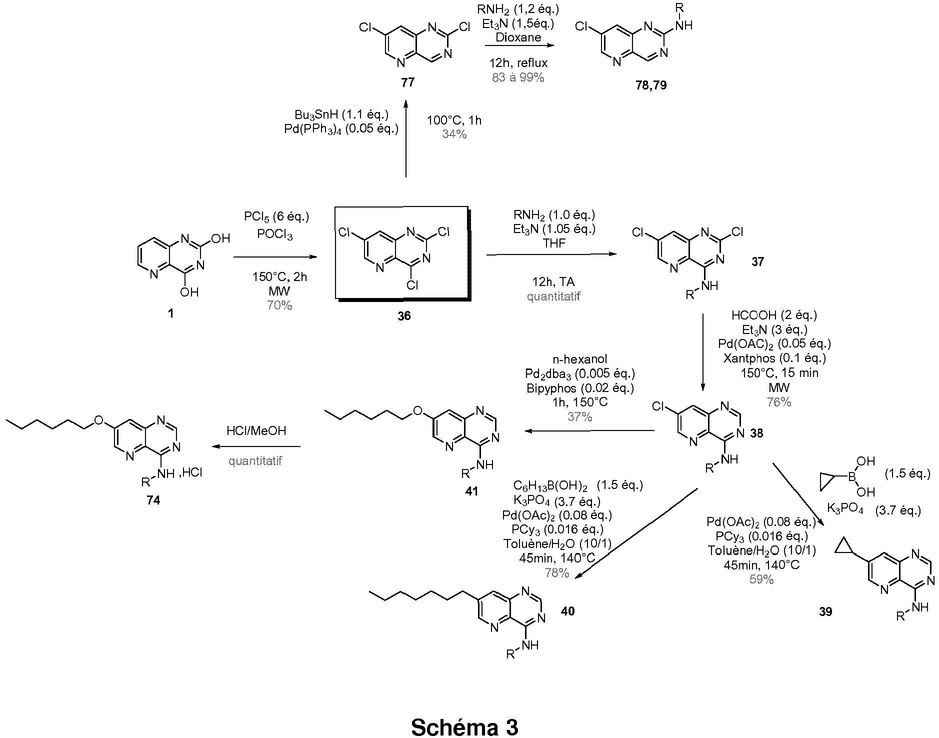

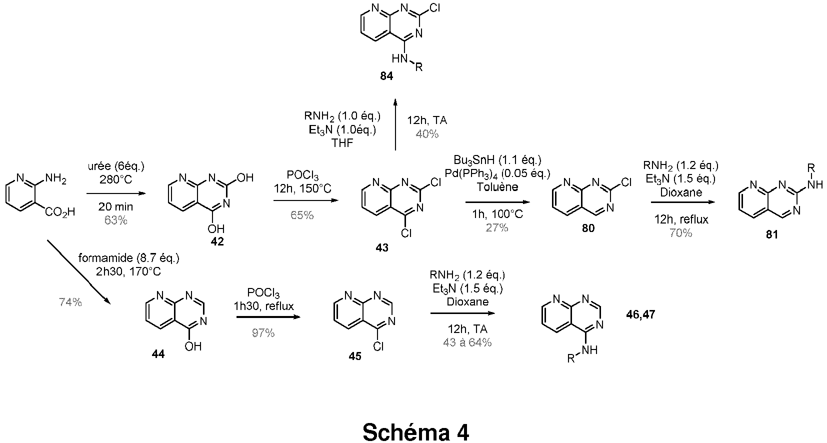

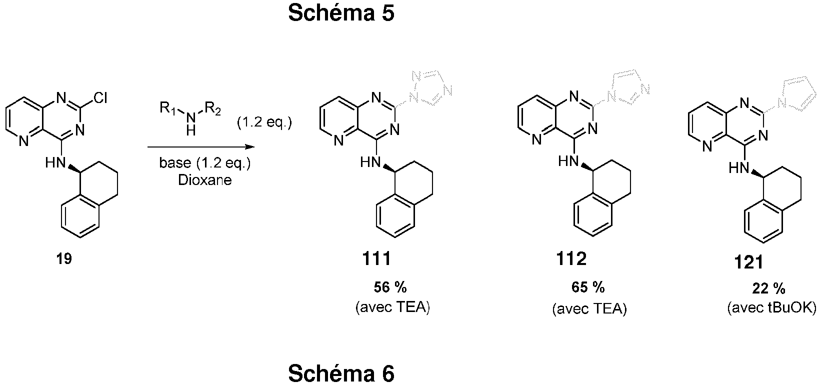

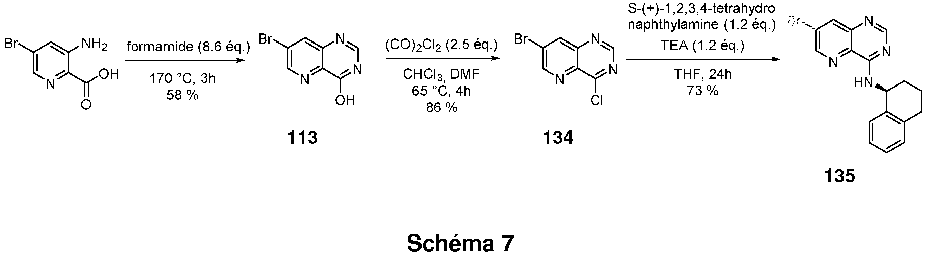

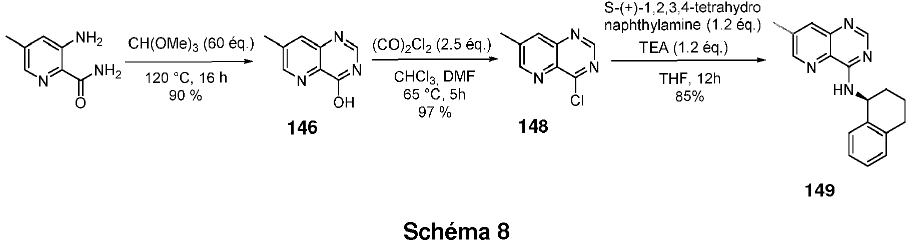

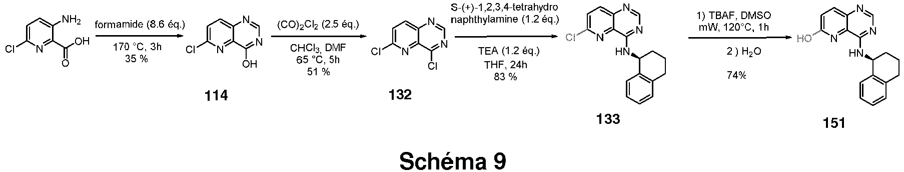

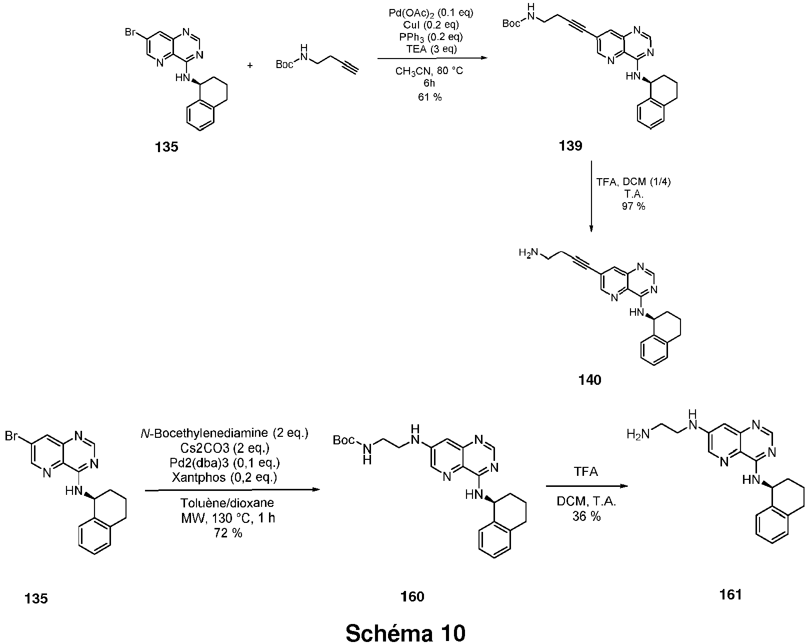

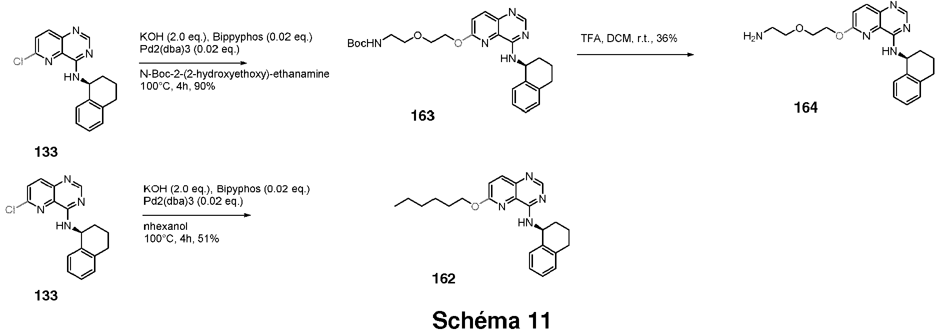

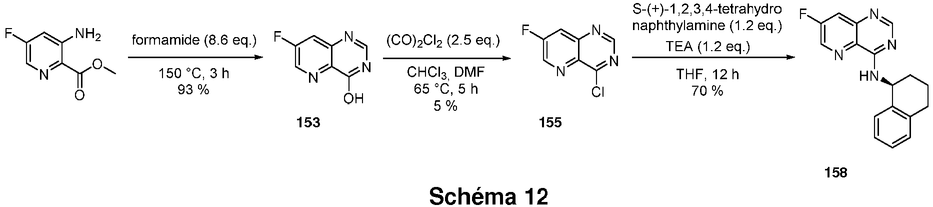

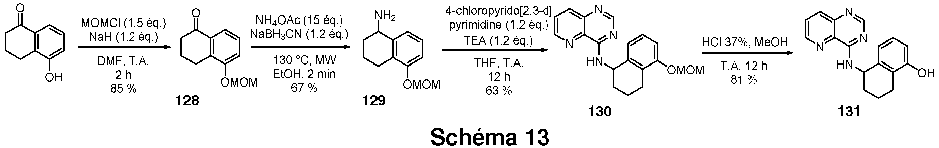

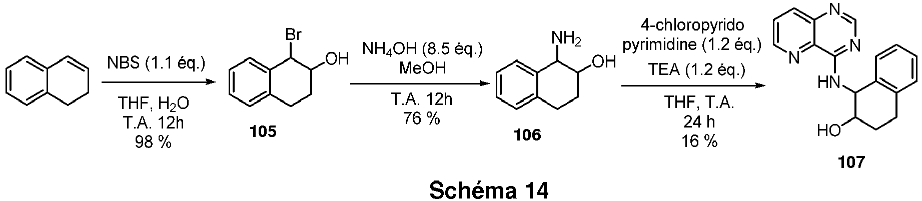

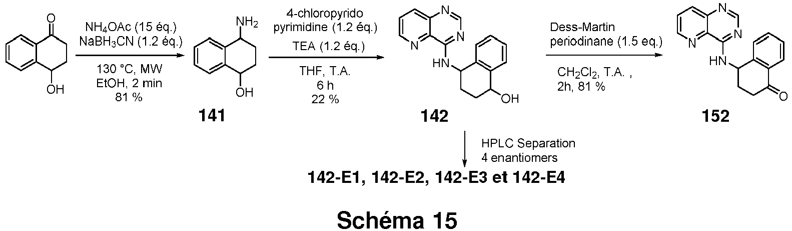

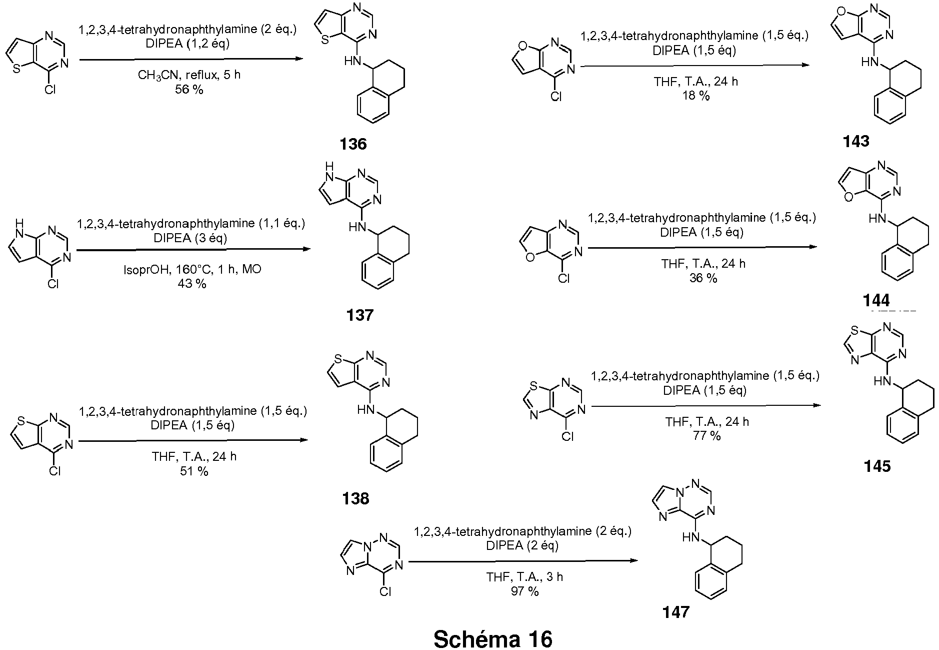

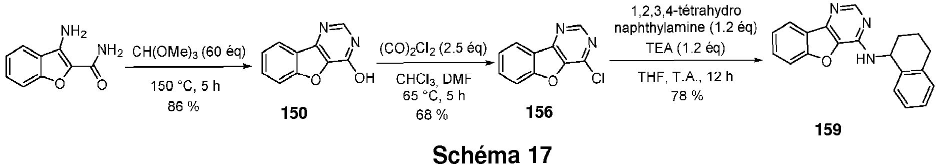

### 1. Préparation des composés intermédiaires

### Pyrido[3,2-d]pyrimidine-2,4(1H,3H)-dione (1)

### CAS number [37538-68-4]

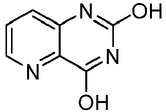

L'acide 3-amino-picolinique (1 g, 7,2 mmol) et l'urée (3 g, 50 mmol, 7 éq.) sont intimement mélangés et broyés au mortier. Le mélange est introduit dans un ballon de 100 mL puis l'ensemble est chauffé au moyen d'un bain de sable à 210°C pendant 20 minutes. La réaction est totale quand le résidu se solidifie. La masse obtenue est alors dissoute dans 4 mL de soude 2N à chaud. Après refroidissement, le mélange est filtré et le filtrat est acidifié à pH 8 par ajout d'acide chlorhydrique concentré. Le précipité formé est filtré, lavé avec un peu d'eau froide et séché pour donner le produit attendu **42** (711 mg, 60%) sous forme d'un solide beige.

### 2,4-Dichloropyrido[3,2-d]pyrimidine (2)

### CAS number [ 39551-54-7]

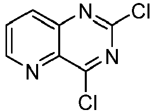

Le produit **1** (1 g, 6,1 mmol) est mis en suspension dans du POCl₃ (10 mL) puis le PCl₅ (5,1 g, 24,5 mmol, 4,0 éq.) est ajouté. L'ensemble est porté à reflux pendant 6 heures puis l'excès de POCl₃ est évaporé sous pression réduite. Le résidu obtenu est repris dans le dichlorométhane 20 mL et versé sur 30 mL d'un mélange eau / glace. Après 30 minutes d'agitation la phase aqueuse est extraite au dichlorométhane 3 x 20 mL. Les phases organiques rassemblées sont séchées sur sulfate de magnésium anhydre puis les solvants sont évaporés pour donner le produit attendu **2** (673 mg, 55%) sous forme d'un solide jaune.

### 2-Chloropyrido[3,2-d]pyrimidine (3)

### CAS number [915302-21-5]

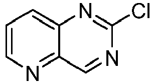

A une solution du produit **2** (540 mg, 2,7 mmol) dans le toluène anhydre (30 mL) sous argon sont successivement ajoutés l'hydrure de tributylétain (0,8 mL, 3,0 mmol, 1,1 éq.) et le Pd(PPh₃)₄ (156 mg, 0,135 mmol, 0,05 éq.). L'ensemble est porté à 100°C pendant une heure. Le toluène est ensuite évaporé puis le résidu obtenu est solubilisé dans le dichlorométhane (20 mL) et est hydrolysé par une solution saturée de fluorure de potassium (20 mL). Le mélange est agité vigoureusement pendant 30 minutes puis filtré sur célite en rinçant au dichlorométhane (2 x 20 mL). La phase aqueuse est extraite au dichlorométhane (20 mL), puis les phases organiques rassemblées sont séchées sur MgSO₄, filtrées et concentrées sous vide. Le brut réactionnel est chromatographié sur gel de silice (gradient EP à EP/AcOEt 80:20) pour donner le produit attendu **3** (180 mg, 40%) sous forme d'un solide jaune.

### 4-Hydroxy-pyrido[3,2-d]pyrimidine (9)

### CAS number [37538-67-3]

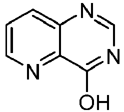

L'acide 3-aminopicolinique (1 g, 7,2 mmol) est introduit dans un ballon de 100 mL puis le formamide (2,3 mL, 57,6 mmol, 8,6 éq.) est versé. La pâte obtenue est porté à 170°C durant 2h30, l'ensemble devient limpide au cours de la réaction. Après refroidissement, le système prend en masse. Le solide est recristallisé dans 15 mL d'eau puis après filtration les cristaux lavés à l'eau froide (10 mL) pour donner le produit attendu **9** (500 mg, 47%) sous forme de cristaux marron.

### 4-Chloropyrido[3,2-d]pyrimidine (10)

### CAS number [51674-77-2]

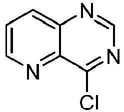

Le produit **9** (1 g, 6.80 mmol) est introduit dans un ballon de 100 mL puis l'oxychlorure de phosphore (30 mL) est coulé. L'ensemble est placé à reflux sous agitation magnétique pendant 4h, le milieu noircit rapidement. L'excès d'oxychlorure est évaporé sous pression réduite, il subsiste un résidu sirupeux noir. Le résidu est placé à 0°C puis environ 20 mL d'un mélange eau/glace ainsi que 20 mL de dichlorométhane sont introduits avec précaution. La mixture est amenée à pH 9/10 par ajout de carbonate de sodium solide. La phase aqueuse est extraite rapidement au dichlorométhane (20 mL). Les extraits organiques rassemblés sont séchés sur MgSO₄ puis les solvants sont évaporés pour donner le produit attendu **10** (310 mg, 28%) sous forme d'un solide violet à utiliser rapidement tel quel.

### 7-Bromo-1,2,3,4-tétrahydronaphthalen-1-amine (15)

### CAS number [865472-04-4]

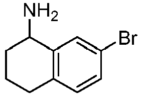

A une solution de 7-bromo-3,4-dihydronaphthalène (100 mg, 0.44 mmol) dans l'éthanol (1 mL) sont successivement ajoutés l'acétate d'ammonium (514 mg, 6.66 mmol, 15.0 éq.) et le cyanoborohydrure de sodium (33 mg, 0.52 mmol, 1.2 éq.). Le mélange est porté à 130°C sous irradiations micro-ondes pendant 2 minutes, puis concentré sous vide. Une solution de soude 2N est ajoutée jusqu'à l'obtention d'un pH > 10, puis le produit est extrait à l'acétate d'éthyle (2x20 mL). Les phases organiques recombinées sont séchées sur MgSO₄, filtrées puis concentrées sous vide pour donner le produit attendu **a** (100 mg, quantitatif) sous forme d'une huile incolore. R_{f} (CH₂Cl₂/MeOH 95:05): 0.25. ¹H RMN (400 MHz, CDCl₃) δ 7.55 (d, *J* = 1.8 Hz, 1H), 7.24 (dd, *J* = 8.2, 2.0 Hz, 1H), 6.94 (d, *J* = 8.2 Hz, 1H), 3.94 - 3.90 (m, 1H), 2.82 - 2.58 (m, 2H), 2.05 - 1.95 (m, 1H), 1.95 - 1.86 (m, 1H), 1.83 - 1.70 (m, 1H), 1.70 - 1.56 (m, 3H). ¹³C RMN (101 MHz, CDCl₃) δ 143.5, 135.8, 130.9, 130.8, 129.7, 119.6, 49.4, 33.5, 29.1, 19.6. IR (ATR Diamant, cm⁻¹) v 3359, 2927, 2858, 1590, 1477, 1266, 1169, 1083, 801. HRMS (EI-MS) m/z calculée pour C₁₀H₁₂BrN [M+H]⁺: 226.022588, trouvée 226.022470.

### 5,7-Diméthyl-1,2,3,4-tétrahydronaphthalen-1-amine (24)

### CAS number [59376-79-3]

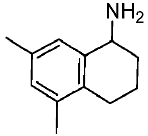

A une solution de 5,7-diméthyl-1-tétralone (100 mg, 0.57 mmol) dans l'éthanol (1.3 mL) sont successivement ajoutés l'acétate d'ammonium (664 mg, 8.61 mmol, 15.0 éq.) et le cyanoborohydrure de sodium (43 mg, 0.68 mmol, 1.2 éq.). Le mélange est porté à 130°C sous irradiations micro-onde pendant 2 minutes, puis concentré sous vide. Une solution de soude 2N est ajoutée jusqu'à l'obtention d'un pH > 10, puis le produit est extrait à l'acétate d'éthyle (2x20 mL). Les phases organiques recombinées sont séchées sur MgSO₄, filtrées puis concentrées sous vide pour donner le produit attendu **24** (100 mg, quantitatif) sous forme d'un solide blanc.

### 1,2,3,4-Tétrahydrophenanthren-4-amine (27)

### CAS number [101561-96-0]

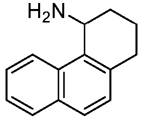

A une solution de 1,2,3,4-tétrahydrophenanthren-4-one (150 mg, 0.96 mmol) dans l'éthanol (2 mL) sont successivement ajoutés l'acétate d'ammonium (1.1 g, 14.4 mmol, 15.0 éq.) et le cyanoborohydrure de sodium (72 mg, 1.15 mmol, 1.2 éq.). Le mélange est porté à 130°C sous irradiations micro-ondes pendant 2 minutes, puis concentré sous vide. Une solution de soude 2N est ajoutée jusqu'à l'obtention d'un pH > 10, puis le produit est extrait à l'acétate d'éthyle. Les phases organiques recombinées sont séchées sur MgSO₄, filtrées puis concentrées sous vide pour donner le produit attendu **27** (140 mg, quantitatif) sous forme d'une huile marron.

### 2,4,7-Trichloropyrido[3,2-d]pyrimidine (36)

### CAS number [1260663-38-4]

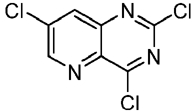

A une solution du produit 1 (1.0 g, 6.1 mmol) dans le POCl₃ (10 mL) est ajouté le PCl₅ (7.65 g, 36.8 mmol, 6.0 éq.). L'ensemble est chauffé sous irradiation micro-ondes à 150°C pendant 2 heures. L'excès de POCl₃ est évaporé, le résidu obtenu est repris dans le dichlorométhane puis versé dans 50 mL d'un mélange eau/glace. Le tout est agité vigoureusement pendant 30 minutes, puis la phase aqueuse est extraite au dichlorométhane. Les phases organiques combinées sont séchées sur MgSO₄, filtrées puis concentrées sous vide pour donner le produit attendu **36** (1 g, 70%) sous forme d'un solide jaune.

### 2,4-Dihydroxy-pyrido[2,3-d]pyrimidine (42)

### CAS number [21038-66-4 ]

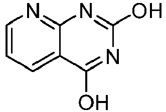

L'acide 2-amino-nicotinique (5.0 g, 36 mmol) et l'urée (11.0 g, 180 mmol, 5 éq.) sont intimement mélangés et broyés au mortier. Le mélange est introduit dans un ballon de 250 mL puis l'ensemble est chauffé au moyen d'un bain de sable à 280°C pendant 20 minutes. La réaction est totale quand le résidu se solidifie. La masse obtenue est alors dissoute dans 100 mL de soude 2N à chaud. Après refroidissement, le mélange est filtré et le filtrat est acidifié à pH 8 par ajout d'acide chlorhydrique concentré. Le précipité est filtré, lavé avec un peu d'eau froide et séché pour donner le produit attendu **42** (3.7 g, 63%) sous forme d'un solide beige.

### 2,4-Dichloro-pyrido[2,3-d]pyrimidine (43)

### CAS number [126728-20-9]

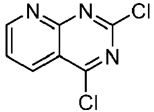

Le produit **42** (10.5 g, 65 mmol) est introduit dans un ballon de 500 mL puis l'oxychlorure de phosphore (250 mL) est coulé. L'ensemble est placé à 150°C sous agitation magnétique pendant 1 nuit. L'excès d'oxychlorure est évaporé sous pression réduite, le résidu est placé à 0°C puis environ 200 mL d'un mélange eau/glace ainsi que 300 mL de dichlorométhane sont introduits avec précaution. Le mélange est amené à pH 8/9 à l'aide de carbonate de sodium solide. La phase aqueuse est extraite au dichlorométhane puis les extraits organiques sont séchés sur MgSO₄ puis les solvants sont évaporés pour donner le produit attendu **43** (8.42 mg, 65%) sous forme d'un solide beige à utiliser rapidement tel quel.

### 4-Hydroxy-pyrido[2,3-d]pyrimidine (44)

### CAS number [24410-19-3]

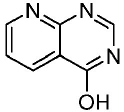

L'acide 2-aminonicotinique (1 g, 7.2 mmol) est introduit dans un ballon de 100 mL puis le formamide (2.3 mL, 57.6 mmol, 8.6 éq.) est versé. La pâte obtenue est porté à 170°C durant 2h30, l'ensemble devient limpide au cours de la réaction. Après refroidissement, le système prend en masse. Le solide est recristallisé dans 15 mL d'eau puis après filtration lavé à l'eau froide pour donner le produit attendu **44** (787 mg, 74%) sous forme de cristaux beige.

### 4-Chloropyrido[2,3-d]pyrimidine (45)

### CAS number [28732-79-8]

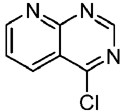

Le produit **44** (787 mg, 5.35 mmol) est introduit dans un ballon de 100 mL puis l'oxychlorure de phosphore (12 mL) est coulé. L'ensemble est placé à reflux sous agitation magnétique pendant 1h30, le milieu noircit rapidement. L'excès d'oxychlorure est évaporé sous pression réduite, il subsiste un résidu sirupeux noir. Le résidu est placé à 0°C puis environ 10 mL d'un mélange eau/glace ainsi que 10 mL de dichlorométhane sont introduits avec précaution. La mixture est amenée à pH 9/10 à l'aide de carbonate de sodium solide. La phase aqueuse est extraite rapidement au dichlorométhane. Les extraits organiques sont séchés sur MgSO₄ puis les solvants sont évaporés pour donner le produit attendu **45** (855 mg, 97%) sous forme d'un solide orange très instable, à utiliser rapidement.

### 1-Nitroso-1,2,3,4-tétrahydroquinoline (50)

### CAS number [5825-44-5]

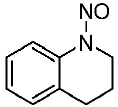

La 1,2,3,4-tétrahydroquinoline (0.94 mL, 7,50 mmol) et une solution d'HCl 10% (3.01 mL, 9,76 mmol, 1.3 éq.) sont mis sous agitation dans un ballon à 0°C, sous atmosphère d'argon. Puis une solution de NaNO₂ (829 mg, 12 mmol, 1.6 éq.) dans l'eau (5 mL) est additionnée goutte à goutte. Après 2 heures d'agitation à température ambiante, le mélange réactionnel est dilué avec 20 mL d'eau et extrait à l'acétate d'éthyle (2x20 mL). La phase organique est séchée sur sulfate de magnésium puis concentrée sous vide mais pas à sec car le produit est sensible. Le résidu est directement engagé dans l'étape suivante sans purification préalable.

### 3,4-Dihydroquinolin-1 (2H)-amine (51)

### CAS number [5825-45-6]

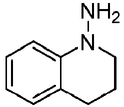

Le produit **50** (1.21 g, 7.51 mmol) est dilué dans le THF anhydre (30 mL) à 0°C et sous atmosphère d'argon. Puis le tétrahydruroaluminate de lithium (1.14 g, 30 mmol, 4.0 éq.) est ajouté par portion. Le mélange réactionnel est ensuite placé sous agitation pendant 1 heure dans un bain d'eau froide (15°C). Après retour à 0°C, de l'eau (2 mL) est ajoutée lentement. Le milieu est filtré sur Célite^{®}, le filtrat est dilué avec de l'eau et extrait au dichlorométhane. La phase organique est séchée sur sulfate de magnésium puis concentrée sous vide. Le brut est ensuite purifié par chromatographie sur gel de silice (éluant : gradient EP à EP/AcOEt 80/20) pour donner le produit attendu **51** sous forme d'un solide jaune/orange (800 mg, 70% sur 2 étapes).

### 6,7,8,9-Tétrahydro-5H-benzo[7]annulen-5-amine (54)

### CAS number [17910-26-8]

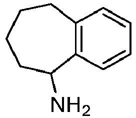

A une solution de 1-benzosuberone (0.62 mmol, 1.0 éq.) dans l'éthanol (1.3 mL) sont successivement ajoutés l'acétate d'ammonium (721 mg, 0.93 mmol, 15.0 éq.) et le cyanoborohydrure de sodium (47 mg, 0.75 mmol, 1.2 éq.). Le mélange est porté à 130°C sous irradiations micro-ondes pendant 2 minutes, puis concentré sous vide. Une solution de soude 2N est ajoutée jusqu'à l'obtention d'un pH > 10, puis le produit est extrait à l'acétate d'éthyle (2x20 mL). Les phases organiques recombinées sont séchées sur MgSO₄, filtrées puis concentrées sous vide pour donner le produit attendu **54** (119 mg, quantitatif) sous forme d'une huile jaune.

### 7-Nitro-1,2,3,4-tétrahydronaphthalen-1-amine (57)

### CAS number [211372-31-5]

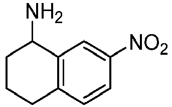

A une solution de 7-nitro-1-tétralone (0.52 mmol, 1.0 éq.) dans l'éthanol (1.3 mL) sont successivement ajoutés l'acétate d'ammonium (605 mg, 7.8 mmol, 15.0 éq.) et le cyanoborohydrure de sodium (39 mg, 0.63 mmol, 1.2 éq.). Le mélange est porté à 130°C sous irradiations micro-ondes pendant 2 minutes, puis concentré sous vide. Une solution de soude 2N est ajoutée jusqu'à l'obtention d'un pH > 10, puis le produit est extrait à l'acétate d'éthyle. Les phases organiques recombinées sont séchées sur MgSO₄, filtrées puis concentrées sous vide. Après, le résidu est purifié avec comme éluant EP/AE (80/20). Le produit a été obtenu sous forme d'un solide marron et avec un rendement de 99 % (100 mg).

### 3,4-Dihydro-2H-1-benzopyran-4-amine (60)

### CAS number [53981-38-7]

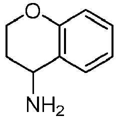

A une solution de 4-Chromanone (3.57 mmol, 1.0 éq.) dans l'éthanol (5.0 mL) sont successivement ajoutés l'acétate d'ammonium (4.0 g, 50.6 mmol, 15 éq.) et le cyanoborohydrure de sodium (269 mg, 4.3 mmol, 1.2 éq.). Le mélange est porté à 130°C sous irradiations micro-ondes pendant 2 minutes, puis concentré sous vide. Une solution de soude 2N est ajoutée jusqu'à l'obtention d'un pH > 10, puis le produit est extrait à l'acétate d'éthyle. Les phases organiques recombinées sont séchées sur MgSO₄, filtrées puis concentrées sous vide. Après, le résidu est purifié avec comme éluant EP/AE (80/20). Le produit **60** a été obtenu sous forme d'un solide marron et avec un rendement de 94 % (500 mg).

### 4,5,6,7-Tétrahydro-1-benzofuran-4-amine (63)

### Numéro CAS [389795-57-7]

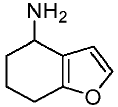

Dans un ballon sous argon, 100 mg du 6-7-dihydro-1-benzofuran-4(5H)-one est solubilisé dans 5 mL de MeOH. 1 g d'acétate d'ammonium (0.014 mol, 15.0 éq.) ainsi que 122 mg de cyanoborohydrure de sodium (1.94 mol, 1.2 éq.) et 2g de tamis moléculaires sont ajoutés au mélange. Au bout de 4h à reflux, le mélange est filtré sur Célite^{®} pour éliminer le tamis. Puis l'excès de MeOH est évaporé. Le pH de la solution est monté au-dessus de 10 avec NaOH 2N. La phase aqueuse est ensuite extraite à l'EtOAc (3x 25 mL). Les phases organiques sont combinées, séchées sur MgSO₄, filtrées sur coton et évaporées sous vide. 215 mg du composé **63** a été obtenu sous forme d'une huile marron avec un rendement de 99 % (215 mg).

### 7-Méthylpyrido[3,2-d]pyrimidine-2,4-diol (68)

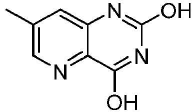

Sous une atmosphère de dioxyde de carbone, dans un vial de 20 mL, 400 mg (3,0 mmol, 1eq) de 3-amino-5-méthylpyridine-2-carbonitrile est dissous dans 8 mL de DMF anhydre. 448 µL (3,0 mmol, 1eq) du 1,8-diazabicyclo[5.4.0]undéc-7-ène (DBU) sont ajoutés . Ensuite, la solution est dégazée pendant 15 minutes et le vial scellé. Le mélange est chauffé à 105 °C pendant 6 heures (précipitation du produit). A 0°C, 2 mL HCl à 1M sont ajoutés. Le précipité est filtré sous vide permettant d'isoler un solide beige avec un rendement de 90%.¹H RMN (400 MHz, DMSO-*d₆*) δ : 11,07 (bs, 2H); 7,93 (s, 1H) 7,34 (s, 1H), 2,34 (s, 3H). ¹³C RMN (101 MHz, DMSO-*d₆*) δ: 161,7, 150.5, 146.2 139.7, 138.5, 129,7, 123.4, 18.6. HRMS (EI-MS): C₈H₇N₃O₂ [M+H]⁺, calculée m/z 178,0617, trouvée m/z 178,0611. IR (ATR diamant, cm⁻¹) v 3052, 1673, 1410, 1127, 846, 820, 686. T_{f} >260°C.

### 2,4-Dichloro-7-méthylpyrido[3,2-d]pyrimidine (69)

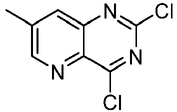

Dans un ballon de 50 mL, 1 g (6,65 mmol; 1,0 éq.) du composé **68** est mis en suspension dans 10 mL d'oxychlorure de phosphore et 4,7 g (22,60 mmol; 4,0 éq.) de PCl₅. L'ensemble est chauffé à 130 °C. Après 12 heures de réaction, l'excès de POCl₃ est évaporé sous pression réduite. Le résidu obtenu est amené à 0 °C au moyen d'un bain de glace puis solubilisé dans le dichlorométhane (150 mL), le mélange est versé dans un mélange eau/glace (200 mL) sans aucune basification. Après retour à température ambiante, la phase aqueuse est extraite au dichlorométhane (1 × 100 mL). Les phases organiques sont séchées sur MgSO₄, filtrées, puis concentrées sous pression réduite. Le résidu ainsi obtenu est purifié par colonne de chromatographie sur gel de silice sous pression (AE/EP, 2/8) pour donner un solide blanc **69** avec un rendement de 70 %. ¹H RMN (400 MHz, CDCl₃) δ : 8.99 (d, *J* = 2,0 Hz, 1H) 8.08 (m, 1H), 2.68 (m, 3H) ; ¹³C RMN (101 MHz, CDCl₃) δ: 166.7, 157.0, 156.6, 150.4, 143.2, 136.4, 135.8, 20.7. HRMS (EI-MS) : C₇H₂Cl₃N₃ [M+H]⁺, calculée m/z 213,9939, trouvée m/z 213,9933 ; IR (ATR diamant, cm⁻¹) v1539, 1439, 1398, 1255, 1137, 1004, 869, 698, 690 ; Tf : 146-168°C.

### 2,4,7-trichloropyrido[3,2-d]pyrimidine (77)

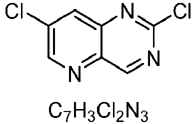

A une solution du produit **36** (310 mg, 1.32 mmol) dans le toluène anhydre (15 mL) sous argon sont successivement ajoutés l'hydrure de tributylétain (0.4 mL, 1.45 mmol, 1.1 éq.) et le Pd(PPh₃)₄ (76 mg, 0.06 mmol, 0.05 éq.). L'ensemble est porté à 100°C pendant une heure. Le toluène est ensuite évaporé puis le résidu obtenu est solubilisé dans le dichlorométhane et est hydrolysé par une solution saturée de fluorure de potassium. Le mélange est agité vigoureusement pendant 30 minutes puis filtré sur célite en rinçant au dichlorométhane. La phase aqueuse est extraite au dichlorométhane, puis séchée sur MgSO₄, filtrée et concentrée sous vide. Le brut est chromatographié sur gel de silice (gradient éther de pétrole -> éther de pétrole/AcOEt 95:05) pour donner le produit attendu **77** (90 mg, 34%) sous forme d'un solide jaune. ¹H NMR (400 MHz, CDCl₃) δ 9.03 (d, *J* = 2.2 Hz, 1H), 8.31 (d, *J* = 2.2 Hz, 1H). ¹³C NMR (101 MHz, CDCl₃) δ 166.0, 157.0, 152.7, 148.8, 138.5, 135.1, 134.2. IR (ATR diamant, cm⁻¹) v 3048, 2167, 1579, 1531, 1430, 1324, 1253, 1136, 1001, 872. HRMS (EI-MS) m/z calculée pour C₇H₂Cl₃N₃ [M+H]⁺: 232.9314, trouvée 232.9323. T_{f}: 166°C.

### 2,4-Dichloro-pyrido[2,3-d]pyrimidine (80)

### CAS Number [1060816-71-8]

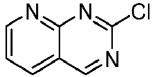

A une solution du produit **43** (450 mg, 2.2 mmol) dans le toluène anhydre (25 mL) sous argon sont successivement ajoutés l'hydrure de tributylétain (0.67 mL, 2.5 mmol, 1.1 éq.) et le Pd(PPh₃)₄ (130 mg, 0.113 mmol, 0.05 éq.). L'ensemble est porté à 100°C pendant une heure. Le toluène est ensuite évaporé puis le résidu obtenu est solubilisé dans le dichlorométhane et est hydrolysé par une solution saturée de fluorure de potassium. Le mélange est agité vigoureusement pendant 30 minutes puis filtré sur célite en rinçant au dichlorométhane. La phase aqueuse est extraite au dichlorométhane, puis séchée sur MgSO₄, filtrée et concentrée sous vide. Le brut est chromatographié sur gel de silice (gradient éther de pétrole -> éther de pétrole/AcOEt 80:20) pour donner le produit attendu **80** (100 mg, 27%) sous forme d'un solide blanc instable, à utiliser rapidement. ¹H NMR (250 MHz, CDCl₃) δ 9.33 (dd, *J* = 2.0, 4.4 Hz, 1H), 8.64 (dd, *J* = 2.0, 8.3 Hz, 1H), 7.72 (dd, *J* = 4.4, 8.3 Hz, 1H). T_{f}: 134-136°C.

### 4-(1H-1,2,4-Triazol-1-yl)pyrido[3,2-d]pyrimidine (85)

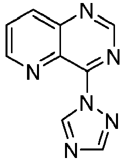

Dans un ballon de 100 mL, le 1,2,4-triazole (3.15 g; 45.6 mmol) est dissous dans 22 mL de CH₃CN puis le ballon est plongé dans un bain de glace. Le POCl₃ (1.4 mL; 15 mmol) est ajouté à 0°C suivi de la triéthylamine (6.34 mL; 45.6 mmol) goutte à goutte. La solution devient blanche et opaque. La solution est agitée à 0 °C pendant 40 minutes puis à température ambiante pendant 30 minutes. Finalement, le composé **9** (1g; 6.8 mmol) est ajouté et la solution jaunâtre est agitée à température ambiante pendant une nuit. La réaction est suivie par CCM (système 98 % CH₂Cl₂ et 2 % MeOH) pour vérifier la disparition du composé de départ. En fin de réaction, la solution est diluée avec 50 mL d'eau puis le produit est extrait avec 5 x 60 mL d'acétate d'éthyle. Les phases organiques sont combinées puis lavées avec une solution aqueuse de NaCl saturée et enfin séchées avec MgSO₄. Après filtration, le solvant est évaporé pour obtenir une poudre jaune. Après un dépôt solide sur silice, le produit est purifié sur colonne de silice avec un système 98% CH₂Cl₂ et 2 % MeOH. Le produit est obtenu sous forme de solide jaune (920 mg; 69%). Rf (EP/AE 50:50): 0.52. ¹H RMN (400 MHz, CDCl₃) δ 10.26 (1H; s), 9.38 (s, 1H), 9.18 (m, 1H), 8.50 (m, 1H), 8.30 (s, 1H), 7.96 (m, 1H). ¹³C RMN (100 MHz, CDCl₃) δ 155.4, 153.4, 153.2, 152.5, 149.2, 149.1, 137.6, 132.2, 129.2. IR (ATR Diamant, cm⁻¹) v 3109, 1514, 1410, 1336, 1197, 1121, 969, 832, 661. HRMS (EI-MS) : m/z calculée pour C₉H₇N₆ [M+H]⁺: 199.0726, trouvée : 199.0728. T_{f} : 184-186°C.

**Procédure générale A (synthèse des amines)** : Dans un tube scellé de 10 mL, la cétone est dissoute dans 6 mL d'éthanol et NH₄OAc (15eq) et NaBH₃CN (1.2 eq) sont successivement ajoutés. La réaction est réalisée aux micro-ondes à 130 °C sous agitation vigoureuse. En fin de réaction, la disparition de la cétone de départ est vérifiée par CCM puis l'éthanol est évaporé. Une solution de soude à 2 M est ajoutée pour atteindre un pH 10 puis le produit est extrait avec 3 x 20 mL d'acétate d'éthyle. Les phases organiques sont combinées et lavées avec une solution aqueuse de NaCl saturée et enfin séchées avec MgSO₄. Après filtration, le solvant est évaporé et le produit est purifié sur colonne de silice.

### 1-Indanamine (96)

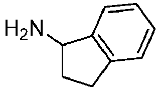

Le composé **96** est obtenu en suivant la procédure A avec l'indanone (200 mg; 1.5 mmol), NH₄OAc (1.75 g; 28 mmol) et NaBH₃CN (114 mg; 1.8 mmol). Le produit est purifié par colonne de silice avec un système 90 % CH₂Cl₂ et 10 % MeOH. Le produit est obtenu sous forme d'un solide blanc (108 mg; 54 %). Rf (CH₂Cl₂/MeOH 90:10): 0.22. ¹H RMN (400 MHz, CDCl₃) δ 7.28 (m, 4H), 4.36 (m, 1H), 2.97 (m, 1H), 2.82 (m, 1H), 2.51 (m, 1H), 1.90 (br s, 2H, NH₂), 1.70 (m, 1H). ¹³C RMN (100 MHz, CDCl₃) δ 147.3, 143.0, 127.2, 126.5, 124.6, 123.3, 57.2, 37.2, 30.1. IR (ATR Diamant, cm⁻¹) v 3280, 2966, 1613, 1477, 1356, 1289, 1258, 1050, 813, 745, 579. HRMS (EI-MS) : m/z calculée pour C₉H₁₂N [M+H]⁺: 134.0964, trouvée: 134.0962. T_{f}: 102-104 °C.

### 5-méthoxy-1,2,3,4-tétrahydronaphthalen-1-amine (97)

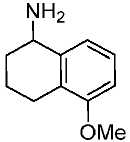

Le composé **97** est obtenu en suivant la procédure A avec le 5-méthoxytétralone (400 mg ; 2,3 mmol), NH₄OAc (2,6 g ; 34 mmol) et NaBH₃CN (174 mg ; 2,8 mmol). Le produit est purifié par colonne de silice avec un système 97% CH₂Cl₂ et 3% MeOH. Le produit est obtenu sous forme d'un solide blanc (352 mg ; 88 %). Rf (CH₂Cl₂/MeOH 98:2): 0,35. ¹H RMN (400 MHz, CD₃OD) δ 7.08 (m, 1H), 6.92 (m, 1H), 6.68 (m, 1H), 3.84 (m, 1H), 3.71 (s, 3H), 2.55 (m, 2H), 1.85 (m, 2H), 1.63 (m, 2H). ¹³C RMN (100 MHz, CD₃OD) δ 158.4, 142.3, 127.4, 126.5, 121.1, 108.9, 55.7, 50.1, 32.7, 24.1, 19.7. IR (ATR Diamant, cm⁻¹) v 3396, 2920, 2840, 1549, 1245, 1066, 780, 717, 640. HRMS (EI-MS) : m/z calculée pour C₁₁H₁₆NO [M+H]⁺: 178.1226, trouvée: 178.1221. T_{f}: 115-117 °C.

### 7-méthoxy-1,2,3,4-tétrahydronaphthalen-1-amine (98)

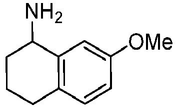

Le composé **98** est obtenu en suivant la procédure A avec le 7-méthoxytétralone (150 mg; 0.85 mmol), NH₄OAc (986 mg; 12.8 mmol) et NaBH₃CN (64 mg; 1.02 mmol). Le produit est purifié par colonne de silice avec un système 90 % CH₂Cl₂ et 10 % MeOH. Le produit est obtenu sous forme d'un solide blanc (150 mg; 99 %). Rf (CH2Cl2/MeOH 90:10): 0.21.¹H RMN (400 MHz, CDCl₃) δ 6.94 (m, 2H), 6.68 (m, 1H), 3.90 (m, 1H), 3.74 (s, 3H), 2.64 (m, 2H), 2.48 (br s, 2H; NH₂), 1.92 (m, 2H), 1.66 (m, 2H). ¹³C RMN (100 MHz, CDCl₃) δ 157.6, 141.4, 129.6, 128.5, 112.8, 112.3, 54.9, 49.3, 33.1, 28.4, 19.6. IR (ATR Diamant, cm⁻¹) v 3341, 2928, 1575, 1470, 1252, 1035, 815, 702. HRMS (EI-MS) : m/z calculée pour C₁₁H₁₆NO [M+H]⁺: 178.1226, trouvée: 178.1223. T_{f}: 99-101°C.

### 7-fluoro-1,2,3,4-tétrahydronaphthalen-1-amine (99)

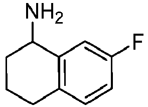

Le composé **99** est obtenu en suivant la procédure A avec le 7-fluorotétralone (400 mg; 2.4 mmol), NH₄OAc (2.8 g; 56.5 mmol) et NaBH₃CN (181 mg; 2.88 mmol). Le produit est purifié par colonne de silice avec un système 97 % CH₂Cl₂ et 3 % MeOH. Le produit est obtenu sous forme d'un solide blanc (271 mg; 68 %). Rf (CH₂Cl₂/MeOH 98:2): 0.20.¹H RMN (250 MHz, CDCl₃) δ 7.08 (m, 1H), 6.96 (m, 1H), 6.78 (m, 1H), 3.86 (m, 1H), 2.66 (m, 2H), 1.98 (m, 1H), 1.87 (m, 3H), 1.72 (m, 1H), 1.58 (m, 1H). ¹³C RMN (62.5 MHz, CDCl₃) δ 161.1 (*J* = 243.0 Hz), 142.9 (*J* = 6.0 Hz), 132.1 (*J* = 3.0 Hz), 130.1 (*J* = 7.0 Hz), 114.0 (*J* = 20.0 Hz), 113.6 (*J* = 20.0 Hz), 49.4, 33.3, 28.7, 19.7. IR (ATR Diamant, cm⁻¹) v 2944, 1551, 1471, 1372, 1250, 807, 705. HRMS (EI-MS) : m/z calculée pour C₁₀H₁₃NF [M+H]⁺: 166.1026, trouvée: 166.1033. T_{f}: 95-97 °C.

### 4,5,6,7-tétrahydrobenzo[b]thiophen-4-amine (100)

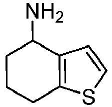

Le composé **100** est obtenu en suivant la procédure A avec le 6,7-dihydro-4-benzo[b]thiophenone (200 mg; 1.32 mmol), NH₄OAc (1.52 g; 19.8 mmol) et NaBH₃CN (100 mg; 1.58 mmol). Le produit est purifié par colonne de silice avec un système 95 % CH₂Cl₂ et 5 % MeOH. Le produit est obtenu sous forme d'un solide jaune (157 mg; 79 %). R_{f} (CH₂Cl₂/MeOH 95:5): 0.28. ¹H RMN (400 MHz, CD₃OD) δ 7.16 (m, 1H), 7.04 (m, 1H), 4.04 (m, 1H), 2.78 (m, 2H), 2.06 (m, 2H), 1.83 (m, 1H), 1.72 (m, 1H). ¹³C RMN (100 MHz, CD₃OD) δ 139.0, 138.2, 127.1, 123.7, 48.1, 32.4, 25.7, 21.8. IR (ATR Diamant, cm⁻¹) v 3307, 2931, 1607, 1474, 1307, 1152, 870, 814, 674. HRMS (EI-MS) : m/z calculée pour C₈H₁₂NS [M+H]⁺: 154.0685, trouvée: 154.0683. T_{f}: 102-104 °C.

### 4,4-diméthyl-1,2,3,4-tétrahydronaphthalen-1-amine (101)

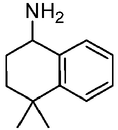

Le composé **101** est obtenu en suivant la procédure A avec le 4,4-diméthyl-1,2,3,4-tétrahydronaphthalen-1-one (500 mg; 2.9 mmol), NH₄OAc (3.32 g; 43.1 mmol) et NaBH₃CN (217 mg; 3.45 mmol). Le produit est purifié par colonne de silice avec un système 95 % CH₂Cl₂ et 5 % MeOH. Le produit est obtenu sous forme d'un solide blanc (240 mg; 48 %). Rf (CH₂Cₗ₂/MeOH 95:5): 0.24. ¹H RMN (250 MHz, CDCl₃) δ 7.41 (m, 1H), 7.35 (m, 1H), 7.19 (m, 2H), 3.97 (m, 1H), 2.65 (br s, 2H), 2.08 (m, 1H), 1.87 (m, 1H), 1.74 (m, 1H), 1.63 (m, 1H), 1.34 (s, 3H), 1.28 (s, 3H). ¹³C RMN (62.5 MHz, CDCl₃) δ 145.3, 139.4, 127.9, 127.00, 126.6, 125.8, 50.0, 35.1, 33.9, 31.7, 29.5. IR (ATR Diamant, cm⁻¹) v 3414, 3303, 2957, 2860, 1636, 1574, 1456, 1291, 1153, 1042, 762, 545, 508. HRMS (EI-MS) : m/z calculée pour C₁₂H₁₈N [M+H]⁺: 176.1433, trouvée: 176.1434. T_{f}: 70-72 °C.

### 6-(Méthoxyméthoxy)-3,4-dihydronaphthalen-1(2H)-one (102)

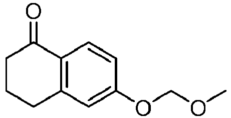

La 6-hydroxytétralone (200 mg, 1.23 mmol, 1.0 eq) est dissoute dans 3 mL de DMF dans un ballon de 25 mL puis celui-ci est plongé dans un bain de glace à 0 °C. Sous agitation, l'hydrure de sodium (60 mg, 1.5 mmol, 1.2 eq) est ajouté puis, après 10 minutes, le chlorure de méthyl méthyl ether (0.14 mL, 1. 85 mmol, 1.5 eq) est également ajouté. Ensuite le mélange est agité à température ambiante pendant 2 heures. En fin de réaction, le mélange est dilué avec 90 mL d'AcOEt puis lavé avec 3 x 70 mL d'eau. La phase organique est lavée avec une solution saturée aqueuse de NaCl (20 mL) puis séchée avec MgSO₄. Après filtration, le solvant est évaporé puis le résidu est purifié par chromatographie sur gel de silice (gradient 100 à 70 % PE et 0 à 30 % AE/). Le produit **102** est obtenu sous forme d'une huile incolore (248 mg, 98 %). R_{f} (ether de pétrole/AcOEt 50:50) 0.90. ¹H RMN (400 MHz, CDCl₃) δ 790 (d, *J* = 87 Hz 1H), 685 (dd, *J* = 8.7, 2.5 Hz, 1H), 6.78 (d, *J* = 2.5 Hz, 1H), 5.14 (s, 2H), 3.40 (s, 3H), 2.3 (t, *J* = 6.0 Hz, 2H), 2.51 (t, *J* = 6.0 Hz, 2H), 2.02 (quin, *J* = 6.0 Hz, 2H). ¹³C RMN (101 MHz, CDCl₃) δ 196.9, 161.0, 146.7, 129.3, 126.9, 114.7, 114.6, 93.8, 56.1, 38.8, 29.9, 23.2. IR (ATR Diamant, cm⁻¹) v 2942, 2828, 1672, 1570, 1491, 1080, 920, 826. HRMS (EI-MS) : m/z calculée pour C₁₂H₁₄N₄NaO₃ [M+Na]⁺: 229.0835, trouvée: 229.0833.

### 6-(Méthoxyméthoxy)-1,2,3,4-tétrahydronaphthalen-1-amine (103)

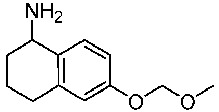

Le composé **103** est obtenu en suivant la procédure A avec le composé **102** (225 mg, 1.1 mmol, 1.0 eq), NH₄OAc (1.27 g, 16.5 mmol, 15.0 eq) et NaBH₃CN (83 mg, 1.32 mmol, 1.2 eq). Le résidu est purifié par chromatographie sur gel de silice (système CH₂Cl₂/MeOH 90:10). Le produit est obtenu sous forme d'un solide blanc (78 mg, 35 %). R_{f} (CH₂Cl₂/MeOH 95:5): 0.21. ¹H RMN (400 MHz, CDCl₃) δ 7,31 (d, *J* = 8,7 Hz, 1H), 6,87 (dd, *J* = 8,7 et 2,5 Hz, 1H), 6,75 (d, *J* = 2.5 Hz, 1H), 5,14 (s, 2H), 3,94 (m, 1H), 3,46 (s, 3H), 2,74 (m, 2H), 1,96 (m, 1H), 1,90 (m, 1H), 1,69 (m, 2H). ¹³C RMN (101 MHz, CDCl₃) δ 155.8, 138.2, 134.7, 129.3, 116.0, 114.5, 94.5, 56.0, 48.9, 33.7, 29.9, 19.5. IR (ATR Diamant, cm⁻¹): 3326, 2937, 2841, 1500, 1318, 1150, 1007, 824, 718. HRMS (EI-MS) : m/z calculée pour C₁₂H₁₇NNaO₂ [M+Na]⁺: 230.1148, trouvée: 230.1151. T_{f}: 99-101 °C.

### 1-Bromo-1,2,3,4-tétrahydronaphthalen-2-ol (105)

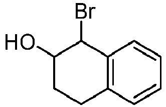

Le 1,2-dihydronaphthalène (1 g, 7.7 mmol) et le N-bromosuccinimide (1.51 g, 8.47 mmol) sont dissous dans 7 mL de THF et 4 mL d'eau puis le mélange est agité à température ambiante pendant une nuit. Le produit de départ disparait et le succinimide précipite sous forme de solide blanc. Le mélange est dilué avec 60 mL d'AcOEt puis lavé avec 2 x 50 mL d'eau. La phase organique est lavée avec une solution saturée aqueuse de NaCl puis séché avec MgSO₄. Après filtration, le solvant est évaporé et le composé **105** est précipité grâce à l'ajout de pentane. Le produit est obtenu sous forme d'un solide blanc (1.7 g, 98 %). R_{f} (PE/AE 60:40): 0.82. ¹H RMN (400 MHz, DMSO-*d₆*) δ 7.37 (m, 1H), 7.19 (m, 2H), 7.10 (m, 1H), 5.85 (br s, 1H, OH), 4.67 (m, 1H), 4.47 (m, 1H), 2.85 (m, 2H), 2.44 (m, 1H), 2.11 (m, 1H). ¹³C RMN (101 MHz, DMSO-*d₆*) δ 136.6, 134.9, 129.4, 128.1, 127.3, 125.9, 71.8, 56.3, 27.7, 26.4. IR (ATR Diamant, cm⁻¹) v 3242, 2910, 1693, 1434, 1188, 949, 878, 749, 652. HRMS (EI-MS) : m/z calculée pour C₁₉H₂₁N₄O₂ [M+H]⁺: 337.1659, trouvée: 337.1660. T_{f}: 104-106 °C.

### 1-Amino-1,2,3,4-tétrahydronaphthalen-2-ol (106)

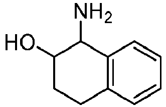

Le composé **105** (770 mg, 3.4 mmol) est dissous dans 2 mL de MeOH puis l'hydroxyde d'ammonium aqueux (4 mL, 29 mmol) est ajouté. Le mélange est agité à température ambiante pendant une nuit. La CCM permet de voir la disparition du produit de départ et le MeOH est évaporé. Le résidu est directement purifié par chromatographie sur gel de silice (système gradient 0 à 10 % MeOH/100 à 90 % CH₂Cl₂). Le produit **106** est obtenu sous forme d'un solide blanc (423 mg, 76 %). R_{f} (AE 100): 0.19. ¹H RMN (400 MHz, DMSO-*d₆*) δ 7.52 (m, 1H), 7.24 (m, 2H), 7.16 (m, 1H), 5.46 (br s, 1H, OH), 4.02 (m, 1H), 3.80 (m, 1H), 2.81 (m, 2H), 2.02 (m, 1H), 1.75 (m, 1H). ¹³C RMN (101 MHz, DMSO-*d₆*) δ 136.6, 132.9, 128.7, 128.0, 127.7, 126.1, 68.5, 55.3, 28.2, 26.3. IR (Diamant ATR, cm⁻¹) v 2929, 1598, 1493, 1043, 774, 742, 552. HRMS (EI-MS) : m/z calculée pour C₁₀H₁₄NO [M+H]⁺: 164.1069, trouvée: 164.1070. T_{f}: 155-160 °C.

### 7-Fluoro-1,2,3,4-tetrahydronaphthalen-1-amine (109)

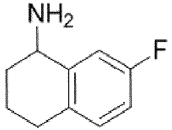

Le composé **109** est obtenu en suivant la procédure B avec la 7-fluorotetralone (400 mg, 24 mmol, 1.0 eq.), NH₄OAc (2.8 g, 56.5 mmol, 15.0 eq.) et NaBH₃CN (181 mg, 2.88 mmol, 1.2 eq.). Le résidu est purifié par chromatographie sur gel de silice (éluant CH₂Cl₂/MeOH 97:3). Le produit est obtenu sous forme d'un solide blanc (271 mg, 68 %). R_{f} (CH₂Cl₂/MeOH 98:2): 0.20. ¹H RMN (400 MHz, CDCl₃) δ 7.08 (dd, J = 10, 3 Hz, 1H), 6.96 (dd, *J* = 8 et 6 Hz, 1H), 6.78 (td, *J* = 8 et 3 Hz, 1H), 3.86 (t, *J* = 6.0 Hz, 1H), 2.66 (m, 2H), 1.98 (m, 1H), 1.87 (m, 1H), 1.86 (br s, 2H, NH₂), 1.72 (m, 1H), 1.58 (m, 1H). ¹³C RMN (101 MHz, CDCl₃) δ 161.1 (*J* = 243.0 Hz), 142.9 (*J* = 6.0 Hz), 132.1 (*J* = 3.0 Hz), 130.1 (*J* = 7.0 Hz), 114.0 (*J* = 20 Hz), 113.6 (*J* = 20.0 Hz), 49.4, 33.3, 28.7, 19.7. IR (ATR diamant, cm⁻¹) n 2944, 1551, 1471, 1372, 1250, 807, 705. HRMS (EI-MS) : m/z calculée pour C₁₀H₁₃NF [M+H]⁺: 166.1026, trouvée: 166.1033. T_{f}: 94-96 °C.

### 7-Bromopyrido[3,2-d]pyrimidin-4-ol (113)

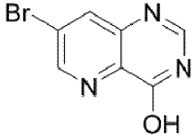

L'acide 3-amino-5-bromopicolinique (500 mg, 2,3 mmol) est dissous dans 5 mL de formamide puis le mélange est chauffé à 170 °C pendant 12 h. En fin de réaction, la solution est refroidie jusqu'à température ambiante et 20 mL d'eau glacée est ajoutée. Le produit précipite sous forme de solide marron qui est isolée par filtration et séchage au four (303 mg, 58 %). R_{f} (CH₂Cl₂/MeOH 90:10): 0.25. ¹H RMN (400 MHz, CDCl₃) δ 12.66 (br s, 1H), 8.87 (d, *J* = 8.0 Hz, 1H), 8.41 (d, *J* = 8.0 Hz, 1H), 8.19 (s, 1H). ¹³C RMN (101 MHz, CDCl₃) δ 159.2, 149.7, 147.5, 146.5, 138.0, 137.3, 124.6. IR (ATR diamant, cm⁻¹) n 3025, 2820, 1719, 1568, 1222, 866, 737, 630, 540. HRMS (EI-MS) : m/z calculée pour C₇H₅BrN₃O [M+H]⁺: 225.9610, trouvée: 225.9614. T_{f}: > 260 °C.

### 6-Chloropyrido[3,2-d]pyrimidin-4-ol (114)

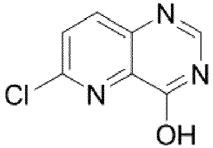

L'acide 3-amino-6-chloropicolinique (500 mg, 2.9 mmol) est dissous dans 5 mL de formamide puis le mélange est chauffé à 170 °C pendant 12 h. En fin de réaction, la solution est refroidie jusqu'à température ambiante et 20 mL d'eau glacée est ajoutée. Le produit précipite sous forme de solide marron qui est isolé par filtration et séchage au four (186 mg, 35 %). R_{f} (CH₂Cl₂/MeOH 90:10): 0.28. ¹H RMN (400 MHz, DMSO-*d*₆) δ 8.18 (s, 1H), 8.14 (d, *J* = 8.0 Hz, 1H), 7.87 (d, *J* = 8.0 Hz, 1H). ¹³C RMN (101 MHz, DMSO-*d*₆) δ 158.6, 148.6, 146.9, 145.3, 139.6, 139.1, 130.0. IR (ATR diamant, cm⁻¹) n 3038, 2907, 1651, 1465, 1275, 1096, 843, 633. HRMS (EI-MS) : m/z calculée pour C₇H₅ClN₃O [M+H]⁺: 182.0115, trouvée: 182.0116. T_{f}: > 260 °C .

### tert-Butyl 4-amino-4,5,6,7-tetrahydroindole-1-carboxylate (115)

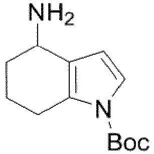

Le composé **115** est obtenu en suivant la procédure A avec le *t*ert-butyl 4-oxo-6,7-dihydro-5*H*-indole-1-carboxylate (200 mg, 0.85 mmol, 1.0 eq.), NH₄OAc (983 mg, 12.8 mmol, 15.0 eq.) et NaBH₃CN (64 mg, 1.02 mmol, 1.2 eq.). Le résidu est purifié par chromatographie sur gel de silice (éluant CH₂Cl₂/MeOH 90:10). Le produit est obtenu sous forme d'une huile marron (85 mg, 42 %). R_{f} (CH₂Cl₂/MeOH 90:10): 0.28. ¹H RMN (400 MHz, CDCl₃) δ 7.12 (d, *J* = 3.0 Hz, 1H), 6.20 (d, *J* = 3.0 Hz, 1H), 3.85 (dt, *J* = 3.0, 7.0 Hz, 1H), 2.79 (t, *J* = 6.0 Hz, 2H), 2.55 (br s, 2H, NH₂), 1.93 (m, 2H), 1.71 (m, 1H), 1.55 (s, 9H), 1,48 (m, 1H). ¹³C RMN (101 MHz, CDCl₃) δ 149.6, 130.2, 126.1, 120.0, 109.3, 83.3, 46.1, 33.3, 28.1, 24.7, 20.6. IR (ATR diamant, cm⁻¹): 2976, 2933, 2400, 1732, 1583, 1368, 1318, 1299, 1141, 1126, 1015, 851, 706. HRMS (EI-MS) : m/z calculée pour C₁₃H₂₀N₂NaO₂ [M+Na]⁺: 259.1417, trouvée: 259.1415.

### tert-Butyl N-(1-aminotetralin-6-yl)carbamate (116)

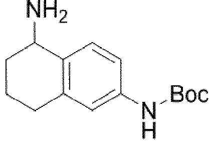

Le composé **116** est obtenu en suivant la procédure A avec le *t*ert-butyl *N-*(1-oxotetralin-6-yl)carbamate (150 mg, 0.57 mmol, 1.0 eq.), NH₄OAc (658 mg, 8.55 mmol, 15.0 eq.) et NaBH₃CN (44 mg, 0.69 mmol, 1.2 eq.). Le résidu est purifié par chromatographie sur gel de silice (éluant CH₂Cl₂/MeOH 90:10). Le produit est obtenu sous forme d'un solide marron (90 mg, 60 %). R_{f} (CH₂Cl₂/MeOH 90:10): 0.25. ¹H RMN (400 MHz, CDCl₃) δ 7.28 (d, *J* = 8.0 Hz, 1H), 7.16 (br s, 1H, NH), 7.05 (dd, *J* = 8.0, 2.0 Hz, 1H), 6.75 (br s, 1H), 3.95 (t, *J* = 5.0 Hz, 1H), 2.70 (m, 2H), 2.57 (br s, 2H, NH₂), 1.97 (m, 1H), 1.89 (m, 1H), 1.70 (m, 2H), 1.49 (s, 9H). ¹³C RMN (101 MHz, CDCl₃) δ 153.0, 137.7, 137.0, 134.9, 128.7, 118.7, 116.8, 80.4, 49.0, 33.1, 29.7, 28.4, 19.5. IR (ATR diamant, cm⁻¹) n 3305, 2929, 1698, 1529, 1239, 1155, 871, 770. HRMS (EI-MS) : m/z calculée pour C₁₅H₂₂N₂NaO₂ [M+Na]⁺: 285.1573, trouvée: 285.1575. T_{f}: 77-79 °C.

### Tetralin-2-amine (117)

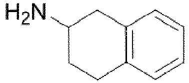

Le composé **117** est obtenu en suivant la procédure A avec la 2-tetralone (0.45 mL, 3.1 mmol, 1.0 eq.), NH₄OAc (3.58 g, 46.5 mmol, 15.0 eq.) et NaBH₃CN (234 mg, 3.72 mmol, 1.2 eq.). Le résidu est purifié par chromatographie sur gel de silice (éluant CH₂Cl₂/MeOH 90:10). Le produit est obtenu sous forme d'un solide marron (325 mg, 71 %). R_{f} (CH₂Cl₂/MeOH 90:10): 0.22. ¹H RMN (400 MHz, CDCl₃) δ 7.10 (m, 4H), 3.17 (m, 1H), 2.99 (m, 1H), 2.87 (m, 2H), 2.56 (dd, *J* = 16.0, 9.0 Hz, 1H), 1.99 (m, 1H), 1.81 (br s, 2H), 1,59 (m, 1H). ¹³C RMN (101 MHz, CDCl₃) δ 135.6, 135.0, 129.1, 128.5, 125.7, 125.6, 47.1, 39.1, 32.6, 27.9. IR (ATR diamant, cm⁻¹) n 3420, 3255, 3018, 2917, 1570, 1394, 738. HRMS (EI-MS) : m/z calculée pour C₁₀H₁₄N [M+H]⁺: 148.1120, trouvée: 148.1121. T_{f}: 100-102 °C.

### 8-Methoxytetralin-1-amine (124)

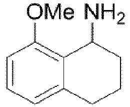

Le composé **124** est obtenu en suivant la procédure A avec la 8-methoxytétralone (300 mg, 1.7 mmol, 1.0 eq.), NH₄OAc (1.97 g, 25.5 mmol, 15.0 eq.) et NaBH₃CN (129 mg, 2.84 mmol, 1.2 eq.). Le résidu est purifié par chromatographie sur gel de silice (éluant CH₂Cl₂/MeOH 90:10). Le produit est obtenu sous forme d'une huile orange (211 mg, 71 %). R_{f} (CH₂Cl₂/MeOH 90:10): 0.19. ¹H RMN (400 MHz, CDCl₃) δ 7.08 (t, *J* = 8.0 Hz, 1H), 6.66 (m, 2H), 4.,18 (t, *J* = 4.0 Hz, 1H), 3.81 (s, 3H), 2.68 (m, 2H), 1.97 (br s, 2H, NH₂), 1.83 (m, 3H), 1.70 (m, 1H). ¹³C RMN (101 MHz, CDCl₃) δ 157.6, 137.6, 129.3, 126.8, 121.4, 107.3, 54.9, 43.4, 31.3, 29.6, 17.9. IR (ATR diamant, cm⁻¹) n 3366, 2930, 2835, 1581, 1467, 1438, 1249, 1093, 1077, 767, 741, 521. HRMS (EI-MS) : m/z calculée pour C₁₁H₁₆NO [M+H]⁺: 178.1226, trouvée: 178.1226.

### 6,7-Dimethoxytetralin-1-amine (125)

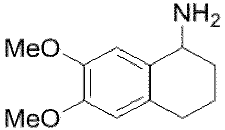

Le composé **125** est obtenu en suivant la procédure A avec la 6,7-dimethoxytetralone (300 mg, 1.45 mmol, 1.0 eq.), NH₄OAc (1.68 g, 21.8 mmol, 15.0 eq.) et NaBH₃CN (110 mg, 1.7 mmol, 1.2 eq.). Le résidu est purifié par chromatographie sur gel de silice (éluant CH₂Cl₂/MeOH 90:10). Le produit est obtenu sous forme d'une huile orange (211 mg, 71 %). R_{f} (CH₂Cl₂/MeOH 90:10): 0.19. ¹H RMN (400 MHz, CDCl₃) δ 6.93 (s, 1H), 6.54 (s, 1H), 6.90 (m, 1H), 3.86 (s, 3H), 3.83 (s, 3H), 2.66 (m, 2H), 2.00 (br s, 2H, NH₂) 1.95 (m, 1H), 1.69 (m, 1H). ¹³C RMN (101 MHz, CDCl₃) δ 147.8, 147.4, 132.6, 128.9, 111.5, 111.1 56.1, 55.9, 49.2, 34.1, 29.2, 19.7. IR (ATR diamant, cm⁻¹) n 3351, 2930, 2835, 1508, 1252, 1216, 1111, 1015, 861, 794. HRMS (EI-MS) : m/z calculée pour C₁₂H₁₇NO₂Na [M+Na]⁺: 230.1151, trouvée: 230.1149.

### 5-(Methoxymethoxy)tetralin-1-one (128)

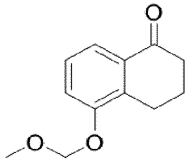

La 5-hydroxytetralone (300 mg, 1.85 mmol, 1.0 eq.) est dissoute dans 3 mL de THF anhydre dans un ballon de 25 mL puis celui-ci est plongé dans un bain de glace à 0 °C. Sous agitation, l'hydrure de sodium (90 mg, 2.22 mmol, 1.2 eq.) est ajouté puis, après 10 minutes, le chlorure de methyl methyl ether (0.21 mL, 2.77 mmol, 1.5 eq.) est également ajouté. Ensuite le mélange est agité à température ambiante pendant 2 heures. En fin de réaction, le mélange est dilué avec 90 mL d'AcOEt puis lavé avec 3 x 70 mL d'eau. La phase organique est lavée avec une solution saturée aqueuse de NaCl (20 mL) puis séchée avec MgSO₄. Après filtration, le solvant est évaporé puis le résidu est purifié par chromatographie sur gel de silice (éluant gradient 0 à 20 % AcOEt/100 à 80 % PE). Le produit **128** est obtenu sous forme d'une huile incolore (334 mg, 85 %). R_{f} (PE/AcOEt 50:50): 0.35. ¹H RMN (400 MHz, CDCl₃) δ 7.66 (d, *J* = 8.0 Hz, 1H), 7.15 (m, 1H), 5.18 (s, 2H), 3.46 (s, 3H), 2.90 (t, *J* = 6.0 Hz, 2H), 2.62 (t, *J* = 6.0 Hz, 2H), 2.10 (quin, *J* = 6.0 Hz, 2H). ¹³C RMN (101 MHz, CDCl₃) δ 198.3, 155.9, 138.3, 133.6, 130.1, 122.8, 113.3, 94.6, 56.2, 39.1, 29.1, 23.5. IR (ATR diamant, cm⁻¹): 2945, 1680, 1606, 1492, 1427, 1322, 1274, 1151, 921, 821. HRMS (EI-MS) : m/z calculée pour C₁₂H₁₅O₃ [M+H]⁺: 207.1015, trouvée: 207.1018.

### 5-(Methoxymethoxy)tetralin-1-amine (129)

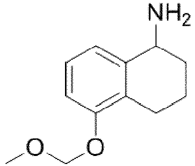

Le composé **129** est obtenu en suivant la procédure A avec le composé **128** (200 mg, 0.97 mmol, 1.0 eq.), NH₄OAc (1.125 g, 14.6 mmol, 15.0 eq.) et NaBH₃CN (74 mg, 1.16 mmol, 1.2 eq.). Le résidu est purifié par chromatographie sur gel de silice (éluant CH₂Cl₂/MeOH 90:10). Le produit est obtenu sous forme d'une huile orange (134 mg, 67 %). R_{f} (CH₂Cl₂/MeOH 90:10): 0.24. ¹H RMN (400 MHz, CDCl₃) δ 7.08 (d, *J* = 3.0 Hz, 1H), 6.98 (d, *J* = 8.0 Hz, 1H), 6.84 (dd, *J* = 8.0, 3.0 Hz, 1H), 5.14 (m, 2H), 3.93 (dd, *J* = 6.0, 5.0 Hz, 1H), 3.46 (s, 3H), 2.70 (m, 2H), 1.94 (m, 2H), 1.69 (m, 2H). ¹³C RMN (101 MHz, CDCl₃) δ 155.5, 142.1, 130.2, 129.9, 115.3, 115.2, 94.6, 55.9, 49.6, 33.4, 28.8, 19.8. IR (ATR diamant, cm⁻¹): 2929, 1681, 1608, 1440, 1315, 1273, 1229, 1149, 999, 920, 814. HRMS (EI-MS) : m/z calculée pour C₁₂H₁₈NO₂ [M+H]⁺: 208.1332, trouvée: 208.1333.

### 4,6-Dichloropyrido[3,2-d]pyrimidine (132)

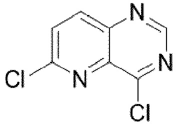

Le composé **114** (190 mg, 1.1 mmol, 1.0 eq.) est dissous dans 10 mL de CHCl₃ et quelques gouttes de DMF sont ajoutées. Puis le ballon est plongé dans un bain de glace à 0 °C et le chlorure d'oxalyle (0.24 mL, 2.76 mmol, 2.5 eq.) est ajouté goutte à goutte. La solution est ensuite chauffée à 65 °C pendant 4 à 5 heures sous agitation vigoureuse. La disparition du réactif de départ est suivie par CCM puis, en fin de réaction, le CHCl₃ et l'excès de chlorure d'oxalyle sont évaporés. Le dépôt obtenu est repris dans 30 mL de dichlorométhane puis le produit est lavé avec 30 mL d'une solution aqueuse de Na₂CO₃ saturée. La phase organique est lavée avec une solution aqueuse de NaCl saturée (40 mL) et enfin séchée sur MgSO₄. Après filtration, le solvant est évaporé et le résidu est purifié sur gel de silice (éluant CH₂Cl₂/Acetone 98:2). Le produit est obtenu sous forme d'un solide marron (107 mg, 51 %). R_{f} (PE/AcOEt 60:40): 0.40. ¹H RMN (400 MHz, CDCl₃) δ 9.13 (s, 1H), 8,34 (d, *J* = 8.0 Hz, 1H), 7.86 (d, *J* = 8.0 Hz, 1H). ¹³C RMN (101 MHz, CDCl₃) δ 163.1, 154.7, 153.7, 146.7, 139.9, 138.1, 131.6. IR (ATR diamant, cm⁻¹) n 3068, 1728, 1643, 1552, 1413, 1306, 1133, 1010, 859, 688. HRMS (EI-MS) : m/z calculée pour C₇H₄Cl₂N₃ [M+H]⁺: 199.9777, trouvée: 199.9776. T_{f}: 161-163 °C.

### 7-Bromo-4-chloro-pyrido[3,2-d]pyrimidine (134)

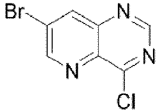

Le composé **134** est obtenu comme décrit pour **132** avec **113** (1.00 g, 4.44 mmol, 1.0 eq.), le chlorure d'oxalyle (0.953 mL, 11.1 mmol, 2.5 eq.). Le produit est purifié par chromatographie sur gel de silice (éluant CH₂Cl₂/Acetone 98:2). Le produit est obtenu sous forme d'un solide blanc (946 mg, 86 %). R_{f} (PE/AcOEt 60:40): 0.82. ¹H RMN (400 MHz, CDCl₃) δ 9.14 (d, *J* = 8.0 Hz, 1H), 9.12 (s, 1H), 8.59 (d, *J* = 8.0 Hz, 1H). ¹³C RMN (101 MHz, CDCl₃) δ 164.6, 155.5, 154.5, 147.9, 138.7, 136.7, 126.5. IR (ATR diamant, cm⁻¹) n 3037, 1553, 1533, 1430, 1361, 1317, 1074, 999, 921, 838, 677, 656. HRMS (EI-MS) : m/z calculée pour C₇H₄ClBrN₃ [M+H]⁺: 243.9271, trouvée: 243.9270. T_{f}: 188-190 °C.

### 4-Aminotetralin-1-ol (141)

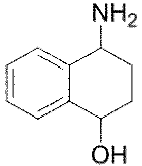

Le composé **141** est obtenu en suivant la procédure A avec la 4-hydroxy-3,4-dihydronaphthalenone (100 mg, 0.62 mmol, 1.0 eq.), NH₄OAc (716 mg, 9.3 mmol, 15.0 eq.) et NaBH₃CN (47 mg, 0.74 mmol, 1.2 eq.). Le résidu est purifié par chromatographie sur gel de silice (système CH₂Cl₂/MeOH 85:15). Le produit est obtenu sous forme d'une huile orange (82 mg, 81 %). R_{f} (CH₂Cl₂/MeOH 90:10): 0.12. ¹H RMN (400 MHz, CDCl₃) δ 7.35 (m, 4H), 4.72 (m, 1H), 3.99 (m, 1H), 2.33 (br s, 2H, NH₂), 2.22 (m, 1H), 1.86 (m, 3H). ¹³C RMN (101 MHz, CDCl₃) δ 140.5, 139.1, 128.4, 128.0, 127.4, 68.2, 49.4, 29.5, 28.8. IR (ATR diamant, cm⁻¹) n 3268, 2861, 1567, 1448, 1330, 1043, 750, 583. HRMS (+ESI) : m/z calculée pour C₁₀H₁₄NO [M+H]⁺: 164.1069, trouvée: 164.1070.

### 7-Methylpyrido[3,2-d]pyrimidin-4-ol (146)

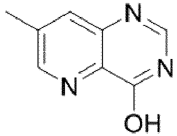

L'acide 3-amino-5-methyl-pyridine-2-carboxylique amide (200 mg, 1.33 mmol) est dissous dans 7.5 mL de triméthylorthoformate (68.6 mmol) puis le mélange est chauffé à 120 °C pendant 16 h. En fin de réaction, la solution est refroidie jusqu'à température ambiante et 20 mL d'eau glacée est ajoutée. Le produit précipite sous forme de solide marron qui est isolé par filtration et séchage au four (192 mg, 90 %). R_{f} (CH₂Cl₂/MeOH 90:10): 0.22. ¹H RMN (400 MHz, CDCl₃) δ 12.44 (br s, 1H), 8.64 (d, *J* = 8.0 Hz, 1H), 8.11 (s, 1H), 7.88 (d, *J* = 8.0 Hz, 1H), 2.47 (s, 3H). ¹³C RMN (101 MHz, CDCl₃) δ 159.5, 150.4, 146.2, 145.6, 139.2, 136.0, 134.6, 18.1. IR (ATR diamant, cm⁻¹) n 3440, 3276, 3033, 2789, 1715, 1595, 1303, 1284, 1212, 1184, 922, 898, 706, 687. HRMS (EI-MS) : m/z calculée pour C₈H₈N₃O [M+H]⁺: 162.0661, trouvée: 162.0663. T_{f}: > 260 °C.

### 4-Chloro-7-methyl-pyrido[3,2-d]pyrimidine (148)

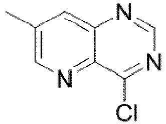

Le composé **148** est obtenu comme décrit pour **132** avec **146** (130 mg, 0.81 mmol, 1.0 eq.), le chlorure d'oxalyle (0.18 mL, 2.13 mmol, 2.5 eq.). Le produit est purifié par chromatographie sur gel de silice (éluant CH₂Cl₂/Acetone 99:1). Le produit est obtenu sous forme d'un solide blanc (946 mg, 86 %). R_{f} (CH₂Cl₂/Acetone 99:01): 0.52. ¹H RMN (400 MHz, CDCl₃) δ 9.07 (s, 1H), 8.98 (d, *J* = 8.0 Hz, 1H), 8.14 (d, *J* = 8.0 Hz, 1H), 2.65 (s, 3H). ¹³C RMN (101 MHz, CDCl₃) δ 163.9, 155.2, 154.5, 147.7, 140.7, 136.4, 135.5, 19.4. IR (ATR diamant, cm⁻¹) n 2624, 1715, 1558, 1371, 1322, 1223, 999, 900, 837, 673. HRMS (EI-MS) : m/z calculée pour C₈H₇ClN₃ [M+H]⁺: 180.0323, trouvée: 180.0322. T_{f}: > 260 °C.

### Benzofuro[3,2-d]pyrimidin-4-ol (150)

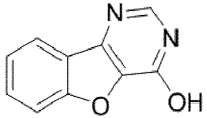

L'acide 3-aminobenzofuran-2-carboxylique amide (100 mg, 0.56 mmol) est dissous dans 5 mL de triméthylorthoformate (45.7 mmol) puis le mélange est chauffé à 150 °C pendant 2 h. En fin de réaction, la solution est refroidie jusqu'à température ambiante et 20 mL d'eau glacée est ajoutée. Le produit précipite sous forme de solide jaune qui est isolée par filtration et séchage au four (100 mg, 86 %). R_{f} (CH₂Cl₂/MeOH 90:10): 0.33. ¹H RMN (400 MHz, DMSO-*d*₆) δ 8.26 (s, 1H), 8.05 (d, *J* = 8.0 Hz, 1H), 7.83 (d, *J* = 8.0 Hz, 1H), 7.68 (t, *J* = 7.0 Hz, 1H), 7.50 (t, *J* = 7.0 Hz, 1H). ¹³C RMN (101 MHz, DMSO-d₆) δ 155.8, 152.5, 146.7, 143.1, 139.1, 129.9, 124.4, 122.1, 121.3, 112.9. IR (ATR diamant, cm⁻¹) n 3326, 2577, 1646, 1444, 1360, 1279, 1230, 1189, 1113, 1081, 959, 864, 743. HRMS (EI-MS) : m/z calculée pour C₁₀H₇N₂O₂ [M+H]⁺: 187.0502, trouvée: 187.0503. T_{f}: > 260 °C.

### 7-Fluoropyrido[3,2-d]pyrimidin-4-ol (153)

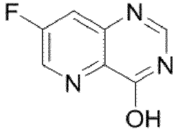

Le méthyl 3-amino-5-fluoropicolinate (100 mg, 0.59 mmol) est dissous dans 6 mL de formamide (151.0 mmol) puis le mélange est chauffé à 150 °C pendant 12 h. En fin de réaction, la solution est refroidie jusqu'à température ambiante et 10 mL d'eau glacée est ajoutée. Le produit précipite sous forme de solide marron qui est isolé par filtration et séchage au four (90 mg, 93 %). R_{f} (CH₂Cl₂/MeOH 90:10): 0.25. ¹H RMN (400 MHz, DMSO-d₆) δ 8.81 (d, *J* = 3.0 Hz, 1H), 8.19 (s, 1H), 8.01 (dd, *J* = 9.0, 3.0 Hz, 1H). ¹³C RMN (101 MHz, DMSO-*d*₆) δ 162.1, 159.5, 158.7, 147.6, 144.6, 139.1, 138.8, 136.3, 120.1, 119.9. IR (ATR diamant, cm⁻¹) n 3044, 2847, 1678, 1593, 1463, 1376, 1301, 1208, 892, 733, 651. HRMS (EI-MS) : m/z calculée pour C₇H₅FN₃O [M+H]⁺: 166.0411, trouvée: 166.0413. T_{f}: > 260 °C.

### 6-Fluorotetralin-1-amine (154)

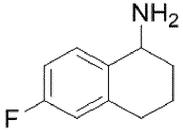

Le composé **154** est obtenu en suivant la procédure A avec le composé 6-fluorotetralone (500 mg, 3.0 mmol, 1.0 eq.), NH₄OAc (3.46 g, 45.0 mmol, 15.0 eq.) et NaBH₃CN (227 mg, 3.6 mmol, 1.2 eq.). Le résidu est purifié par chromatographie sur gel de silice (éluant CH₂Cl₂/MeOH 95:5). Le produit est obtenu sous forme d'une poudre jaune (498 mg, 81 %). R_{f} (CH₂Cl₂/MeOH 95:5): 0.18. ¹H RMN (400 MHz, CDCl₃) δ 7.28 (dd, *J* = 8.0, 6.0 Hz, 1H), 6.77 (td, *J* = 8.0, 3.0 Hz, 1H), 6.67 (dd, *J* = 9.0, 3.0 Hz, 1H), 3.85 (t, *J* = 6.0 Hz, 1H), 2.66 (m, 2H), 1.87 (m, 2H), 1.64 (m, 1H), 1.62 (m, 1H), 1.50 (br s, 2H, NH₂), ¹³C RMN (101 MHz, CDCl₃) δ 162.3, 159.9, 138.8, 138.7, 136.6, 129.6, 129.5, 114.7, 114.5, 112.8, 112.6, 48.7, 33.5, 29.5, 19.1. IR (ATR diamant, cm⁻¹): 2929, 1617, 1473, 1357, 813, 572. HRMS (EI-MS) : m/z calculée pour C₁₀H₁₃FN [M+H]⁺: 166.1026, trouvée: 166.1029. T_{f}: 104-106 °C.

### 4-Chloro-7-fluoro-pyrido[3,2-d]pyrimidine (155)

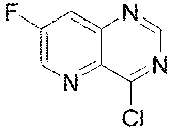

Le composé **155** est obtenu comme décrit pour **132** avec **153** (100 mg, 0.61 mmol, 1.0 eq.), le chlorure d'oxalyle (0.13 mL, 1.52 mmol, 2.5 eq.). Le produit est purifié par chromatographie sur gel de silice (éluant gradient 0 à 40 % AcOEt/100 à 60 % PE). Le produit est obtenu sous forme d'un solide beige (5 mg, 5 %). R_{f} (PE/AcOEt 50:50): 0.91. ¹H RMN (400 MHz, CDCl₃) δ 9.09 (s, 1H), 9.01 (d, *J* = 3.0 Hz, 1H), 8.00 (dd, *J* = 8.0, 3.0 Hz, 1H). ¹³C RMN (101 MHz, CDCl₃) δ 164.0, 160.9 (d, *J* = 270.0 Hz), 155.4, 148.7 (d, *J* = 10.0 Hz), 144.7 (d, *J* = 28.0 Hz), 135.4 (d, *J* = 2.0 Hz), 119.9 (d, *J* = 17.0 Hz). IR (ATR diamant, cm⁻¹) n 3025, 2582, 1678, 1646, 1411, 1301, 1076, 867, 732, 584. HRMS (EI-MS) : m/z calculée pour C₇H₄ClFN₃ [M+H]⁺: 184.0072, trouvée: 184.0073. T_{f}: 148-150 °C.

### 4-Chlorobenzofuro[3,2-d]pyrimidine (156)

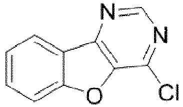

Le composé **156** est obtenu comme décrit pour **132** avec **150** (75 mg, 0.40 mmol, 1.0 eq.), le chlorure d'oxalyle (0.10 mL, 1.0 mmol, 2.5 eq.). Le produit est purifié par chromatographie sur gel de silice (éluant gradient 0 à 20 % AcOEt/100 à 80 % PE). Le produit est obtenu sous forme d'un solide blanc (56 mg, 68 %).R_{f} (PE/AcOEt 70:30): 0.49. ¹H RMN (400 MHz, CDCl₃) δ 8.99 (s, 1H), 8.25 (m, 1H), 7.75 (m, 1H), 7.54 (m, 1H). ¹³C RMN (101 MHz, CDCl₃) δ 158.1, 153.1, 151.4, 144.5, 142.8, 132.7, 125.0, 122.9, 121.6, 113.2. IR (ATR diamant, cm⁻¹) n 3059, 1626, 1538, 1488, 1380, 1213, 1187, 1141, 1066, 874, 635. HRMS (EI-MS) : m/z calculée pour C₁₀H₆ClN₂O [M+H]⁺: 205.0163, trouvée: 205.0163. T_{f}: 133-135 °C.

### 2. Préparation des composés de formule (I) selon l'invention

### Exemple 1 : N-(3,4-Dichlorobenzyl)pyrido[3,2-d]pyrimidin-2-amine (4)

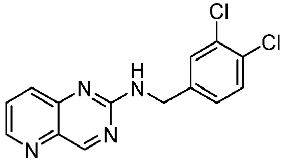

Le produit **3** (100 mg, 0,60 mmol) est dissous dans le 1,4-dioxane (7 mL) puis la 3,4-dichlorobenzylamine (0.10 mL, 0.75 mmol, 1.2 éq.) et la triéthylamine (0.11 mL, 0.79 mmol, 1.5 éq.) sont successivement additionnées et le mélange est agité à reflux pendant une nuit. Le 1,4-dioxane est évaporé, le résidu obtenu est repris à l'eau (20 mL) puis extrait au dichlorométhane (2x20mL). La phase organique rassemblée est ensuite séchée sur sulfate de magnésium, filtrée puis concentrée sous vide. Le brut est chromatographié sur gel de silice (gradient EP à EP/AcOEt 50:50) pour donner le produit attendu **4** (110 mg, 60%) sous forme d'un solide jaune. R_{f} (EP/AcOEt 60:40) : 0,13. ¹H RMN (400 MHz, CDCl₃) δ 9.20 (s, 1H), 8.66 (dd, *J* = 4.1, 1.4 Hz, 1H), 7.89 (d, *J* = 8.5 Hz, 1H), 7.57 (dd, *J* = 8.6, 4.1 Hz, 1H), 7.50 (d, *J* = 1.5 Hz, 1H), 7.39 (d, *J* = 8.2 Hz, 1H), 7.23 (dd, *J* = 8.2, 1.7 Hz, 1H), 5.93 (s, 1H), 4.73 (d, *J* = 6.2 Hz, 2H). ¹³C RMN (101 MHz, CDCl₃) δ 163.7, 159.3, 148.3, 147.5, 139.5, 137.2, 133.9, 132.8, 131.4, 130.7, 129.6, 128.5, 127.0, 44.8. IR (ATR diamant, cm⁻¹) v 3231, 3034, 2862, 1585, 1541, 1465, 1397, 1351, 1250, 1142, 1035, 936, 821. HRMS (EI-MS) m/z calculée pour C₁₄H₁₀Cl₂N₄ [M+H]⁺: 305,035528 ; trouvée 305,035355. T_{f}: 163-165°C.

### Exemple 2 : N-(1,2,3,4-Tétrahydronaphthalen-1-yl)pyrido[3,2-d]pyrimidin-2-amine (5)

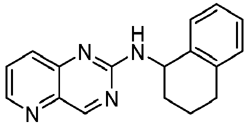

Le produit **5** est obtenu selon le même opératoire que **4** en partant de la 1,2,3,4-tétrahydronaphthylamine (0.12 mL, 0.83 mmol, 1.2 éq.) pour donner le produit attendu **5** (190 mg, quantitatif) sous forme d'un solide jaune. R_{f} (EP/AcOEt 60:40) : 0.18. ¹H RMN (250 MHz, CDCl₃) δ 9.12 (s, 1H), 8.62 (dd, *J* = 4.1, 1.5 Hz, 1H), 7.88 (s, 1H), 7.55 (dd, *J* = 8.6, 4.1 Hz, 1H), 7.39 (d, *J* = 7.2 Hz, 1H), 7.24-7.08 (m, 3H), 5.85 (d, *J* = 8.5 Hz, 1H), 5.44 (dd, *J* = 13.6, 5.9 Hz, 1H), 2.84 (dd, *J* = 11.7, 5.9 Hz, 2H), 2.21-2.14 (m, 1H), 2.02-1.92 (m, 3H). ¹³C RMN (101 MHz, CDCl₃) δ 164,7, 163.6, 148.6, 146.9, 137.7, 137.4, 137.1, 133.8, 129.5, 129.3, 128.9, 128.4, 127.4, 126.3, 47.5, 29.5, 20.2. IR (ATR diamant, cm⁻¹) v 3238, 3046, 2927, 2858, 1607, 1585, 1536, 1407, 1313, 1248, 1099, 916, 821. HRMS (EI-MS) m/z calculée pour C₁₇H₁₆N₄ [M+H]⁺: 277.144773 ; trouvée 277.144824. T_{f}: 120-122°C.

### Exemple 3: 2-Chloro-N-(1,2,3,4-tétrahydronaphthalen-1-yl)pyrido[3,2-d]pyrimidin-4-amine (6)

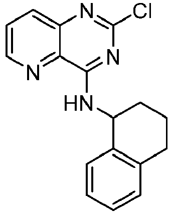

Le produit **2** (673 mg, 3.36 mmol) est dissous dans le THF (32 mL) puis le mélange est placé à 0°C La 1,2,3,4-tétrahydronaphthylamine (0.48 mL, 3.36 mmol, 1.0 éq.) puis la triéthylamine (0.50 mL, 3.53 mmol, 1.05 éq.) sont successivement additionnées et le mélange est agité à température ambiante pendant une nuit. Le THF est évaporé, le résidu obtenu est repris à l'eau (20 mL) puis extrait au dichlorométhane (2x20 mL). La phase organique rassemblée est ensuite séchée sur sulfate de magnésium, filtrée puis concentrée sous vide. Le brut est chromatographié sur gel de silice (gradient EP à EP/AcOEt 80:20) pour donner le produit attendu **6** (869 mg, 83%) sous forme d'un solide blanc. R_{f} (CH₂Cl₂/MeOH 99:01): 0.55. ¹H RMN (250 MHz, CDCl₃) δ 8.61 (dd, *J* = 4.3, 1.5 Hz, 1H), 8.03 (dd, *J* = 8.5, 1.5 Hz, 1H), 7.64 (dd, *J* = 8.5, 4.3 Hz, 1H), 7.58 (d, *J* = 8.6 Hz, 1H), 7.32 (d, *J* = 7.2 Hz, 1H), 7.23-7.14 (m, 3H), 5.63 (dd, *J* = 14.7, 5.7 Hz, 1H), 2.87 (q, *J* = 6.3 Hz, 2H), 2.30-2.14 (m, 1H), 2.11-2.03 (m, 1H), 1.99-1.92 (m, 2H). ¹³C RMN (101 MHz, CDCl₃) δ 160.3, 158.7, 148.4, 145.9, 137.9, 135.9, 135.2, 130.8, 129.5, 129.0, 128.5, 127.8, 126.6, 49.0, 29.7, 29.3, 20.1. IR (ATR diamant, cm⁻¹) v 3388, 2944, 1574, 1542, 1466, 1397, 1375, 1283, 1127, 1033, 957, 826. HRMS (EI-MS) m/z calculée pour C₁₇H₁₅ClN₄ [M+H]⁺: 311.105801, trouvée 311.105823. T_{f}: 146-148°C.

### Exemple 4 : N-(1,2,3,4-Tétrahydronaphthalen-1-yl)pyrido[3,2-d]pyrimidin-4-amine (7)

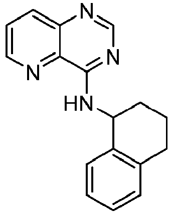

Le produit **6** (100 mg, 0,32 mmol) est dissous dans le THF anhydre (1 mL) sous atmosphère d'argon, puis la triéthylamine (0.13 mL, 0.97 mmol, 3.0 éq.) et l'acide formique (0.02 mL, 0,64 mmol, 2.0 éq.) sont respectivement ajoutés. Après 5 minutes d'agitation à température ambiante, l'acétate de palladium (7 mg, 0.032 mmol, 0.1 éq.) et le Xantphos (37 mg, 0.064 mmol, 0.2 éq.) sont ajoutés puis le mélange est porté à 150°C sous irradiation micro-ondes pendant 15 minutes. Le mélange réactionnel est évaporé puis le résidu obtenu est repris avec de l'eau (20 mL) et extrait au dichlorométhane (2x20 mL). La phase organique rassemblée est séchée sur sulfate de magnésium puis concentrée sous vide. Le brut est ensuite purifié par chromatographie sur gel de silice (gradient CH₂Cl₂ à CH₂Cl₂/MeOH 99,5:0,5) pour donner le produit attendu **7** (70 mg, 79%) sous forme d'un solide jaune. R_{f} (CH₂Cl₂/MeOH 98:02): 0.28. ¹H RMN (250 MHz, CDCl₃) δ 8.68 (s, 1H), 8.63 (dd, *J* = 4.2, 1.5 Hz, 1H), 8.10 (dd, *J* = 8.5, 1.4 Hz, 1H), 7.63 (dd, *J* = 8.5, 4.2 Hz, 1H), 7.46 (d, *J* = 8.0 Hz, 1H), 7.34 (d, *J* = 7.0 Hz, 1H), 7.24-7.08 (m, 3H), 5.63 (dd, *J* = 14.3, 5.7 Hz, 1H), 2.87 (q, *J* = 6.4 Hz, 2H), 2.28-2.16 (m, 1H), 2.13-1.86 (m, 3H). ¹³C RMN (101 MHz, CDCl₃) δ 159.2, 156.6, 148.2, 144.5, 137.8, 136.5, 135.9, 132.1, 129.4, 129.0, 127.8, 127.6, 126.4, 48.7, 29.8, 29.4, 20.1. IR (ATR diamant, cm⁻¹) v 3213, 2938, 1577, 1538, 1480, 1388, 1313, 1268, 1153, 917, 899. HRMS (EI-MS) m/z calculée pour C₁₇H₁₆N₄ [M+H]⁺: 277.144773, trouvée 277.145010. T_{f}: 96-98°C.

### Exemple 5 : 2-Chloro-N-(3,4-dichlorobenzyl)pyrido[3,2-d]pyrimidin-4-amine (8)

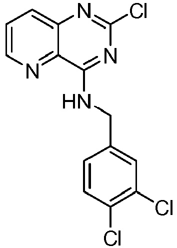

Le produit **8** est obtenu comme décrit pour **6** en partant de **2** et de la 3,4 dichlorobenzylamine (0.10 mL, 0.75 mmol, 1.0 éq.) pour donner le produit attendu **8** (163 mg, 64%) sous forme d'un solide blanc. R_{f} (CH₂Cl₂/MeOH 95:05): 0.65. ¹H RMN (250 MHz, CDCl₃) δ 8.67 (dd, *J* = 4.3, 1.5 Hz, 1H), 8.05 (dd, *J* = 8.5, 1.5 Hz, 1H), 7.68 (dd, *J* = 8.5, 4.3 Hz, 1H), 7.60 (s, 1H), 7.49 (d, *J* = 2.0 Hz, 1H), 7.43 (d, *J* = 8.2 Hz, 1H), 7.25 (dd, *J* = 7.7, 2.6 Hz, 1H), 4.83 (d, *J* = 6.2 Hz, 2H). ¹³C RMN (101 MHz, CDCl₃) δ 160.9, 158.4, 148.7, 145.9, 137.6, 135.3, 133.0, 132.2, 130.9, 130.6, 130.0, 128.7, 127.5, 43.9. IR (ATR diamant, cm⁻¹) v 3372, 1587, 1546, 1427, 1375, 1279, 1209, 1130, 1028, 922, 869, 822. HRMS (EI-MS) m/z calculée pour C₁₄H₉Cl₃N₄ [M+H]⁺: 338.996556, trouvée 338.996507. T_{f}: 120-122°C.

### Exemple 6 : N-(4-Chlorobenzyl)pyrido[3,2-d]pyrimidin-4-amine (11)

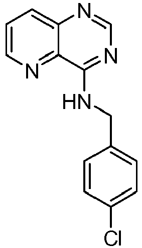

Le produit **10** (43 mg, 0.26 mmol) est dissous dans le dioxane (2 mL) puis le mélange est placé à 0°C La 4-chlorobenzylamine (0.03 mL, 0.26 mmol, 1.0 éq.) puis la triéthylamine (0.04 mL, 0.26 mmol, 1.0 éq.) sont successivement additionnées et le mélange est agité à température ambiante pendant une nuit. Le dioxane est évaporé, le résidu obtenu est repris à l'eau (20 mL) puis extrait au dichlorométhane (2x20 mL). La phase organique rassemblée est ensuite séchée sur sulfate de magnésium, filtrée puis concentrée sous vide. Le brut est chromatographié sur gel de silice (gradient EP à EP/AcOEt 40:60) pour donner le produit attendu **11** (35 mg, 50%) sous forme d'un solide blanc. R_{f} (EP/AcOEt 50:50): 0.18. ¹H RMN (400 MHz, CDCl₃) δ 8.74-8.58 (m, 2H), 8.11 (dd, *J* = 8.5, 1.5 Hz, 1H), 7.65 (dd, *J* = 8.5, 4.2 Hz, 1H), 7.50 (s, 1H), 7.36-7.26 (m, 4H), 4.83 (d, *J* = 6.1 Hz, 2H). ¹³C RMN (101 MHz, CDCl₃) δ 159.8, 156.4, 148.5, 144.5, 136.6, 136.1, 133.6, 132.1, 129.3 (x2), 129.0 (x2), 127.9, 44.1. IR (ATR diamant, cm⁻¹) v 3399, 3253, 2923, 1585, 1551, 1490, 1362, 1312, 1112, 1011, 920, 803. HRMS (EI-MS) m/z calculée pour C₁₄H₁₁ClN₄ [M+H]⁺: 271.074501, trouvée 271.074754. T_{f}: 141-143°C

### Exemple 7 : N-(3-Chlorobenzyl)pyrido[3,2-d]pyrimidin-4-amine (12)

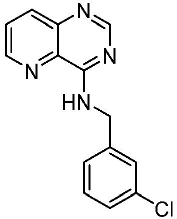

Le produit **12** est obtenu comme décrit pour **11** en utilisant le produit **10** et la 3-chlorobenzylamine (0.03 mL, 0.22 mmol, 1.0 éq.) pour donner le produit attendu **12** (30 mg, 50%) sous forme d'une huile incolore. R_{f} (EP/AcOEt 70:30): 0.13. ¹H RMN (400 MHz, CDCl₃) δ 8.77-8.60 (m, 2H), 8.12 (dd, *J* = 8.5, 1.4 Hz, 1H), 7.66 (dd, *J* = 8.5, 4.2 Hz, 1H), 7.53 (s, 1H), 7.39 (s, 1H), 7.31-7.24 (m, 3H), 4.85 (d, *J* = 6.1 Hz, 2H). ¹³C RMN (101 MHz, CDCl₃) δ 159.8, 156.4, 148.5, 144.5, 140.1, 136.1, 134.8, 132.0, 130.2, 128.0 (x2), 126.0, 44.1. IR (ATR diamant, cm⁻¹) v 3248, 1581, 1553, 1482, 1416, 1310, 1196, 1077, 991, 894, 825. HRMS (EI-MS) m/z calculée pour C₁₄H₁₁ClN₄ [M+H]⁺: 271.074501, trouvée 271.074641. T_{f}: 87-89°C.

### Exemple 8 : N-Benzylpyrido[3,2-d]pyrimidin-4-amine (13)

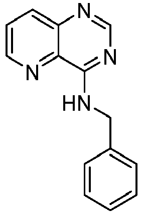

Le produit **13** est obtenu comme décrit pour **11** au départ de **10** (100 mg, 0.37 mmol) et de la benzylamine et la 3-chlorobenzylamine (0.03 mL, 0.37 mmol, 1.0 éq.) pour donner le produit attendu **13** (30 mg, 34%) sous forme d'un solide jaune. R_{f} (CH₂Cl₂/MeOH 99:01): 0.13. ¹H RMN (400 MHz, CDCl₃) δ 8.68 (d, 1H) 8.67 (s, 1H), 8.11 (dd, *J* = 8.5, 1.5 Hz, 1H), 7.65 (dd, *J* = 8.5, 4.3 Hz, 1H), 7.50 (s, 1H), 7.46-7.28 (m, 5H), 4.87 (d, *J* = 5.9 Hz, 2H). ¹³C RMN (101 MHz, CDCl₃) δ 159.9, 156.6, 148.5, 144.5, 138.1, 136.1, 132.3, 129.0 (x2), 128.1 (x2), 128.0, 127.9, 45.0. IR (ATR diamant, cm⁻¹) v 3257, 3057, 1578, 1483, 1421, 1382, 1352, 1312, 1142, 1023, 890, 825, 800. HRMS (EI-MS) m/z calculée pour C₁₄H₁₂N₄ [M+H]⁺: 237.113473, trouvée 237.113550. T_{f}: 84-86°C.

### Exemple 9 : N-(3,4-Dichlorobenzyl)pyrido[3,2-d]pyrimidin-4-amine (14)

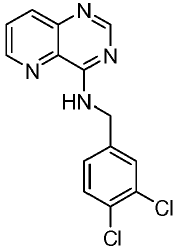

Le produit **14** est obtenu comme décrit pour **11** en utilisant le produit **10** (110 mg, 0.66 mmol) et la 3,4 dichlorobenzylamine (0.18 mL, 1.33 mmol, 2 éq.) pour donner le produit attendu **14** (59 mg, 30%) sous forme d'un solide blanc. R_{f} (CH₂Cl₂/MeOH 97:03): 0.33. ¹H RMN (400 MHz, CDCl₃) δ 8.67 (dt, *J* = 4.2, 1.5 Hz, 1H), 8.64 (s, 1H), 8.10 (dt, *J* = 8.5, 1.5 Hz, 1H), 7.65 (ddd, *J* = 8.5, 4.2, 1.4 Hz, 1H), 7.56 (s, 1H), 7.46 (s, 1H), 7.37 (dd, *J* = 8.2, 1.4 Hz, 1H), 7.24-7.16 (m, 1H), 4.81 (d, *J* = 6.2 Hz, 2H). ¹³C RMN (101 MHz, CDCl₃) δ 159.8, 156.3, 148.5, 144.5, 138.5, 136.1, 132.8, 132.0, 131.7, 130.7, 129.7, 128.0, 127.1, 43.5. IR (ATR diamant, cm⁻¹) v 3257, 3066, 1578, 1551, 1469, 1371, 1310, 1127, 1027, 897, 827, 804. HRMS (EI-MS) m/z calculée pour C₁₄H₁₀Cl₂N₄ [M+H]⁺: 305.035528, trouvée 305.035598. T_{f}: 140-142°C.

### Exemple 10 : N-(7-Bromo-1,2,3,4-tétrahydronaphthalen-1-yl)pyrido[3,2-d]pyrimidin-4-amine (16)

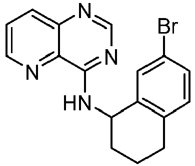

Le produit **16** est obtenu comme décrit pour **11** au départ de l'amine **15** (217 mg, 0.96 mmol, 1 éq.) pour donner le produit attendu **16** (149 mg, 43%) sous forme d'une huile incolore. R_{f} (EP/AcOEt 50:50): 0,33. ¹H RMN (400 MHz, CDCl₃) δ 8.65 (dd, *J* = 6.9, 3.7 Hz, 2H), 8.10 (d, *J* = 8.4 Hz, 1H), 7.64 (dd, *J* = 8.5, 4.2 Hz, 1H), 7.44 (dd, *J* = 15.5, 4.9 Hz, 2H), 7.29 (dd, *J* = 8.2, 1.8 Hz, 1H), 7.00 (d, *J* = 8.2 Hz, 1H), 5.61 (dd, *J* = 14.4, 6.2 Hz, 1H), 2.78 (dt, *J* = 12.8, 6.4 Hz, 2H), 2.23 - 2.10 (m, 1H), 2.05 - 1.86 (m, 3H). ¹³C RMN (101 MHz, CDCl₃) δ 159.2, 156.5, 148.3, 144.5, 138.8, 136.7, 136.0, 132.0, 131.5, 131.0, 130.7, 127.9, 119.8, 48.3, 29.6, 28.9, 20.0. IR (ATR diamant, cm⁻¹) v 3380, 2930, 2858, 1672, 1578, 1531, 1478, 1360, 1299, 1119, 1001, 873, 828, 802. HRMS (EI-MS) m/z calculée pour C₁₇H₁₅BrN₄ [M+H]⁺: 355.055285, trouvée 355.055432.

### Exemple 11 : (R)-2-Chloro-N-(1,2,3,4-tétrahydronaphthalen-1-yl)pyrido-[3,2-d]pyrimidin-4-amine (17)

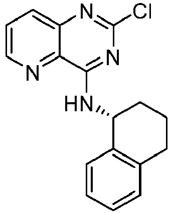

Le produit **17** est obtenu comme décrit pour **6** au départ de **2** (150 mg, 0.75 mmol) et de (*R*)-1,2,3,4-tétrahydronaphthylamine (0.10 mL, 0.75 mmol, 1.0 éq.) pour donner le produit attendu **17** (150 mg, 64%) sous forme d'un solide blanc. R_{f} (CH₂Cl₂/MeOH 99:01): 0.55. ¹H RMN (400 MHz, CDCl₃) δ 8.57 (d, *J* = 4.2 Hz, 1H), 8.00 (d, *J* = 8.5 Hz, 1H), 7.69-7.48 (m, 2H), 7.31 (d, *J* = 7.6 Hz, 1H), 7.22-7.12(m, 3H), 5.61 (dd, *J* = 14.3, 5.9 Hz, 1H), 2.99-2.68 (m, 2H), 2.24-2.16 (m, 1H), 2.07-1.81 (m, 3H). ¹³C RMN (101 MHz, CDCl₃) δ 160.1, 158.6, 148.3, 145.7, 137.7, 135.8, 135.0, 130.7, 129.4, 128.9, 128.4, 127.7, 126.4, 48.9, 29.6, 29.2, 20.0. IR (ATR diamant, cm⁻¹) v 3256, 2927, 1578, 1466, 1394, 1377, 1321, 1270, 1192, 1127, 958, 873, 824. HRMS (EI-MS) m/z calculée pour C₁₇H₁₅ClN₄ [M+H]⁺: 311.105801, trouvée 311.106246. [α]_{D} = +67.2 (c 1.0, CHCl₃). T_{f}: 180-182°C.

### Exemple 12 : (R)-N-(1,2,3,4-Tétrahydronaphthalen-1-yl)pyrido[3,2-d] pyrimidin-4-amine (18)

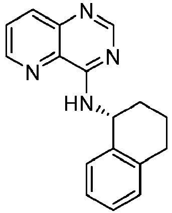

Le produit **18** est obtenu comme décrit pour **7** au départ de **17** (100 mg, 0.32 mmol) et de pour donner le produit attendu **18** (60 mg, 67%) sous forme d'un solide jaune. R_{f} (CH₂Cl₂/MeOH 98:02): 0.28. ¹H RMN (400 MHz, CDCl₃) δ 8.68 (s, 1H), 8.62 (dd, *J* = 4.2, 1.4 Hz, 1H), 8.11 (dd, *J* = 8.5, 1.4 Hz, 1H), 7.63 (dd, *J* = 8.5, 4.2 Hz, 1H), 7.48 (d, *J* = 8.2 Hz, 1H), 7.34 (d, *J* = 7.2 Hz, 1H), 7.22-7.11 (m, 3H), 5.63 (dd, *J* = 14.3, 5.9 Hz, 1H), 3.05-2.65 (m, 2H), 2.23-2.17 (m, 1H), 2.10-1.84 (m, 3H). ¹³C RMN (101 MHz, CDCl₃) δ 159.2, 156.6, 148.2, 144.4, 137.8, 136.5, 135.9, 132.1, 129.4, 129.0, 127.8, 127.6, 126.4, 48.8, 29.8, 29.4, 20.2. IR (ATR diamant, cm⁻¹) v 3373, 2934, 1582, 1536, 1365, 1311, 1137, 1111, 1001, 928, 830. HRMS (EI-MS) m/z calculée pour C₁₇H₁₆N₄ [M+H]⁺: 277.144773, trouvée 277.145111. [α]_{D} = +8.3 (c 1.1, CHCl₃). T_{f}: 85-87°C.

### Exemple 13 : (S)-2-Chloro-N-(1,2,3,4-tétrahydronaphthalen-1-yl)pyrido [3,2-d]pyrimidin-4-amine (19)

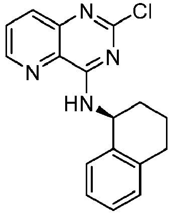

Le produit **19** est obtenu comme décrit pour **6** au départ de **2** (200 mg, 1.00 mmol) et de (*S*)-1,2,3,4-tétrahydronaphthylamine (0.15 mL, 1.05 mmol, 1.0 éq.) pour donner le produit attendu **19** (165 mg, 53%) sous forme d'un solide blanc. R_{f} (CH₂Cl₂/MeOH 99:01): 0.55. ¹H RMN (400 MHz, CDCl₃) δ 8.59 (dd, *J* = 4.2, 1.5 Hz, 1H), 8.02 (dd, *J* = 8.5, 1.5 Hz, 1H), 7.71-7.52 (m, 2H), 7.32 (d, *J* = 7.5 Hz, 1H), 7.23-7.14 (m, 3H), 5.62 (dd, *J* = 14.2, 5.9 Hz, 1H), 2.98-2.71 (m, 2H), 2.25-2.17 (m, 1H), 2.12-1.80 (m, 3H). ¹³C RMN (101 MHz, CDCl₃) δ 160.2, 158.7, 148.3, 145.8, 137.8, 135.8, 135.1, 130.8, 129.5, 129.0, 128.4, 127.8, 126.5, 49.0, 29.6, 29.3, 20.0. IR (ATR diamant, cm⁻¹) v 3256, 2927, 1578, 1533, 1394, 1321, 1270, 1127, 958, 873, 824. HRMS (EI-MS) m/z calculée pour C₁₇H₁₅ClN₄ [M+H]⁺: 311.105801, trouvée 311.106098. [α]_{D} = -68.0 (c 1.0, CHCl₃). T_{f}: 179-181°C.

### Exemple 14 : (S)-N-(1,2,3,4-Tétrahydronaphthalen-1-yl)pyrido[3,2-d]pyrimidin-4-amine (20)

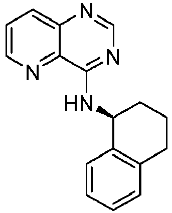

Le produit **20** est obtenu comme décrit pour **7** au départ de **19** (100 mg, 0.32 mmol) pour donner le produit attendu **20** (70 mg, 79%) sous forme d'un solide jaune. R_{f} (CH₂Cl₂/MeOH 98:02): 0.28. ¹H RMN (400 MHz, CDCl₃) δ 8.69 (s, 1H), 8.64 (dd, *J* = 4.2, 1.4 Hz, 1H), 8.11 (dd, *J* = 8.5, 1.4 Hz, 1H), 7.64 (dd, *J* = 8.5, 4.2 Hz, 1H), 7.46 (d, *J* = 8.3 Hz, 1H), 7.34 (d, *J* = 7.3 Hz, 1H), 7.24-7.16 (m, 3H), 5.64 (dd, *J* = 14.3, 6.0 Hz, 1H), 3.03-2.65 (m, 2H), 2.28-2.14 (m, 1H), 2.11-1.89 (m, 3H). ¹³C RMN (101 MHz, CDCl₃) δ 159.3, 156.7, 148.3, 144.5, 137.9, 136.6, 136.0, 132.2, 129.4, 129.0, 127.8, 127.6, 126.5, 48.8, 29.8, 29.4, 20.2. IR (ATR diamant, cm⁻¹) v 3373, 2934, 1582, 1536, 1365, 1311, 1137, 1111, 1001, 928, 830. HRMS (EI-MS) m/z calculée pour C₁₇H₁₆N₄ [M+H]⁺: 277.144773, trouvée 277.145203. [α]_{D} = -8.1 (c 1.2, CHCl₃). T_{f}: 80-82°C

### Exemple 15 : N-Benzyl-2-chloropyrido[3,2-d]pyrimidin-4-amine (21)

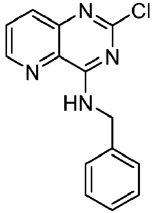

Le produit **21** est obtenu comme décrit pour **6** au départ de **2** (200 mg, 1.00 mmol) et de benzylamine (0.11 mL, 1.00 mmol, 1 éq.) pour donner le produit attendu **21** (181 mg, 67%) sous forme d'un solide blanc. R_{f} (EP/AcOEt 70:30): 0.40. ¹H RMN (400 MHz, CDCl₃) δ 8.62 (dd, *J* = 4.3, 1.5 Hz, 1H), 8.01 (dd, *J* = 8.5, 1.5 Hz, 1H), 7.68-7.55 (m, 2H), 7.42-7.29 (m, 5H), 4.84 (d, *J* = 5.9 Hz, 2H). ¹³C RMN (101 MHz, CDCl₃) δ 160.8, 158.5, 148.4, 145.7, 137.2, 135.1, 130.7, 129.0 (x2), 128.5, 128.2 (x2), 128.0, 45.2. IR (ATR diamant, cm⁻¹) v 3401, 3036, 1578, 1543, 1456, 1377, 1323, 1282, 1220, 1132, 956, 873, 808. HRMS (EI-MS) m/z calculée pour C₁₄H₁₁ClN₄ [M+H]⁺: 271.074501, trouvée 271.074680. T_{f}: 130-132°C.

### Exemple 16 : 2-Chloro-N-cyclohexylpyrido[3,2-d]pyrimidin-4-amine (22)

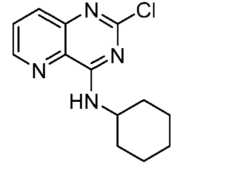

Le produit **22** est obtenu comme décrit pour **6** au départ de **2** (100 mg, 0.50 mmol) et de cyclohexylamine (0.06 mL, 0.50 mmol, 1.0 éq.) pour donner le produit attendu **22** (94 mg, 72%) sous forme d'un solide blanc. R_{f} (EP/AcOEt 80:20): 0.44. ¹H RMN (400 MHz, CDCl₃) δ 8.63 (dd, *J* = 4.2, 1.4 Hz, 1H), 7.98 (dd, *J* = 8.5, 1.4 Hz, 1H), 7.61 (dd, *J* = 8.5, 4.3 Hz, 1H), 7.24 (s, 1H), 4.31-4.07 (m, 1H), 2.09 (dd, *J* = 12.1, 3.1 Hz, 2H), 1.85-1.74 (m, 2H), 1.74-1.60 (m, 1H), 1.57-1.23 (m, 5H). ¹³C RMN (101 MHz, CDCl₃) δ 160.0, 158.7, 148.1 (x2), 145.6, 135.1 (x2), 130.8, 128.3 (x2), 49.5, 32.7, 25.61, 24.8. IR (ATR diamant, cm⁻¹) v 3267, 2924, 2854, 1585, 1538, 1470, 1381, 1323, 1272, 1133, 1066, 951, 871, 820. HRMS (EI-MS) m/z calculée pour C₁₃H₁₅ClN₄ [M+H]⁺: 263.105801, trouvée 263.106066. T_{f}: 80-82°C.

### Exemple 17 : N-Cyclohexylpyrido[3,2-d]pyrimidin-4-amine (23)

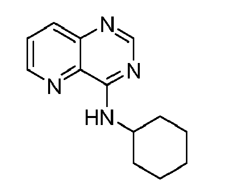

Le produit **23** est obtenu comme décrit pour **7** au départ de **22** (70 mg, 0.27 mmol) pour donner le produit attendu **23** (30 mg, 50%) sous forme d'une huile jaune. R_{f} (EP/AcOEt 50:50): 0.23. ¹H RMN (400 MHz, CDCl₃) δ 8.70-8.63 (m, 1H), 8.61 (s, 1H), 8.08 (d, *J* = 8.4 Hz, 1H), 7.63 (dd, *J* = 8.4, 4.2 Hz, 1H), 7.16 (d, *J* = 6.3 Hz, 1H), 4.19 (dt, *J* = 14.5, 8.4 Hz, 1H), 2.21-2.02 (m, 2H), 1.90-1.75 (m, 2H), 1.69 (dd, *J* = 9.1, 3.8 Hz, 1H), 1.59-1.29 (m, 5H). ¹³C RMN (101 MHz, CDCl₃) δ 159.0, 156.5, 148.0 (x2), 144.2, 135.8, 132.2, 127.7 (x2), 49.4, 32.9, 25.7, 24.9. IR (ATR diamant, cm⁻¹) v 386, 2929, 2853, 1581, 1541, 1438, 1364, 1317, 1276, 914, 836, 828. HRMS (EI-MS) m/z calculée pour C₁₃H₁₆N₄ [M+H]⁺: 229.144773, trouvée 229.145034.

### Exemple 18: 2-Chloro-N-(5,7-diméthyl-1,2,3,4-tétrahydronaphthalen-1-yl)pyrido[3,2-d]pyrimidin-4-amine (25)

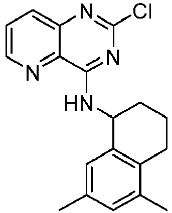

Le produit **25** est obtenu comme décrit pour **6** au départ de 2 (200 mg, 1.00 mmol) et de l'amine **24** (175 mg, 1.0 mmol, 1.0 éq.) pour donner le produit attendu **25** (230 mg, 68%) sous forme d'un solide blanc. R_{f} (EP/AcOEt 70:30): 0.55. ¹H RMN (400 MHz, CDCl₃) δ 8.55 (dd, *J* = 4.2, 1.1 Hz, 1H), 8.00 (dd, *J* = 8.5, 1.1 Hz, 1H), 7.67 (d, *J* = 8.6 Hz, 1H), 7.60 (dd, J = 8.5, 4.2 Hz, 1H), 6.98 (s, 1H), 6.91 (s, 1H), 5.61 - 5.46 (m, 1H), 2.73 - 2.50 (m, 2H), 2.23 (s, 3H), 2.20 (s, 3H), 2.15 - 1.99 (m, 2H), 1.99 - 1.89 (m, 2H). ¹³C RMN (101 MHz, CDCl₃) δ 159.9, 158.6, 148.2, 145.6, 136.7, 135.4, 135.3, 134.9, 133.0, 130.7, 130.2, 128.2, 127.2, 49.3, 28.9, 26.2, 20.9, 19.6, 19.5. IR (ATR diamant, cm⁻¹) v 3271, 2930, 2861, 1577, 1537, 1466, 1389, 1322, 1271, 1190, 1127, 1035, 956, 873, 822. HRMS (EI-MS) m/z calculée pour C₁₉H₁₉ClN₄ [M+H]⁺: 339.137101, trouvée 339.137019. T_{f}: 55-57°C.

### Exemple 19 : N-(5,7-Diméthyl-1,2,3,4-tétrahydronaphthalen-1-yl)pyrido [3,2-d]pyrimidin-4-amine (26)

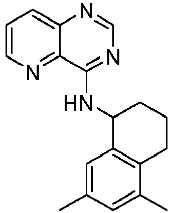

Le produit **26** est obtenu comme décrit pour **7** au départ de **25** (100 mg, 0.30 mmol) pour donner le produit attendu **26** (52 mg, 58%) sous forme d'une huile incolore. R_{f} (EP/AcOEt 50:50): 0.32. ¹H RMN (400 MHz, CDCl₃) δ 8.69 (s, 1H), 8.63 (dd, *J* = 4.2, 1.5 Hz, 1H), 8.11 (dd, *J* = 8.5, 1.5 Hz, 1H), 7.63 (dd, *J* = 8.5, 4.2 Hz, 1H), 7.48 (d, *J* = 8.3 Hz, 1H), 7.02 (s, 1H), 6.94 (s, 1H), 5.61 - 5.49 (m, 1H), 2.76 - 2.69 (m, 1H), 2.67 - 2.56 (m, 1H), 2.24 (s, 6H), 2.18 - 2.04 (m, 2H), 1.99 - 1.94 (m, 2H). ¹³C RMN (101 MHz, CDCl₃) δ 159.1, 156.7, 148.2, 144.4, 136.8, 136.2, 135.9, 135.5, 133.2, 132.2, 130.2, 127.8, 127.3, 49.1, 29.2, 26.4, 21.0, 19.8, 19.7. (ATR diamant, cm⁻¹) v 2929, 2861, 1578, 1533, 1478, 1439, 1360, 1305, 1192, 1112, 908, 857, 827, 802.HRMS (EI-MS) m/z calculée pour C₁₉H₂₀N₄ [M+H]⁺: 305.176073, trouvée 305.176343. IR

### Exemple 20 : 2-Chloro-N-(1,2,3,4-tétrahydrophenanthren-4-yl)pyrido[3,2-d]pyrimidin-4-amine (28)

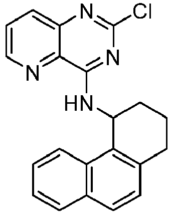

Le produit **28** est obtenu comme décrit pour **6** au départ de **2** (200 mg, 1.00 mmol) et de l'amine **27** (197 mg, 1.0 mmol, 1.0 éq.) pour donner le produit attendu **28** (240 mg, 67%) sous forme d'un solide blanc. R_{f} (EP/AcOEt 80:20): 0.43. ¹H RMN (400 MHz, CDCl₃) δ 8.48 (d, *J* = 4.2 Hz, 1H), 8.02 (d, *J* = 8.5 Hz, 1H), 7.95 - 7.87 (m, 1H), 7.84 - 7.79 (m, 1H), 7.76 (d, *J* = 8.5 Hz, 1H), 7.65 - 7.54 (m, 2H), 7.42 (dd, *J* = 9.5, 4.9 Hz, 2H), 7.27 (d, *J* = 8.3 Hz, 1H), 6.15 (d, *J* = 8.2 Hz, 1H), 3.12 - 2.89 (m, 2H), 2.52 (d, *J* = 8.6 Hz, 1H), 2.11 - 1.92 (m, 3H). ¹³C RMN (101 MHz, CDCl₃) δ 159.4, 158.7, 148.3, 145.8, 136.3, 135.1, 132.6, 132.4, 130.8, 129.2, 128.7, 128.7, 128.4, 128.1, 127.0, 125.4, 123.0, 45.0, 30.1, 28.7, 18.2. IR (ATR diamant, cm⁻¹) v 3383, 2930, 1575, 1534, 1466, 1435, 1389, 1324, 1273, 1190, 1127, 959, 874, 805. HRMS (EI-MS) m/z calculée pour C₂₁H₁₇ClN₄ [M+H]⁺: 361.121451, trouvée 361.121633. T_{f}: 70-72°C.

### Exemple 21 : N-(1,2,3,4-Tétrahydrophenanthren-4-yl)pyrido[3,2-d] pyrimidin-4-amine (29)

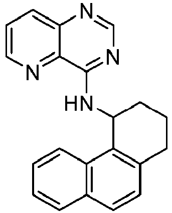

Le produit **29** est obtenu comme décrit pour **7** au départ de **28** (100 mg, 0.28 mmol) pour donner le produit attendu **29** (70 mg, 77%) sous forme d'une huile jaune. R_{f} (EP/AcOEt 70:30): 0.18. ¹H RMN (400 MHz, CDCl₃) δ 8.81 (s, 1H), 8.52 (dd, *J* = 4.2, 1.5 Hz, 1H), 8.11 (dd, *J* = 8.5, 1.5 Hz, 1H), 7.93 - 7.85 (m, 1H), 7.80 (dd, *J* = 6.2, 3.1 Hz, 1H), 7.75 (d, *J* = 8.5 Hz, 1H), 7.59 (dd, *J* = 8.5, 4.2 Hz, 1H), 7.50 (d, *J* = 8.1 Hz, 1H), 7.43 - 7.33 (m, 2H), 7.31 - 7.24 (m, 1H), 6.18 (d, *J* = 8.3 Hz, 1H), 3.14 - 2.87 (m, 2H), 2.57 - 2.41 (m, 1H), 2.09 - 1.92 (m, 3H). ¹³C RMN (101 MHz, CDCl₃) δ 158.4, 156.7, 148.3, 144.5, 136.3, 135.9, 132.6, 132.5, 132.2, 129.9, 128.6, 128.5, 128.1, 127.8, 127.0, 125.3, 123.1, 44.6, 30.2, 28.8, 18.3. IR (ATR diamant, cm⁻¹) v 3384, 3049, 2930, 1575, 1533, 1466, 1436, 1389, 1325, 1273, 1190, 1126, 959, 874, 805. HRMS (EI-MS) m/z calculée pour C₂₁H₁₈N₄ [M+H]⁺: 327.160423, trouvée 327.160571.

### Exemple 22 : 2-Méthyl-N-(1,2,3,4-tétrahydronaphthalen-1-yl)pyrido[3,2-d]pyrimidin-4-amine (30)

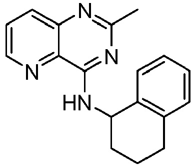

Le produit **6** (100 mg, 0.32 mmol) est dissous dans le THF (3.5 mL) sous argon puis une solution de triméthylaluminium dans le toluène (2M, 0.35 mL, 0.70 mmol, 2.2 éq.) est ajoutée goutte à goutte. Le Pd(PPh₃)₄ (36 mg, 0.032 mmol, 0.1 éq.) est ensuite additionné puis le milieu est chauffé à reflux pendant une nuit. Après refroidissement, le solvant est évaporé. Le brut réactionnel est purifié par chromatographie sur gel de silice (gradient CH₂Cl₂ à CH₂Cl₂/MeOH 99:01) pour donner le produit attendu **30** (65 mg, 70%) sous forme d'un solide blanc. R_{f} (CH₂Cl₂/MeOH 99:01): 0.20. ¹H RMN (400 MHz, CDCl₃) δ 8.57 (dd, *J* = 4.3, 1.5 Hz, 1H), 8.03 (dd, *J* = 8.5, 1.6 Hz, 1H), 7.59 (dd, *J* = 8.4, 4.2 Hz, 1H), 7.44-7.32 (m, 2H), 7.25-7.11 (m, 3H), 5.70 (dd, *J* = 14.1, 6.2 Hz, 1H), 2.97-2.79 (m, 2H), 2.70 (s, 3H), 2.29-2.16 (m, 1H), 2.10-1.88 (m, 3H). ¹³C RMN (101 MHz, CDCl₃) δ 166.0, 158.9, 147.2, 144.7, 137.7, 136.9, 135.0, 130.6, 129.3, 128.9, 127.6, 127.4, 126.3, 48.3, 29.8, 29.4, 27.0, 20.2. IR (ATR diamant, cm⁻¹) v 3385, 2928, 1578, 1530, 1440, 1413, 1366, 1339, 1297, 1162, 1114, 1001, 825. HRMS (EI-MS) m/z calculée pour C₁₈H₁₈N₄ [M+H]⁺: 291.160423, trouvée 291.160771. T_{f}: 95-97°C.

### Exemple 23 : 2-Ethoxy-N-(1,2,3,4-tétrahydronaphthalen-1-yl)pyrido[3,2-d]pyrimidin-4-amine (31)

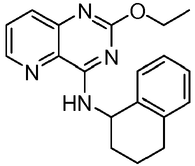

Une suspension du produit **6** (100 mg, 0.32 mmol), de MeB(OH)₂ (29 mg, 0.48 mmol, 1.5 éq.), de Pd(PPh₃)₄ (18 mg, 0.016 mmol, 0.05 éq.) et de Cs₂CO₃ (313 mg, 0.96 mmol, 3 éq.) dans un mélange toluène/éthanol 3 :1 (4 mL) est chauffé sous irradiation micro-ondes à 150°C pendant 1 heure. Après refroidissement, de l'eau (20 mL) est ajoutée au mélange puis le produit est extrait au dichlorométhane (2x20 mL). La phase organique rassemblée est ensuite séchée sur sulfate de magnésium, filtrée et concentrée sous vide. Le brut est ensuite purifié par chromatographie sur gel de silice (gradient EP à EP/AcOEt 85 :15) pour donner le produit **31** (60 mg, 58%) sous forme d'un solide blanc. R_{f} (EP/AcOEt 80:20): 0.37. ¹H RMN (400 MHz, CDCl₃) δ 8.43 (dd, *J* = 4.2, 1.5 Hz, 1H), 7.88 (dd, *J* = 8.5, 1.4 Hz, 1H), 7.52 (dd, *J* = 8.5, 4.2 Hz, 1H), 7.42 (d, *J* = 8.5 Hz, 1H), 7.36-7.30 (m, 1H), 7.22-7.13 (m, 3H), 5.65 (dd, *J* = 14.4, 6.0 Hz, 1H), 4.51 (q, *J* = 7.1 Hz, 2H), 2.86 (dt, *J* = 13.3, 6.4 Hz, 2H), 2.26-2.11 (m, 1H), 2.08-1.83 (m, 3H), 1.47 (t, *J* = 7.1 Hz, 3H). ¹³C RMN (101 MHz, CDCl₃) δ 162.9, 160.9, 146.3, 145.3, 137.8, 136.6, 134.2, 130.3, 129.3, 129.1, 127.8, 127.5, 126.4, 63.3, 48.6, 29.9, 29.4, 20.2, 14.8. IR (ATR diamant, cm⁻¹) v3379, 2939, 1570, 1423, 1333, 1311, 1219, 1059, 825, 807. HRMS (EI-MS) m/z calculée pour C₁₉H₂₀N₄O [M+H]⁺: 321.170988, trouvée 321.171136. T_{f}: 187-189°C.

### Exemple 24 : 4-(1,2,3,4-tétrahydronaphthalen-1-ylamino)pyrido[3,2-d]pyrimidine-2-carbonitrile (32)

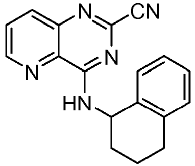

Sous atmosphère d'argon, dans un tube scellé, le produit 6 (100 mg, 0.32 mmol) est dissous dans le DMF sur tamis (1 mL) puis le Zn(CN)₂ (38 mg, 0.32 mmol, 1.0 éq.) et le Pd(PPh₃)₄ (37 mg, 0.032 mmol, 0.1 éq.) sont ajoutés. Le tout est porté à 150°C sous irradiation micro-ondes pendant 5 minutes. Le DMF est évaporé puis le résidu obtenu est repris avec de l'eau (20 mL) et extrait à l'acétate d'éthyle (2x20 mL). Ensuite la phase organique est lavée avec une solution saturée de NaCl (20 mL), séchée sur MgSO₄ puis concentrée sous pression réduite. Le brut est purifié par chromatographie sur gel de silice (gradient EP à EP/AcOEt 85 :15) pour donner le produit attendu **32** (70 mg, 72%) sous forme d'un solide beige. R_{f} (CH₂Cl₂/MeOH 99:01): 0.55. ¹H RMN (400 MHz, CDCl₃) δ 8.74 (dd, *J* = 4.2, 1.4 Hz, 1H), 8.16 (dd, *J* = 8.5, 1.4 Hz, 1H), 7.73 (dd, *J* = 8.5, 4.3 Hz, 1H), 7.64 (d, *J* = 8.2 Hz, 1H), 7.33-7.07 (m, 4H), 5.64 (dd, *J* = 14.3, 5.8 Hz, 1H), 3.04-2.66 (m, 2H), 2.37-2.14 (m, 1H), 2.14-1.84 (m, 3H). ¹³C RMN (101 MHz, CDCl₃) δ 159.3, 150.4, 144.3, 142.3, 137.9, 136.3, 135.7, 131.9, 129.6, 128.9, 128.7, 127.9, 126.6, 116.6, 49.2, 29.6, 29.3, 20.0. IR (ATR diamant, cm⁻¹) v 3280, 2938, 2359, 1399, 1557, 1467, 1440, 1387, 1324, 1291, 1163, 1116, 1072, 1039, 996, 888, 801. HRMS (EI-MS) m/z calculée pour C₁₈H₁₅N₅ [M+H]⁺: 302.140022, trouvée 302.139997. T_{f}: 159-161°C.

### Exemple 25 : N⁴-(1,2,3,4-tétrahydronaphthalen-1-yl)pyrido[3,2-d] pyrimidine-2,4-diamine (33)

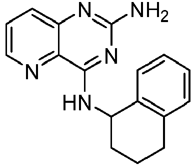

Dans un tube scellé, le produit **6** (100 mg, 0.32 mmol) est dissous dans le dioxane (1 mL) puis l'ammoniaque (2 mL) est ajouté. Le tout est porté à 100°C pendant 3 jours. Après refroidissement, le mélange est concentré sous vide. Le brut est repris à l'eau (20 mL) puis extrait au dichlorométhane (20 mL). Les phases organiques rassemblées sont séchées sur MgSO₄, filtrées puis concentrées sous vide. Le brut est purifié par chromatographie sur gel de silice (gradient CH₂Cl₂ à CH₂Cl₂/MeOH 98:02) pour donner le produit attendu 33 (90 mg, 96%) sous forme d'un solide blanc. R_{f} (CH₂Cl₂/MeOH 97:03): 0.20. ¹H RMN (400 MHz, CDCl₃) δ 8.29 (dd, *J* = 4.2, 1.4 Hz, 1H), 7.68 (dd, *J* = 8.5, 1.4 Hz, 1H), 7.44 (dd, *J* = 8.5, 4.2 Hz, 1H), 7.34 (d, *J=* 7.3 Hz, 1H), 7.30 (d, *J=* 8.6 Hz, 1H), 7.22-7.12 (m, 3H), 5.54 (dd, *J* = 14.3, 6.2 Hz, 1H), 5.05 (s, 2H), 2.97-2.75 (m, 2H), 2.20-2.13 (m, 1H), 2.08-1.83 (m, 3H). ¹³C RMN (101 MHz, CDCl₃) δ 160.7, 159.9, 146.6, 143.7, 137.8, 136.9, 132.6, 129.3 (x2), 129.0, 127.8, 127.5, 126.4, 48.4, 29.9, 29.5, 20.3. IR (ATR diamant, cm⁻¹) v 3311, 3153, 2929, 1600, 1567, 1537, 1447, 1381, 1103, 906, 818. HRMS (EI-MS) m/z calculée pour C₁₇H₁₇N₅ [M+H]⁺: 292.155672, trouvée 292.155978. T_{f}: 152-154°C.

### Exemple 26: 4-(1,2,3,4-tétrahydronaphthalen-1-ylamino)pyrido[3,2-d]pyrimidin-2-ol(34)

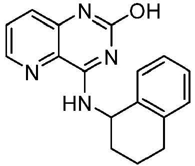

Le produit **6** (100 mg, 0.32 mmol) est dissous dans l'éthanol (2 mL) dans un tube scellé puis de l'ammoniac gazeux est bullé dans cette solution à 0°C pendant 15 minutes. Le mélange est ensuite chauffé à 100°C pendant 4 jours. Après refroidissement, le brut est concentré sous vide puis purifié par chromatographie sur gel de silice (gradient CH₂Cl₂ à CH₂Cl₂/MeOH 96:04) pour donner le produit **34** (62 mg, 66%) sous forme d'un solide blanc. R_{f} (CH₂Cl₂/MeOH 97:03): 0.10. ¹H RMN (400 MHz, CDCl₃) δ 12.80 (s, 1H), 8.32 (dd, *J* = 4.4, 1.4 Hz, 1H), 7.82 (dd, *J* = 8.4, 1.4 Hz, 1H), 7.66 (d, *J* = 8.9 Hz, 1H), 7.50 (dd, *J* = 8.4, 4.4 Hz, 1H), 7.40-7.34 (m, 1H), 7.27-7.14 (m, 3H), 5.80 (dt, *J* = 8.6, 5.9 Hz, 1H), 3.00-2.75 (m, 2H), 2.29-2.22 (m, 1H), 2.09-2.03 (m, 1H), 1.99-1.92 (m, 2H). ¹³C RMN (101 MHz, CDCl₃) δ 160.7, 159.5, 143.8, 138.0, 137.9, 136.1, 129.0, 129.2, 128.4, 127.6, 126.8, 126.5, 124.4, 48.5, 29.8, 29.4, 20.1. IR (ATR diamant, cm⁻¹) v 3354, 2923, 2857, 1654, 1597, 1535, 1466, 1333, 1160, 1089, 1002, 920, 804. HRMS (EI-MS) m/z calculée pour C₁₇H₁₆N₄O [M+H]⁺: 293.139688, trouvée 293.139965. T_{f} > 260°C.

### Exemple 27 : N²-méthyl-N⁴-(1,2,3,4-tétrahydronaphthalen-1-yl)pyrido[3,2-d]pyrimidine-2,4-diamine (35)

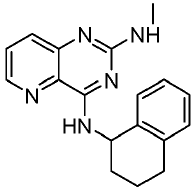

Dans un tube scellé sont introduits le produit **6** (100 mg, 0.32 mmol), le dioxane (1 mL) et la méthylamine (40% dans l'eau, 3 mL). Le tout est chauffé à 100°C pendant 3 jours. Le mélange est ensuite évaporé puis le produit est extrait au dichlorométhane (2 x20 mL), les phases organiques rassemblées séchées sur MgSO₄, filtrées puis concentrées sous vide. Le brut est purifié par chromatographie sur gel de silice (gradient EP à EP/AcOEt 40 :60) pour donner le produit attendu 35 (93 mg, 95%) sous forme d'un solide jaune. R_{f} (EP/AcOEt 40:60): 0.33. ¹H RMN (250 MHz, CDCl₃) δ 8.24 (dd, *J* = 4.2, 1.5 Hz, 1H), 7.73 (d, *J* = 8.5 Hz, 1H), 7.46-7.29 (m, 2H), 7.25-7.10 (m, 4H), 5.54 (dd, *J* = 13.8, 6.1 Hz, 1H), 5.13 (s, 1H), 3.08 (d, *J* = 5.0 Hz, 3H), 2.85 (dd, *J* = 13.5, 6.8 Hz, 2H), 2.22-2.08 (m, 1H), 2.02-1.77 (m, 3H). ¹³C RMN (101 MHz, CDCl₃) δ 160.5, 159.3, 146.8, 142.8, 137.6, 137.1, 132.4, 129.2 (x2), 128.9, 127.5, 127.3, 126.3, 48.3, 29.8, 29.4, 28.6, 20.2. IR (ATR diamant, cm⁻¹) v 3365, 3289, 2919, 2851, 1569, 1476, 1394, 1365, 1320, 1131, 815. HRMS (EI-MS) m/z calculée pour C₁₈H₁₉N₅ [M+H]⁺: 306.171322, trouvée 306.171542. T_{f}: 145-147°C.

### Exemple 28 : 2,7-dichloro-N-(1,2,3,4-tétrahydronaphthalen-1-yl)pyrido [3,2-d]pyrimidin-4-amine (37)

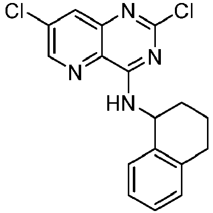

Le produit **36** (250 mg, 1.07 mmol) est dissous dans le THF (10 mL) puis le mélange est placé à 0°C La 1,2,3,4-tétrahydronaphthylamine (0.15 mL, 1.07 mmol, 1 éq.) puis la triéthylamine (0.16 mL, 1.12 mmol, 1.05 éq.) sont successivement additionnées et le mélange est agité à température ambiante pendant une nuit. Le THF est évaporé, le résidu obtenu est repris à l'eau (20 mL) puis extrait au dichlorométhane (2x20 mL). La phase organique rassemblée est ensuite séchée sur sulfate de magnésium, filtrée puis concentrée sous vide. Le brut est chromatographié sur gel de silice (gradient EP à EP/AcOEt 98:02) pour donner le produit attendu **37** (378 mg, quantitatif) sous forme d'un solide blanc. R_{f} (EP/AcOEt 95:05): 0.25. ¹H RMN (400 MHz, CDCl₃) δ 8.49 (d, *J* = 2.2 Hz, 1H), 7.97 (d, *J* = 2.2 Hz, 1H), 7.50 (d, *J* = 8.5 Hz, 1H), 7.29 (d, *J* = 7.6 Hz, 1H), 7.24-7.14 (m, 3H), 5.61 (dt, *J* = 11.7, 5.9 Hz, 1H), 2.95-2.73 (m, 2H), 2.29-2.12 (m, 1H), 2.11-1.82 (m, 3H). ¹³C RMN (101 MHz, CDCl₃) δ 159.8 (x2), 147.6, 146.2, 137.8, 136.2, 135.6, 133.5, 129.5, 128.9, 128.8, 127.9, 126.5, 49.1, 29.6, 29.3, 20.0. IR (ATR diamant, cm⁻¹) v3384, 3062, 2939, 1564, 1438, 1360, 1318, 1280, 1178, 1126, 1086, 959, 906, 791. HRMS (EI-MS) m/z calculée pour C₁₇H₁₄Cl₂N₄ [M+H]⁺: 345.066828, trouvée 345.066837. T_{f}: 185-187°C.

### Exemple 29 : 7-Chloro-N-(1,2,3,4-tétrahydronaphthalen-1-yl)pyrido[3,2-d]pyrimidin-4-amine (38)

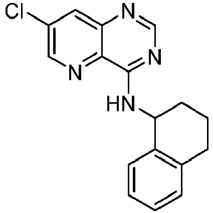

Le produit **38** est obtenu comme décrit pour **7** au départ de **37** (50 mg, 0.14 mmol) pour donner le produit attendu **38** (32 mg, 76%) sous forme d'une huile jaune. R_{f} (CH₂Cl₂/MeOH 95:05): 0.50. ¹H RMN (400 MHz, CDCl₃) δ 8.67 (s, 1H), 8.55 (d, *J* = 1.4 Hz, 1H), 8.11 (s, 1H), 7.40 (d, *J* = 8.1 Hz, 1H), 7.32 (d, *J* = 7.6 Hz, 1H), 7.25 - 7.13 (m, 3H), 5.63 (dd, *J* = 13.7, 6.1 Hz, 1H), 2.98 - 2.77 (m, 2H), 2.26 - 2.16 (m, 1H), 2.13-1.86 (m, 3H). ¹³C RMN (101 MHz, CDCl₃) δ 159.1, 157.6, 147.6, 144.8, 137.9, 136.2, 135.5, 134.2, 130.1, 129.5, 128.9, 127.8, 126.5, 48.9, 29.7, 29.4, 20.1. IR (ATR diamant, cm⁻¹) v 3393, 2949, 2918, 1574, 1533, 1446, 1350, 1301, 1159, 1079, 936, 890. HRMS (EI-MS) m/z calculée pour C₁₇H₁₅ClN₄ [M+H]⁺: 311.105801, trouvée 311.105890. T_{f}: 145147°C.

### Exemple 30 : 7-Cyclopropyl-N-(1,2,3,4-tétrahydronaphthalen-1-yl)pyrido [3,2-d]pyrimidin-4-amine (39)

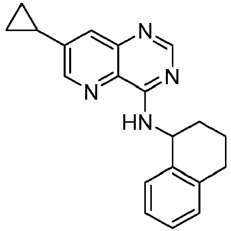

A une solution du produit **38** (50 mg, 0.16 mmol) dans un mélange toluène/eau (10:1, 0.66 mL) sont successivement ajoutés l'acide cyclopropylboronique (18 mg, 0.21 mmol, 1.3 éq.), le phosphate de potassium (119 mg, 0.56 mmol, 3.5 éq.), la tricyclohexylphosphine (7 mg, 0.026 mmol, 0.16 éq.) et l'acétate de palladium (3 mg, 0.013 mmol, 0.08 éq.). Le mélange est porté à 140°C sous irradiation micro-ondes pendant 45 minutes, puis filtré sur célite et rincé à l'acétate d'éthyle (2x20 mL). Le filtrat est lavé à l'eau (20 mL), séché sur sulfate de magnésium, filtré puis concentré sous vide. Le brut est purifié par chromatographie sur gel de silice (gradient EP à EP/AcOEt 60:40) pour donner le produit attendu **39** (30 mg, 59%) sous forme d'un solide blanc. R_{f} (EP/AcOEt 70:30): 0.18. ¹H RMN (400 MHz, CDCl₃) δ 8.59 (s, 1H), 8.43 (d, *J* = 1.4 Hz, 1H), 7.60 (d, *J* = 1.7 Hz, 1H), 7.33 (d, *J* = 6.9 Hz, 2H), 7.23-7.19 (m, 1H), 7.18 - 7.13 (m, 2H), 5.66 (dd, *J* = 13.7, 5.7 Hz, 1H), 3.09 - 2.89 (m, 2H), 2.38 - 2.30 (m, 1H), 2.23 - 2.15 (m, 2H), 2.14 - 2.01 (m, 2H), 1.36 - 1.26 (m, 2H), 1.05 (td, *J* = 6.0, 2.8 Hz, 2H). ¹³C RMN (100 MHz, CDCl₃) δ 158.8, 156.4, 147.9, 144.8, 144.3, 137.6, 136.5, 129.9, 129.8, 129.2, 128.8, 127.4, 126.3, 48.8, 30.1, 29.6, 20.4, 13.9, 10.7 (x2). IR (ATR diamant, cm⁻¹) v 3374, 2952, 2906, 1577, 1463, 1490, 1454, 1441, 1369, 1300, 1215, 1164, 1136, 1020, 968, 910, 874. HRMS (EI-MS) m/z calculée pour C₂₀H₂₀N₄ [M+H]⁺: 317.176073, trouvée 317.176340. T_{f}: 135-137°C.

### Exemple 31: 7-Hexyl-N-(1,2,3,4-tétrahydronaphthalen-1-yl)pyrido[3,2-d]pyrimidin-4-amine (40)

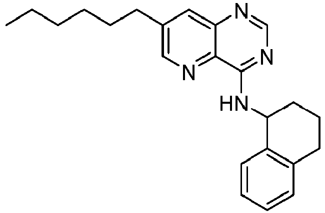

Le produit **40** est obtenu comme décrit pour **39** au départ de **38** (50 mg, 0.16 mmol) et d'acide *n*-hexylboronique (27 mg, 0.21 mmol, 1.3 éq.) pour donner le produit attendu **40** (50 mg, 78%) sous forme d'une huile jaune. R_{f} (EP/AcOEt 70:30): 0.50. ¹H RMN (400 MHz, CDCl₃) δ 8.62 (s, 1H), 8.44 (d, *J* = 1.9 Hz, 1H), 7.85 (d, *J* = 1.9 Hz, 1H), 7.39 (d, *J* = 8.5 Hz, 1H), 7.34 (d, *J* = 6.8 Hz, 1H), 7.24 - 7.18 (m, 1H), 7.18 - 7.11 (m, 2H), 5.67 (dd, *J* = 13.7, 5.9 Hz, 1H), 3.08 - 2.96 (m, 2H), 2.96 - 2.86 (m, 2H), 2.41 - 2.30 (m, 1H), 2.24 - 2.14 (m, 1H), 2.14 - 2.03 (m, 2H), 1.84 (dt, *J* = 14.9, 7.3 Hz, 2H), 1.58 - 1.40 (m, 6H), 1.10 - 0.99 (m, 3H). ¹³C RMN (100 MHz, CDCl₃) δ 158.9, 156.3, 149.4, 144.2, 143.0, 137.6, 136.4, 133.9, 130.0, 129.2, 128.8, 127.4, 126.3, 48.8, 33.5, 31.9, 31.1, 30.0, 29.6, 29.1, 22.9, 20.4, 14.4. IR (ATR diamant, cm⁻¹) v 3391, 2926, 2856, 1577, 1528, 1445, 1366, 1305, 1203, 1159, 1119, 907, 848. HRMS (EI-MS) m/z calculée pour C₂₃H₂₈N₄ [M+H]⁺: 361.238673, trouvée 361.238688.

### Exemple 32 : 7-(hexyloxy)-N-(1,2,3,4-tétrahydronaphthalen-1-yl)pyrido [3,2-d]pyrimidin-4-amine (41)

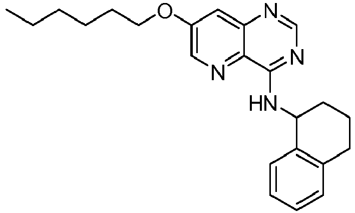

Une solution de Pd₂(dba)₃ (1.4 mg, 16.10⁻⁴ mmol, 0.005 éq.) et de Bippyphos (3.2 mg, 0.0064 mmol, 0.02 éq.) dans le n-hexanol (0.6 mL) est agitée à température ambiante pendant 5 minutes, puis la potasse (27 mg, 0.48 mmol, 1.5 éq.) et le produit **38** (100 mg, 0.32 mmol) sont ajoutés et le mélange est porté à 150°C sous irradiation micro-ondes pendant une heure. De l'eau (20 mL) est ajoutée puis le produit est extrait au dichlorométhane (2 x 20 mL). La phase organique rassemblée est séchée sur MgSO₄, filtrée puis concentrée sous vide. Le brut est purifié par chromatographie sur gel de silice (gradient EP à EP/AcOEt 60:40) pour donner le produit attendu **41** (45 mg, 37%) sous forme d'un solide blanc. R_{f} (EP/AcOEt 70:30): 0.35. ¹H RMN (400 MHz, CDCl₃) δ 8.57 (s, 1H), 8.31 (d, *J* = 2.6 Hz, 1H), 7.35 - 7.32 (m, 2H), 7.23 (dd, *J* = 7.2, 5.0 Hz, 1H), 7.19 (dd, *J* = 6.7, 1.6 Hz, 1H), 7.15 (t, *J* = 6.3 Hz, 2H), 5.69 - 5.61 (m, 1H), 4.17 (t, *J* = 6.4 Hz, 2H), 3.07 - 2.88 (m, 2H), 2.39 - 2.28 (m, 1H), 2.22 - 2.04 (m, 3H), 2.04 - 1.93 (m, 2H), 1.64 (dt, *J* = 14.2, 7.0 Hz, 2H), 1.51 (td, *J* = 7.0, 3.6 Hz, 4H), 1.10 - 1.05 (m, 3H). ¹³C RMN (100 MHz, CDCl₃) δ 158.5, 158.1, 156.8, 145.9, 141.8, 137.6, 136.5, 129.2, 128.8, 127.4, 126.2, 125.5, 113.2, 69.0, 48.7, 31.8, 30.1, 29.6, 29.1, 25.9, 22.9, 20.4, 14.4. IR (ATR diamant, cm⁻¹) v 3419, 3050, 2930, 2855, 1577, 1526, 1441, 1389, 1329, 1281, 1136, 1073, 999, 891. HRMS (EI-MS) m/z calculée pour C₂₃H₂₃N₄O[M+H]⁺: 377.233588, trouvée 377.233647. T_{f}: 92-94°C.

### Exemple 33 : N-(3,4-dichlorobenzyl)pyrido[2,3-d]pyrimidin-4-amine (46)

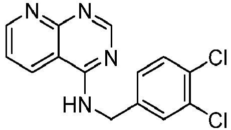

Le produit **45** (200 mg, 1.20 mmol) est dissous dans le 1,4-dioxane (12 mL) puis la 3,4 dichlorobenzylamine (0.32 mL, 2.40 mmol, 2.0 éq.) est additionnée et le mélange est agité à température ambiante pendant une nuit. Le 1,4-dioxane est évaporé, le résidu obtenu est repris à l'eau (20 mL) puis extrait au dichlorométhane (2x20 mL). La phase organique est ensuite séchée sur sulfate de magnésium, filtrée puis concentrée sous vide. Le brut est chromatographié sur gel de silice (gradient CH₂Cl₂ à CH₂Cl₂/MeOH 95:05) pour donner le produit attendu **46** (234 mg, 64%) sous forme d'un solide blanc. R_{f} (CH₂Cl₂/MeOH 97:03): 0.13. ¹H RMN (250 MHz, DMSO-*d*₆) δ 9.14 (t, *J* = 5.5 Hz, 1H), 9.01 (dd, *J* = 4.4, 1.8 Hz, 1H), 8.73 (dd, *J* = 8.3, 1.8 Hz, 1H), 8.60 (s, 1H), 7.64 (d, *J* = 1.8 Hz, 1H), 7.58 (dt, *J* = 8.2, 2.1 Hz, 2H), 7.36 (dd, *J* = 8.3, 2.0 Hz, 1H), 4.78 (d, *J=* 5.8 Hz, 2H). ¹³C RMN (101 MHz, DMSO-*d*₆) δ 160.7, 158.2 (x2), 155.8, 140.3, 132.5, 130.9, 130.5, 129.4, 129.3, 127.6, 121.5, 109.7, 42.9. IR (ATR diamant, cm⁻¹) v 3195, 2972, 2359, 1574, 1472, 1414, 1334, 1276, 1207, 1112, 1032, 977, 897. HRMS (EI-MS) m/z calculée pour C₁₄H₁₀Cl₂N₄ [M+H]⁺: 305.035528, trouvée 305.035763. T_{f} > 260°C.

### Exemple 34 : N-(1,2,3,4-Tétrahydronaphthalen-1-yl)pyrido[2,3-d] pyrimidin-4-amine (47)

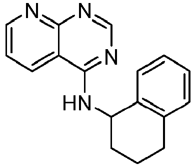

Le produit **47** est synthétisé comme décrit pour **46** au départ de **45** (152 mg, 0.92 mmol) pour donner le produit attendu **47** (120 mg, 43%) sous forme d'un solide blanc. R_{f} (CH₂Cl₂/MeOH 98:02): 0.38. ¹H RMN (400 MHz, DMSO-d₆) δ 8.98 (dd, *J* = 4.3, 1.8 Hz, 1H), 8.85-8.74 (m, 2H), 8.64 (s, 1H), 7.50 (dd, *J* = 8.2, 4.3 Hz, 1H), 7.24-7.06 (m, 4H), 5.79-5.66 (m, 1H), 2.92-2.70 (m, 2H), 2.12-1.73 (m, 4H). ¹³C RMN (101 MHz, DMSO-d₆) δ 160.4, 158.5, 158.4, 155.6, 137.4, 137.0, 132.7, 128.9, 128.0, 126.9, 125.9, 121.2, 109.6, 48.5, 29.3, 28.9, 20.4. IR (ATR diamant, cm⁻¹) v 3248, 2929, 1560, 1529, 1416, 1334, 1279, 1110, 924, 848. HRMS (EI-MS) m/z calculée pour C₁₇H₁₆N₄ [M+H]⁺: 277.144773, trouvée 277.145128. T_{f}: 254-256°C.

### Exemple 35 : (S)-2-Méthyl-N-(1,2,3,4-tétrahydronaphthalen-1-yl)pyrido [3,2-d]pyrimidin-4-amine (48)

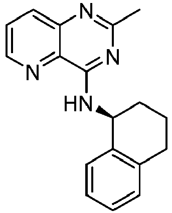

Le produit **48** est obtenu comme décrit pour **30** au départ du produit **19** (100 mg, 0.32 mmol) es pour donner le produit attendu **48** sous forme d'un solide blanc (93 mg, 65%). ¹H RMN (250 MHz, CDCl₃) δ 1.81-1.98 (m, 3H), 2.06-2.25 (m, 1H), 2.64 (s, 3H), 2.71-2.92 (m, 2H), 5.64 (m, 2H), 7.03-7.18 (m, 2H), 7.26-7.39 (m, 3H), 7.53 (dd, *J* = 8.4, 4.2 Hz, 1H), 7.99 (dd, *J* = 8.5, 1.6 Hz, 1H), 8.51 (dd, *J* = 4.3, 1.6 Hz, 1H). ¹³C RMN (101 MHz, CDCl₃) δ 166.0, 158.9, 147.2, 144.7, 137.7, 136.9, 135.0, 130.6, 129.3, 128.9, 127.6, 127.4, 126.3, 48.3, 29.8, 29.4, 27.0, 20.2. IR (ATR diamant, cm⁻¹) v 3385, 2928, 1578, 1530, 1440, 1413, 1366, 1339, 1297, 1162, 1114, 1001, 825. [α]²⁰_{D} = -31.3 (c=0.003 , CH₂Cl₂). T_{f}: 103-105°C.

### Exemple 36 : (S)-2-Fluoro-N-(1,2,3,4-tétrahydronaphthalen-1-yl)pyrido [3,2-d]pyrimidin-4-amine (49)

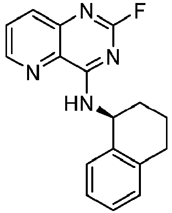

Le produit **19** (200 mg, 0.64 mmol) est dissous dans la NMP (4 mL) sous atmosphère d'argon, puis le fluorure de potassium anhydre (187 mg, 3.21 mmol, 5.0 éq.) et le 18-crown-6 (17 mg, 0.064 mmol, 0.1 éq.) sont ajoutés. Puis le mélange est porté à 230°C sous irradiation micro-ondes pendant 2 heures. Le mélange réactionnel est repris avec de l'eau (20 mL) et extrait à l'acétate d'éthyle (2x20 mL). La phase organique est séchée sur sulfate de magnésium puis concentrée sous vide. Le brut est ensuite purifié par chromatographie sur gel de silice (éluant : gradient EP à EP/AcOEt 80/20) pour donner le produit attendu **49** sous forme d'un solide blanc (95 mg, 50%). ¹H RMN (400 MHz, CDCl₃) δ 8.58 (dd, *J* = 4.3, 1.5 Hz, 1H), 8.01 (dd, *J* = 8.5, 1.5 Hz, 1H), 7.68 (d, *J* = 8.5 Hz, 1H), 7.63 (dd, *J* = 8.5, 4.3 Hz, 1H), 7.36-7.29 (m, 1H), 7.24-7.14 (m, 3H), 5.60 (dt, *J* = 8.7, 5.8 Hz, 1H), 2.97-2.78 (m, 2H), 2.28-2.18 (m, 1H), 2.09-1.89 (m, 3H). ¹³C RMN (101 MHz, CDCl₃) δ 19.9, 29.1, 29.5, 49.0, 126.4, 127.7, 128.3, 128.8, 129.3, 130.5, 130.8, 135.0, 135.0, 135.6, 137.7, 145.9, 146.1, 147.3, 147.3, 159.5, 161.6, 162.2, 162.4. ¹⁹F RMN (400 MHz, CDCl₃) δ -43,33. IR (ATR diamant, cm⁻¹) v3371, 3353, 2929, 1611, 1586, 1568, 1550, 1489, 1438, 1405, 1350, 1328, 1297, 1085, 819, 769, 736. HRMS (EI-MS) m/z calculée pour C₁₇H₁₆FN₄ [M+H]⁺: 295.135207, trouvée 295.135351. [α]²⁰_{D} = -25.6 (c=0.003 , CH₂Cl₂). T_{f}: 154-156 °C.

### Exemple 37 : 2-Chloro-N-(3,4-dihydroquinolin-1(2H)-yl)pyrido[3,2-d] pyrimidin-4-amine (52)

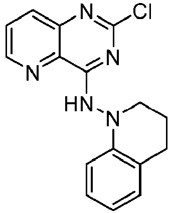

Le produit **52** est synthétisé comme décrit pour **6** au départ de **2** (205 mg, 1.02 mmol) et du composé **50** (159 mg, 1,07 mmol, 1.05 éq.) pour donner le produit attendu **52** sous forme d'un solide jaunâtre (191 mg, 60%). ¹H RMN (400 MHz, DMSO-d₆) δ 10.85 (s, 1H), 8.87 (dd, *J* = 4.2, 1.6 Hz, 1H), 8.13 (dd, *J* = 8.5, 1.6 Hz, 1H), 7.92 (dd, *J* = 8.5, 4.2 Hz, 1H), 7.00 (d, *J* = 7.3 Hz, 1H), 6.94 (t, *J* = 7.7 Hz, 1H), 6.66 (t, *J* = 7.3 Hz, 1H), 6.60 (d, *J* = 8.2 Hz, 1H), 3.54 (s, 2H), 2.78 (t, *J* = 6.3 Hz, 2H), 2.14-2.01 (m, 2H). ¹³C RMN (101 MHz, DMSO-*d*₆) δ 22.2, 27.0, 50.4, 112.5, 118.7, 122.9, 127.1, 129.3, 129.6, 130.2, 135.1, 145.7, 146.2, 149.6, 157.8, 161.3. IR (ATR diamant, cm⁻¹) v 3238, 3063, 2945, 2921, 2888, 2856, 1597, 1577, 1515, 1488, 1473, 1455, 1282, 1233, 1212, 1170, 1091, 803. HRMS (EI-MS) m/z calculée pour C₁₆H₁₅ClN₅ [M+H]⁺: 312,09 , trouvée 312,101050. T_{f}: 85-87°C.

### Exemple 38 : N-(3,4-Dihydroquinolin-1(2H)-yl)pyrido[3,2-d]pyrimidin-4-amine (53)

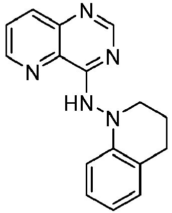

Le produit **53** est obtenu comme décrit pour **7** au départ de **52** (67 mg, 0,21 mmol) pour donner le produit attendu **53** sous forme d'un solide jaune (40 mg, 68%).¹H RMN (400 MHz, CDCl₃) δ 2.20-2.36 (m, 2H), 2.91 (t, *J* = 6.4 Hz, 2H), 3.65 (s, 2H), 6.75-6.90 (m, 2H), 6.99-7.12 (m, 2H), 7.75 (dd, *J* = 8.5, 4.2 Hz, 1H), 8.20 (dd, *J* = 8.5, 1.6 Hz, 1H), 8.64 (s, 1H), 8.76 (s, 1H), 8.78 (dd, *J* = 4.3, 1.6 Hz, 1H). ¹³C RMN (101 MHz, CDCl₃) δ 22.2, 26.9, 51.5, 76.7, 77.0, 77.2, 77.3, 113.1, 119.9, 123.7, 127.1 128.0, 129.3, 131.2, 136.0, 144.5, 145.3, 148.6, 156.6, 159.0. IR (ATR diamant, cm⁻¹) v 3238, 3063, 2945, 2921, 2888, 2856, 1597, 1577, 1515, 1488, 1473, 1455, 1435, 1370, 1282, 1233, 1212, 1170, 1091, 803, 757. HRMS (EI-MS) m/z calculée pour C₁₆H₁₆N₅ [M+H]⁺: 278.13, trouvée 278.140203. T_{f} : 179-180°C.

### Exemple 39: 2-Ghloro-N-{6,7,8,9-tétrahydro-5H-benzo[7]annulen-5-yl} pyrido [3,2-d] pyrimidin-4-amine (55)

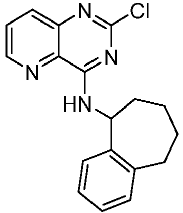

Le produit **55** est synthétisé comme décrit pour **6** au départ de **2** (122 mg, 0.61 mmol) et du composé **54** (119 mg, 0.81 mmol, 1.05 éq.) puis la triéthylamine (0, 94 mL, 0.67 mmol, 1.05 éq.) donner le produit attendu **55** sous forme d'un solide marron (116 mg, 60%). Rf (EP/AcOEt 70:30): 0.28. ¹H RMN (250 MHz, CDCl₃) δ 8.53 (m, 1H), 7.84 (m, 1H), 7.80-7.71 (m, 1H), 7.50 (m, 1H), 7.20-7.12 (m, 1H), 7.03-6.91 (m, 3H), 3.96 (q, *J* = 7.1 Hz, 6H), 3.55 (q, *J* = 7.0 Hz, 1H), 2.83 (t, *J* = 5.6 Hz, 2H). ¹³C RMN (100 MHz, CDCl₃) ppm: 159.7, 158.5, 148.3, 145.6, 141.5, 140.7, 135.0, 130.7, 130.4, 128.3, 127.6, 126.9, 126.4, 55.1, 36.3, 33.3, 27.6, 2159.7, 158.5, 148.3, 145.6, 141.5, 140.7, 135.0, 130.7, 130.4, 128.3, 127.6, 126.9, 126.4, 55.1, 36.3, 33.3, 27.6, 27.5. IR (ATR diamant, cm⁻¹) v 3368, 2919, 2851, 1585, 1544, 1190, 1152, 1129, 976, 871. HRMS (EI-MS) m/z calculée pour C₁₈H₁₇ClN₄ [M+H]⁺ :325.1213, trouvée m/z 325.1214. T_{f}: 122-124°C.

### Exemple 40 : N-{6,7,8,9-tétrahydro-5H-benzo[7]annulen-5-yl}pyrido[3,2-d] pyrimidin-4-amine (56)

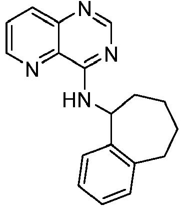

Le produit **56** est synthétisé comme décrit pour **7** au départ de **55** (60 mg, 0.19 mmol) pour donner le produit attendu **56** sous forme d'un solide jaune amorphe (40 mg, 68%). Rf (EP/AcOEt 70:30): 0.3 ¹H RMN (250 MHz, CDCl₃) δ 8.84-8.72 (m, 1H), 8.63 (s, 1H), 8.13 (d, *J* = 8.4 Hz, 1H), 7.70 (dd, *J* = 8.7, 4.4 Hz, 2H), 7.32 (d, *J* = 7.2 Hz, 2H), 7.24-7.08 (m, 3H), 5.65 (t, *J* = 8.8 Hz, 1H), 3.15-2.87 (m, 2H), 2.24-1.94 (m, 4H). ¹³C RMN (100 MHz, CDCl₃) δ 158.5, 156.4, 148.0, 144.1, 141.3, 141.2, 135.7, 131.9, 130.1, 127.5, 127.1, 126.2, 125.6, 54.3, 36.0, 33.9, 28.1, 27.4. IR (ATR diamant, cm⁻¹) v 3403, 2923, 1574, 1534, 1359, 827, 747, 685. HRMS (EI-MS) m/z calculée pour C₁₈H₁₈N₄[M+H]⁺: 291.1605, trouvée m/z 291.1604. T_{f}: 104-106°C

### Exemple 41 : 2-Chloro-N-(7-nitro-1,2,3,4-tétrahydronaphthalen-1-yl)pyrido[3,2-d]pyrimidin-4-amine (58)

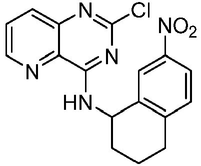

Le produit **58** est synthétisé comme décrit pour **6** au départ de **2** (95 mg, 0.47 mmol) et du composé **57** (96 mg, 0.50 mmol, 1.05 éq.) pour donner le produit attendu 58 sous forme d'un solide jaunâtre (132 mg, 78%). Rf (EP/AcOEt 70:30): 0.6. ¹H RMN (250 MHz, CDCl₃) δ 8.60 (dt, *J* = 4.3, 1.5 Hz, 1H), 8.20 (d, *J* = 2.2 Hz, 1H), 8.05-7.93 (m, 2H), 7.64 (ddd, J = 8.5, 4.3, 0.9 Hz, 1H), 7.56 (d, *J* = 9.1 Hz, 1H), 7.31-7.20 (m, 1H), 5.64 (t, *J* = 8.1 Hz, 1H), 2.90 (dq, *J* = 14.3, 6.7 Hz, 2H), 2.32-2.16 (m, 1H), 1.28-1.13 (m, 2H), 0.91-0.75 (m, 1H). ¹³C RMN (100 MHz, CDCl₃) δ 165.94, 160.1, 158.1, 155.4, 153.1, 148.8, 148.5, 146.4, 145.7, 145.6, 137.5, 136.8, 136.0, 135.0, 130.4, 128.5, 123.9, 122.3, 48.6, 29.5, 29.4, 29.2, 19.6. IR (ATR diamant, cm⁻¹) v 3395, 3059, 3027, 2929, 2907, 2843, 1601, 1556, 1493, 1452, 1376, 1276, 1142, 1128, 1069, 995, 901, 889, 756. T_{f}: 134-136°C.

### Exemple 42 : 2N-(7-nitro-1,2,3,4-tétrahydronaphthalen-1-yl)pyrido[3,2-d]pyrimidin-4-amine (59)

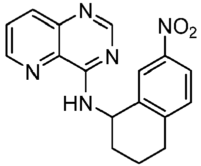

Le produit **59** est synthétisé comme décrit pour **7** au départ de **58** (85 mg, 0,24 mmol) pour donner le produit attendu **59** sous forme d'un solide jaune (40 mg, 68%). Rf (EP/AcOEt 50:50): 0.7. ¹H RMN (250 MHz, CDCl₃) δ 8.79-8.65 (m, 2H), 8.30-8.24 (m, 1H), 8.17 (dd, *J* = 8.5, 1.6 Hz, 1H), 8.06 (dd, *J* = 8.5, 2.5 Hz, 1H), 7.70 (dd, *J* = 8.5, 4.3 Hz, 1H), 7.45 (m, 2H), 5.76 (q, *J* = 7.7, 6.5 Hz, 1H), 3.12-2.88 (m, 2H), 2.29-1.77 (m, 4H).¹³C RMN (100 MHz, CDCl₃) δ 148.3, 138.4, 130.2, 127.9, 123.8, 122.2, 48.2, 29.5, 29.4. IR (ATR diamant, cm⁻¹) v3384, 2951, 2871, 1559, 1537, 1515, 1343, 1311. HRMS (EI-MS) m/z calculée pour C₁₇H₁₅N₅O₂ [M+H]⁺: 322.1299, trouvée m/z 322.1298. T_{f}: 160-162°C.

### Exemple 43: 2-Chloro-N-(3,4-dihydro-2H-1-benzopyran-4-yl)pyrido[3,2-d]pyrimidin-4-amine (61)

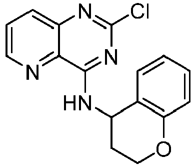

Le produit **61** est synthétisé comme décrit pour **6** au départ de 2 (3.04 mmol, 1.0 éq.) est solubilisé dans 90 mL de THF et du composé **60** (3.38 mmol, 1.0 éq.) pour donner le produit **61** sous forme d'un solide rosé avec un rendement de 62 % (615 mg). Rf (EP/AcOEt 50:50): 0.32. ¹H RMN (250 MHz, CDCl₃) δ 8.35 (tt, *J* = 3.9, 1.7 Hz, 1H, NH), 7.78 (dt, *J* = 8.6, 1.8 Hz, 1H), 7.59-7.35 (m, 2H), 7.12-6.87 (m, 2H), 6.73-6.51 (m, 2H), 5.34 (m, 1H, CH), 2.28-1.95 (m, 2H, CH₂), 1.31 -0.89 (m, 1H, CH). ¹³C RMN (101 MHz, CDCl₃) δ 160.0, 158.4, 155.2, 148.5, 145.8, 135.1, 130.6, 129.7, 129.5, 128.5, 121.2, 121.0, 117.4, 63.2, 45.0, 28.5. IR (ATR diamant, cm⁻¹) v 3389, 1573, 1437, 1390, 1278, 1224, 1126, 936, 873. HRMS (EI-MS) m/z calculée pour C₁₆H₁₃ClN₄O [M+H]⁺: 313.0853, trouvée m/z 313.0851. T_{f}: 152-154°C.

### Exemple 44 : N-(3,4-dihydro-2H-1-benzopyran-4-yl)pyrido[3,2-d] pyrimidin-4-amine (62)

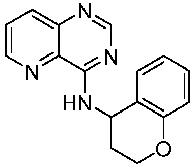

Le produit **62** est synthétisé comme décrit pour **7** au départ de **61** (300 mg, 0.96 mmol) pour donner le produit attendu **62** sous forme d'un solide blanc (25 mg, 10%). Rf (EP/AcOEt 50:50): 0.33. ¹H RMN (250 MHz, CDCl₃) δ 8.72-8.57 (m, 1H), 8.10 (dd, *J* = 8.5, 1.6 Hz, 1H), 7.64 (dd, *J* = 8.5, 4.2 Hz, 1H), 7.42 (d, *J* = 7.8 Hz, 1H), 7.31-7.14 (m, 2H), 6.94-6.81 (m, 2H), 5.61-5.49 (m, 1H), 2.50-2.16 (m, 1H), 1.35-1.11 (m,2H), 0.93-0.76 (m, 2H). ¹³C RMN (101 MHz, CDCl₃) δ 159.0, 156.3, 155.2, 148.3, 144.4, 135.9, 131.9, 129.6, 129.5, 127.8, 121.9, 120.9, 117.3, 77.3, 63.3, 44.7, 29.7, 28.6. IR (ATR diamant, cm⁻¹) v3389, 2925, 1577, 1487, 1529, 1453, 1435, 1361, 1304, 1250, 1224, 1115, 1065, 1021, 828. HRMS (EI-MS) m/z calculée pour C₁₆H₁₄N₄O [M+H]⁺: 279.1240, trouvée m/z 279.1239.T_{f}: 86-88°C.

### Exemple 45 : 2-chloro-N-(4,5,6,7-tétrahydro-1-benzofuran-4-yl)pyrido[3,2-d]pyrimidin-4-amine (64)

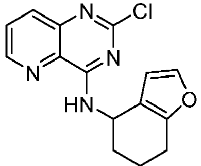

Le produit **64** est synthétisé comme décrit pour **6** au départ de **2** (0.99 mmol, 1.0 éq.) et du composé **63** (1.56 mmol, 1.0 éq.) pour donner le produit **64** sous forme d'un solide beige avec un rendement de 62 % (185 mg). Rf (EP/AcOEt 50:50): 0.71. ¹H RMN (250 MHz, CDCl₃) δ 8.61 (dd, *J* = 4.3, 1.5 Hz, 1H) δ 8.05-7.96 (m, 1H), 7.71-7.55 (m, 1H), 7.30-7.19 (m, 3H), 6.33 (d, *J* = 2.0 Hz, 1H), 2.65 (d, *J* = 5.9 Hz, 2H), 1.94 (m, 4H). ¹³C RMN (101 MHz, CDCl₃) δ 160.1, 158.5, 153.0, 148.2, 145.7, 141.3, 135.0, 130.7, 128.3, 117.2, 109.4, 44.8, 30.9, 29.6, 22.9, 19.9. IR (ATR diamant, cm⁻¹) v3394, 1601, 1576, 1549, 1469, 1398, 1375, 1283, 1137, 1036, 891, 872, 694, 568,510. HRMS (EI-MS) C₁₅H₁₃ClN₄O [M+H] ⁺, calculée m/z 301.0850, trouvée m/z 301.0851. T_{f}: 168-170°C.

### Exemple 46 : N-(4,5,6,7-Tétrahydro-1-benzofuran-4-yl)pyrido[3,2-d] pyrimidin-4-amine (65)

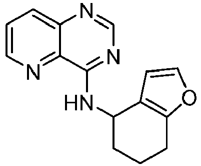

Le produit **65** est synthétisé comme décrit pour **7** au départ de **64** (100 mg, 0.33 mmol) pour donner le produit attendu **65** sous forme d'un solide blanc (83 mg, 95%). Rf (EP/AcOEt 50:50): 0.42. ¹H RMN (250 MHz, CDCl₃) δ 8.80-8.60 (m, 1H), 8.12 (dd, *J* = 8.5, 1.6 Hz, 1H), 7.66 (dd, *J* = 8.5, 4.2 Hz, 1H), 7.40-7.24 (m, 1H), 6.37 (d, *J* = 1.9 Hz, 1H), 5.44 (ddd, *J* = 7.7, 6.0, 4.1 Hz, 1H), 2.81-2.09 (m, 2H), 2.08-1.71 (m, 2H), 1.44-1.13 (m, 2H). ¹³C RMN (100 MHz, CDCl₃) δ 148.1, 141.1, 135.8, 132.0, 127.6, 109.4, 44.4, 29.6, 22.9, 19.9. IR (ATR diamant, cm⁻¹) v392, 2930, 1579, 1537, 1476, 1362, 1294, 1105, 1026, 828, 686.HRMS (EI-MS) C₁₅H₁₅N₄O [M+H]⁺, calculée m/z 267.1240, trouvée m/z 267.1239. T_{f}: 142-144°C.

### Exemple 47 : N-Cyclohexyl-2-(3,5-diméthylpyrazol-1-yl)pyrido[3,2-d]pyrimidin-4-amine (66)

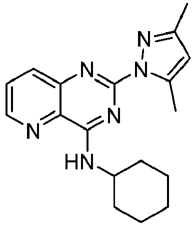

Le produit **22** (151 mg, 0.576 mmol) est dissous dans 10 mL de dioxane suivit de l'ajout d'hydrazine monohydrate (0.976 mL, 20,7 mmol, 36 éq.). Au bout d'une nuit à reflux, le mélange réactionnel est mis à sec sous vide. Le brut est repris dans 10 mL d'acétate d'éthyle. La phase organique est lavé avec de l'eau (3x10mL), séchée avec du MgSO₄, filtrée et évaporée sous vide. Ensuite, le brut est dissous dans 10 mL d'éthanol et la 2,4-pendadione (0.063mL, 0.67 mmol, 1.05 éq.) est ajoutée. Au bout d'une nuit à reflux, le solvant est évaporé sous pression réduite et suivit d'une purification flash sur silice avec comme éluant ether de pétrole/acétate d'éthyle (50/50). Le produit **66** est obtenu avec un rendement de 72% sous forme une huile incolore. Rf (EP/AcOEt 30:70): 0.33. ¹H RMN (400 MHz, CDCl₃) δ 8.57 (m, 1H), 8.21 (m, 1H), 7.63-7.51 (m, 1H), 7.20 (d, *J =* 8.4 Hz, 1H), 6.06-5.98 (m, 1H), 4.12 (sl, 1H), 2.73 (s, 3H), 2.35 (s, 3H), 2.18-2.08 (m, 2H), 1.90-1.78 (m, 2H), 1.73-1.64 (m, 1H), 1.41 (m, 4H), 1.33-1.19 (m, 1H). ¹³C RMN (101 MHz, CDCl₃) δ 159.5, 154.5, 150.8, 146.8, 145.7, 142.6, 135.9, 130.3, 127.8, 110.0, 77.4, 77.3, 77.1, 76.7, 50.1, 32.6, 25.5, 24.9, 15.7, 13.9. IR (ATR diamant, cm⁻¹) v 3380, 2924, 2851, 1604, 1584, 1541, 1441, 1412, 1376, 1123, 1035, 972, 946, 873, 822. HRMS (EI-MS) C₁₈H₂₂N₆ [M+H] ⁺, calculée m/z 345.179815, trouvée m/z 345.179809.

### Exemple 48 : -2-(3,5-Diméthylpyrazol-1-yl)-N-tétralin-1-yl-pyrido[3,2-d] pyrimidin-4-amine (67)

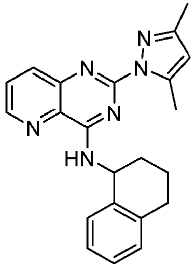

Le produit **67** est obtenu comme décrit pour **66** (76 mg, 0.24 mmol) au départ de 6 pour donner le produit attendu **67** avec un rendement de 85% sous forme une huile incolore. Rf (DCM/MeOH 96:4): 0.5. ¹H RMN (250 MHz, CDCl₃) δ 8.53 (dd, *J* = 4.3, 1.5 Hz, 1H), 8.24 (dd, *J* = 8.5, 1.5 Hz, 1H), 7.65-7.47 (m, 2H), 7.34 (d, *J =* 7.0 Hz, 1H), 7.18 (m, 3H), 5.58 (dt, *J* = 8.5, 5.8 Hz, 1H), 2.93-2.79 (m, 2H), 2.74 (s, 3H), 2.36 (s, 3H), 2.26-1.82 (m, 5H). .¹³C NMR (63 MHz, CDCl₃) δ 159.8, 154.5, 151.0, 147.0, 145.9, 142.7, 137.7, 2 x 136.0, 130.3, 129.3, 128.8, 127.9, 127.6, 126.4, 110.1, 77.5, 77.2, 77.0, 76.5, 49.2, 29.69, 29.2, 20.0, 15.9, 13.9. IR (ATR diamant, cm⁻¹) v 3356, 2922, 1604, 1583, 1558, 1540, 1469, 1440, 1411, 1385, 1357, 1300, 1158, 1123, 1035, 971, 874. HRMS (EI-MS) C₂₂H₂₂N₆ [M+H]⁺, calculée m/z 371.197871 , trouvée m/z 371.197768.

### Exemple 49 : 2-Chloro-N-cyclohexyl-7-méthyl-pyrido[3,2-d]pyrimidin-4-amine (70)

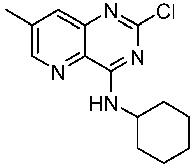

Le produit **69** (80 mg, 0.375 mmol) est dissous dans le THF anhydre (6 mL) puis le mélange est placé à 0°C. La cyclohexanamine (0.046 mL, 0,41 mmol, 1,1 éq.) puis la triéthylamine (0.48 mL, 0.41 mmol, 1.1 éq.) sont successivement additionnées et le mélange est agité à température ambiante pendant une nuit. Le THF est évaporé, le résidu obtenu est repris dans une solution saturée en NaHCO₃ puis extrait au dichlorométhane. La phase organique est ensuite séchée sur sulfate de magnésium, filtrée puis concentrée sous vide. Le brut est chromatographié sur gel de silice (éluant : gradient EP à EP/AcOEt 80:20) pour donner le produit attendu **70** sous forme d'un solide jaunâtre (70 mg, 67%). Rf (EP/AcOEt 30:70): 0.7. ¹H RMN (400 MHz, CDCl₃) δ 8.50 (d, *J=* 2.0 Hz, 1H), 7.77 (dd, *J =* 2.0, 0.9 Hz, 1H), 7.19 (d, *J =* 8.6 Hz, 1H), 4.22 (m, 1H), 2.53 (d, *J* = 0.9 Hz, 3H), 2.17-2.07 (m, 2H), 1.84 (m,1H), 1.76-1.62 (m, 2H), 1.57-1.23 (m, 5H).¹³C RMN (101 MHz, CDCl₃) δ 159.9, 158.6, 149.6, 145.5, 138.9, 133.8, 128.6, 77.3, 77.2, 77.0, 76.7, 49.3, 32.7, 25.5, 24.7, 18.9. IR (ATR diamant, cm⁻¹) v 3381, 2922, 2849, 1578, 1442, 1368, 1329, 1275, 1202, 1156, 1124, 943, 728.HRMS (EI-MS) C₁₄H₁₇ClN₄ [M+H] ⁺, calculée m/z 277.121451, trouvée m/z 277.121264. T_{f}: 144-146°C

### Exemple 50 : N-Cyclohexyl-2-(3,5-diméthylpyrazol-1-yl)-7-méthyl-pyrido[3,2-d]pyrimidin-4-amine (71)

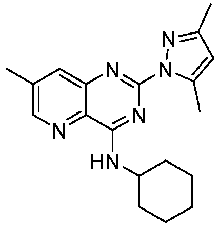

Le produit **70** (60 mg, 0.217 mmol) est dissous dans 10 mL de dioxane suivit de l'ajout d'hydrazine monohydrate (0.367 mL, 7.8 mmol, 36 éq.). Au bout d'une nuit à reflux, le mélange réactionnel est mis à sec sous vide. Le brut est repris dans 10 mL d'acétate d'éthyle. La phase organique est lavée avec de l'eau (3x10 mL), séchée avec du MgSO₄, filtrée et évaporée sous vide. Ensuite, le brut est dissous dans 10 mL d'éthanol et la 2,4-pendadione (0.025mL, 0.29 mmol, 1.05 éq.) est ajoutée. Au bout d'une nuit à reflux, le solvant est évaporé sous pression réduite. La phase organique est lavée avec une solution saline (5x15mL), séchée avec MgSO₄, filtrée et évaporation sous vide. Pour finir, le brut est purifié sur silice avec comme éluant EP/acétate d'éthyle (50/50). Le produit **71** est obtenu avec un rendement de 41% sous forme d'une huile incolore. Rf (EP/AcOEt 30:70): 0.33. ¹H RMN (400 MHz, CDCl₃) δ 8.41 (d, *J* = 2.0 Hz, 1H), 7.97 (dd, *J* = 2.0, 1.1 Hz, 1H), 7.11 (d, *J =* 8.3 Hz, 1H), 6.02 (s, 1H), 4.11 (m, 1H), 2.73 (s, 3H), 2.48 (s, 3H), 2.35 (d, *J =* 1.1 Hz, 3H), 2.13 (m, 2H), 1.83 (m, 2H), 1.69 (m, 1H), 1.54-1.22 (m, 5H). ¹³C RMN (101 MHz, CDCl₃) δ 159.5, 154.6, 150.7, 148.5, 145.6, 142.5, 138.3, 134.9, 128.2, 109.9, 77.4, 77.3, 77.10, 76.8, 50.0, 32.7, 25.6, 24.9, 18.9, 15.6, 13.9. IR (ATR diamant, cm⁻¹) v 3390, 2926, 2852, 1712, 1585, 1538, 1448, 1407, 1376, 1351, 1152, 1104, 972, 940, 888. HRMS (EI-MS) C₁₉H₂₄N₆ [M+H]⁺, calculée m/z 337.213521, trouvée m/z 337.213503.

### Exemple 51 : 2-Chloro-7-méthyl-N-tétralin-1-yl-pyrido[3,2-d]pyrimidin-4-amine (72)

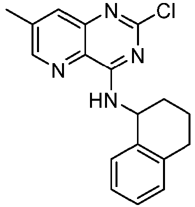

Le produit **69** (80 mg, 0.375mmol) est dissous dans le THF anhydre (6 mL) puis le mélange est placé à 0°C. La 1,2,3,4-tétrahydronaphthylamine (0.06 mL, 0.041 mmol, 1.1 éther de pétrole puis la triéthylamine (0.48 mL, 0.41 mmol, 101 éq.) sont successivement additionnées et le mélange est agité à température ambiante pendant une nuit. Le THF est évaporé, le résidu obtenu est repris dans une solution saturée en NaHCO₃ (20 mL) puis extrait au dichlorométhane (2x20 mL). La phase organique rassemblée est ensuite séchée sur sulfate de magnésium, filtrée puis concentrée sous vide. Le brut est chromatographié sur gel de silice (éluant : gradient EP à EP/AcOEt 80:20) pour donner le produit attendu **72** sous forme d'un solide jaunâtre (70 mg, 67%). Rf (EP/AcOEt 30:70): 0.68. ¹H RMN (400 MHz, CDCl₃) δ 8.49-8.38 (m, 1H), 7.76 (dt, *J =* 2.0, 1.0 Hz, 1H), 7.46 (d, *J* = 9.0 Hz, 1H), 7.35-7.06 (m, 4H), 5.59 (dt, *J* = 9.0, 5.9 Hz, 1H), 2.85 (q, *J =* 5.9 Hz, 2H), 2.48 (s, 3H), 2.29-1.81 (m, 4H). ¹³C RMN (63 MHz, CDCl₃) δ 160.2, 158.7, 150.0, 145.8, 139.2, 137.9, 136.1, 134.0, 129.5, 129.0, 128.7, 127.8, 126.5, 48.9, 29.8, 29.4, 20.1, 19.1. IR (ATR diamant, cm⁻¹) v 3271, 2930, 2861, 1577, 1537, 1466, 1389, 1322, 1271, 1190, 1127, 1035, 956, 873, 822.HRMS (EI-MS) C₁₈H₁₇ClN₄ [M+H] ⁺,calculée m/z 325.121451, trouvée m/z 325.121362. T_{f}: 185-166°C.

### Exemple 52 : 2-(3,5-Diméthylpyrazol-1-yl)-7-méthyl-N-tétralin-1-yl-pyrido[3,2-d]pyrimidin-4-amine (73)

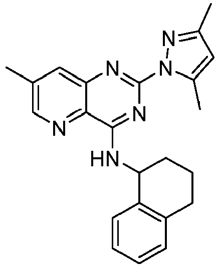

Le produit **72** (67 mg, 0.206 mmol) est dissous dans 10 mL de dioxane suivit de l'ajout d'hydrazine monohydrate (0.357 mL, 7,4 mmol, 36 éq.). Au bout d'une nuit à reflux, le mélange réactionnel est mis à sec sous vide. Le brut est repris dans 10 mL d'acétate d'éthyle. La phase organique est lavée avec de l'eau (3 x 10 mL), séchée avec du MgSO₄, filtrée et évaporée sous vide. Ensuite, le brut est dissous dans 10 mL d'éthanol et la 2,4-pendadione (0.023 mL, 0.23 mmol, 1.05 éq.) est ajoutée. Au bout d'une nuit à reflux, le solvant est évaporé sous pression réduite. La phase organique est lavée avec une solution saline (5x15mL), séchée avec MgSO₄, filtrée et évaporation sous vide. Pour finir, le brut est purifié sur silice avec comme éluant EP/acétate d'éthyle (50/50). Le produit **73** est obtenu avec un rendement de 38% sous forme d'une huile jaunâtre. Rf (EP/AcOEt 30:70): 0.25. ¹H RMN (400 MHz, CDCl₃) δ 8.40 (d, *J =* 2.0 Hz, 1H), 8.03 (dd, *J =* 2.0, 1.1 Hz, 1H), 7.48 (d, *J* = 8.7 Hz, 1H), 7.41-7.33 (m, 1H), 7.30-7.13 (m, 3H), 6.05 (s, 1H), 5.61 (m, 1H), 2.89 (m, 2H), 2.76 (s, 3H), 2.50 (s, 3H), 2.38 (s, 3H), 2.29-2.04 (m, 2H), 2.07-1.89 (m, 2H). ¹³C RMN (101 MHz, CDCl₃) δ 159.7, 154.6, 150.8, 148.8, 145.9, 142.6, 138.5, 137.7, 136.2, 134.9, 129.3, 128.9, 128.9, 128.2, 127.6, 126.4, 110.00, 77.4, 77.3, 77.0, 76.7, 49.1, 29.7, 29.3, 20.0, 19.0, 15.9, 13.9. IR (ATR diamant, cm⁻¹) v 3392, 2930, 2862, 1732, 1595, 1568, 1458, 1419, 1380, 1351, 1152, 1104, 972, 940, 890.HRMS (EI-MS) C₂₃H₂₄N₆ [M+H] ⁺, calculée m/z 385.213521, trouvée m/z 385.213551.

### Exemple 53 : 7-Hexoxy-N-tétralin-1-yl-pyrido[3,2-d]pyrimidin-3-ium-4-amine chloride (74)

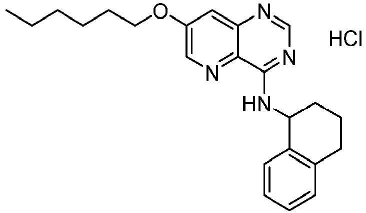

Le composé **41** (26 mg, 0.069 mmol) est solubilisé dans le 5 mL de méthanol. Une solution de HCl dans le méthanol (0.056 mL, 0.069 mmol, 1.0 éq., C = 1.27 M) est ajouté goutte à goutte. Après une nuit d'agitation à température ambiante, le solvant est évaporé sous pression réduite. Le brut est repris dans 5 mL d'éther diéthylique. Après trituration, le solvant est évaporé sous vide. Cette opération est effectuée 3 fois et le produit **74** est obtenu sous forme d'un solide blanc avec rendement quantitatif. Rf (EP/AcOEt 80:20): 0.00. ¹H RMN (400 MHz, DMSO-*d*₆) δ 10.34 (d, *J=* 8.4 Hz, 1H), 8.91 (s, 1H), 8.70 (d, *J =* 2.5 Hz, 1H), 7.67 (d, *J=* 3.6 Hz, 1H), 7.17 (m, 4H), 5.87-5.77 (m, 1H), 4.25 (t, *J =* 6.4 Hz, 2H), 2.91-2.72 (m, 2H), 2.08 (m, 3H), 1.83 (p, *J* = 6.9 Hz, 3H), 1.48 (p, *J* = 6.9 Hz, 2H), 1.34 (m, 4H), 0.94-0.86 (m, 3H). ¹³C RMN (101 MHz, DMSO-*d*₆) δ 159.9, 159.6, 143.5, 138,0 136.0, 129.5, 127.8, 127.6, 126.3, 123.0, 69.8, 50.3, 40.6, 40.4, 40.2, 40.0, 39.8, 39.6, 39.4, 31.4, 29.2, 29.1, 28.5, 25.4, 22.5, 21.2, 14.4. IR (ATR diamant, cm⁻¹) v 3330, 2929, 1626, 1606, 1565, 1435, 1417, 1369, 1348, 1268, 1236, 1041, 883. HRMS (EI-MS) C₂₃H₂₉N₄O [M+H] ⁺, calculée m/z 377,2335, trouvée m/z 377,2334. T_{f}: 121-123°C.

### Exemple 54 : chlorure de N-Tétralin-1-ylpyrido[3,2-d]pyrimidin-3-ium-4-amine (75)

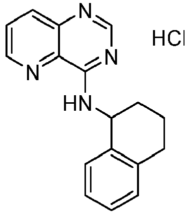

Le composé **20** (42 mg, 0.151 mmol) est solubilisé dans le 3 mL de méthanol. Une solution de HCl dans le méthanol (0.122 mL, 0.151 mmol, 1.0 éq., C = 1.27 M) est ajouté goutte à goutte. Après une nuit d'agitation à température ambiante, le solvant est évaporé sous pression réduite. Le brut est repris dans 5 mL d'éther diéthylique. Après trituration, le solvant est évaporé sous vide. Cette opération est effectuée 3 fois et le produit **75** est obtenu sous forme d'un solide blanc avec rendement quantitatif. Rf (EP/AcOEt 80:20): 0.00. ¹H RMN (400 MHz, DMSO-d₆) δ 10.51 (d, *J* = 9.2 Hz, 1H), 9.02-8.95 (m, 2H), 8.40-8.32 (m, 1H), 8.07 (dd, *J* = 8.6, 4.3 Hz, 1H), 7.26-7.07 (m, 4H), 5.85 (q, *J =* 8.0 Hz, 1H), 2.93-2.73 (m, 2H), 2.22-1.99 (m, 3H), 1.81 (m, 1H). ¹³C RMN (101 MHz, DMSO-*d*₆) δ 160.6, 152.6, 150.9, 138.0, 135.8, 130.9, 129.9, 129.5, 128.9, 127.9, 127.6, 126.3, 50.5, 29.2, 28.9, 21.1. IR (ATR diamant, cm⁻¹) v 3330, 2929, 1626, 1606,1565, 1435, 1417, 1369, 1348, 1268, 1236, 1041, 883. HRMS (EI-MS) C₁₇H₁₇N₄ [M+H] ⁺, calculée m/z 277.1447, trouvée m/z 277.1448. T_{f} : 121-123°C.

### Exemple 55 : N-(1,2,3,4-tétrahydronaphthalen-1-yl)pyrimidin-2-amine (76)

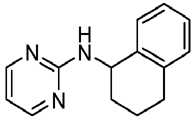

La 2-chloropyrimidine (200 mg, 1.75 mmol) est dissous dans l'éthanol (2,2 mL). La 1,2,3,4-tétrahydronaphthylamine (0.325 mL, 2.27 mmol, 1.05 éq.) puis la triéthylamine (0.35 mL, 2.62 mmol, 1.05 éq.) sont successivement additionnées et le mélange est porté à 120°C sous irradiation micro-ondes pendant 5 minutes. Le milieu est repris dans une solution saturée en NaHCO₃ puis extrait au dichlorométhane. La phase organique est ensuite séchée sur sulfate de magnésium, filtrée puis concentrée sous vide. Le brut est chromatographié sur gel de silice (éluant : EP/AcOEt 80:20) pour donner le produit attendu **76** sous forme d'un solide jaunâtre (244 mg, 62%). ¹H RMN (400 MHz, CDCl₃) δ 8.34 - 8.08 (m, 2H), 7.39 (dd, *J* = 7.3, 1.8 Hz, 1H), 7.25- 7.10 (m, 3H), 6.53 (t, *J* = 4.8 Hz, 1H), 5.76 (s, 1H), 5.35 - 5.21 (m, 1H), 2.94 - 2.73 (m, 2H), 2.18 - 2.08 (m, 1H), 2.01 - 1.79 (m, 3H). ¹³C RMN (101 MHz, CDCl₃) δ 19.9, 29.3, 29.9, 49.0, 76.7, 77.0, 77.2, 77.34, 110.5, 126.1, 127.1, 128.8, 129.0, 137.5, 137.6, 158.1, 161.8. IR (ATR diamant, cm⁻¹) : 3227, 3098, 3008, 2923, 1586, 1523, 1421, 1360, 1316, 1270, 1257, 1232, 1106, 1089, 1035, 994, 951, 925,881. HRMS (EI-MS) m/z calculée pour C₁₄H₁₆N₃ [M+H]⁺: 225.13, trouvée 226.134048. T_{f}:: 94-96°C.

### Exemple 56 : 2,7-dichloropyrido[3,2-d]pyrimidine (78)

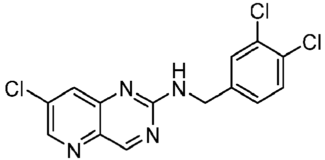

Le produit **77** (90 mg, 0.45 mmol) est dissous dans le 1 ,4-dioxane (4 mL) puis la 3,4 dichlorobenzylamine (0.07 mL, 0.54 mmol, 1,2 éq.) et la triéthylamine (0.10 mL, 0.68 mmol, 1.5 éq.) sont successivement additionnées et le mélange est agité à reflux pendant une nuit. Le 1,4-dioxane est évaporé, le résidu obtenu est repris à l'eau puis extrait au dichlorométhane. La phase organique est ensuite séchée sur sulfate de magnésium, filtrée puis concentrée sous vide. Le brut est chromatographié sur gel de silice (gradient éther de pétrole -> éther de pétrole/AcOEt 70:30) pour donner le produit attendu **78** (138 mg, 90%) sous forme d'un solide blanc. ¹H NMR (250 MHz, DMSO-*d₆*) δ 9.68 (s, 1H), 9.17 (d, *J=* 2.3 Hz, 1H), 8.68 (d, *J =* 2.3 Hz, 1H). ¹³C NMR (62.5 MHz, DMSO-*d*₆) δ 164.9, 157.5, 152.7, 148.0, 137.1, 136.5, 133.7. IR (ATR diamant, cm⁻¹) v 3043, 2167, 1594, 1538, 1433, 1353, 1215, 1117, 1072, 910. HRMS (EI-MS) m/z calculée pour C₇H₃Cl₂N₃ [M+H]⁺: 198.9704, trouvée 198.9715. T_{f}: 178-180°C.

### Exemple 57 : 7-chloro-N-(3,4-dichlorobenzyl)pyrido[3,2-d]pyrimidin-2-amine (79)

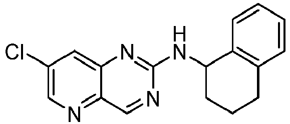

Le produit **77** (70 mg, 0.35 mmol) est dissous dans le 1 ,4-dioxane (3 mL) puis la 1,2,3,4-tétrahydronaphthylamine (0.06 mL, 0.42 mmol, 1,2 éq.) et la triéthylamine (0.07 mL, 0.53 mmol, 1.5 éq.) sont successivement additionnées et le mélange est agité à reflux pendant une nuit. Le 1,4-dioxane est évaporé, le résidu obtenu est repris à l'eau puis extrait au dichlorométhane. La phase organique est ensuite séchée sur sulfate de magnésium, filtrée puis concentrée sous vide. Le brut est chromatographié sur gel de silice (gradient éther de pétrole -> éther de pétrole/AcOEt 96:04) pour donner le produit attendu **79** (90 mg, 83%) sous forme d'un solide jaune. R_{f} (éther de pétrole/AcOEt 80:20): 0.18. ¹H NMR (250 MHz, CDCl₃) δ 9.16 (s, 1H), 8.54 (d, *J* = 2.2 Hz, 1H), 7.88 (s, 1H), 7.48 (d, *J* = 1.8 Hz, 1H), 7.40 (d, *J =* 8.2 Hz, 1H), 7.22 (dd, *J* = 8.2, 1.9 Hz, 1H), 5.95 (s, 1H), 4.71 (d, *J =* 6.2 Hz, 2H). ¹³C NMR (101 MHz, CDCl₃) δ 166.7, 163.4, 148.6, 146.7, 139.1, 136.3, 135.3, 132.8, 132.2, 131.5, 130.7, 129.6, 127.0, 44.8. IR (ATR diamant, cm⁻¹) v 3266, 3094, 1602, 1584, 1538, 1392, 1269, 1130, 1080, 964, 893, 821. HRMS (EI-MS) m/z calculée pour C₁₄H₉Cl₃N₄ [M+H]⁺: 338.996556, trouvée 338.996638. T_{f}: 154-156°C.

### Exemple 58 : 2-Chloro-pyrido[2,3-d]pyrimidine (81)

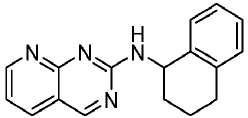

Le produit **80** (100 mg, 0.60 mmol) est dissous dans le 1,4-dioxane (7 mL) puis la 1,2,3,4-tétrahydronaphthylamine (0.10 mL, 0.75 mmol, 1,2 éq.) et la triéthylamine (0.11 mL, 0.79 mmol, 1.5 éq.) sont successivement additionnées et le mélange est agité à reflux pendant une nuit. Le 1,4-dioxane est évaporé, le résidu obtenu est repris à l'eau puis extrait au dichlorométhane. La phase organique est ensuite séchée sur sulfate de magnésium, filtrée puis concentrée sous vide. Le brut est chromatographié sur gel de silice (gradient CH₂Cl₂ -> CH₂Cl₂/MeOH 99.5:0.5) pour donner le produit attendu **81** (116 mg, 70%) sous forme d'un solide jaune. R_{f} (CH₂Cl₂/MeOH 98:02): 0.28. ¹H NMR (250 MHz, CDCl₃) δ 9.32 (s, 1H), 9.24 (dd, *J* = 4.1, 1.9 Hz, 1H), 8.34 (dd, *J* = 7.9, 1.9 Hz, 1H), 7.65 (dd, *J* = 7.9, 4.2 Hz, 1H). ¹³C NMR (101 MHz, CDCl₃) δ 163.0, 161.1, 160.3, 157.5, 138.0, 137.1, 136.7, 129.7, 129.2, 127.3, 126.3, 118.3, 114.2, 49.1, 29.5, 29.3, 19.7. IR (ATR diamant, cm⁻¹) v 3242, 3021, 2919, 1595, 1540, 1402, 1294, 1228, 1094, 932, 883. HRMS (EI-MS) m/z calculée pour C₁₇H₁₆N₄ [M+H]⁺: 277.144773, found 277.145002. T_{f}: 180-182°C

### Exemple 59 : 2-Chloro-N-(4-fluorophenyl)pyrido[3,2-d]pyrimidin-4-amine (82)

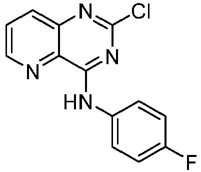

Le produit **2** (200 mg, 1,0 mmol) est dissous dans le THF anhydre (15 mL) puis le mélange est placé à 0°C Le 4-fluoroaniline (122 mg, 1,1 mmol, 1,1 éq) puis la triéthylamine (0,15 mL, 1,1 mmol, 1,1 éq.) sont successivement additionnées et le mélange est agité à température ambiante pendant une nuit. Le THF est évaporé, le résidu obtenu est repris dans une solution saturée en NaHCO₃ puis extrait au dichlorométhane. La phase organique est ensuite séchée sur sulfate de magnésium, filtrée puis concentrée sous vide. Le brut est chromatographié sur gel de silice (éluant : gradient EP -> EP/AcOEt 80:20) pour donner le produit attendu **82** sous forme d'un solide jaunâtre (201 mg, 73%). Rf (éther de pétrole/AcOEt 30:70): 0.34. ¹H RMN (400 MHz, CDCl₃) δ 9.24 (s, 1H), 8.79 (dd, *J* = 4.3, 1.5 Hz, 1H), 8.12 (dd, *J* = 8.5, 1.5 Hz, 1H), 7.94 - 7.87 (m, 2H), 7.75 (dd, *J* = 8.5, 4.2 Hz, 1H), 7.20 - 7.13 (m, 2H). ¹³C RMN (101 MHz, CDCl₃) δ 160.8, 158.4, 157.9, 157.9, 148.7, 145.8, 135.4, 133.4, 133.4, 130.4, 128.6, 122.3, 122.2, 116.0, 115.8, 77.3, 77.2, 77.0, 76.7. ¹⁹F RMN (376 MHz, CDCl₃) δ -117.05. IR (ATR diamant, cm⁻¹) v3380, 1670, 1640, 1552, 1469, 1398, 1375, 1283, 1237, 1136, 971, 877. HRMS (EI-MS) C₁₃H₈ClN₄ [M+H]⁺, calculée m/z 275.0494 trouvée m/z 275.0497. T_{f}: 156-158°C.

### Exemple 60 : 2-(3,5-Diméthylpyrazol-1-yl)-N-(4-fluorophenyl)pyrido[3,2-d] pyrimidin-4-amine (83)

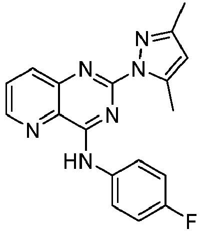

Le produit **82** (85, 0.576 mmol) est dissous dans 10 mL de dioxane suivit de l'ajout d'hydrazine monohydrate (0.976 mL, 20,7 mmol, 36 éq.). Au bout d'une nuit à reflux, le mélange réactionnel est mis à sec sous vide. Le brut est repris dans 10 mL d'acétate d'éthyle. La phase organique est lavé avec de l'eau (3x10mL), séchée avec du MgSO₄, filtrée et évaporée sous vide. Ensuite, le brut est dissous dans 10 mL d'éthanol et la 2,4-pendadione (0.063 mL, 0.67 mmol, 1.05 éq.) est ajoutée. Au bout d'une nuit à reflux, le solvant est évaporé sous pression réduite. La phase organique est lavée avec une solution saline (5x15mL), séchée avec MgSO₄, filtrée et évaporation sous vide. Pour finir, le brut est purifié sur silice avec comme éluant éther de pétrole/acétate d'éthyle (50/50). Le produit **83** est obtenu avec un rendement de 32% sous forme d'un solide jaunâtre. Rf (éther de pétrole/AcOEt 30:70): 0.33. ¹H RMN (400 MHz, CDCl₃) δ 9.17 (s, 1H), 8.71 (dd, *J* = 4.3, 1.5 Hz, 1H), 8.28 (dd, *J* = 8.5, 1.5 Hz, 1H), 7.84 - 7.74 (m, 2H), 7.70 (dd, *J* = 8.5, 4.3 Hz, 1H), 7.19 - 7.06 (m, 2H), 6.04 (s, 1H), 2.59 (s, 3H), 2.37 (s, 3H). ¹³C RMN (101 MHz, CDCl₃) δ 161.0, 158.5, 158.3, 154.0, 151.2, 147.6, 145.9, 142.6, 136.2, 133.5, 133.5, 130.0, 128.3, 123.6, 123.5, 115.8, 115.6, 110.0, 77.3, 77.2, 77.0, 76.7, 29.69, 15.4, 13.9. ¹⁹F RMN (376 MHz, CDCl₃) δ -117.05. IR (ATR diamant, cm⁻¹) v 3356, 2922, 1606, 1587, 1567, 1544, 1505, 1470, 1444, 1420, 1380, 1351, 1311, 1283, 1137, 1036, 891. HRMS (EI-MS) C₁₈H₁₅FN₆ [M+H] ⁺, calculée m/z 335.141499, trouvée m/z 335.141422. T_{f}: 176-178°C.

### Exemple 61 : 2-Chloro-N-(1,2,3,4-tétrahydronaphthalen-1-yl)pyrido[2,3-d] pyrimidin-4-amine (84)

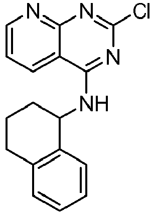

Le produit **43** (280 mg, 1.4 mmol) est dissous dans le THF anhydre (6 mL) puis le mélange est placé à 0°C. La 1,2,3,4-tétrahydronaphthylamine (0.19 mL, 1,4 mmol, 1.0 éq.) puis la triéthylamine (0.14 mL, 1,4 mmol, 1.0 éq.) sont successivement additionnées et le mélange est agité à température ambiante pendant une nuit. Le THF est évaporé, le résidu obtenu est repris dans une solution saturée en NaHCO₃ puis extrait au dichlorométhane. La phase organique est ensuite séchée sur sulfate de magnésium, filtrée puis concentrée sous vide. Le brut est chromatographié sur gel de silice (gradient CH₂Cl₂ à CH₂Cl₂/MeOH 99:1) pour donner le produit attendu **84** sous forme d'un solide jaunâtre (162 mg, 40%). R_{f} (CH₂Cl₂/MeOH 99:1): 0.22. ¹H RMN (400 MHz, DMSO-d6) δ 9.27 (d, *J =* 8.2 Hz, 1H), 8.98 (dd, *J* = 4.4, 1.9 Hz, 1H), 8.84 (dd, *J* = 8.2, 1.9 Hz, 1H), 7.55 (dd, *J* = 8.2, 4.4 Hz, 1H), 7.27 - 7.10 (m, 4H), 5.69 - 5.58 (m, 1H), 2.93 - 2.74 (m, 2H), 2.15 - 1.75 (m, 4H). ¹³C RMN (101 MHz, DMSO-*d₆*) δ 162.0, 160.7, 159.5, 156.7, 137.9, 133.7, 129.4, 128.5, 127.6, 126.5, 122.0, 109.0, 49.5, 29.5, 29.3, 20.7. IR (ATR diamant, cm⁻¹) v 3294, 2942, 2359, 1600, 1559, 1526, 1478, 1452, 1351, 1284, 1240, 1202, 1161, 1047, 955, 942. HRMS (EI-MS) C₁₇H₁₅ClN₄ [M+H]⁺, calculée m/z 311.1058, trouvée m/z 311.1060.

**Procédure générale B** : Le composé **85** est dissous dans 5 mL de THF anhydre pour donner une solution jaune à laquelle l'amine désirée est ajoutée. La solution devient trouble. La triéthylamine est ajoutée et la solution reste à température ambiante pendant 2 heures sous agitation vigoureuse. La disparition du réactif de départ est suivie par CCM puis, en fin de réaction, le THF est évaporé. Le dépôt obtenu est repris dans 30 mL d'eau puis le produit est extrait avec 3 x 20 mL d'acétate d'éthyle. Les phases organiques sont combinées et lavées avec une solution aqueuse de NaCl saturée et enfin séchées avec MgSO₄. Après filtration, le solvant est évaporé et le produit est purifié sur colonne de silice.

### Exemple 62 : N-(1-Naphtyl)pyrido[3,2-d]pyrimidin-4-amine (86)

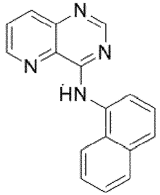

Le composé **86** est obtenu en suivant la procédure B avec les composés **85** (200 mg; 1 mmol), naphthylamine (246 mg; 1.7 mmol) et la triéthylamine (0.28 mL; 2 mmol). Le produit est purifié par colonne de silice avec un système 60 % éther de pétrole et 40 % acétate d'éthyle. Le produit est obtenu sous forme d'un solide jaune (106 mg; 38 %). Rf (EP/AcOEt 50:50): 0.41. ¹H RMN (400 MHz, DMSO-*d₆*) δ 10.48 (1H; br s; NH), 8.99 (1H; m), 8.44 (1H; s), 8.23 (1H; m), 7.95 (4H; m), 7.83 (1H; m), 7.55 (3H; m). ¹³C RMN (100 MHz, DMSO-*d₆*) δ 159.4, 155.7, 149.4, 144.6, 135.8, 134.1, 134.0, 131.5, 129.2, 128.7, 128.3, 126.4, 126.3, 126.2, 125.8, 123.7, 122.9. IR (ATR Diamant, cm⁻¹) v 3347, 3056, 1584, 1538, 1372, 1302, 783, 761, 678, 598. HRMS (EI-MS) : m/z calculée pour C₁₇H₁₃N₄ [M+H]⁺: 273.1134, trouvée: 273.1136. T_{f}: 180 -182°C.

### Exemple 63 : N-(2,3-dihydro-1H-inden-1-yl)pyrido[3,2-d]pyrimidin-4-amine (87)

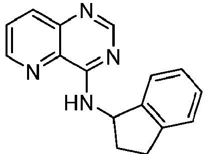

Le composé **87** est obtenu en suivant la procédure B avec les composés 1-Indanamine **96** (60 mg ; 0.45 mmol), **85** (53 mg ; 0.27 mmol) et la triéthylamine (0.08 mL ; 0.53 mmol). Le produit est purifié par colonne de silice avec un système 99 % CH₂Cl₂ et 1 % MeOH. Le produit est obtenu sous forme d'un solide jaune (57 mg; 81 %). Rf (EP/AE 50:50): 0.52. ¹H RMN (400 MHz, CDCl₃) δ 8.69 (s, 1H), 8.65 (m, 1H), 8.10 (m, 1H), 7.64 (m, 1H), 7.30 (m, 5H), 5.93 (m, 1H), 3.09 (m, 1H), 2.99 (m, 1H), 2.77 (m, 1H), 2.05 (m, 1H). ¹³C RMN (100 MHz, CDCl₃) δ 159.7, 156.6, 148.3, 144.5, 143.7, 143.1, 135.9, 132.1, 128.3, 127.8, 126.9, 125.0, 124.4, 55.9, 34.1, 30.5. IR (ATR Diamant, cm⁻¹) v 3367, 2950, 1581, 1481, 1446, 1379, 1362, 1313, 763, 741, 594. HRMS (EI-MS) : m/z calculée pour C₁₆H₁₅N₄ [M+H]⁺: 263.1291, trouvée: 263.1292. T_{f} : 135-137 °C.

### Exemple 64 : N-(5-méthoxy-1,2,3,4-tétrahydronaphthalen-1-yl)pyrido[3,2-d]pyrimidin-4-amine (88)

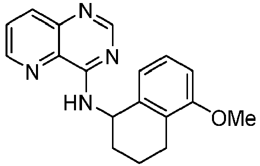

Le composé **88** est obtenu en suivant la procédure B avec les composés 5-méthoxy-1,2,3,4-tétrahydro-1-Naphthalenamine **97** (50 mg ; 0.28 mmol), **85** (33 mg ; 0.17 mmol) et la triéthylamine (0.05 mL ; 0.33 mmol). Le produit est purifié par colonne de silice avec un système 99% CH₂Cl₂ et 1 % MeOH. Le produit est obtenu sous forme d'un solide jaune (41 mg; 80 %). Rf (CH₂Cl₂/MeOH 98:2): 0.35. ¹H RMN (400 MHz, CDCl₃) δ 8.68 (s, 1H), 8.63 (m, 1H), 8.09 (m, 1H), 7.63 (m, 1H), 7.45 (m, 1H), 7.14 (m, 1H), 6.97 (m, 1H), 6.77 (m, 1H), 5.61 (m, 1H), 3.85 (s, 3H), 2.75 (m, 2H), 2.15 (m, 1H), 2.05 (m, 1H), 1.94 (m, 2H). ¹³C RMN (100 MHz, CDCl₃) δ 159.2, 157.3, 156.7, 148.2, 144.5, 137.7, 135.9, 132.2, 127.7, 127.0, 126.7, 120.9, 108.7, 55.5, 48.8, 29.2, 23.1, 19.5. IR (ATR Diamant, cm⁻¹) v 3352, 2943, 1578, 1461, 1380, 1309, 1249, 1094, 1014, 764, 656. HRMS (EI-MS) : m/z calculée pour C₁₈H₁₉N₄O [M+H]⁺: 307.1553, trouvée: 307.1552. T_{f}: 135-137 °C.

### Exemple 65 : N-(7-méthoxy-1,2,3,4-tétrahydronaphthalen-1-yl)pyrido[3,2-d]pyrimidin-4-amine (89)

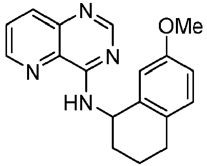

Le composé **89** est obtenu en suivant la procédure B avec les composés 7-méthoxy-1,2,3,4-tétrahydronaphthalen-1-amine **98** (70 mg; 0.4 mmol), **85** (47 mg; 0.24 mmol) et la triéthylamine (0.07 mL; 0.47 mmol). Le produit est purifié par colonne de silice avec un système 99 % CH₂Cl₂ et 1 % MeOH. Le produit est obtenu sous forme d'un solide blanc (65 mg; 92 %). Rf (CH₂Cl₂/MeOH 98:2): 0.32. ¹H RMN (400 MHz, CDCl₃) δ 8.66 (1H; s), 8.57 (1H; m), 8.06 (1H; m), 7.58 (1H; m), 7.49 (1H; m), 7.11 (1H; m), 6.95 (1H; m), 6.73 (1H; m), 5.59 (1H; m), 3.82 (3H; s), 2.69 (2H; m), 2.13 (1H; m), 2.00 (1H; m), 1.89 (2H; m). ¹³C RMN (100 MHz, CDCl₃) δ 159.1, 157.2, 156.6, 148.1, 144.4, 137.6, 135.8, 132.1, 127.6, 126.8, 126.6, 120.8, 108.6, 55.4, 48.7, 29.1, 23.0, 19.4. IR (ATR Diamant, cm⁻¹) v 3352, 2942, 1577, 1541, 1309, 1094, 797, 720, 527. HRMS (EI-MS) : m/z calculée pour C₁₈H₁₉N₄O [M+H]⁺: 307.1553, trouvée: 307.1554. T_{f}: 143-145 °C.

### Exemple 66 : N-(7-fluoro-1,2,3,4-tétrahydronaphthalen-1-yl)pyrido[3,2-d]pyrimidin-4-amine (90)

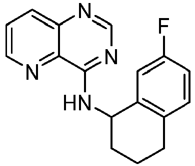

Le composé **90** est obtenu en suivant la procédure B avec les composés 7-fluoro-1,2,3,4-tétrahydro-1-Naphthalen-1-amine **99** (200 mg ; 1.2 mmol), **85** (142 mg ; 0,72 mmol) et la triéthylamine (0.2 mL ; 1,44 mmol). Le produit est purifié par colonne de silice avec un système 99 % CH₂Cl₂ et 1 % MeOH. Le produit est obtenu sous forme d'une huile incolore (196 mg; 93 %). Rf (CH₂Cl₂/MeOH 98:2) : 0.30. ¹H RMN (250 MHz, CDCl₃) δ 8.66 (s, 1H), 8.63 (m, 1H), 8.09 (m, 1H), 7.62 (m, 1H), 7.43 (m, 1H), 7.05 (m, 2H), 6.87 (m, 1H), 5.61 (m, 1H), 2.78 (m, 2H), 2.19 (m, 1H), 1.94 (m, 3H). ¹³C RMN (62.5 MHz, CDCl₃) δ 161.3 (*J* = 244.0 Hz), 159.3, 156.5, 148.3, 144.5, 138.5 (*J* = 6.0 Hz), 135.9, 133.1 (*J* = 3.0 Hz), 132.0, 130.6 (*J* = 8.0 Hz), 127.8, 114.9 (*J* = 20.0 Hz), 114.6 (*J* = 20.0 Hz), 48.6, 29.6, 28.7, 20.4. IR (ATR Diamant , cm⁻¹) v 3249, 2929, 1577, 1531, 1361, 1105, 919, 827, 685. HRMS (EI-MS) : m/z calculée pour C₁₇H₁₆N₄F [M+H]⁺: 295.1354, trouvée : 295.1353. T_{f}: 79-81 °C.

### Exemple 67 : N-(4,5,6,7-tétrahydrobenzo[b]thiophen-4-yl)pyrido[3,2-d]pyrimidin-4-amine (91)

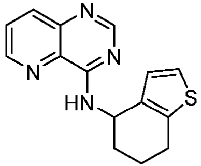

Le composé **91** est obtenu en suivant la procédure B avec les composés 4,5,6,7-tétrahydrobenzo[b]thiophen-4-amine **100** (130 mg ; 0.85 mmol), **85** (99 mg ; 0.5 mmol) et la triéthylamine (0.14 mL ; 1 mmol). Le produit est purifié par colonne de silice avec un système 96% CH₂Cl₂ et 4% MeOH. Le produit est obtenu sous forme d'un solide blanc (98 mg ; 70%). Rf (CH₂Cl₂/MeOH 95:5): 0.77. ¹H RMN (400 MHz, CDCl₃) δ 8.65 (s, 1H), 8.60 (m, 1H), 8.07 (m, 1H), 7.61 (m, 1H), 7.40 (m, 1H), 7.06 (m, 1H), 6.91 (m, 1H), 5.52 (m, 1H), 2.84 (m, 2H), 2.18 (m, 1H), 1.98 (m, 3H). ¹³C RMN (100 MHz, CDCl₃) δ 159.1, 156.5, 148.2, 144.4, 139.1, 135.9, 135.2, 132.1, 127.7, 126.8, 122.9, 46.3, 29.5, 25.0, 21.00. IR (ATR Diamant, cm⁻¹) v 3379, 2924, 1600, 1583, 1364, 1150, 1111, 829, 686, 582. HRMS (EI-MS) : m/z calculée pour C₁₅H₁₅N₄S [M+H]⁺: 283.1012, trouvée: 283.1012. T_{f}: 149-151 °C.

### Exemple 68 : N-(6-méthoxy-1,2,3,4-tétrahydronaphthalen-1-yl)pyrido[3,2-d]pyrimidin-4-amine (92)

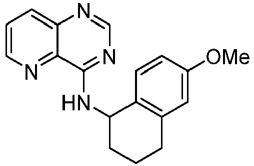

Le composé **92** est obtenu en suivant la procédure B avec les composés 6-méthoxy-1,2,3,4-tétrahydronaphthalen-1-amine (100 mg; 0.565 mmol), **85** (66 mg ; 0.33 mmol) et la triéthylamine (0.1 mL ; 0.66 mmol). Le produit est purifié par colonne de silice avec un système 96 % CH₂Cl₂ et 4 % MeOH. Le produit est obtenu sous forme d'un solide blanc (91 mg ; 89 %). Rf (CH₂Cl₂/MeOH 95:5): 0.62. ¹H RMN (250 MHz, CDCl₃) δ 8.64 (m, 1H), 8.57 (m, 1H), 8.05 (m, 1H), 7.57 (m, 1H), 7.42 (m, 1H), 7.22 (m, 1H), 6.68 (m, 1H), 6.64 (m, 1H), 5.53 (m, 1H), 3.75 (m, 3H), 2.78 (m, 2H), 2.16 (m, 1H), 2.09 (m, 1H), 1.95 (m, 2H). ¹³C RMN (62.5 MHz, CDCl₃) δ 159.0, 158.8, 156.5, 148.1, 144.4, 139.1, 135.8, 132.1, 130.2, 128.6, 127.6, 113.6, 112.7, 55.3, 48.2, 29.7, 29.6, 19.9. IR (ATR Diamant, cm⁻¹) v 3414, 2956, 2830, 1609, 1575, 1294, 1234, 1111, 920, 838, 685, 632. HRMS (EI-MS) : m/z calculée pour C₁₈H₁₉N₄O [M+H]⁺: 307.1553, trouvée: 307.1554. T_{f}: 104-106 °C.

### Exemple 69 : N-(4,4-diméthyl-1,2,3,4-tétrahydronaphthalen-1-yl)pyrido[3,2-d]pyrimidin-4-amine 90 (93)

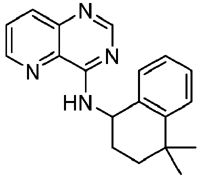

Le composé **93** est obtenu en suivant la procédure B avec les composés 4,4-diméthyl-1,2,3,4-tétrahydronaphthalen-1-amine **101** (111 mg; 0.64 mmol), **85** (74 mg; 0.37 mmol) et la triéthylamine (0.11 mL; 0.74 mmol). Le produit est purifié par colonne de silice avec un système isocratique 100 % CH₂Cl₂. Le produit est obtenu sous forme d'un solide jaune (10 mg; 9 %). Rf (CH₂Cl₂/MeOH 95:5): 0.42. ¹H RMN (250 MHz, CDCl₃) δ 8.65 (s, 1H), 8.69 (m, 1H), 8.12 (m, 1H), 7.65 (m, 1H), 7.43 (m, 2H), 7.29 (m, 2H), 7.15 (m, 1H), 5.60 (m, 1H), 2.26 (m, 1H), 2.07 (m, 1H), 1.84 (m, 2H), 1.40 (s, 3H), 1.34 (s, 3H). ¹³C RMN (62.5 MHz, CDCl₃) δ 159.4, 156.7, 148.3, 146.5, 144.5, 136.0, 135.5, 132.2, 128.6, 128.0, 127.8, 127.1, 126.3, 49.6, 35.8, 34.1, 31.8, 31.7, 26.2. IR (ATR Diamant, cm⁻¹) v 3227, 2960, 1578, 1479, 1270, 1148, 807, 686, 612. HRMS (EI-MS) : m/z calculée pour C₁₉H₂₁N₄ [M+H]⁺: 305.1760, trouvée: 305.1757. T_{f}: 200-202 °C.

**Procédure générale C** : Le composé **19** est dissous dans 5 mL de dioxane pour donner une solution jaune à laquelle l'amine désirée et la triéthylamine sont ajoutées. La solution devient trouble et est chauffée à 90 °C pendant 2 jours. La disparition du réactif de départ est suivie par CCM puis, en fin de réaction, la solution est diluée avec 30 mL d'acétate d'éthyle. Après deux lavages à l'eau, les phases organiques sont combinées et lavées avec une solution aqueuse de NaCl saturée et enfin séchées avec MgSO₄. Après filtration, le solvant est évaporé et le produit est purifié sur colonne de silice.

### Exemple 70 : ((S)-N-(1,2,3,4-tétrahydronaphthalen-1-yl)-2-(1H-1,2,4-triazol-1-yl)pyrido[3,2-d]pyrimidin-4-amine (94)

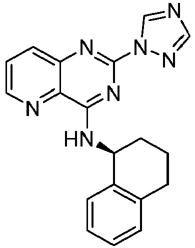

Le composé **94** est obtenu en suivant la procédure C avec les composés **19** (100 mg ; 0.32 mmol), 1,2,4-triazole (30 mg ; 0.4 mmol) et la triéthylamine (0.05 mL ; 0.4 mmol). Le produit est purifié par colonne de silice avec un système 97% CH₂Cl₂ et 3% MeOH. Le produit est obtenu sous forme d'un solide blanc (62 mg; 56 %). Rf (CH₂Cl₂/MeOH 94:6): 0.38. ¹H RMN (400 MHz, CDCl₃) δ 9.30 (s, 1H), 8.64 (m, 1H), 8.26 (m, 1H), 8.18 (s, 1H), 7.72 (m, 1H), 7.68 (m, 1H), 7.36 (m, 1H), 7.22 (m, 3H), 5.69 (m, 1H), 2.92 (m, 2H), 2.27 (m, 1H), 2.15 (m, 1H), 1.99 (m, 2H). ¹³C RMN (100 MHz, CDCl₃) δ 160.3, 153.5, 151.4, 148.1, 145.3, 144.2, 137.8, 136.1, 135.7, 131.4, 129.5, 128.8, 128.6, 127.9, 126.6, 49.4, 29.5, 29.3, 20.1. IR (ATR Diamand, cm⁻¹) v 3500, 2929, 1585, 1503, 1423, 1386, 1000, 940, 870, 734, 670. HRMS (EI-MS) : m/z calculée pour C₁₉H₁₈N₇ [M+H]⁺: 344.1618, trouvée : 344.1617. T_{f}: 171-173 °C.

### Exemple 71 : (S)-2-(1H-imidazol-1-yl)-N-(1,2,3,4-tétrahydronaphthalen-1-yl)pyrido[3,2-d]pyrimidin-4-amine (95)

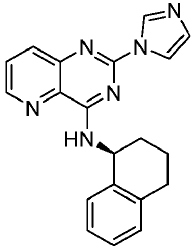

Le composé **95** est obtenu en suivant la procédure C avec les composés **19** (100 mg ; 0.32 mmol), l'imidazole (30 mg ; 0.4 mmol) et la triéthylamine (0.05 mL ; 0.4 mmol). Le produit est purifié par colonne de silice avec un système 94 % CH₂Cl₂ et 6 % MeOH. Le produit est obtenu sous forme d'un solide blanc (71 mg; 65 %). Rf (CH₂Cl₂/MeOH 90:10): 0.75. ¹H RMN (250 MHz, CDCl₃) δ 8.70 (s, 1H), 8.56 (m, 1H), 8.03 (m, 1H), 7.99 (s, 1H), 7.63 (m, 2H), 7.36 (m, 1H), 7.19 (m, 4H), 5.65 (m, 1H), 2.90 (m, 2H), 2.26 (m, 1H), 2.15 (m, 1H), 1.97 (m, 2H). ¹³C RMN (62.5 MHz, CDCl₃) δ 160.2, 151.8, 147.4, 145.5, 137.8, 136.7, 136.1, 135.2, 131.0, 130.2, 129.5, 128.9, 128.3, 127.8, 126.5, 117.0, 49.2, 29.6, 29.4, 20.1. IR (ATR Diamant, cm⁻¹) v 3340, 2934, 1549, 1426, 1315, 1036, 1014, 819, 737, 649. HRMS (EI-MS) : m/z calculée pour C₂₀H₁₉N₆ [M+H]⁺: 343.1665, trouvée: 343.1666. T_{f}: 187-189 °C.

### Exemple 72 : N-(6-(Méthoxyméthoxy)-1,2,3,4-tétrahydronaphthalen-1-yl)pyrido[3,2-d]pyrimidin-4-amine (104)

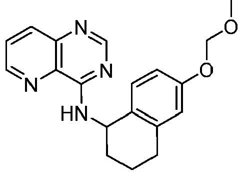

Le composé **104** est obtenu en suivant la procédure B avec le composé **103** (70 mg, 0.34 mmol, 1.7 eq), **85** (40 mg, 0. 20 mmol, 1.0 eq) et la triéthylamine (0,06 mL, 0.40 mmol, 2.0 eq). Le résidu est purifié par chromatographie sur gel de silice (système gradient 0 à 90 % AE/100 à 10 % PE). Le produit est obtenu sous forme d'un solide blanc (55 mg, 81 %). R_{f} (PE/AE 10:90): 0.42. ¹H RMN (400 MHz, CDCl₃) δ 8.67 (s, 1H), 8.65 (dd, *J =* 4.0, 2.0 Hz, 1H), 8.10 (dd, *J* = 8.0, 2.0 Hz, 1H), 7.64 (dd, *J* = 8.0, 4.0 Hz, 1H), 7.41 (dd, *J* = 8.0 Hz, 1H, NH), 7.27 (d, *J=* 3.0 Hz, 1H), 6.85 (d, *J =* 3.0 Hz, 1H), 6.83 (s, 1H), 5.57 (m, 1H), 5.16 (s, 2H), 3.48 (s, 3H), 2.83 (m, 2H), 2.17 (m, 1H), 2.07 (m, 1H), 1.93 (m, 2H). ¹³C RMN (101 MHz, CDCl₃) δ 159.1, 156.7, 156.5, 148.2, 144.5, 139.4, 135.9, 132.2, 130.4, 130.0, 127.7, 116.2, 115.0, 94.5, 56.1, 48.3, 29.8, 29.7, 19.9. IR (ATR Diamant, cm⁻¹) v 3405, 2926, 2903, 2869, 1575, 1436, 1317, 1043, 924, 863, 643. HRMS (EI-MS) : m/z calculée pour C₁₉H₂₁N₄O₂ [M+H]⁺: 337.1659, trouvée: 337.1660. **T_{f}** : 109-111 °C.

### Exemple 73: 5-(pyrido[3,2-d]pyrimidin-4-ylamino)-5,6,7,8-tétrahydro-naphthalen-2-ol (104')

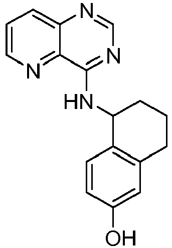

Le composé **104** est dissous dans 5 mL de MeOH et quelques gouttes d'une solution de HCl concentrée (37%) sont ajoutées à température ambiante. Le mélange est agité pendant une nuit. Le mélange est dilué avec 20 mL d'AcOEt puis lavé avec une solution saturée aqueuse de Na₂CO₃ (20 mL). La phase organique est lavée avec une solution saturée aqueuse de NaCl puis séchée avec MgSO₄. Après filtration, le solvant est évaporé et le produit **104'** est obtenu par précipitation dans le pentane sous forme de solide blanc (91 mg, 62 %). R_{f} (CH₂Cl₂/MeOH 90:10): 0.51. ¹H RMN (400 MHz, DMSO-*d₆*) δ 9.22 (s, 1H, OH), 8.76 (dd, *J* = 4.0, 2.0 Hz, 1H), 8.54 (s, 1H), 8.12 (m, 2H), 7.83 (dd, *J =* 8.0, 4.0 Hz, 1H), 6.99 (d, *J =* 9.0 Hz, 1H, NH), 6.52 (m, 2H), 5.50 (m, 1H), 2.71 (m, 2H), 1.99 (m, 2H), 1.91 (m, 1H), 1.73 (m, 1H). ¹³C RMN (101 MHz, DMSO-d₆) δ 158.8, 156.1, 156.0, 148.5, 144.1, 138.5, 135.4, 131.3, 129.0, 128.2, 127.3, 114.8, 113.5, 47.7, 29.2, 29.0, 20.2. IR (ATR Diamant, cm⁻¹) v 3343, 2929, 1584, 1545, 1439, 1237, 742. HRMS (EI-MS) : m/z calculée pour C₁₇H₁₇N₄O [M+H]⁺: 293.1396, trouvée: 293.1397. T_{f}: 233-235 °C.

### Exemple 74 : 1-(pyrido[3,2-d]pyrimidin-4-ylamino)-1,2,3,4-tétrahydro-naphthalen-2-ol (107)

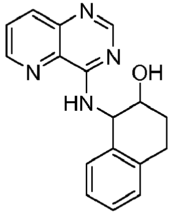

Le composé **107** est obtenu en suivant la procédure B avec le composé **106** (135 mg, 0.83 mmol, 1.7 eq), **85** (100 mg, 0.50 mmol, 1.0 eq) et la triéthylamine (0.14 mL, 1.0 mmol, 2.0 eq). Le résidu est purifié par chromatographie sur gel de silice (système gradient 0 à 2 % MeOH/100 à 98 % CH₂Cl₂). Le produit est obtenu sous forme d'un solide blanc (24 mg, 16 %). R_{f} (CH₂Cl₂/MeOH 95:5): 0.40. ¹H RMN (400 MHz, CDCl₃) δ 8.64 (dd, *J =* 4.0, 2.0 Hz, 1H), 8.62 (s, 1H), 8.10 (dd, *J* = 8.0, 2.0 Hz, 1H), 7.65 (dd, *J* = 8.0, 4.0 Hz, 1H), 7.53 (d, *J =* 7.5 Hz, 1H, NH), 7.35 (m, 1H), 7.22 (m, 3H), 5.44 (t, *J =* 7.5 Hz, 1H), 5.15 (br s, 1H, OH), 4.,12 (m, 1H), 2.94 (m, 2H), 2.25 (m, 1H), 1.99 (m, 1H). ¹³C RMN (101 MHz, CDCl₃) δ 160.6, 155.8, 148.7, 144.5, 137.5, 136.0, 134.9, 131.9, 129.0, 128.4, 128.1, 127.9, 127.0, 73.9, 58.0, 30.2, 27.7. IR (Diamant ATR, cm⁻¹) v 3264, 2928, 1583, 1438, 1304, 1111, 827, 684. HRMS (EI-MS) : m/z calculée pour C₁₇H₁₇N₄O [M+H]⁺: 293.1396, trouvée: 293.1398. T_{f}: 90-92 °C.

### Exemple 75 : N-(7-Fluoro-1,2,3,4-tetrahydronaphthalen-1-yl)pyrido[3,2-d]pyrimidin-4-amine (110)

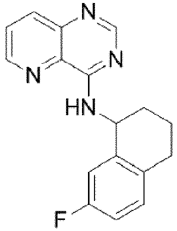

Le composé **110** est obtenu en suivant la procédure B avec les composés **109** (200 mg, 1.2 mmol, 1.7 eq.), **85** (142 mg, 0.72 mmol, 1.0 eq.) et la triéthylamine (0.2 mL, 1.44 mmol, 2.0 eq.). Le résidu est purifié par chromatographie sur gel de silice (éluant CH₂Cl₂/MeOH 99:1). Le produit est obtenu sous forme d'une huile incolore (196 mg, 93 %). **R_{f}** (CH₂Cl₂/MeOH 98:2): 0.30. ¹H RMN (400 MHz, CDCl₃) δ 8.66 (s, 1H), 8.63 (m, 1H), 8.09 (dd, *J* = 8.0, 1.7 Hz, 1H), 7.62 (dd, *J* = 8.0, 4.0 Hz, 1H), 7.43 (br d, *J* = 5.0 Hz, 1H, NH), 7.05 (m, 2H), 6.87 (td, *J* = 8.0, 3.0 Hz, 1H), 5.61 (q, *J =* 8.0 Hz, 1H), 2.78 (m, 2H), 2.19 (m, 1H), 1.94 (m, 3H). ¹³C RMN (101 MHz, CDCl₃) δ 161.3 (*J* = 244.0 Hz), 159.3, 156.5, 148.3, 144.5, 138.5 (*J* = 6.0 Hz), 135.9, 133.1 (J = 3.0 Hz), 132.0, 130.6 (*J* = 8.0 Hz), 127.8, 114.9 (*J* = 20.0 Hz), 114.6 (*J* = 20.0 Hz), 48.6, 29.6, 28.7, 20.4. IR (ATR diamant, cm⁻¹) n 3249, 2929, 1577, 1531, 1361, 1105, 919, 827, 685. HRMS (EI-MS) : m/z calculée pour C₁₇H₁₆N₄F [M+H]⁺: 295.1354, trouvée: 295.1353. T_{f}: 78-80 °C.

### Exemple 76 : (S)-N-(1,2,3,4-Tetrahydronaphthalen-1-yl)-2-(1H-1,2,4-triazol-1-yl)pyrido[3,2-d]pyrimidin-4-amine (111)

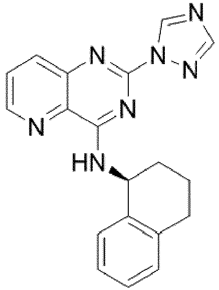

Le composé **111** est obtenu en suivant la procédure C avec les composés **19** (100 mg, 0.32 mmol, 1.0 eq.), 1,2,4-triazole (30 mg, 0.4 mmol, 1.0 eq.) et la triéthylamine (0.05 mL, 0.4 mmol, 1.0 eq.). Le résidu est purifié par chromatographie sur gel de silice (éluant CH₂Cl₂/MeOH 97:3). Le produit est obtenu sous forme d'un solide blanc (62 mg, 56 %). **R_{f}** (CH₂Cl₂/MeOH 94:6): 0.38. T_{f}: 170-172 °C. ¹H RMN (400 MHz, CDCl₃) δ 9.30 (s, 1H), 8.64 (m, 1H), 8.26 (dt, *J* = 8.0, 1.0 Hz, 1H), 8.18 (s, 1H), 7.72 (br d, *J* = 5.0 Hz, 1H, NH), 7.68 (m, 1H), 7.36 (d, *J* = 8.0 Hz, 1H), 7.22 (m, 3H), 5.69 (q, *J =* 6.0 Hz, 1H), 2.92 (m, 2H), 2.27 (m, 1H), 2.15 (m, 1H), 1.99 (m, 2H). ¹³C RMN (101 MHz, CDCl₃) δ 160.3, 153.5, 151.4, 148.1, 145.3, 144.2, 137.8, 136.1, 135.7, 131.4, 129.5, 128.8, 128.6, 127.9, 126.6, 49.4, 29.5, 29.3, 20.1. IR (ATR diamant, cm⁻¹) n 3500, 2929, 1585, 1503, 1423, 1386, 1000, 940, 870, 734, 670. HRMS (EI-MS) : m/z calculée pour C₁₉H₁₈N₇ [M+H]⁺: 344.1618, trouvée : 344.1617.

### Exemple 77 : (S)-2-(1H-Imidazol-1-yl)-N-(1,2,3,4-tetrahydronaphthalen-1-yl)pyrido[3,2-d]pyrimidin-4-amine (112)

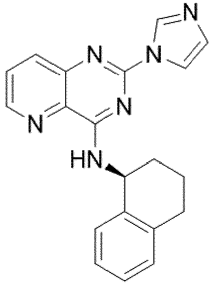

Le composé **112** est obtenu en suivant la procédure C avec les composés **19** (100 mg, 0.32 mmol, 1.0 eq.), l'imidazole (30 mg, 0.4 mmol, 1.0 eq.) et la triéthylamine (0.05 mL, 0.4 mmol, 1.0 eq.). Le résidu est purifié par chromatographie sur gel de silice (éluant CH₂Cl₂/MeOH 94:6). Le produit est obtenu sous forme d'un solide blanc (71 mg, 65 %). R_{f} (CH₂Cl₂/MeOH 90:10): 0.75. ¹H RMN (400 MHz, CDCl₃) δ 8.70 (s, 1H), 8.56 (m, 1H), 8.03 (dd, *J* = 8.0, 2.0 Hz, 1H), 7.99 (s, 1H), 7.63 (m, 2H), 7.36 (d, *J =* 8.0 Hz, 1H), 7.19 (m, 4H), 5.65 (q, *J =* 6.0 Hz, 1H), 2.90 (m, 2H), 2.26 (m, 1H), 2.15 (m, 1H), 1.97 (m, 2H). ¹³C RMN (101 MHz, CDCl₃) δ 160.2, 151.8, 147.4, 145.5, 137.8, 136.7, 136.1, 135.2, 131.0, 130.2, 129.5, 128.9, 128.3, 127.8, 126.5, 117.0, 49.2, 29.6, 29.4, 20.1. IR (ATR diamant, cm⁻¹) n 3340, 2934, 1549, 1426, 1315, 1036, 1014, 819, 737, 649. HRMS (EI-MS) : m/z calculée pour C₂₀H₁₉N₆ [M+H]⁺: 343.1665, trouvée: 343.1666. T_{f}: 186-188 °C.

### Exemple 78 : N-Tetralin-2-ylpyrido[3,2-d]pyrimidin-4-amine (118)

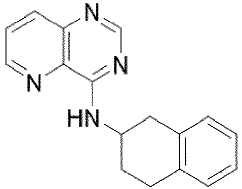

Le composé **118** est obtenu en suivant la procédure B avec le composé **117** (200 mg, 1.36 mmol, 1.7 eq.), **85** (158 mg, 0.8 mmol, 1.0 eq.) et la triéthylamine (0.22 mL, 16. mmol, 2.0 eq.). Le résidu est purifié par chromatographie sur gel de silice (éluant gradient 0 à 80 % AcOEt/100 à 20 % PE). Le produit est obtenu sous forme d'un solide blanc (205 mg, 93 %). R_{f} (PE/AcOEt 20:80): 0.34. ¹H RMN (400 MHz, CDCl₃) δ 8.64 (m, 2H), 8.09 (dd, *J* = 8.0, 2.0 Hz, 1H), 7.63 (dd, *J* = 8.0, 4.0 Hz, 1H), 7.27 (br d, *J* = 8.0 Hz, 1H, NH), 7.14 (m, 4H), 4.70 (m, 1H), 3.35 (dd, *J* = 16.0, 5.0 Hz, 1H), 3.01 (m, 2H), 2.92 (dd, *J* = 16.0, 8.0 Hz, 1H), 2.30 (m, 1H), 2.01 (m, 1H). ¹³C RMN (101 MHz, CDCl₃) δ 159.3, 156.5, 148.1, 144.3, 135.9, 135.5, 134.1, 132.1, 129.5, 128.9, 127.7, 126.3, 126.0, 46.5, 35.6, 28.7, 27.5. IR (ATR diamant, cm⁻¹) n 3380, 2927, 1586, 1542, 1367, 1311, 1137, 776. HRMS (EI-MS) : m/z calculée pour C₁₇H₁₇N₄ [M+H]⁺: 277.1447, trouvée: 277.1448. T_{f}: 123-125 °C.

### Exemple 79 : tert-Butyl N-[1-(pyrido[3,2-d]pyrimidin-4-ylamino)tetralin-6-yl]carbamate (119)

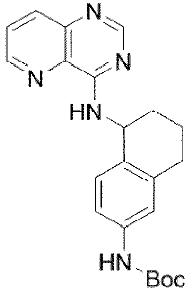

Le composé **119** est obtenu en suivant la procédure B avec le composé **116** (80 mg, 0.35 mmol, 1.7 eq.), **85** (44 mg, 0,22 mmol, 1 eq.) et la triéthylamine (0,06 mL, 0,45 mmol, 2 eq.). Le résidu est purifié par chromatographie sur gel de silice (éluant gradient 0 à 90 % AcOEt/100 à 10 % PE). Le produit est obtenu sous forme d'une huile incolore (50 mg, 57 %). R_{f} (PE/AcOEt 90:10): 0.35. ¹H RMN (400 MHz, CDCl₃) δ 8.64 (s, 1H), 8.62 (dd, *J =* 4.0, 2.0 Hz, 1H), 8.08 (dd, *J* = 8.0, 2.0 Hz, 1H), 7.62 (dd, *J* = 8.0, 4.0 Hz, 1H), 7.39 (d, *J=* 8.0 Hz, 1H, NH), 7,29 (s, 1H), 7,23 (d, *J=* 3.0 Hz, 1H), 7.02 (d, *J=* 3.0 Hz, 1H), 6.65 (s, 1H), . (m, 1H), 2.82 (m, 2H), 2.14 (m, 1H), 2.03 (m, 1H), 1.90 (m, 2H), 1.49 (s, 9H). ¹³C RMN (101 MHz, CDCl₃) δ 159.1, 156.6, 152.9, 148.2, 144.5, 138.7, 137.7, 135.9, 132.1, 131.1, 129.6, 127.7, 118.8, 117.0, 80.6, 60.5, 48.4, 29.8, 29.6, 28.4, 20.0, 14.3. IR (ATR diamant, cm⁻¹) n 2931, 1732, 1537, 1298, 1154, 828, 686. HRMS (EI-MS) : m/z calculée pour C₂₂H₂₅N₅O₂ [M+H]⁺: 392.2080, trouvée: 392.2081.

### Exemple 80 : tert-Butyl 4-(pyrido[3,2-d]pyrimidin-4-ylamino)-4,5,6,7-tetrahydroindole-1-carboxylate (120)

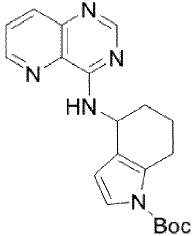

Le composé **120** est obtenu en suivant la procédure B avec le composé **115** (74 mg, 0.31 mmol, 1.7 eq.), **85** (36 mg, 0.18 mmol, 1.0 eq.) et la triéthylamine (0.05 mL, 0.36 mmol, 2.0 eq.). Le résidu est purifié par chromatographie sur gel de silice (éluant gradient 0 à 90 % AcOEt/100 à 10 % PE). Le produit est obtenu sous forme d'une huile incolore (60 mg, 91 %). R_{f} (PE/AcOEt 90:10): 0.38. ¹H RMN (400 MHz, CDCl₃) δ 8.64 (s, 1H), 8.62 (d, *J* = 4.0 Hz, 1H), 8,06 (dd, *J* = 8.0 , 2.0 Hz, 1H), 7.60 (ddd, *J* = 8.0, 4.0, 2.0 Hz, 1H), 7.33 (d, *J* = 8.0 Hz, 1H, NH), 7.14 (d, *J* = 4.0 Hz, 1H), 6.13 (m, 1H), 5.39 (m, 1H), 2.88 (m, 2H), 2.11 (m, 1H), 1.92 (m, 3H), 1.58 (s, 9H). ¹³CRMN (101 MHz, CDCl₃) δ 159.2, 156.5, 149.5, 148.1, 144.4, 135.8, 132.2, 131.8, 127.6, 122.3, 120.3, 109.9, 83.6, 45.1, 29.3, 28.2, 24.6, 20.3. IR (ATR diamant, cm⁻¹) n 3389, 2975, 2937, 1733, 1534, 1297, 1119, 828, 716, 685. HRMS (EI-MS) : m/z calculée pour C₂₀H₂₄N₅O₂ [M+H]⁺: 366.1924, trouvée: 366.1925.

### Exemple 81 : 2-Pyrrolidin-1-yl-N-[(1S)-tetralin-1-yl]pyrido[3,2-d]pyrimidin-4-amine (121)

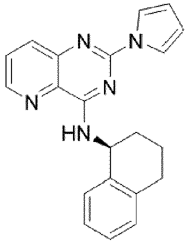

Le composé **121** est obtenu en suivant la procédure C avec les composés **19** (100 mg, 0.32 mmol, 1.0 eq.), le pyrrole (0.03 mL, 0.4 mmol, 1 eq.) et la *tert*-butylate de potassium (45 mg, 0.4 mmol, 1.0 eq.). Le résidu est purifié par chromatographie sur gel de silice (éluant PE/AcOEt 90:10). Le produit est obtenu sous forme d'un solide blanc (24 mg, 22 %). R_{f} (PE/AcOEt 90:10): 0.21. ¹H RMN (400 MHz, CDCl₃) δ 8.51 (dd, *J=* 4.0, 2.0 Hz, 1H), 8.01 (dd, *J* = 8.0, 2.0 Hz, 1H), 7.92 (m, 2H), 7.58 (dd, *J =* 8.0, 4.0 Hz, 1H), 7.49 (d, *J =* 8.0 Hz, 1H, NH), 7.39 (m, 1H), 7.20 (m, 3H), 6.34 (m, 2H), 5.70 (m, 1H), 2.90 (m, 2H), 2.24 (m, 1H), 2.13 (m, 1H), 1.97 (m, 2H). ¹³C RMN (101 MHz, CDCl₃) δ 160.0, 153.3, 146.4, 145.9, 137.8, 136.5, 135.0, 130.8, 129.4, 129.1, 128.0, 127.7, 126.5, 119.6, 111.4, 48.9, 31.0, 29.7, 20.2. IR (ATR diamant, cm⁻¹) n 3851, 3743, 3381, 2935, 2853, 1545, 1430, 1064, 855, 758. HRMS (EI-MS) : m/z calculée pour C₂₁H₂₀N₅ [M+H]⁺: 342.1713, trouvée: 342.1714. T_{f}: 131-133 °C.

### Exemple 82 : N-(4,5,6,7-Tetrahydro-1H-indol-4-yl)pyrido[3,2-d]pyrimidin-4-amine (122)

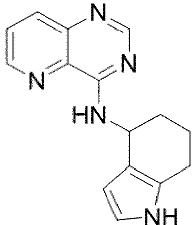

Le composé **120** (51 mg, 0.14 mmol) est dissous dans 4 mL de CH₂Cl₂ puis 1 mL de TFA est ajouté dans le ballon. Après une nuit à température ambiante sous agitation vigoureuse, quelques millilitres d'une solution de NaOH 2 M sont ajoutés et le mélange est agité à température ambiante pendant 1 heure. Le produit est ensuite extrait avec 3 x 15 mL d'AcOEt et les phases organiques sont combinées et séchées avec MgSO₄. Après filtration et évaporation, le résidu est purifié par chromatographie sur gel de silice (éluant gradient 0 à 5 % MeOH/100 à 95 % CH₂Cl₂). Le produit est obtenu sous forme d'un solide orange (22 mg, 60 %).

Rf (CH2Cl2/MeOH 95:5): 0.22. RMN 1H (400 MHz, CDCl3) δ 8.70-8.55 (m, 2H), 8.06 (dd, *J* = 8.5, 1.6 Hz, 1H), 7.66-7.52 (m, 1H), 7.39 (m, 1H), 7.13-7.11 (d, *J* = 8.1 Hz, 1H), 6.55 - 6.47 (m, 2H), 5.55 - 5.47 (m, 1H), 2.67-84 (m, 2H), 2.151.94 (m, 3H), 1.85-1.92 (m, 2H). RMN 13C (101 MHz, CDCl3) δ :.159.0, 156.7, 148.1, 145.8, 139.9, 135.9, 132.2, 130.3, 127.7, 126.5, 114.9, 48.4, 29.8, 28.5 ; 19.9.IR (ATR diamant, cm⁻¹) n ): 3396, 3190, 2921, 1577, 1541, 1357, 1306, 826, 685. HRMS (+ESI) : m/z calculée pour C15H16N5 [M+H]+:, trouvée:. Tf : 166-168 °C.

### Exemple 83 : N1-pyrido[3,2-d]pyrimidin-4-yltetralin-1,6-diamine (123)

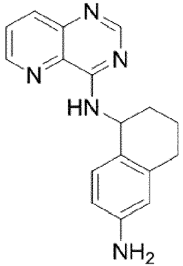

Le composé **119** (60 mg, 0.15 mmol) est dissous dans 4 mL de CH₂Cl₂ puis 1 mL de TFA est ajouté dans le ballon. Après une nuit à température ambiante sous agitation vigoureuse, quelques millilitres d'une solution de NaOH 2 M sont ajoutés et le mélange est agité à température ambiante pendant 1 heure. Le produit est ensuite extrait avec 3 x 15 mL d'AcOEt et les phases organiques sont combinées et séchées avec MgSO₄. Après filtration et évaporation, le résidu est purifié par chromatographie sur gel de silice (éluant gradient 0 à 5 % MeOH/100 à 95 % CH₂Cl₂). Le produit est obtenu sous forme d'un solide orange (28 mg, 68 %). R_{f} (CH₂Cl₂/MeOH 95:5): 0.26. ¹H RMN (400 MHz, CDCl₃) δ 8.65 (s, 1H), 8.63 (dd, *J* = 4.0, 2.0 Hz, 1H), 8.09 (dd, *J* = 8.0, 2.0 Hz, 1H), 7.63 (dd, *J* = 8.0, 4.0 Hz, 1H), 7.40 (br d, 1H, NH), 7.12 (d, *J=* 8.0 Hz, 1H), 6.51 (dd, *J* = 8.0, 2.0 Hz, 1H), 6.49 (d, *J* = 2.0 Hz, 1H), 5.50 (dt, *J* = 8.0, 5.0 Hz, 1H), 3.63 (br s, 2H, NH₂), 2,76 (m, 2H), 2.13 (m, 1H), 2.05 (m, 1H), 1.90 (m, 2H). ¹³C RMN (101 MHz, CDCl₃) δ 159.1, 156.7, 148.2, 145.8, 144.5, 139.0, 135.9, 132.3, 130.3, 127.7, 126.6, 114.9, 113.9, 48.4, 29.9, 29.6, 20.0. IR (ATR diamant, cm⁻¹) n 3438, 3395, 3306, 3190, 2921, 1541, 1306, 1119, 826, 685, 550. HRMS (EI-MS) : m/z calculée pour C₁₇H₁₈N₅ [M+H]⁺: 292.1556, trouvée: 292.1558. T_{f}: 181-183°C.

### Exemple 84 : N-(8-Methoxytetralin-1-yl)pyrido[3,2-d]pyrimidin-4-amine (126)

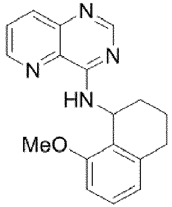

Le composé **126** est obtenu en suivant la procédure B avec le composé **124** (167 mg, 0.38 mmol, 1.7 eq.), **85** (46 mg, 0,23 mmol, 1.0 eq.) et la triéthylamine (0.07 mL, 0.45 mmol, 2.0 eq.). Le résidu est purifié par chromatographie sur gel de silice (éluant gradient 0 à 90 % AcOEt/100 à 10 % PE). Le produit est obtenu sous forme d'un solide orange (58 mg, 82 %). R_{f} (PE/AcOEt 80:20): 0.29. ¹H RMN (400 MHz, CDCl₃) δ 8.70 (s, 1H), 8.58 (dd, *J =* 4.0, 2.0 Hz, 1H), 8.07 (dd, *J* = 8.0, 2.0 Hz, 1H), 7.59 (dd, *J* = 8.0, 4.0 Hz, 1H), 7.26 (br d, *J* = 3.0 Hz, 1H, NH), 7.21 (t, *J* = 8.0 Hz, 1H), 6.77 (d, *J =* 8.0 Hz, 1H), 6.69 (d, *J =* 8.0 Hz, 1H), 5.69 (m, 1H), 3.64 (s, 3H), 2.84 (m, 2H), 2.36 (m, 1H), 1.85 (m, 3H). ¹³C RMN (101 MHz, CDCl₃) δ 158.7, 158.5, 156.7, 147.9, 144.3, 139.5, 135.8, 132.3, 128.6, 127.5, 124.2, 121.5, 107.9, 55.6, 43.2, 29.3, 28.5, 18.3. IR (ATR diamant, cm⁻¹) n 3386, 2927, 1466, 1436, 1250, 977, 892, 780, 585. HRMS (EI-MS) : m/z calculée pour C₁₈H₁₉N₄O [M+Na]⁺: 307.1553, trouvée: 307.1553. T_{f}: 147-149 °C.

### Exemple 85 : N-(6,7-Dimethoxytetralin-1-yl)pyrido[3,2-d]pyrimidin-4-amine (127)

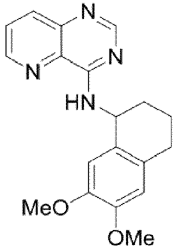

Le composé **127** est obtenu en suivant la procédure B avec le composé **125** (105 mg, 0.51 mmol, 1.7 eq.), **85** (60 mg, 0.30 mmol, 1.0 eq.) et la triéthylamine (0.08 mL, 0.60 mmol, 2.0 eq.). Le résidu est purifié par chromatographie sur gel de silice (éluant gradient 0 à 90 % AcOEt/100 à 10 % PE). Le produit est obtenu sous forme d'un solide blanc (73 mg, 72 %). R_{f} (PE/AcOEt 80:20): 0.32. ¹H RMN (400 MHz, CDCl₃) δ 8.64 (s, 1H), 8.62 (dd, *J =* 4.0, 2.0 Hz, 1H), 8.08 (dd, *J* = 8.0, 2.0 Hz, 1H), 7.62 (dd, *J* = 8.0, 4.0 Hz, 1H), 7,43 (br d, *J=* 3.0 Hz, 1H, NH), 6.81 (s, 1H), 6.61 (s, 1H), 5.51 (m, 1H), 3.85 (s, 3H), 3.74 (s, 3H), 2.76 (m, 2H), 2.10 (m, 2H), 1.90 (m, 2H). ¹³CRMN(101 MHz, CDCl₃) δ 159.0, 156.5, 148.7, 148.2, 147.7, 144.4, 135.9, 132.2, 130.2, 128.0, 127.7, 111.8, 111.6, 56.1, 56.0, 48.4, 29.5, 29.0, 19.8. IR (ATR diamant, cm⁻¹) n 3409, 2828, 1671, 1537, 1437, 1294, 1165, 1020, 942, 870, 789, 685. HRMS (EI-MS) : m/z calculée pour C₁₉H₂₁N₄O₂ [M+Na]⁺: 337.1659, trouvée: 337.1659. T, : 117-119 °C.

### Exemple 86 : N-[5-(Methoxymethoxy)tetralin-1-yl]pyrido[3,2-d]pyrimidin-4-amine (130)

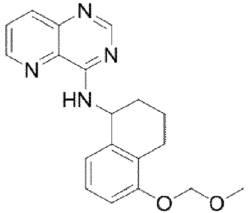

Le composé **130** est obtenu en suivant la procédure B avec le composé **129** (120 mg, 0.58 mmol, 1.7 eq.), **85** (68 mg, 0.34 mmol, 1.0 eq.) et la triéthylamine (0.10 mL, 0.68 mmol, 2.0 eq.). Le résidu est purifié par chromatographie sur gel de silice (éluant gradient 0 à 90 % AcOEt/100 à 10 % PE). Le produit est obtenu sous forme d'une huile incolore (73 mg, 63 %). R_{f} (PE/AcOEt 20:80): 0.42. ¹H RMN (400 MHz, CDCl₃) δ 8.65 (s, 1H), 8.62 (dd, *J =* 4.0, 2.0 Hz, 1H), 8.09 (dd, *J* = 8.0, 2.0 Hz, 1H), 7.62 (dd, *J* = 8.0, 4.0 Hz, 1H), 7.46 (br d, *J* = 3.0 Hz, 1H, NH), 7.06 (t, *J* = 8.0 Hz, 1H), 7.00 (t, *J =* 2.0 Hz, 1H), 6.92 (t, *J =* 8.0, 2.0 Hz, 1H), 5.58 (m, 1H), 5.07 (m, 2H), 3.39 (s, 3H), 2.79 (m, 2H), 2.16 (m, 1H), 2.00 (m, 1H), 1.95 (m, 2H). ¹³C RMN (101 MHz, CDCl₃) δ 159.2, 156.6, 155.7, 148.2, 144.5, 137.6, 135.9, 132.1, 131.2, 130.3, 127.7, 116.5, 116.0, 94.7, 56.0, 48.9, 29.6, 28.6, 20.3. IR (ATR diamant, cm⁻¹) n 3384, 2929, 1578, 1477, 1360, 1075, 1000, 920, 828, 803, 686. HRMS (EI-MS) : m/z calculée pour C₁₉H₂₁N₄O₂ [M+H]⁺: 337.1659, trouvée: 337.1658.

### Exemple 87 : 1-(Pyrido[3,2-d]pyrimidin-4-ylamino)tetralin-5-ol (131)

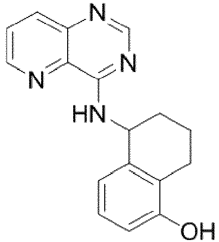

Le composé **130** (60 mg, 0.18 mmol) est dissous dans 5 mL de MeOH et quelques gouttes de HCl concentré (37% dans l'eau) sont ajoutés à température ambiante. Le mélange est agité pendant une nuit. Le mélange est dilué avec 20 mL d'AcOEt puis lavé avec une solution saturée aqueuse de Na₂CO₃. La phase organique est lavée avec une solution saturée aqueuse de NaCl puis séchée avec MgSO₄. Après filtration, le solvant est évaporé et le produit **131** est obtenu par précipitation dans le pentane sous forme de solide blanc (42 mg, 81 %). R_{f} (AcOEt 100): 0.29. ¹H RMN (400 MHz, DMSO-*d*₆) δ 8.98 (br s, 1H, OH), 8.79 (dd, *J =* 4.0, 2.0 Hz, 1H), 8.54 (s, 1H), 8.37 (br d, *J* = 3.0 Hz, 1H, NH), 8.14 (dd, *J* = 8.0, 2.0 Hz, 1H), 7.84 (dd, *J* = 8.0, 4.0 Hz, 1H), 6.93 (d, *J* = 8.0 Hz, 1H), 6.58 (m, 2H), 5.56 (m, 1H), 2.69 (m, 2H), 2.02 (m, 2H), 1.93 (m, 1H), 1.76 (m, 1H). ¹³C RMN (101 MHz, DMSO-d₆) δ 159.1, 156.0, 155.3, 148.5, 144.2, 138.1, 135.4, 131.4, 129.7, 128.3, 127.3, 114.4, 113.4, 59.7, 48.4, 29.0, 20.8. IR (ATR diamant, cm⁻¹) n 3362, 2928, 2680, 2593, 1584, 1509, 1451, 1361, 1237, 1189, 1158, 1130, 870, 825, 800, 616, 529. HRMS (EI-MS) : m/z calculée pour C₁₂H₁₈NO₂ [M+H]⁺: 293.1396, trouvée: 293.1398. T_{f}: 249-251 °C.

### Exemple 88 : 6-Chloro-N-[(1S)-tetralin-1-yl]pyrido[3,2-d]pyrimidin-4-amine (133)

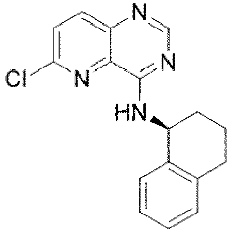

Le composé **132** (120 mg, 0,6 mmol) est dissous dans 5 mL de THF anhydre et (*S*)-(+)-1,2,3,4-tetrahydro-1-naphthylamine (0.09 mL, 0.6 mmol) et triéthylamine (0.1 mL; 0.72 mmol) sont ajoutés. La solution est agitée pendant 24 h à température ambiante. La disparition du réactif de départ est suivie par CCM puis, en fin de réaction, le THF est évaporé. Le dépôt obtenu est repris dans 30 mL d'eau puis le produit est extrait avec 3 x 20 mL d'acétate d'éthyle. Les phases organiques sont combinées et lavées avec une solution aqueuse de NaCl saturée (20 mL) et enfin séchées sur MgSO₄. Après filtration, le solvant est évaporé et le résidu est purifié sur gel de silice (éluant gradient 0 à 40 % AcOEt/100 à 60 % PE). Le produit est obtenu sous forme d'un solide beige (154 mg, 83 %). R_{f} (PE/AcOEt 50:50): 0.32. ¹H RMN (400 MHz, CDCl₃) δ 8.67 (s, 1H), 8.04 (d, *J =* 8.0 Hz, 1H), 7,59 (d, *J* = 8.0 Hz, 1H), 7.32 (m, 1H), 7.18 (m, 4H), 5.64 (m, 1H), 2.87 (m, 2H), 2.20 (m, 1H), 1.99 (m, 3H). ¹³C RMN (101 MHz, CDCl₃) δ 158.3, 156.9, 148.8, 143.8, 139.1, 137.9, 136.2, 131.5, 129.5, 129.2, 128.9, 127.7, 126.5, 48.9, 29.7, 29.4, 20.2. IR (ATR diamant, cm⁻¹) n 3373, 2922, 2859, 1583, 1540, 1471, 1374, 1342, 1124, 864, 753, 636. HRMS (EI-MS) : m/z calculée pour C₁₇H₁₆ClN₄ [M+H]⁺: 311.1058, trouvée: 311.1056. T_{f}: 115-117 °C.

### Exemple 89 : 7-Bromo-N-[(1S)-tetralin-1-yl]pyrido[3,2-d]pyrimidin-4-amine (135)

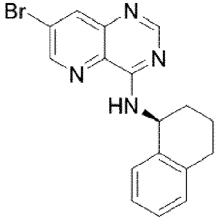

Le composé **134** (540 mg, 2.12 mmol) est dissous dans 5 mL de THF anhydre et de la (*S*)-(+)-1,2,3,4-tetrahydro-1-naphthylamine (0.31 mL, 2.12 mmol) mais aussi de la triethylamine (0.35 mL, 2.54 mmol) sont ajoutées. La solution est agitée pendant une nuit à 80 °C. La disparition du réactif de départ est suivie par CCM puis, en fin de réaction, le THF est évaporé. Le dépôt obtenu est repris dans 30 mL d'une solution aqueuse de NaHCO₃ saturée puis le produit est extrait avec 3 x 20 mL d'acétate d'éthyle. Les phases organiques sont combinées et lavées avec une solution aqueuse de NaCl saturée (20 mL) et enfin séchées sur MgSO₄. Après filtration, le solvant est évaporé et le résidu est purifié sur gel de silice (éluant gradient 0 à 50 % AcOEt/100 à 50 % PE). Le produit est obtenu sous forme d'un solide jaune (574 mg, 73 %). R_{f} (PE/AcOEt 50:50): 0.51. ¹H RMN (400 MHz, CDCl₃) δ 8.66 (s, 1H), 8.60 (d, *J* = 8.0 Hz, 1H), 8.24 (d, *J =* 8.0 Hz, 1H), 7. 40 (br d, *J =* 3.0 Hz, 1H, NH), 7.31 (m, 1H), 7.16 (m, 3H), 5.62 (m, 1H), 2.85 (m, 2H), 2.20 (m, 1H), 2.02 (m, 1H), 1.93 (m, 2H). ¹³CRMN(101 MHz, CDCl₃) δ 159.1, 157.6, 149.3, 145.3, 137.8, 137.6, 136.2, 130.3, 129.4, 128.8, 127.7, 126.4, 124.1, 48.8, 29.7, 29.3, 20.1. IR (ATR diamant, cm⁻¹) n 3946, 1571, 1524, 1452, 1531, 1299, 1122, 873, 748. HRMS (EI-MS) : m/z calculée pour C₁₇H₁₆BrN₄ [M+H]⁺: 355.0553, trouvée: 355.0552. T, : 82-84 °C.

### Exemple 90 : N-Tetralin-1-ylthieno[3,2-d]pyrimidin-4-amine (136)

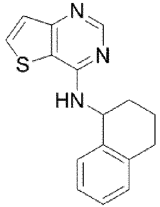

La 4-chlorothieno[3,2-*d*]pyrimidine (200 mg, 1.17 mmol, 1.0 eq.) est dissoute dans 5 mL de CH₃CN anhydre et de la 1,2,3,4-tetrahydro-1-naphthylamine (0.34 mL, 2.35 mmol, 2.0 eq.) mais aussi de la diisopropylethylamine (0.25 mL; 1.41 mmol, 1.2 eq.) sont ajoutées. La solution est agitée pendant 5 h à reflux. La disparition du réactif de départ est suivie par CCM puis, en fin de réaction, le CH₃CN est évaporé. Le dépôt obtenu est repris dans 30 mL d'une solution aqueuse de NaHCO₃ saturée puis le produit est extrait avec 3 x 20 mL d'acétate d'éthyle. Les phases organiques sont combinées et lavées avec une solution aqueuse de NaCl saturée (20 mL) et enfin séchées sur MgSO₄. Après filtration, le solvant est évaporé et le résidu est purifié sur gel de silice (éluant gradient 0 à 50 % AcOEt/100 à 50 % PE). Le produit est obtenu sous forme d'un solide blanc (184 mg, 56 %). R_{f} (PE/AcOEt 50:50): 0.25. ¹H RMN (400 MHz, CDCl₃) δ 8.68 (s, 1H), 7.70 (d, *J=* 8.0 Hz, 1H), 7.44 (d, *J =* 8.0 Hz, 1H), 7.36 (m, 1H), 7.20 (m, 3H), 5.69 (m, 1H), 5.07 (br d, *J =* 3.0 Hz, 1H, NH), 2.87 (m, 2H), 2.21 (m, 1H), 2.03 (m, 1H), 1.93 (m, 2H). ¹³C RMN (101 MHz, CDCl₃) δ 160.1, 156.8, 155.3, 138.1, 136.8, 130.9, 129.5, 129.1, 127.7, 126.6, 125.7, 115.2, 49.0, 30.2, 29.5, 20.1. IR (ATR diamant, cm⁻¹): 2928, 1578, 1535, 1496, 1442, 1302, 1040, 792, 719. HRMS (EI-MS) : m/z calculée pour C₁₆H₁₆N₃S [M+H]⁺: 282.1059, trouvée: 282.1058. T_{f}: 178-180 °C.

### Exemple 91 : N-Tetralin-1-yl-7H-pyrrolo[2,3-d]pyrimidin-4-amine (137)

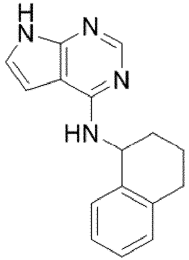

Dans un tube scellé, la 4-chloro-7H-pyrrolo[3,2-*d*]pyrimidine (200 mg, 1.31 mmol, 1.0 eq.) est dissoute dans 6 mL d'isopropanol et de la 1,2,3,4-tetrahydro-1-naphthylamine (0.22 mL, 1.5 mmol, 1.2 eq.) maiscaussi de la diisopropylethylamine (0.68 mL; 3.93 mmol, 3.0 eq.) sont ajoutés. La réaction est lancée au micro-onde pendant 1 h à 160 °C. La disparition du réactif de départ est suivie par CCM puis, en fin de réaction, la solution est diluée avec 50 mL d'AcOEt. La solution est ensuite lavée avec 50 mL d'eau puis 50 mL d'une solution aqueuse de NaCl saturée et séchée avec MgSO₄. Après filtration, le solvant est évaporé et le résidu est purifié sur gel de silice (éluant gradient 0 à 80 % AcOEt/100 à 20 % PE). Le produit est obtenu sous forme d'un solide blanc (148 mg, 43 %). R_{f} (PE/AcOEt 20:80): 0.20. ¹H RMN (400 MHz, CD₃OD) δ 8.16 (s, 1H), 7.28 (m, 1H), 7.14 (m, 3H), 7.07 (d, *J* = 8.0 Hz, 1H), 6.62 (d, *J=* 8.0 Hz, 1H), 5.57 (m, 1H), 2.88 (m, 2H), 2.16 (m, 1H), 1.97 (m, 3H). ¹³C RMN (101 MHz, CD₃OD) δ 157.6, 152.2, 150.5, 138.8, 130.0, 129.5, 128.0, 127.0, 122.2, 104.3, 100.2, 31.5, 30.4, 21.6. IR (ATR diamant, cm⁻¹) n 3189, 3095, 2931, 2852, 1584, 1473, 1354, 1321, 1160, 1138, 897, 819, 656. HRMS (EI-MS) : m/z calculée pour C₁₆H₁₇N₄ [M+H]⁺: 265.1447, trouvée: 265.1445. T_{f}: 226-228 °C.

### Exemple 92 : N-Tetralin-1-ylthieno[2,3-d]pyrimidin-4-amine (138)

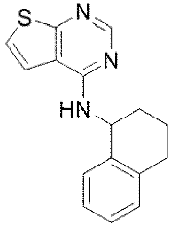

La 4-chlorothieno[2,3-*d*]pyrimidine (200 mg, 1.18 mmol, 1.0 eq.) est dissoute dans 6 mL de THF anhydre et la 1,2,3,4-tetrahydro-1-naphthylamine (0.25 mL, 1.77 mmol, 1.5 eq.) mais aussi la diisopropylethylamine (0.34 mL, 1.77 mmol, 1.5 eq.) sont ajoutées. La solution est agitée pendant 48 h à température ambiante. La disparition du réactif de départ est suivie par CCM puis, en fin de réaction, le THF est évaporé. Le dépôt obtenu est repris dans 30 mL d'une solution aqueuse de NaHCO₃ saturée puis le produit est extrait avec 3 x 20 mL d'acétate d'éthyle. Les phases organiques sont combinées et lavées avec une solution aqueuse de NaCl saturée (20 mL) et enfin séchées sur MgSO₄. Après filtration, le solvant est évaporé et le résidu est purifié sur gel de silice (éluant gradient 0 à 40 % AcOEt/100 à 60 % PE). Le produit est obtenu sous forme d'un solide blanc (168 mg, 51 %). R_{f} (PE/AcOEt 50:50): 0.32. ¹H RMN (400 MHz, CDCl₃) δ 8.55 (s, 1H), 7.34 (m, 1H), 7,25 (d, *J =* 8.0 Hz, 1H), 7.20 (m, 3H), 7.08 (d, *J =* 8.0 Hz, 1H), 5.67 (m, 1H), 5.40 (br d, *J =* 3.0 Hz, 1H, NH), 2.86 (m, 2H), 2.19 (m, 1H), 2.02 (m, 1H), 1.93 (m, 2H). ¹³C RMN (101 MHz, CDCl₃) δ 166.8, 156.5, 154.3, 138.0, 136.9, 129.5, 129.1, 127.7, 126.5, 123.2, 117.1, 116.2, 48.8, 30.0, 29.5, 20.1. IR (ATR diamant, cm⁻¹) n 3234, 3052, 2929, 2858, 1578, 1536, 1493, 1437, 1354, 1306, 1079, 1023, 880, 738. HRMS (EI-MS) : m/z calculée pour C₁₆H₁₆N₃S [M+H]⁺: 282.1059, trouvée: 282.1055. T_{f}: 176-178 °C.

### Exemple 93 : tert-Butyl N-[4-[4-[[(1S)-tetralin-1-yl]amino]pyrido[3,2-d]pyrimidin-7-yl]but-3-ynyl]carbamate (139)

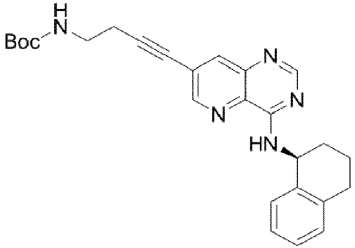

Le composé **135** (200 mg, 0.56 mmol) est dissous dans 10 mL de CH₃CN et le *tert*-butyl but-3-yn-1-ylcarbamate (0.11 mL, 0.62 mmol) est ajouté ainsi que l'iodure de cuivre (6.0 mg, 0.03 mmol), la triphénylphosphine (8.0 mg, 0.03 mmol) et la triéthylamine (0.25 mL, 3.4 mmol) sous agitation vigoureuse puis en dernier le palladium acetate (1.5 mg, 0.0056 mmol). Le mélange est agité à 80 °C pendant 2 heures. La disparition du réactif de départ est suivie par CCM puis, en fin de réaction, le CH₃CN est évaporé. Le dépôt obtenu est repris dans 30 mL d'eau puis le produit est extrait avec 3 x 20 mL d'acétate d'éthyle. Les phases organiques sont combinées et lavées avec une solution aqueuse de NaCl saturée (20 mL) et enfin séchées sur MgSO₄. Après filtration, le solvant est évaporé et le résidu est purifié sur gel de silice (éluant gradient 0 à 60 % AcOEt/100 à 40 % PE). Le produit est obtenu sous forme d'une huile incolore (152 mg, 61 %). R_{f} (PE/AcOEt 50:50): 0,29. ¹H RMN (400 MHz, CDCl₃) δ 8.66 (s, 1H), 8.57 (d, *J* = 2.0 Hz, 1H), 8.05 (d, *J* = 2.0 Hz, 1H), 7.35 (br d, *J=* 3.0 Hz, 1H, NH), 7.32 (m, 1H), 7.18 (m, 3H), 5.61 (m, 1H), 4.92 (br s, 1H, NH), 3.41 (q, *J =* 6.0 Hz, 2H), 2.87 (qt, *J =* 17.0, 6.0 Hz, 2H), 2.69 (t, *J =* 6.0 Hz, 2H), 2.19 (m, 1H), 2.00 (m, 1H), 1.98 (m, 2H), 1.46 (s, 9H). ¹³C RMN (101 MHz, CDCl₃) δ 159.0, 157.2, 155.9, 150.4, 143.8, 137.9, 137.8, 136.5, 130.5, 129.4, 129.0, 127.7, 126.5, 124.7, 93.6, 79.8, 78.5, 48.8, 39.4, 29.8, 29.4, 28.5, 21.4, 20.2. IR (ATR diamant, cm⁻¹) n 3373, 2931, 2231, 1698, 1574, 1525, 1389, 1364, 1319, 1283, 1162, 909, 754. HRMS (EI-MS) : m/z calculée pour C₂₆H₃₀N₅O₂ [M+H]⁺: 444.2394, trouvée: 444.2394.

### Exemple 94 : 7-(4-Aminobut-1-ynyl)-N-[(1S)-tetralin-1-yl]pyrido[3,2-d]pyrimidin-4-amine (140)

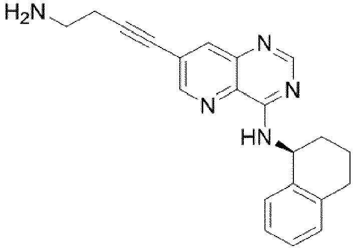

Le composé **139** (130 mg, 0.3 mmol) est dissous dans 4 mL de CH₂Cl₂ puis 1 mL de TFA est ajouté dans le ballon. Après une nuit à température ambiante sous agitation vigoureuse, quelques millilitres d'une solution de NaOH 2 M sont ajoutés et le mélange est agité à température ambiante pendant 1 heure. Puis le produit est extrait avec 3 x 15 mL d'AcOEt et les phases organiques sont combinées et séchées avec MgSO₄. Après filtration et évaporation, le produit est obtenu sous forme d'un solide orange (98 mg, 97 %). R_{f} (CH₂Cl₂/MeOH 90:10): 0.14. ¹H RMN (400 MHz, CDCl₃) δ 8.65 (s, 1H), 8.56 (d, *J =* 2.0 Hz, 1H), 8.05 (d, *J* = 2.0 Hz, 1H), 7.35 (br d, *J* = 3.0 Hz, 1H, NH), 7.32 (m, 1H), 7.16 (m, 3H), 5.60 (m, 1H), 2.91 (q, *J* = 6.0 Hz, 2H), 2,87 (qt, *J =* 17.0, 6.0 Hz, 2H), 2.62 (t, *J =* 6.0 Hz, 2H), 2.18 (m, 1H), 1.99 (m, 1H), 1.97 (m, 2H), 1.67 (br s, 2H, NH₂), ¹³C RMN (101 MHz, CDCl₃) δ 159.0, 157.1, 150.4, 143.8, 137.8, 137.7, 136.4, 130.4, 129.4, 128.9, 127.6, 126.4, 124.9, 94.4, 78.5, 48.7, 41.0, 29.7, 29.4, 24.7, 20.1. IR (ATR diamant, cm⁻¹) n 3367, 2934, 2225, 1577, 1530, 1322, 750, 733. HRMS (EI-MS) : m/z calculée pour C₂₁H₂₂N₅ [M+H]⁺: 344.1869, trouvée: 344.1870. T_{f} : 88-90 °C.

### Exemple 95 : 4-(Pyrido[3,2-d]pyrimidin-4-ylamino)tetralin-1-ol (142)

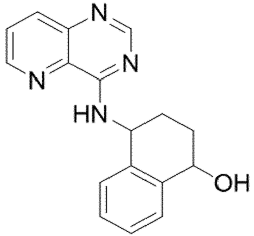

Le composé **10** (100 mg, 0.6 mmol, 1.2 eq.) est dissous dans 5 mL de THF anhydre pour donner une solution jaune à laquelle l'amine **141** (80 mg, 0.50 mmol, 1.0 eq.) est ajoutée. La solution devient trouble. La triéthylamine (0.08 mL, 0.60 mmol, 1,2 eq.) est ajoutée et la solution reste à température ambiante pendant 2 à 12 heures sous agitation vigoureuse. La disparition du réactif de départ est suivie par CCM puis, en fin de réaction, le THF est évaporé. Le dépôt obtenu est repris dans 3 x 20 mL d'acétate d'éthyle puis le produit est lavé avec 30 mL d'une solution aqueuse saturée de NaHCO₃. La phase organique est lavée avec une solution aqueuse de NaCl saturée (40 mL) et enfin séchée sur MgSO₄. Après filtration, le solvant est évaporé et le résidu est purifié sur gel de silice (éluant gradient 0 à 60 % AcOEt/100 à 40 % PE). Le produit est obtenu sous forme d'une poudre blanche (32 mg, 22 %). R_{f} (PE/AcOEt 30:70): 0.22. ¹H RMN (400 MHz, CDCl₃) δ 8.64 (dd, *J=* 4.0 , 2.0 Hz, 1H), 8.62 (s, 1H), 8.09 (dd, *J* = 8.0, 2.0 Hz, 1H), 7.64 (dd, *J* = 8.0, 4.0 Hz, 1H), 7.57 (br d, *J=* 3.0 Hz, 1H, NH), 7.51 (m, 1H), 7.32 (m, 3H), 5.57 (m, 1H), 4.86 (m, 1H), 2.67 (br s, 1H, OH), 2.29 (m, 1H), 2.16 (m, 3H). ¹³C RMN (101 MHz, CDCl₃) δ 159.4, 156.5, 148.4, 144.5, 139.6, 136.7, 136.0, 132.1, 128.8, 128.6, 128.3, 128.2, 127.9, 67.9, 48.9, 29.8, 25.3. IR (ATR diamant, cm⁻¹) n 3372, 3253, 2925, 2859, 1584, 1436, 1317, 1063, 924, 826, 802, 764, 684, 590. HRMS (EI-MS) : m/z calculée pour C₁₇H₁₇N₄O [M+H]⁺: 293.1396, trouvée: 293.1395. T, : 159-161 °C.

4 énantiomères sont obtenus et séparés (cf. schéma 15) : 142-E1, 142-E2, 142-E3 et 142-E4.

### Exemple 96 : N-Tetralin-1-ylfuro[2,3-d]pyrimidin-4-amine (143)

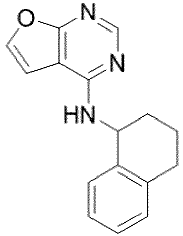

La 4-chlorofuro[2,3-*d*]pyrimidine (200 mg, 1.3 mmol, 1.0 eq.) est dissoute dans 5 mL de THF anhydre et de la 1,2,3,4-tetrahydro-1-naphthylamine (0.28 mL, 1.95 mmol, 1.5 eq.) mais aussi de la diisopropylethylamine (0.35 mL, 1.95 mmol, 1.5 eq.) sont ajoutées. La solution est agitée pendant 24 h à température ambiante. La disparition du réactif de départ est suivie par CCM puis, en fin de réaction, le THF est évaporé. Le dépôt obtenu est repris dans 30 mL d'une solution aqueuse de NaHCO₃ saturée puis le produit est extrait avec 3 x 20 mL d'acétate d'éthyle. Les phases organiques sont combinées et lavées avec une solution aqueuse de NaCl saturée (20 mL) et enfin séchées sur MgSO₄. Après filtration, le solvant est évaporé et le résidu est purifié sur gel de silice (éluant gradient 0 à 30 % AcOEt/100 à 70 % PE). Le produit est obtenu sous forme d'un solide blanc (61 mg, 18 %). R_{f} (PE/AcOEt 60:40): 0.33. ¹H RMN (400 MHz, CDCl₃) δ 8.40 (s, 1H), 7.47 (d, *J =* 2.0 Hz, 1H), 7.33 (m, 1H), 7.18 (m, 3H), 6.61 (d, *J =* 2.0 Hz, 1H), 5.51 (br s, 1H), 5.49 (br s, 1H) 2.85 (m, 2H), 2.16 (m, 1H), 2.02 (m, 1H), 1.90 (m, 2H). ¹³C RMN (101 MHz, CDCl₃) δ 166.9, 157.1, 154.3, 141.2, 137.9, 136.7, 129.4, 129.1, 127.7, 126.5, 102.9, 100.7, 49.2, 30.2, 29.4, 19.9. IR (ATR diamant, cm⁻¹) n 3281, 3103, 2929, 1589, 1488, 1343, 1143, 760, 739. HRMS (EI-MS) : m/z calculée pour C₁₆H₁₆N₃O [M+H]⁺: 266.1287, trouvée: 266.1286. T_{f} : 166-168 °C.

### Exemple 97 : N-Tetralin-1-ylfuro[3,2-d]pyrimidin-4-amine (144)

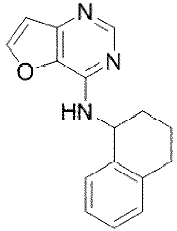

La 4-chlorofuro[3,2-*d*]pyrimidine (200 mg, 1.30 mmol, 1.0 eq.) est dissoute dans 5 mL de THF anhydre et de la 1,2,3,4-tetrahydro-1-naphthylamine (0.28 mL, 1.95 mmol, 1.2 eq.) mais aussi de la diisopropylethylamine (0.35 mL; 1.95 mmol, 1.2 eq.) sont ajoutées. La solution est agitée pendant 24 h à température ambiante. La disparition du réactif de départ est suivie par CCM puis, en fin de réaction, le THF est évaporé. Le dépôt obtenu est repris dans 30 mL d'une solution aqueuse de NaHCO₃ saturée puis le produit est extrait avec 3 x 20 mL d'acétate d'éthyle. Les phases organiques sont combinées et lavées avec une solution aqueuse de NaCl saturée (20 mL) et enfin séchées sur MgSO₄. Après filtration, le solvant est évaporé et le résidu est purifié sur gel de silice (éluant gradient 0 à 40 % AcOEt/100 à 60 % PE). Le produit est obtenu sous forme d'un solide blanc (124 mg, 36 %). R_{f} (PE/AcOEt 50:50): 0.42. ¹H RMN (400 MHz, CDCl₃) δ 8.51 (s, 1H), 7.69 (d, *J =* 2.0 Hz, 1H), 7.35 (m, 1H), 7.19 (m, 3H), 6.85 (d, *J =* 2.0 Hz, 1H), 5.61 (m, 1H), 5.45 (br d, *J =* 3.0 Hz, 1H, NH), 2.85 (m, 2H), 2.19 (m, 1H), 2.02 (m, 1H), 1.97 (m, 2H). ¹³C RMN (101 MHz, CDCl₃) δ 154.1, 149.7, 148.2, 148.1, 137.8, 136.8, 134.1, 129.4, 129.0, 127.7, 126.5, 108.3, 48.7, 30.4, 29.4, 20.0. IR (ATR diamant, cm⁻¹) n 3169, 3117, 3046, 2923, 2858, 1621, 1330, 1110, 898, 795, 588. HRMS (EI-MS) : m/z calculée pour C₁₆H₁₆N₃O [M+H]⁺: 266.1288, trouvée: 266.1287. T_{f}: 149-151 °C.

### Exemple 98 : N-Tetralin-1-ylthiazolo[5,4-d]pyrimidin-7-amine (145)

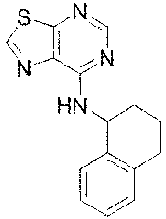

La 7-chlorothiazolo[5,4-*d*]pyrimidine (200 mg, 1.16 mmol, 1.0 eq.) est dissous dans 6 mL de THF anhydre et de la 1,2,3,4-tetrahydro-1-naphthylamine (0.33 mL, 2.32 mmol, 2.0 eq.) mais aussi de la triéthylamine (0.97 mL, 7.0 mmol, 6.0 eq.) sont ajoutées. La solution est agitée pendant 48 h à température ambiante. La disparition du réactif de départ est suivie par CCM puis, en fin de réaction, le THF est évaporé. Le dépôt obtenu est repris dans 30 mL d'une solution aqueuse de NaHCO₃ saturée puis le produit est extrait avec 3 x 20 mL d'acétate d'éthyle. Les phases organiques sont combinées et lavées avec une solution aqueuse de NaCl saturée (20 mL) et enfin séchées sur MgSO₄. Après filtration, le solvant est évaporé et le résidu est purifié sur gel de silice (éluant gradient 0 à 40 % AcOEt/100 à 60 % PE). Le produit est obtenu sous forme d'un solide blanc (257 mg, 78 %). R_{f} (PE/AcOEt 50:50): 0.38. ¹H RMN (400 MHz, CDCl₃) δ 8.67 (s, 1H), 8.57 (s, 1H), 7.33 (m, 1H), 7.17 (m, 3H), 6.49 (br d, *J=* 3.0 Hz, 1H, NH), 5,64 (m, 1H), 2.85 (m, 2H), 2.19 (m, 1H), 1.96 (m, 1H), 1.92 (m, 2H). ¹³C RMN (101 MHz, CDCl₃) δ 161.3, 155.7, 155.4, 150.4, 137.8, 136.5, 130.9, 129.4, 128.9, 127.6, 126.4, 48.7, 30.0, 29.4, 20.0. IR (ATR diamant, cm⁻¹) n 3438, 3044, 2930, 2860, 1576, 1531, 1422, 1098, 996, 805, 746. HRMS (EI-MS) : m/z calculée pour C₁₅H₁₅N₄S [M+H]⁺: 283.1012, trouvée: 283.1010. T_{f} : 111-113 °C.

### Exemple 99 : N-Tetralin-1-ylimidazo[2,1-f][1,2,4]triazin-4-amine (147)

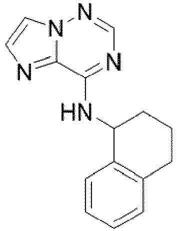

La 4-chlorofuro[3,2-*d*]pyrimidine (200 mg, 1.30 mmol, 1.0 eq.) est dissoute dans 5 mL de THF anhydre et de la 1,2,3,4-tetrahydro-1-naphthylamine (0.37 mL, 2.6 mmol, 2.0 eq.) mais aussi de la diisopropylethylamine (0.45 mL; 2.6 mmol, 2.0 eq.) est ajoutée. La solution devient trouble et prend en masse en 3 h. La disparition du réactif de départ est suivie par CCM puis, en fin de réaction, la solution est diluée avec 50 mL d'acétate d'éthyle. La solution est lavée avec 30 mL d'une solution aqueuse de NaHCO₃ saturée puis avec 30 mL d'une solution aqueuse de NaCl saturée (20 mL) et enfin séchée sur MgSO₄. Après filtration, le solvant est évaporé et le résidu est purifié sur gel de silice (éluant gradient 0 à 40 % AcOEt/100 à 60 % PE). Le produit est obtenu sous forme d'un solide blanc (335 mg, 97 %). R_{f} (PE/AcOEt 50:50): 0.34. ¹H RMN (400 MHz, CDCl₃) δ 8.13 (s, 1H), 7.63 (d, *J =* 2.0 Hz, 1H), 7.43 (br d, *J* = 3.0 Hz, 1H, NH), 7.25 (m, 1H), 7.23 (d, *J* = 2.0 Hz, 1H), 7.16 (m, 1H), 7.08 (m, 2H), 5.58 (m, 1H), 2.77 (t, *J =* 6.0 Hz, 2H), 2.15 (m, 1H), 1.92 (m, 1H), 1.90 (m, 2H). ¹³C RMN (101 MHz, CDCl₃) δ 151.7, 149.4, 137.6, 135.6, 130.9, 129.3, 129.2, 128.7, 127.6, 126.2, 117.8, 48.7, 29.7, 29.2, 20.0. IR (ATR diamant, cm⁻¹) n 3154, 2941, 1603, 1477, 1147, 922, 738, 509. HRMS (EI-MS) : m/z calculée pour C₁₅H₁₆N₅ [M+H]⁺: 266.1400, trouvée: 266.1399. T_{f}: 135-137 °C.

### Exemple 100 : 7-Methyl-N-[(1S)-tetralin-1-yl]pyrido[3,2-d]pyrimidin-4-amine (149)

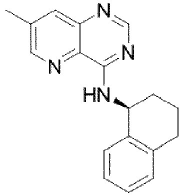

Le composé **148** (80 mg, 0.50 mmol, 1.0 eq.) est dissous dans 5 mL de THF anhydre et de la (*S*)-(+)-1,2,3,4-tetrahydro-1-naphthylamine (0.1 mL, 0.67 mmol, 1.3 eq.) mais aussi de la triethylamine (0.1 mL, 0.67 mmol, 1.3 eq.) sont ajoutées. La solution est agitée pendant 3 h à température ambiante. La disparition du réactif de départ est suivie par CCM puis, en fin de réaction, le THF est évaporé. Le dépôt obtenu est repris dans 30 mL d'une solution aqueuse de NaHCO₃ saturée puis le produit est extrait avec 3 x 20 mL d'acétate d'éthyle. Les phases organiques sont combinées et lavées avec une solution aqueuse de NaCl saturée (20 mL) et enfin séchées sur MgSO₄. Après filtration, le solvant est évaporé et le résidu est purifié sur gel de silice (éluant gradient 0 à 50 % AcOEt/100 à 50 % PE). Le produit est obtenu sous forme d'un solide jaune (110 mg, 85 %). R_{f} (PE/AcOEt 50:50): 0.40. ¹H RMN (400 MHz, CDCl₃) δ 8.64 (s, 1H), 8.44 (m, 1H), 7.84 (m, 1H), 7.39 (br d, *J* = 3.0 Hz, 1H, NH), 7.33 (m, 1H), 7.16 (m, 3H), 5.62 (m, 1H), 2.84 (m, 2H), 2.49 (s, 3H), 2.19 (m, 1H), 2.01 (m, 1H), 1.95 (m, 2H). ¹³C RMN (101 MHz, CDCl₃) δ 159.1, 156.7, 149.8, 144.4, 138.2, 137.8, 136.6, 134.7, 130.0, 129.3, 128.9, 127.5, 126.4, 48.6, 29.8, 29.4, 20.1, 19.0. IR (ATR diamant, cm⁻¹) n 3386, 2926, 1577, 1529, 1312, 882, 684. HRMS (EI-MS) : m/z calculée pour C₁₈H₁₉N₄ [M+H]⁺: 291.1604, trouvée: 291.1602. T_{f}: 129-131 °C.

### Exemple 101 : 4-[[(1S)-Tetralin-1-yl]amino]pyrido[3,2-d]pyrimidin-6-ol (151)

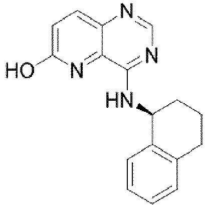

Dans un tube scellé, le composé **133** (50 mg, 0.16 mmol) est dissous dans 5 mL de DMSO puis le TBAF (1 mL, 1 M in THF) est ajouté. Le tube est chauffé au micro-onde à 120 °C pendant 1 h. En fin de réaction, la solution est refroidie jusqu'à température ambiante et 30 mL d'acétate d'éthyle est ajoutée. La solution est lavée avec 20 mL d'eau puis 20 mL d'une solution aqueuse de NaCl saturée puis enfin séchée avec MgSO₄. Après filtration, le solvant est évaporé pour obtenir le produit sous forme d'un solide jaune (35 mg, 74 %). R_{f} (PE/AcOEt 50:50): 0.25. ¹H RMN (400 MHz, CDCl₃) δ 8.51 (s, 1H), 7.76 (d, *J* = 8.0 Hz, 1H), 7.49 (br d, *J* = 7.0 Hz, 1H, NH), 7.22 (m, 3H), 7.08 (m, 1H), 6.19 (d, *J =* 8.0 Hz, 1H), 5.56 (m, 1H), 2.89 (m, 2H), 2.10 (m, 3H), 1.89 (m, 1H). ¹³C RMN (101 MHz, CDCl₃) δ 163.8, 153.5, 151.0, 142.8, 138.6, 138.0, 136.3, 129.6, 129.2, 127.5, 126.6, 126.2, 119.7, 49.2, 29.4, 29.2, 19.4. IR (ATR diamant, cm⁻¹) n 3360, 3022, 2931, 2854, 1633, 1591, 1439, 1102, 907, 848, 748. HRMS (EI-MS) : m/z calculée pour C₁₇H₁₇N₄O [M+H]⁺: 293.1396, trouvée: 293.1397. T_{f}: 253-255 °C.

### Exemple 102 : 4-(Pyrido[3,2-d]pyrimidin-4-ylamino)tetralin-1-one (152)

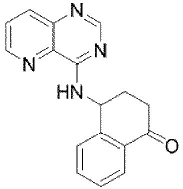

Le composé **142** (50 mg, 0.17 mmol) est dissous dans 7 mL de CH₂Cl₂ puis le Dess-Martin periodinane (110 mg, 0.26 mmol, 1.5 eq.) est ajouté. La solution est agitée à température ambiante pendant 2 h. En fin de réaction, la solution est diluée avec 10 mL d'une solution aqueuse de NaHCO₃ saturée et 10 mL d'une solution aqueuse de Na₂S₂O₃ saturée et le produit est extrait avec 30 mL de CH₂Cl₂. La phase organique est séchée avec MgSO₄ et après filtration, le solvant est évaporé. Le produit est purifié par chromatographie sur gel de silice (éluant CH₂Cl₂/MeOH 95:5). Le produit est obtenu sous forme d'un solide blanc (42 mg, 84 %). R_{f} (PE/AcOEt 20:80): 0.23. ¹H RMN (400 MHz, CDCl₃) δ 8.70 (dd, *J =* 4.0, 2.0 Hz, 1H), 8.69 (s, 1H), 8.13 (dd, *J* = 8.0, 2.0 Hz, 1H), 7.69 (dd, *J* = 8.0, 4.0 Hz, 1H), 7.54 (m, 1H), 7.45 (m, 3H), 5.88 (td, *J* = 49.0, 4.0 Hz, 1H), 2.92 (m, 1H), 2.80 (m, 1H), 2.57 (m, 1H), 2.39 (m, 1H). ¹³C RMN (101 MHz, CDCl₃) δ 196.9, 159.7, 156.4, 148.6, 144.7, 143.3, 136.2, 134.3, 132.3, 131.9, 128.5, 128.1, 127.7, 127.4, 48.7, 36.6, 29.7. IR (ATR diamant, cm⁻¹) n 3375, 2872, 1677, 1577, 1356, 1146, 776, 683. HRMS (EI-MS) : m/z calculée pour C₁₇H₁₅N₄O [M+H]⁺: 291.1240, trouvée: 291.1239. T_{f}: 158-160 °C.

### Exemple 103 : N-(6-Fluorotetralin-1-yl)pyrido[3,2-d]pyrimidin-4-amine (157)

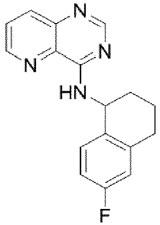

Le composé **10** (150 mg, 0.91 mmol, 1.2 eq.) est dissous dans 5 mL de THF anhydre pour donner une solution jaune à laquelle **154** (90 mg, 0.54 mmol, 1.0 eq.) est ajouté. La solution devient trouble. La triéthylamine (0.16 mL, 1.1 mmol, 2.0 eq.) est ajoutée et la solution reste à température ambiante pendant 2 à 12 heures sous agitation vigoureuse. La disparition du réactif de départ est suivie par CCM puis, en fin de réaction, le THF est évaporé. Le dépôt obtenu est repris dans 3 x 20 mL d'acétate d'éthyle puis le produit est lavé avec 30 mL d'une solution aqueuse saturée de NaHCO₃. La phase organique est lavée avec une solution aqueuse de NaCl saturée (40 mL) et enfin séchée sur MgSO₄. Après filtration, le solvant est évaporé et le résidu est purifié sur gel de silice (éluant gradient 0 à 60 % AcOEt/100 à 40 % PE). Le produit est obtenu sous forme d'un solide jaune (132 mg, 83 %). R_{f} (PE/AcOEt 50:50): 0.31. ¹H RMN (400 MHz, CDCl₃) δ 8.66 (s, 1H), 8.61 (dd, *J =* 4.0, 2.0 Hz, 1H), 8.08 (dd, *J* = 8.0, 2.0 Hz, 1H), 7.62 (dd, *J* = 8.0, 4.0 Hz, 1H), 7.40 (br d, *J* = 7.0 Hz, 1H, NH), 7.29 (m, 1H), 6.82 (m, 2H), 5.58 (m, 1H), 2.82 (m, 2H), 2.16 (m, 1H), 2.03 (m, 1H), 1.92 (m, 2H). ¹³C RMN (101 MHz, CDCl₃) δ 162.0 (d, *J =* 246.0 Hz), 159.1, 156.5, 148.2, 144.5, 140.0 (d, *J =* 7.0 Hz), 135.9, 132.3(d, *J* = 3.0 Hz), 132.0, 130.7 (d, *J =* 8.0 Hz), 127.8, 115.4 (d, *J =* 21.0 Hz), 113.6 (d, *J =* 20.0 Hz), 48.1, 29.7, 29.53 (d, *J =* 2.0 Hz), 19.8. RMN ¹⁹F (376 MHz, CDCl₃) δ -115.1 (1F). IR (ATR diamant, cm⁻¹) n 3400, 2954, 2928, 1576, 1498, 1356, 1302, 1134, 870, 841, 798, 682. HRMS (EI-MS) : m/z calculée pour C₁₇H₁₆FN₄ [M+H]⁺: 295.1354, trouvée: 295.1353. T_{f}: 135-137 °C.

### Exemple 104 : 7-Fluoro-N-[(1S)-tetralin-1-yl]pyrido[3,2-d]pyrimidin-4-amine (158)

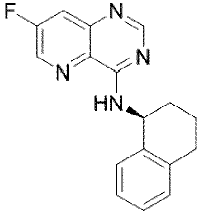

Le composé **155** (5 mg, 0,027 mmol, 1 eq.) est dissous dans 5 mL de THF anhydre et de la (*S*)-(+)-1,2,3,4-tetrahydro-1-naphthylamine (0.005 mL, 0.033 mmol, 1.2 eq.) mais aussi de la triethylamine (0.005 mL, 0.033 mmol, 1.2 eq.) sont ajoutées. La solution est agitée pendant une nuit à température ambiante. La disparition du réactif de départ est suivie par CCM puis, en fin de réaction, le THF est évaporé. Le dépôt obtenu est repris dans 30 mL d'une solution aqueuse de NaHCO₃ saturée puis le produit est extrait avec 3 x 20 mL d'acétate d'éthyle. Les phases organiques sont combinées et lavées avec une solution aqueuse de NaCl saturée (20 mL) et enfin séchées sur MgSO₄. Après filtration, le solvant est évaporé et le résidu est purifié sur gel de silice (éluant gradient 0 à 40 % AcOEt/100 à 60 % PE). Le produit est obtenu sous forme d'un solide blanc (5,6 mg, 70 %). R_{f} (PE/AcOEt 50:50): 0.49. ¹H RMN (400 MHz, CDCl₃) δ 8.66 (s, 1H), 8.51 (d, *J* = 3.0 Hz, 1H), 7.73 (dd, *J* =9.0, 3.0 Hz, 1H), 7.36 (br d, *J* = 7.0 Hz, 1H, NH), 7.33 (m, 1H), 7.18 (m, 3H), 5.63 (m, 1H), 2.88 (m, 2H), 2.21 (m, 1H), 2.00 (m, 1H), 1.97(m, 2H). ¹³C RMN (101 MHz, CDCl₃) δ 161.3 (d, *J* = 263.0 Hz), 158.9, 157.8, 146.0 (d, *J* = 9.0 Hz), 139.0, 138.7, 137.9, 136.4, 129.2 (d, *J* = 56.0 Hz), 129.0 (d, *J* = 2.0 Hz), 127.7, 126.5, 119.4 (d, *J* = 16.0 Hz), 48.8, 29.8, 29.4, 20.2. RMN ¹⁹F (376 MHz, CDCl₃) δ - 118,8 (1F). IR (ATR diamant, cm⁻¹) n 3294, 3052, 2922, 2852, 1615, 1527, 1439, 1269, 1119, 942, 890, 748, 674, 557. HRMS (EI-MS) : m/z calculée pour C₁₇H₁₆FN₄ [M+H]⁺: 295.1353, trouvée: 295.1354. T_{f}: 108-110 °C.

### Exemple 105 : N-Tetralin-1-ylbenzofuro[3,2-d]pyrimidin-4-amine (159)

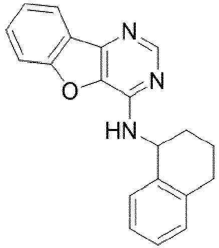

Le composé **156** (50 mg, 0.25 mmol, 1.0 eq.) est dissous dans 5 mL de THF anhydre et de la 1,2,3,4-tetrahydro-1-naphthylamine (0.042 mL, 0.3 mmol, 1.2 eq.) mais aussi de la triéthylamine (0.041 mL, 0.3 mmol, 1.2 eq.) sont ajoutées. La solution est agitée pendant 12 h à température ambiante. La disparition du réactif de départ est suivie par CCM puis, en fin de réaction, le THF est évaporé. Le dépôt obtenu est repris dans 30 mL d'une solution aqueuse de NaHCO₃ saturée puis le produit est extrait avec 3 x 20 mL d'acétate d'éthyle. Les phases organiques sont combinées et lavées avec une solution aqueuse de NaCl saturée (20 mL) et enfin séchées sur MgSO₄. Après filtration, le solvant est évaporé et le résidu est purifié sur gel de silice (éluant gradient 0 à 30 % AcOEt/100 à 70 % PE). Le produit est obtenu sous forme d'un solide orange (60 mg, 78 %). R_{f} (PE/AcOEt 40:60): 0.42. ' H RMN (400 MHz, CDCl₃) δ 8.66 (s, 1H), 8.16 (dt, *J* = 8.0, 1.0 Hz, 1H), 7.56 (m, 2H), 7.41 (m, 2H), 7.20 (m, 3H), 5.68 (m, 1H), 5.60 (br d, *J* = 8.0 Hz, 1H, NH), 2.87 (m, 2H), 2.21 (m, 1H), 2.08 (m, 1H), 1.95 (m, 2H). ¹³C RMN (101 MHz, CDCl₃) δ 156.5, 153.9, 148.8, 145.9, 137.9, 136.8, 135.0, 129.9, 129.4, 129.1, 127.7, 126.5, 123.9, 121.8, 112.5, 48.7, 30.2, 29.4, 19.9. IR (ATR diamant, cm⁻¹) n 3230, 3026, 2932, 1602, 1498, 1400, 1158, 1099, 988, 741. HRMS (EI-MS) : m/z calculée pour C₂₀H₁₈N₃O [M+H]⁺: 316.1444, trouvée: 316.1445. T_{f}: 152-154 °C.

### Exemple 106 : tert-Butyl N-[2-[[4-[[(1S)-tetralin-1-yl]amino]pyrido[3,2-d]pyrimidin-7-yl]amino]ethyl]carbamate (160)

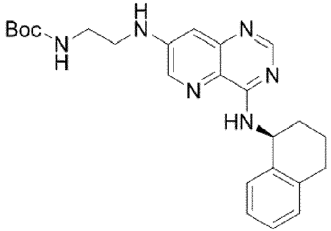

Dans un tube scellé de 10 mL, le composé **135** (120 mg, 0.34 mmol, 1.0 eq.) est dissous dans 2.5 mL de toluène et 2.5 mL de dioxane avant d'ajouter successivement la *N*-bocéthylènediamine (0.11 mL, 0.68 mmol, 2.0 eq.), le Cs₂CO₃ (221 mg, 0.68 mmol, 2.0 eq.), le xantphos (40 mg, 0.06 mmol, 0.2 eq.) et le Pd₂(dba)₃ (31 mg, 0.034 mmol, 0.1 eq.). La réaction est réalisée aux micro-ondes à 130 °C sous agitation vigoureuse pendant une heure. En fin de réaction, la disparition du composé bromé de départ est vérifiée par CCM puis la solution est diluée avec 5 mL d'acétate d'éthyle puis filtrée sur célite. Après la filtration, le solvant est évaporé et le résidu est purifié sur gel de silice (gradient éluent 0 à 90 % AcOEt/100 à 10 % PE). Le produit est obtenu sous forme d'un solide orange (105 mg, 72 %). R_{f} (PE/AcOEt 10:90): 0.24. ¹H RMN (400 MHz, CDCl₃) δ 8.52 (s, 1H), 8.04 (d, *J* = 3.0 Hz, 1H), 7.33 (m, 1H), 7.12 (m, 4H), 6.87 (m, 1H), 5.57 (m, 1H), 5.27 (br s, 1H, NH), 5.08 (br s, 1H, NH), 3.44 (m, 2H), 3.30 (m, 2H), 2.83 (m, 2H), 2.18 (m, 1H), 1.97 (m, 1H), 1.95 (m, 2H), 1.43 (s, 9H). ¹³C RMN (101 MHz, CDCl₃) δ 158.7, 157.3, 156.9, 147.6, 146.3, 139.8, 137.8, 137.0, 129.3, 129.0, 127.4, 126.3, 123.4, 108.7, 80.2, 48.4, 44.9, 39.6, 30.0, 29.4, 28.5, 20.2. IR (ATR diamant, cm⁻¹): 2929, 1689, 1575, 1515, 1330, 1163, 731. HRMS (EI-MS) : m/z calculée pour C₂₄H₃₁N₆O₂ [M+H]⁺: 435.2503, trouvée: 435.2502. T_{f} : 87-89 °C.

### Exemple 107 : N7-(2-Aminoethyl)-N4-[(1S)-tetralin-1-yl]pyrido[3,2-d]pyrimidine-4,7-diamine (161)

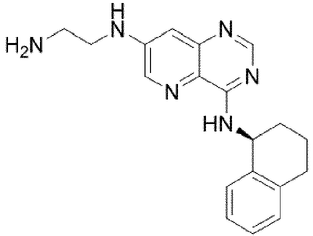

Le composé 160 (80 mg, 0.18 mmol) est dissous dans 4 mL de CH₂Cl₂ puis 1 mL de TFA est ajouté dans le ballon. Après une nuit à température ambiante sous agitation vigoureuse, quelques millilitres d'une solution de NaOH 2 M sont ajoutés et le mélange est agité à température ambiante pendant 1 heure. Le produit est extrait avec 3 x 15 mL de AcOEt et les phases organiques sont combinées et séchées avec MgSO₄. Après filtration et évaporation, le résidu est purifié par chromatographie sur gel de silice (gradient 0 à 100 % AcOEt/100 à 0 %PE). Le produit est obtenu sous forme d'un solide blanc (22 mg, 36 %). Rf (PE/AcOEt 70:30): 0.15. ¹H RMN (400 MHz, CDCl₃) δ 8.31 (s, 1H), 8.18 (d, *J* = 3.0 Hz, 1H), 7.23 (d, *J* = 8.0 Hz, 1H), 7.11 (m, 3H), 6.81 (d, *J* = 3.0 Hz, 1H), 5.51 (t, *J* = 6.0 Hz, 1H), 3.28 (t, *J* = 6 .0 Hz, 2H), 2.91 (t, *J* = 6.0 Hz, 2H), 2.81 (m, 2H), 2.13 (m, 1H), 1.95 (m, 2H), 1.86 (m, 1H). ¹³CRMN (101 MHz, CDCl₃) δ 159.9, 156.9, 150.2, 147.0, 141.8, 138.7, 137.8, 130.2, 129.3, 128.3, 127.1, 123.2, 107.3, 49.9, 46.3, 41.0, 31.0, 30.2, 21.5 HRMS (+ESI) : m/z calculée pour C₁₉H₂₃N₆ [M+H]+: 335.1979, trouvée: 335.1977.

### Exemple 108 : 6-Hexoxy-N-[(1S)-tetralin-1-yl]pyrido[3,2-d]pyrimidin-4-amine (162)

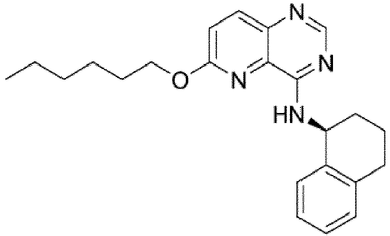

Le composé **133** (100 mg, 0.32 mmol, 1.0 eq.) est dissous dans 1 mL de n-hexanol puis le Pd2(dba)3 (1.4 mg, 0.002 mmol, 0.02 eq.), le KOH (27 mg, 0.28 mmol, 2.0 eq.) et le Bippyphos (3.2 mg, 0.006 mmol, 0.02 eq.) sont ajoutés successivement dans le ballon. Le mélange est chauffé à 100 °C pendant 4 h. Puis, le milieu réactionnel est dilué avec 20 mL d'eau et le produit est extrait avec 3 x 20 mL de CH2Cl2. Les phases organiques sont combinées puis séchées avec le MgSO4 et le solvant est évaporé après filtration. Le résidu est purifié par chromatographie sur gel de silice (gradient 0 à 100 % AcOEt/100 à 0 % PE). Le produit est obtenu sous forme d'une huile orangée (61 mg, 51 %). Rf (PE/AcOEt 50:50): 0.11.¹H RMN (400 MHz, CDCl₃) δ 8.61 (s, 1H), 8.00 (d, *J* = 9.0 Hz, 1H), 7.43 - 7.36 (m, 1H), 7.29 - 7.12 (m, 4H), 6.93 (d, *J* = 9.0 Hz, 1H), 5.73 - 5.62 (m, 1H), 4.31 (t, *J* = 6.6 Hz, 2H), 3.02 - 2.82 (m, 2H), 2.37 - 2.23 (m, 1H), 2.08 - 1.90 (m, 3H), 1.85 - 1.73 (m, 2H), 1.51 - 1.14 (m, 9H). ¹³C RMN (101 MHz, CDCl3) δ 161.4, 158.2, 154.0, 141.2, 138.9, 137.7, 137.2, 129.2, 128.4, 128.3, 127.3, 126.3, 119.3, 77.3, 77.2, 77.0, 76.7, 66.8, 48.5, 31.5, 30.2, 29.7, 29.4, 29.3, 28.6, 25.7, 22.5, 20.5, 13.9. IR (ATR diamant, cm⁻¹) : 3401, 2927, 2857, 1613, 1582, 1526, 1486, 1405, 1348, 1265, 1103, 841, 745. HRMS (+ESI) : m/z calculée pour C23H29N4O [M+H]+ : 377.2336, trouvée: 377.2335.

### Exemple 109 : tert-Butyl N-[2-[2-[4-(tetralin-1-ylamino)pyrido[3,2-d]pyrimidin-6-yl]oxyethoxy]ethyl]carbamate (163)

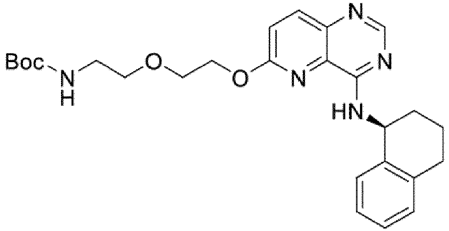

Le composé **133** (110 mg, 0.35 mmol, 1.0 eq.) est dissous dans 0.5 mL de N-boc-2-(2-hydroxyethoxy)-ethylamine puis le Pd2(dba)3 (7.3 mg, 0.008 mmol, 0.02 eq.), le KOH (40 mg, 0.7 mmol, 2.0 éq.) et le Bippyphos (3.5 mg, 0.007 mmol, 0.02 eq.) sont ajoutés successivement dans le ballon. Le mélange est chauffé à 100 °C pendant 4 h. Puis, le milieu réactionnel est dilué avec 20 mL d'eau et le produit est extrait avec 3 x 20 mL de CH₂Cl₂. Les phases organiques sont combinées puis séchées avec le MgSO4 et le solvant est évaporé après filtration. Le résidu est purifié par chromatographie sur gel de silice (gradient 0 à 40 % AcOEt/100 à 60 % PE). Le produit est obtenu sous forme d'une huile incolore (153 mg, 90 %). Rf (PE/AcOEt 50:50): 0.62. ¹H RMN 1H (400 MHz, CDCl₃) δ 8.62 (s, 1H), 8.03 (d, *J* = 9.0 Hz, 1H), 7.38 (m, 1H), 7.21 (m, 4H), 6.88 (d, *J* = 9.0 Hz, 1H), 5.68 (q, *J* = 7.2, 6.1, 6.1 Hz, 1H), 4.93 (s, 1H), 4.47 (m, 2H), 3.83 (m, 2H), 3.60 (m, 2H), 3.34 (q, *J* =5.4, 5.3, 5.3 Hz, 2H), 3.91 (d, *J* = 7.6 Hz, 2H), 2.29 (m, 1H), 1.98 (m, 2H), 1.45 (s, 9H), 1.23 (m, 2H).¹³C RMN (101 MHz, CDCl₃) 160.9, 158.1, 154.2, 141.5, 139.2, 137.7, 137.1, 129.2, 128.4, 127.3, 126.3, 119.2, 77.3, 77.2, 77.0, 76.6, 70.3, 68.9, 65.7, 48.5, 30.1, 29.3, 28.3, 20.5. HRMS (+ESI) : m/z calculée pour C₂₆H₃₄N₅O₄ [M+H]+: 480.2605, trouvée: 480.2605.

### Exemple 110 : 6-[2-(2-Aminoethoxy)ethoxy]-N-tetralin-1-yl-pyrido[3,2-d]pyrimidin-4-amine (164)

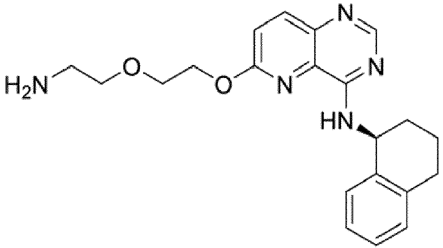

Le composé **163** (150 mg, 0.31 mmol) est dissous dans 4 mL de CH₂Cl₂ puis 1 mL de TFA est ajouté dans le ballon. Après une nuit à température ambiante sous agitation vigoureuse, quelques millilitres d'une solution de NaOH 2 M sont ajoutés et le mélange est agité à température ambiante pendant 1 heure. Puis le produit est extraire avec 3 x 15 mL de AcOEt et les phases organiques sont combinées et séchées avec MgSO₄. Après filtration et évaporation, le résidu est purifié par chromatographie sur gel de silice (gradient 0 à 60 % AcOEt/100 à 40% PE). Le produit est obtenu sous forme d'une huile incolore (42 mg, 36 %). **Rf** (PE/ AcOEt 50:50): 0.10.

### Exemple 111 : N-(5,6,7,8-tetrahydroquinolin-8-yl)pyrido[3,2-d]pyrimidin-4-amine (165)

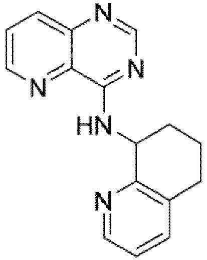

Le composé **165** est obtenu en suivant la procédure B avec la 5,6,7,8-tetrahydroquinolin-8-amine (70 mg, 0.47 mmol, 1.2 eq.), **10** (60 mg, 0.37 mmol, 1.0 eq.) et la triéthylamine (0.07 mL, 0.47 mmol, 1.2 eq.). Le résidu est purifié par chromatographie sur gel de silice (gradient 0 à 50 % AcOEt 100 à 50 % PE). Le produit est obtenu sous forme d'un solide gris (82 mg, 82 %). Rf (PE/AcOEt 50:50): 0.29. ¹H RMN (400 MHz, CDCl3) δ 8.69 (m, 2H), 7.49 (dd, *J* = 4.7, 1.6 Hz, 1H), 8.10 (dd, *J* = 8.5, 1.6 Hz, 1H), 8.01 (d, *J* = 6.8 Hz, 1H), 7.64 (dd, *J* = 8.4, 4.2 Hz, 1H), 7.49 (m, 1H), 7.17 (dd, *J* = 7.7, 4.6 Hz, 1H), 5.43 (m, 1H), 2.90 (m, 2H), 2.68 (m, 2H), 1.92 (m, 4H). ¹³C RMN (101 MHz, CDCl3) δ 159.6, 156.4, 155.2, 148.1, 147.5, 144.2, 137.1, 135.7, 133.1, 132.4, 127.5, 122.5, 76.7 51.5, 29.1, 28.4, 19.8. IR (Diamant Smart iTR, cm-1, neat): 3402, 2043, 2946, 1574, 1531, 1442, 1380, 1357, 1294, 804, 684. HRMS (+ESI) : m/z calculée pour C₁₆H₁₆N₅ [M+H]+: 278.1400, trouvée: 278.1398. Tf : 155-157 °C.

### PROPRIÉTÉS BIOLOGIQUES DES COMPOSÉS SELON L'INVENTION

### MATERIELS ET METHODES

### Lignées cellulaires utilisées par cribler les molécules sur l'activité des canaux SKCa (small conductance calcium activated potassium channel ; SK1, SK2 et SK3) et IKCa (intermediate conductance calcium activated potassium channel, SK4)

On a utilisé la lignée HEK 293 T, une lignée rénale foetale, qui a été immortalisée à l'aide de l'antigène T du virus simien SV40. Cette lignée cellulaire est largement utilisée du fait de la facilité avec laquelle ces cellules se transfectent à l'aide de plasmides ou autres vecteurs de transfection. Quatre lignées stables ont été utilisées pour les expériences de patch clamp : HEK-hSK1, HEK-rSK2, HEK-rSK3 et HEK-IKCa/SK4 qui surexpriment respectivement les canaux SK1, SK2, SK3 et SK4. Les lignées ont été obtenues par transduction stable (Girault et al., Current Cancer Drug Targets, 2011, 11, 1111-25) et sont celles déjà utilisées en routine pour tester les modulateurs de SK3 (Potier et al. Br J Pharmacol. 2011 Jan;162(2):464-79 ; Sevrain et al., Med. Chem. Commun., 2012, 3, 1471-1478; Sevrain et al., Organic & Biomolecular Chemistry, 2013 Jul 21;11(27):4479-87, Berthe W, Sevrain CM, Chantôme A, Bouchet AM, Gueguinou M, Fourbon Y, Potier-Cartereau M, Haelters JP, Couthon-Gourvès H, Vandier C, Jaffrès PA.ChemMedChem. 2016 Jul 19;11(14):1531-9). Un autre avantage de cette lignée réside dans le fait qu'elle n'exprime pas, de manière native, les canaux SKCa. Ces lignées nous ont donc permis d'étudier facilement l'effet des molécules sur le canal SK3 mais également leurs sélectivités à l'aide de la technique de patch clamp. L'activité inhibitrice des molécules non toxiques est testée sur l'activité du canal SK3. La sélectivité des molécules est déterminée en patch clamp sur les modèles cellulaires HEK exprimant de façon stable le canal SK3 mais également les canaux SK1, SK2, SK3 ou IKCa/SK4.

### Electrophysiologie/Patch-Clamp

Le patch clamp est une technique qui permet de mesurer l'activité de canaux ioniques en enregistrant les courants ioniques macroscopiques (configuration whole cell) ou microscopique (ex : configuration inside out) au travers de la membrane plasmique. Pour tester l'effet de molécules sur les canaux SKCa/IKCa, deux technologies complémentaires ont été utilisées : le patch clamp conventionnel et manuel et l'automate de patch clamp Nanion NPC-16 Patchliner Quattro (4 canaux) (Potier et al., Mol Cancer Ther 2006, 5, 2946-53 ; Chantome et al., Exp Cell Res 2009, 315, 3620-30 ; Potier et al., Biochem Biophys Res Commun 2010, 397, 42-7; Girault et al., Current Cancer Drug Targets, 2011, 11, 1111-25 ; Sevrain et al., Med. Chem. Commun., 2012, 3, 1471-1478; Sevrain et al., Organic & Biomolecular Chemistry, 2013 Jul 21;11(27):4479-87; Chantome et al., Cancer Research, 2013 Aug 1;73(15):4852-61, Gueguinou M, Crottés D, Chantôme A, Rapetti-Mauss R, Potier-Cartereau M, Clarysse L, Girault A, Fourbon Y, Jézéquel P, Guérin-Charbonnel C, Fromont G, Martin P, Pellissier B, Schiappa R, Chamorey E, Mignen O, Uguen A, Borgese F, Vandier C, Soriani O.Oncogene. 2017 Jan 23. doi: 10.1038/onc.2016.501, Berthe W, Sevrain CM, Chantôme A, Bouchet AM, Gueguinou M, Fourbon Y, Potier-Cartereau M, Haelters JP, Couthon-Gourvès H, Vandier C, Jaffrès PA. ChemMedChem. 2016 Jul 19;11(14):1531-9, Guéguinou M, Harnois T, Crottes D, Uguen A, Deliot N, Gambade A, Chantome A, Haelters JP, Jaffrès PA, Jourdan ML, Weber G, Soriani O, Bougnoux P, Mignen O, Bourmeyster N, Constantin B, Lecomte T, Vandier C, Potier-Cartereau M.Oncotarget. 2016 Jun 14;7(24):36168-36184. doi: 10.18632/oncotarget.8786).

Pour le patch clamp conventionnel la micropipette est remplie d'un milieu intrapipette ou MIP (voir composition après) et elle permettra la mesure des courants ioniques en même temps qu'elle permet le maintien du potentiel de membrane (voltage clamp). Le dispositif expérimental est constitué d'une table anti-vibration sur laquelle repose un microscope inversé (Nikon, Eclispe TE 300). La boite de Pétri (Corning incorporated, USA), contenant les cellules est posée sur une plaque de plexiglas fixée à la platine du microscope. La pipette est reliée à l'amplificateur (Axopatch 200B) comme l'électrode de référence qui permet de fermer le circuit électrique. L'amplificateur, qui assure la conversion courant-tension est lui-même relié à une carte analogique-numérique (Digidata 1322A, Axon Instruments, USA). Le contrôle du potentiel ainsi que l'enregistrement et le suivi des courants sont assistés par ordinateur et pilotés grâce au logiciel PClamp 8.1 (Axon Instruments, USA). Les pipettes sont réalisées en verre dur sur une étireuse verticale (PP-830, Narishige) et ont une résistance en générale comprise entre 3-5 M'Ω pour la configuration whole-cell. Pour la configuration whole cell, les cellules sont maintenues dans une solution physiologique saline ou PSS composé (en mM) : NaCl 140, KCI 4, MgCl₂ 1, CaCl₂ 2, D-Glc 11.1, HEPES 10. Le pH est ajusté à 7,4 avec du NaOH et la pipette de verre remplie de MIP composé (en mM) de K-glutamate 125, KCI 20, CaCl₂ 0.37, MgCl₂ 1, MgATP 1, EGTA 1, HEPES 10 et le pH est ajusté à 7,2 avec du KOH. Le milieu intrapipette (MIP) est composé d'une solution contenant (en mM) KCI 140, EGTA 5, HEPES 10 et MgCl₂ 1 (pH 7,4 / KOH). Plusieurs protocoles sont utilisés : i) un protocole de créneaux de potentiel successifs (pulse) de 10 mV (500 msec) qui permet de construire les relations courants-voltages (I/V) de -100 mV à +100 mV à partir d'un potentiel de holding (maintien) de -70 mV et un protocole de rampes permettant d'incrémenter le potentiel imposé de -100 mV à +100 mV en un temps donné (500 msec).

La technique de choix pour découvrir des molécules inhibitrices de l'activité de canaux ioniques est la technique de patch-clamp. Cependant, cette technique qui est manuelle prend du temps et ne permet que de faibles rendements. La robotisation du patch-clamp a révolutionné l'étude et le criblage des canaux ioniques, permettant d'envisager des essais avec un débit et un rendement beaucoup plus élevés. Il existe plusieurs robots de patch clamp dont le fonctionnement est basé sur l'utilisation de puces. Ces derniers appelés « planar » patch-clamp ou en 2D (plan) ont été développés par la société Nanion et en particulier le Patchliner qui consiste à remplacer la pipette de patch classiquement utilisée par une puce possédant un trou et mimant l'extrémité de la pipette de patch. C'est cet automate et plus précisément le NPC-16 Patchliner Quattro (4 canaux) qui est utilisé pour tester les molécules sur les canaux SK1/SK2/IKCa. L'activité du canal SK3 est testée à l'aide de la technique de patch clamp conventionnel.

### Toxicité in vivo aigüe et répétées.

Pour la toxicité aigüe, le composé **20** est préparé dans un mélange DMSO/PEG300/Eau (5/45/50) pour y être injecté par voie intra péritonéale à 10 ml/kg et pour des doses de 0, 1, 5, 10, 25 et 50 mg/kg à raison de 2 souris par groupe de dose. Tout au long de l'étude, les animaux sont pesés et sont observés quotidiennement pendant 12 jours. Après 12 jours, les animaux sont euthanasiés et une observation macroscopique des organes est effectuée.

Pour la toxicité répétée, l'étude se décompose en deux étapes sur une durée de quatre semaines. Le produit testé est administré par injection intrapéritonéale de souris NMRI femelles de 6 semaines non cancéreuses (Janvier, France). Les injections se font quotidiennes pendant 15 jours. A ce point, une partie des souris est sacrifiée afin d'observer les effets macroscopiques des composés sur les organes internes. Le groupe restant est conservé à l'animalerie pour les deux semaines suivantes. Pendant cette période, le comportement des souris est contrôlé. A la fin de cette deuxième étape, le sacrifice nous permet également de contrôler les effets des molécules sur les organes internes et de tester l'aspect réversible ou non de la toxicité éventuellement détectée.

Six groupes de 4 souris ont été établis et le composé **20** a été préparé dans un mélange DMSO/PEG300/Eau (5/45/50) à 10 ml/kg :
- Groupe **20** 5 : groupe traité par injection intrapéritonéale du composé **20** à 5 mg/kg, 5 fois par semaine pendant 15 jours. La dose totale injectée est de 50 mg/kg (5 x 10 jours).
- Groupe **20** 7.5 : groupe traité par injection intrapéritonéale du composé **20** à 7.5 mg/kg, 5 fois par semaine pendant 15 jours. La dose totale injectée est de 75 mg/kg (7.5 x 10 jours).
- Groupe **20** 12.5 : groupe traité par injection intrapéritonéale du composé **20** à 12.5 mg/kg, 5 fois par semaine pendant 15 jours. La dose totale injectée est de 125 mg/kg (12.5 x 10 jours).
- Groupe **20** 17.5 : groupe traité par injection intrapéritonéale du composé **20** à 17.5 mg/kg, 5 fois par semaine pendant 15 jours. La dose totale injectée est de 175 mg/kg (17.5 x 10 jours).
- Groupe **20** 25 : groupe traité par injection intrapéritonéale du composé **20** à 25 mg/kg, 5 fois par semaine pendant 15 jours. La dose totale injectée est de 250 mg/kg (25 x 10 jours).
- Groupe Témoin : groupe recevant des injections intrapéritonéales de DMSO/PEG300/Eau à 10 ml/kg, 5 fois par semaine pendant 15 jours.

A la fin des 2 semaines, des prélèvements sanguins sont effectués sur la moitié des animaux et des observations macroscopiques des organes sont réalisées après euthanasie. Après les 4 semaines, les animaux restants sont euthanasiés et une observation macroscopique des organes est effectuée.

### Modèle murin in vivo de xénogreffe orthotopique : test de la capacité des molécules à réduire le développement tumoral

Ce modèle permet l'étude d'un cancer métastatique dans lequel il y a développement de la tumeur primaire et formation des métastases en parallèle du développement de la tumeur primaire. Pour réaliser ce test, des souris NMRI nude femelles âgées de quatre semaines sont utilisées. En préparation de la greffe, le bourgeon mammaire des souris est cautérisé à l'aide du bistouri électrique puis 2 millions de cellules exprimant le canal SK3 et la luciférase (MDA-MB-435s-Luc) y sont implantées. La luciférase permet de suivre *in vivo* le développement tumoral par imagerie optique (Girault et al., Current Cancer Drug Targets, 2011, 11, 1111-25 ; Chantome et al., Cancer Research, 2013 Aug 1;73(15):4852-61).

Deux groupes de souris ont été établis et le composé **20** est préparé dans un mélange DMSO/PEG300/eau (5/45/50) à 10 ml/kg :
- Groupe **20** 1 : groupe traité par injection intrapéritonéale du composé **20** à 1 mg/kg, trois fois par semaine pendant 14 semaines ; et
- Groupe Témoin : groupe recevant des injections avec la dilution équivalente de véhicule (DMSO/éthanol), trois fois par semaine pendant 15 semaines.

Le développement de la tumeur primaire a été suivi de deux manières : la mesure de la taille au pied à coulisse (longueur x largeur x épaisseur) et la mesure de la bioluminescence au bio-imageur.

Lorsque la tumeur primaire atteint la taille de 900 mm³, une exérèse totale est réalisée afin de se rapprocher de la situation clinique et de s'affranchir de cette variable qui pourrait influencer le développement des métastases. L'expérimentation s'est poursuivie avec les injections de composé **20** et le suivi du développement des métastases par des mesures de BLI sur le ventre puis sur le dos des souris.

Le poids des souris ainsi que les mesures de bioluminescence et de la taille de la tumeur ont été réalisées chaque semaine afin de suivre l'évolution de la tumeur et de ses métastases. Pour réaliser l'imagerie, les souris sont anesthésiées avec de l'isoflurane.

Après environ 15 semaines, les souris ont été sacrifiées. Une injection de luciférine a été administrée avant le sacrifice afin de pouvoir réaliser l'imagerie de l'animal entier puis pour chaque organe prélevé (os, foie, ganglion, cerveau, colon).

### RESULTATS

### Effet du composé 20 sur le développement de la tumeur primaire et de métastases.

Parmi les différentes variables recueillies, le poids des souris permet de vérifier l'absence de toxicité du composé **20** au cours du temps. Le poids n'a plus été pris en compte à partir de l'exérèse de la première tumeur afin de ne pas biaiser la moyenne. Nous avons obtenu une progression identique du poids des souris en fonction des groupes. A 10 semaines, il n'existe pas de différence de poids significative entre les deux groupes. Cela confirme sur les souris xénogreffées l'absence de toxicité apparente du composé **20** à 1 mg/kg.

Le volume et la BLI de la tumeur primaire ont été mesurés de façon hebdomadaire. La BLI reflète le nombre de cellules MDA-MB-435s au site d'implantation. Une croissance progressive des tumeurs primaires a été constatée dans les différents groupes avec les deux méthodes de suivi : BLI et pied à coulisse. Le volume tumoral de la semaine 7 a été mesuré par un opérateur différent d'où l'inflexion de la courbe. Le volume de la tumeur ne diffère pas selon le groupe au moment de l'exérèse de la première tumeur.

Après 15 semaines, les animaux sont euthanasiés et les métastases sont visualisées *ex vivo* dans différents organes.

L'ensemble des souris (Témoins et composé **20** à 1 mg/kg) a développé des métastases pulmonaires.

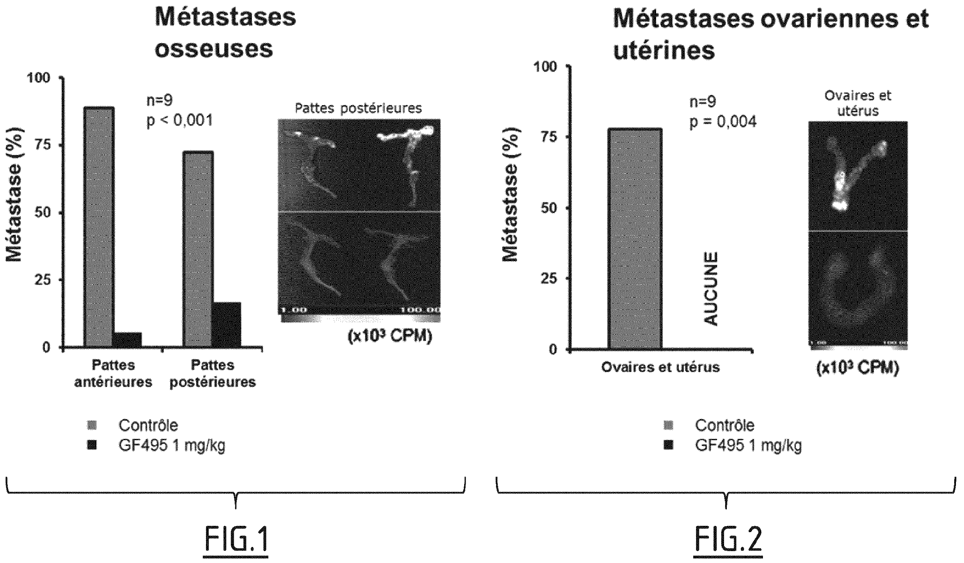

Cependant, le composé **20** à 1 mg/kg a réduit le nombre de métastases osseuses et a totalement aboli le développement de métastases ovariennes et utérines (Figures 1 et 2). Les Figures 1 et 2 représentent le pourcentage de métastases chez les souris témoins et chez les souris ayant reçu du composé **20** à 1 mg/kg.

| **Composé** | **% d'inhibition SK3** | **% d'inhibition SK2** |
|---|---|---|
| **NS8593** | **IC50** : 87nM ± 12 | ND |
| | Stroebaek et al, 2006 : 90 nM±8 | |
| | Jenkins et al. 2011 : 104nMi34 | |
| **4** | **10 µM** : 3.6% ± 3.6 ((N=2) | ND |
| **5** | **10 µM** : 7.7% ±2 (N=4) | ND |
| **79** | **10 µM** : 27% ± 3.9% (N=4) | ND |
| **7** | **10 µM** : 83.4±3.2 (N=8) | **10 µM** 95.6±1.2 (N=9) |
| | **100 nM** : 81.3 ± 1.0 (N=3) | **100 nM** : 91.5 ± 1.6 (N=9) |
| | **10 nM** : 56.1 ± 4.8 (N=3) | **10 nM** : 89.3 ± 3.2 (N=8) |
| | **1 nM** : 42.2 ± 6.4 (N=6) | **1 nM** : 35.0 ± 9.2 (N=7) |
| **6** | **10 µM** 2.65% ±4.8 (N=2) | ND |
| **47** | **10 µM** : 7.54 ± 10.1 (N=3) | ND |
| **14** | **10 µM** : 1.52 ± 8.81 (N=2) | **ND** |
| **18** | **10 µM** : 89.9 ± 3.1 (N=3) | **10 µM** : 97.05±1.08 (N=5) |
| | **100 nM** : 96.0 ± 0.9 (N=4) | **100 nM** : 92.9 ± 1.9 (N=5) |
| | **10 nM** : 59.7 ± 4.1 (N=3) | **10 nM** : 81.4 ± 7.8 (N=4) |
| | **1 nM** : 44.7 ± 5.5 (N=4) | **1 nM** : 36.7 ± 9.9 (N=3) |
| **20** | **10 µM** : 92.8 ±1.4 (N=8) | **10 µM** : 91.8 ±4.1 (N=5) |
| | **10 nM** : 45.5 ± 8.8 (N=3) | **100 nM** : 91.2 ±1.6 (N=10) |
| **30** | **10 µM** : 87.2 ±3.0 (N=7) | **10 µM** : 95.05 ±2.2 (N=6) |
| | **500 nM** : 52.9± 9.1 (N=5) | |
| | **50 nM** : 35.5 ± 6.7 (N=5) | |
| **32** | **500 nM** : 27.73 ± 6.13 (N=4) | ND |
| | **50 nM** : 23.57 ± 1.81 (N=4) | |
| **34** | **500 nM** : 40.4 ± 9.6 (N=4) | **10 µM** : 94.8±1.2 (N=7) |
| | **50 nM** : 32.9 ± 10.0 (N=5) | **100 nM** : 92.7 ± 4.8 (N=2) |
| **33** | **10 µM** : 71.5±7.4 (N=5) | |
| | **100 nM** : 53.2±10.9 (N=5) | ND |
| | **50 nM** : 30.5±6.9 (N=5) | |
| **35** | ND | **10 µM** : 91.4±2.3 (N=4) |
| | | **100 nM** : 83.7 ± 4.2 (N=2) |
| **38** | **10 µM** : 73.98 ± 2.93 | ND |
| | **100 nM** : 51.9 ± 4.39 | |
| | **50 nM** : 31.36 ± 6.09 | |
| **23** | **10 µM** : 8.4±5.5 (N=2) | **10 µM** : 1±22 (N=2) |
| **29** | **10 µM** : 46.0±2.5 (N=3) | **10 µM** : 16.2±12.7 (N=2) |
| **40** | **10 µM** : 82.2±2.2 (N=4) | **10 µM** : 88.1 (N=1) |
| | **100 nM** : 11.9 ±1.9 (N=2) | **100 nM** : 25.6 (N=1) |

| **Composé** | **% d'inhibition SK3** | **% d'inhibition SK2** |
|---|---|---|
| **53** | **10 µM** 62.2 ± 3.9 (N=5) | **50 nM** : 29.25 ± 2.68 (N=12) |
| | **100 nM** 41.9 ± 6.9 (N=8) | |
| | **50 nM** 29.2 ± 2.7 (N=12) | **100 nM** : 41.92 ± 6.99 (N=5) |
| | **500 nM** : 94.2 ± 1.4 (N=4) | **500 nM** : 43.17 ± 2.97 (N=8) |
| | **10 nM** : 42.6 ± 3.7 (N=3) | **10 µM** : 62.21 ± 3.95 (N=5) |
| | **1 nM** : 7.4 ± 3.0 (N=3) | |
| **62** | **50 nM** : 32.09 ± 2.3 (N=3) | ND |
| | **100 nM** : 46.84 ± 6.82 (N=3) | |
| | **10 µM** : 54.38 ± 4.85 (N=3) | |
| **67** | **100 nM** : 35.3 ± 9.4 (N=5) | ND |
| | **1 µM** : 82.4 ± 7.1 (N=4) | |
| | **500 nM** : 39.3 ± 10.4 (N=3) | |
| **49** | **500 nM** 75.8 ± 8.6 (N=5) | ND |
| | **250 nM** 83.1 ± 3.0 (N=4) | |
| | **100 nM** 37.1 ± 9.4 (N=5) | |
| **74** | **500 nM** : 48.55±8.8 (N=3) | ND |
| | **50 nM** : 18.45±2.45 (N=3) | |
| **75** | **10 µM** : 88.81±1.16 (N=2) | **10 µM** : 93.3 (N=1) |
| | **100 nM** : 85.42±6.35 (N=2) | |
| | **10 nM:** 92.43±0.78 (N=2) | **100 nM** : 80 (N=1) |
| **86** | **500 nM** : 18±2.3 (N=5) | ND |
| | **50 nM** : -9.01 ± 2.2 | |
| **87** | **500 nM** : 47.44±2.3 (N=3) | ND |
| | **50 nM** : 22.92±4.6 (N=3) | |
| **88** | **500 nM** : 54.81±8.43 (N=4) | ND |
| | **50 nM** : 25.75±11.01 (N=4) | |
| **90** | **500 nM** : 39.87±7.82 (N=4) | ND |
| | **50 nM** : 16.07±2.47(N=4) | |
| **93** | **50 nM** : 45.85 ± 11.2 | ND |
| | **100 nM :** 53.55 ± 9.92 | |
| | **10 µM** : 71.83 ± 7.37 | |
| **103** | **10 µM** : 83.83±6.71 (N=4) | ND |
| | **500 nM** : 46.91±6.45 (N=3) | |
| | **100 nM** :57.85±7.82 (N=3) | |
| | **50 nM** : 30.69±6.94 (N=6) | |
| **107** | **10 µM** : 54.74±6.82 (N=6) | ND |
| | **100 nM** : 46.78±7.7 (N=8) | |
| | **50 nM** : 29.74±5.5 (N=8) | |
| **133** | **50 nM** : 38.97 ± 4.68 (N=5) | ND |
| | **100 nM** : 56.34 ± 7.3 (N=4) | |
| | **10 µM** : 67.31 ± 10.04 (N=3) | |

| **Composé** | **% d'inhibition SK3** | **% d'inhibition SK2** |
|---|---|---|
| **142-E1** | **50 nM** : 35.27 ± 3.63 (N=5) | ND |
| | **100 nM** : 44.55±5.67 (N=5) | |
| | **10 µM** : 57.16 ± 5.51 (N=4) | |
| **142-E2** | **50 nM** : 35.72 ± 1.79 (N=4) | ND |
| | **100 nM** : 59.42 ± 3.43 (N=4) | |
| | **10 µM** : 75.11 ± 2.87 (N=4) | |
| **142-E3** | **50 nM** : 30.69 ± 3.07 (N=4) | ND |
| | **100 nM** : 49.71 ± 4.48 (N=4) | |
| | **10 µM** : 58.9 ± 6.08 (N=4) | |
| **142-E4** | **50 nM** : 21.9 ± 2.32 (N=3) | ND |
| | **100 nM** : 37.97 ± 3.99 (N=3) | |
| | **10 µM** : 48.28 ± 5.99 (N=3) | |
| **152** | **50 nM** : 24.42 ± 1.68 (N=3) | ND |
| | **100 nM** : 41.48 ±8.7 (N=3) | |
| | **10 µM** : 61.19 ± 8.1 (N=3) | |
| **157** | **50 nM** : 32.04 ± 1.39 (N=3) | ND |
| | **100 nM** : 51.13 ± 1.01 (N=3) | |
| | **10 µM** : 53.99 ± 2.19 (N=3) | |
| **158** | **50 nM** : 35.84 ± 5.97 (N=4) | ND |
| | **100 nM** : 64.14 ± 3.06 (N=4) | |
| | **10 µM** : 84.83 ± 5.56 (N=4) | |

## Revendications

1. Composé de formule (III-4) suivante : 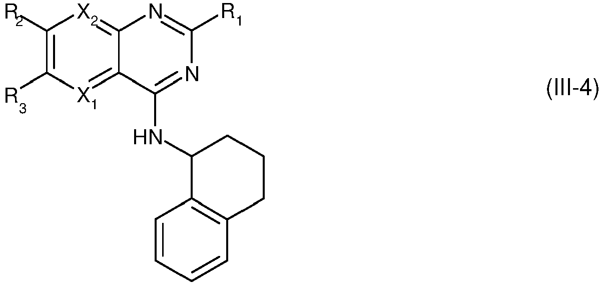 dans laquelle :
- R₁ représente H, un groupe (C₁-C₆)alkyle ou un radical choisi dans le groupe constitué de : -ORₐ, -NHRₐ, -NRₐR_{b}, -NH-C(=O)Rₐ, -C(=O)Rₐ, -C(=O)ORₐ, -NH-CN, -C(=O)NRₐR_{b}, Cl, F, CN et des hétérocycles azotés et/ou oxygénés, Rₐ et R_{b}, indépendamment l'un de l'autre, représentant H, un groupe (C₁-C₆)alkyle ou un groupe (C₃-C₆)cycloalkyle ;
- soit X₁ représente un atome d'azote et X₂ représente un groupe C(R₄),
- soit X₁ représente un groupe C(R₅) et X₂ représente un atome d'azote,
- soit X₁ et X₂ représentent un atome d'azote,
- R₄ et R₅, représentent, indépendamment les uns des autres, H ou un substituant choisi dans le groupe constitué des atomes d'halogène, des groupes (C₁-C₆)(cyclo)alkyles, des groupes NRₐR_{b} et des groupes ORₐ, Rₐ et R_{b}, indépendamment l'un de l'autre, représentant H ou un groupe (C₁-C₆)alkyle,
- R₂ et R₃ représentent, indépendamment les uns des autres, H ou un substituant choisi dans le groupe constitué des atomes d'halogène, des groupes (C₁-C₆)(cyclo)alkyles, des groupes NRₐR_{b} et des groupes ORₐ, Rₐ et R_{b}, indépendamment l'un de l'autre, représentant H ou un groupe (C₁-C₆)alkyle,
ainsi que ses sels pharmaceutiquement acceptables,
ledit composé de formule (III-4) étant sous forme de stéréoisomère pur ou sous forme de mélange d'énantiomères et/ou de diastéréoisomères, y compris de mélanges racémiques,
pour son utilisation pour le traitement des cancers.

2. Composé pour son utilisation selon la revendication 1, dans lequel R₃ est H.

3. Composé pour son utilisation selon la revendication 1, dans lequel R₂ et R₃ sont H.

4. Composé pour son utilisation selon l'une quelconque des revendications 1 à 3, ledit composé étant choisi parmi :

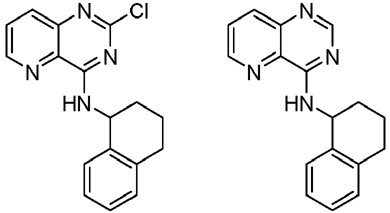

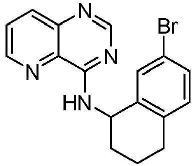

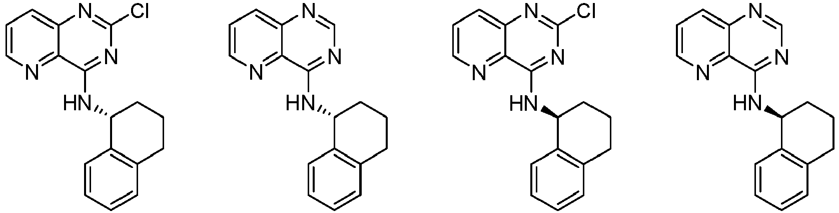

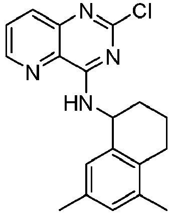

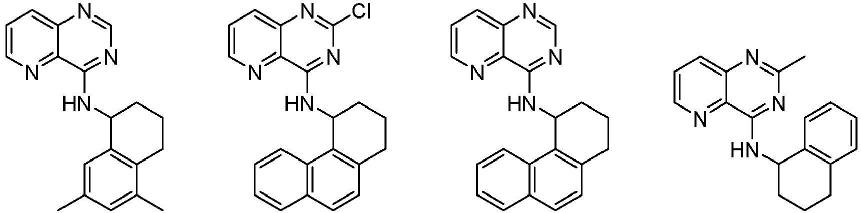

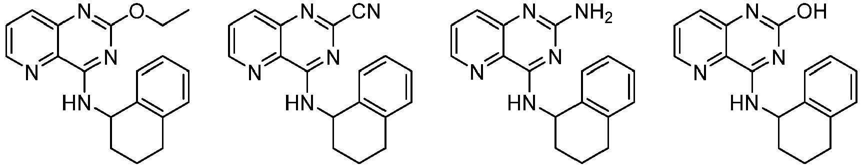

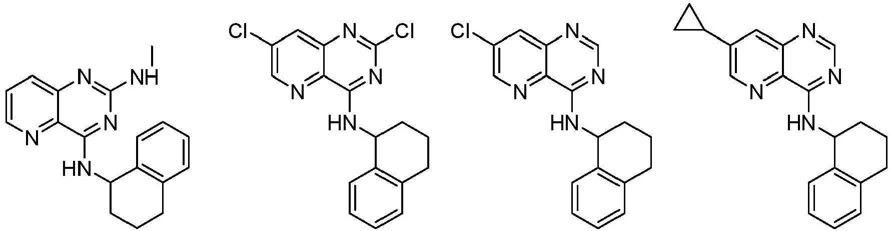

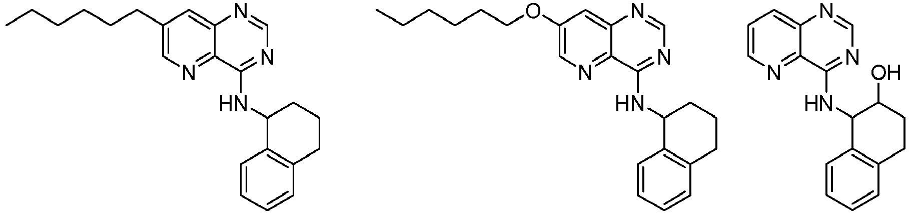

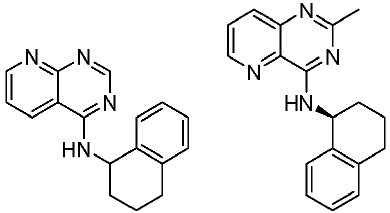

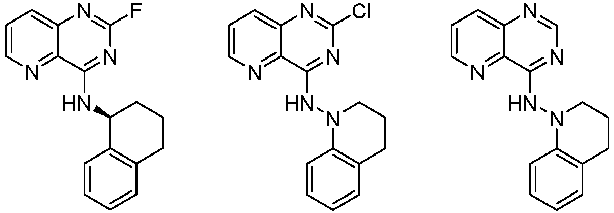

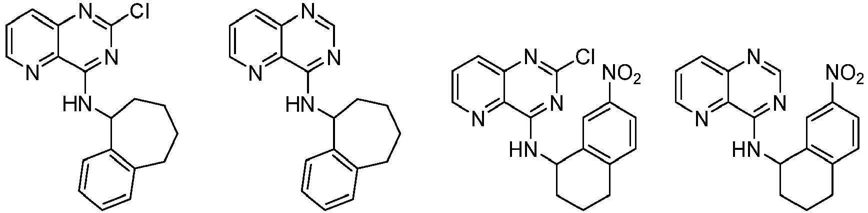

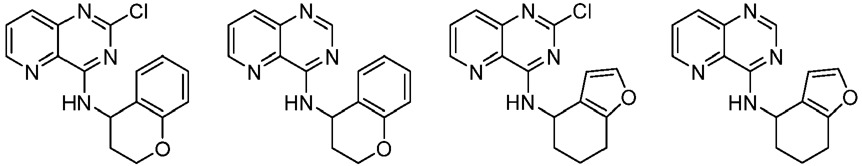

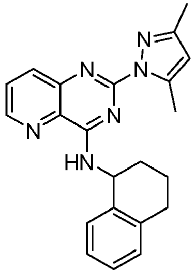

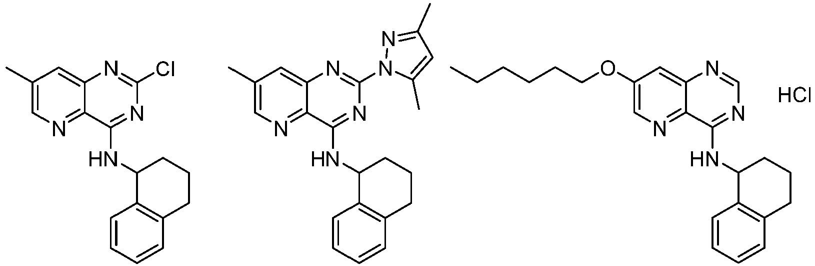

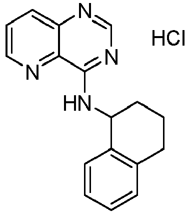

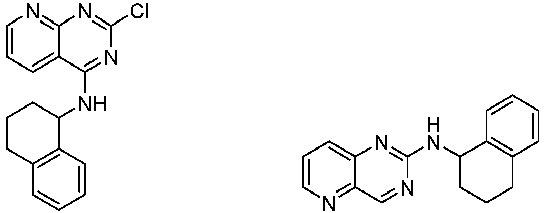

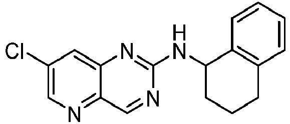

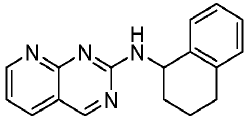

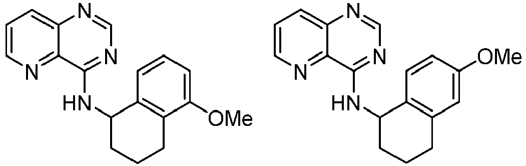

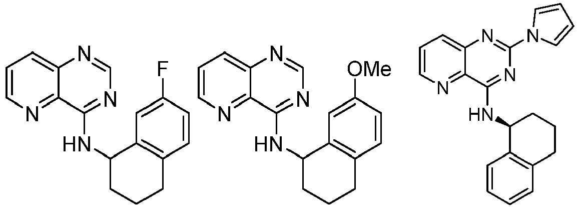

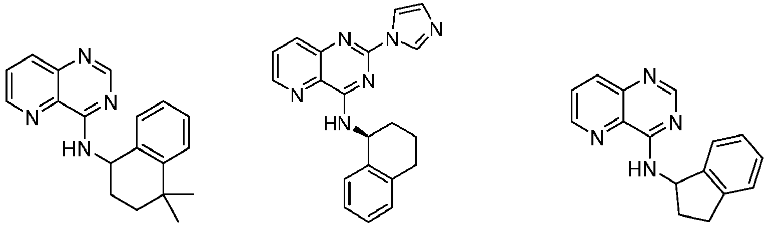

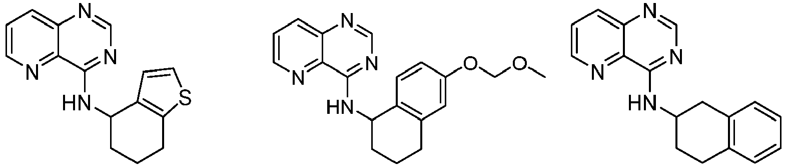

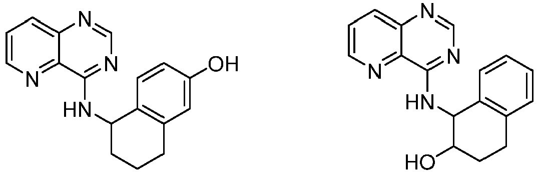

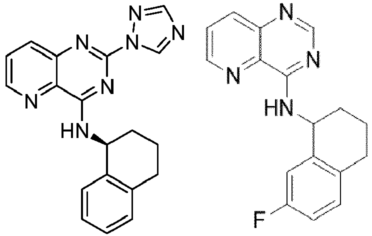

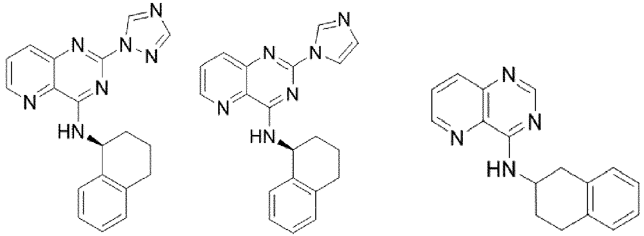

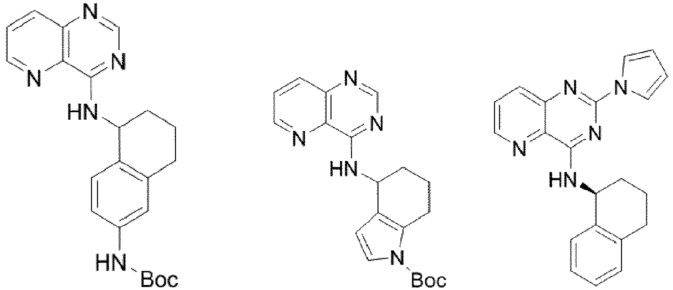

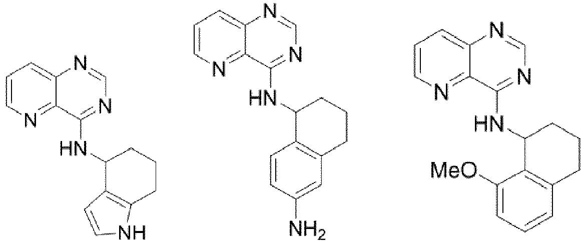

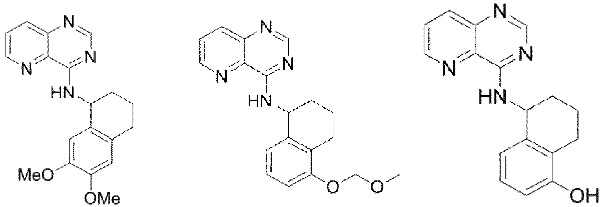

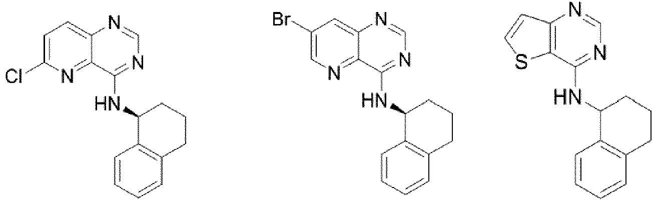

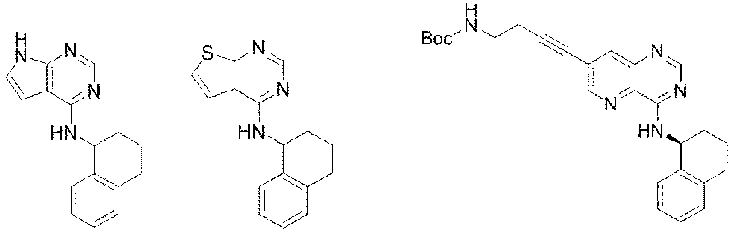

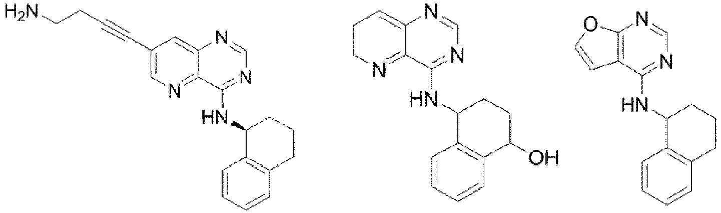

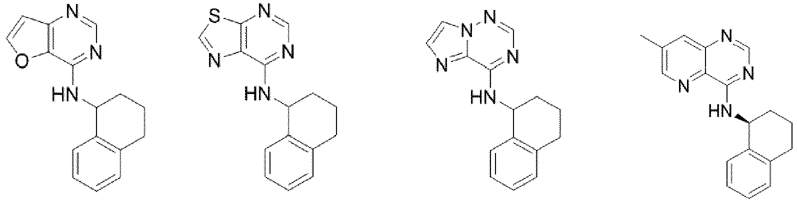

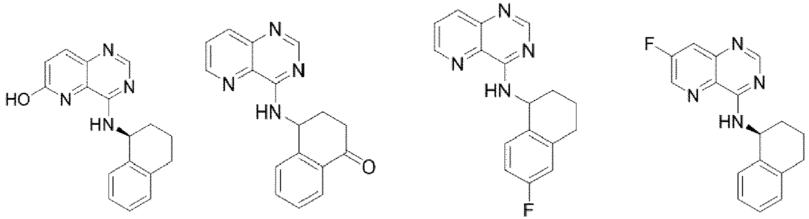

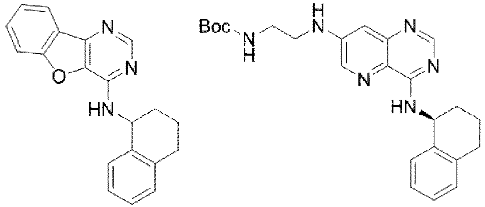

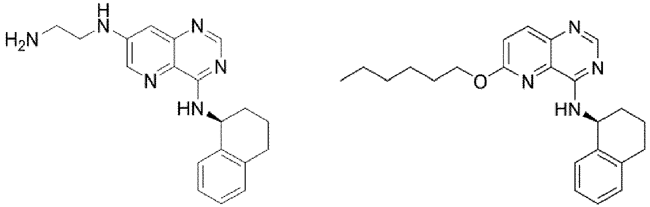

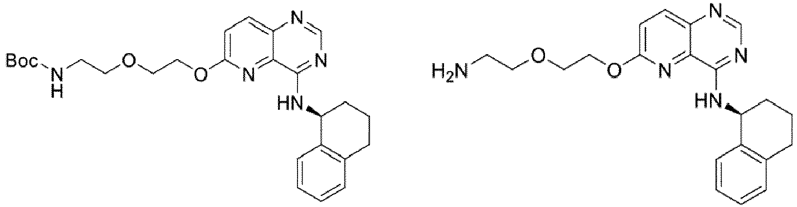

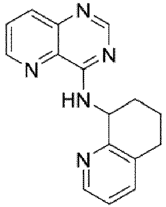

5. Composé pour son utilisation selon l'une quelconque des revendications 1 à 4, pour le traitement du cancer du sein.

6. Composé de formule (III-4) : 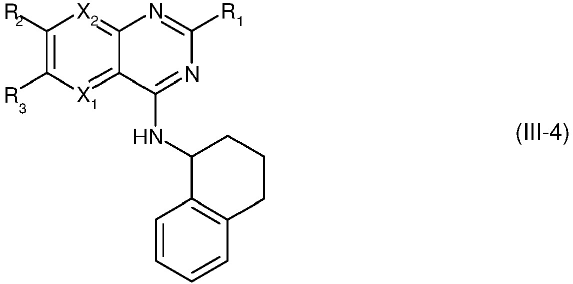 dans laquelle :
- soit X₁ représente un atome d'azote et X₂ représente un groupe C(R₄),
- soit X₁ représente un groupe C(R₅) et X₂ représente un atome d'azote,
- R₁ représente H, un groupe (C₁-C₆)alkyle ou un radical choisi dans le groupe constitué de : -ORₐ, -NHRₐ, -NRₐR_{b}, -NH-C(=O)Rₐ, -C(=O)Rₐ, -C(=O)ORₐ, -NH-CN, -C(=O)NRₐR_{b}, Cl, F, CN et des hétérocycles azotés et/ou oxygénés, Rₐ et R_{b}, indépendamment l'un de l'autre, représentant H, un groupe (C₁-C₆)alkyle ou un groupe (C₃-C₆)cycloalkyle ;
- R₂, R₃, R₄ et R₅, représentent, indépendamment les uns des autres, H ou un substituant choisi dans le groupe constitué des atomes d'halogène, des groupes (C₁-C₆)(cyclo)alkyles, des groupes NRₐR_{b} et des groupes ORₐ, Rₐ et R_{b}, indépendamment l'un de l'autre, représentant H ou un groupe (C₁-C₆)alkyle,
ainsi que ses sels pharmaceutiquement acceptables,
ledit composé de formule (III-4) étant sous forme de stéréoisomère pur ou sous forme de mélange d'énantiomères et/ou de diastéréoisomères, y compris de mélanges racémiques.

7. Composé de formule (VI-1) : 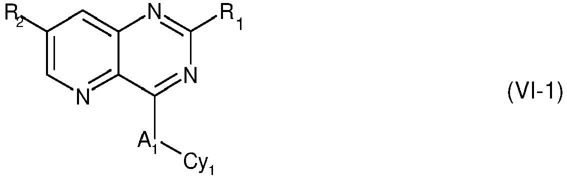 dans laquelle :
- R₁ représente H, un groupe (C₁-C₆)alkyle ou un radical choisi dans le groupe constitué de : -ORₐ, -NHRₐ, -NRₐR_{b}, -NH-C(=O)Rₐ, -C(=O)Rₐ, -C(=O)ORₐ, -NH-CN, -C(=O)NRₐR_{b}, Cl, F, CN et des hétérocycles azotés et/ou oxygénés, Rₐ et R_{b}, indépendamment l'un de l'autre, représentant H, un groupe (C₁-C₆)alkyle ou un groupe (C₃-C₆)cycloalkyle ;
- A₁ représente un radical -NH- ;
- Cy₁ représente un cycle aliphatique comprenant au moins 5 atomes de carbone, éventuellement substitué, et fusionné à un cycle (hétéro)aryle comprenant de 2 à 6 atomes de carbone, soit un cycle hétéroaliphatique comprenant au moins 4 atomes de carbone, éventuellement substitué, et éventuellement fusionné à un cycle (hétéro)aryle comprenant de 2 à 6 atomes de carbone ;
lesdits cycles (hétéro)aliphatiques pouvant être éventuellement substitués par au moins un substituant choisi parmi les groupes Rₐ, ORₐ, OCH₂OCH₃, C(=O)Rₐ, C(=O)ORₐ, NRₐR_{b} ou F, Rₐ et R_{b} étant tels que définis ci-dessus,
- R₂ représente H ou un substituant choisi dans le groupe constitué des atomes d'halogène, des groupes (C₁-C₆)(cyclo)alkyles, des groupes NRₐR_{b} et des groupes ORₐ, Rₐ et R_{b}, indépendamment l'un de l'autre, représentant H ou un groupe (C₁-C₆)alkyle.

8. Composé de formule (III-4) selon la revendication 6, dans laquelle :
- R₃ est H ; et
- X₁ représente un groupe CH et X₂ représente un atome d'azote.

9. Composé selon l'une quelconque des revendications 6 à 8, pour son utilisation comme médicament.

## Patentansprüche

1. Verbindung der folgenden Formel (III-4): 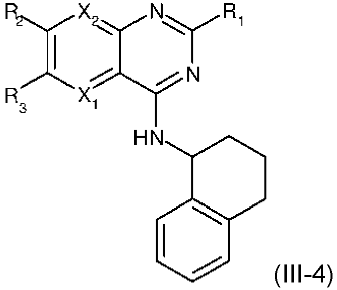 wobei:
- R₁ H, eine (C₁-C₆)-Alkylgruppe oder ein Radikal darstellt, die/das aus der Gruppe ausgewählt ist, bestehend aus: -ORₐ, -NHRₐ, -NRₐR_{b}, -NH-C(=O)Rₐ, -C(=O)Rₐ, -C(=O)ORₐ, -NH-CN, -C(=O)NRₐR_{b}, Cl, F, CN und Stickstoff- und/oder Sauerstoffheterocyclen, wobei Rₐ und R_{b} unabhängig voneinander H, eine (C₁-C₆)-Alkylgruppe oder eine (C₃-C₆)-Cycloalkylgruppe darstellen;
- oder X₁ ein Stickstoffatom darstellt und X₂ eine C(R₄)-Gruppe darstellt,
- oder X₁ eine C(R₅)-Gruppe darstellt und X₂ ein Stickstoffatom darstellt,
- oder X₁ und X₂ ein Stickstoffatom darstellen,
- R₄ und R₅ unabhängig voneinander H oder einen Substituenten, der aus der Gruppe ausgewählt ist, bestehend aus Halogenatomen, (C₁-C₆)-(Cyclo)alkylgruppen, NRₐR_{b}-Gruppen und ORₐ-Gruppen, darstellen, wobei Rₐ und R_{b} unabhängig voneinander H oder eine (C₁-C₆)-Alkylgruppe darstellen,
- R₂ und R₃, unabhängig voneinander H oder einen Substituenten, der aus der Gruppe ausgewählt ist, bestehend aus Halogenatomen, (C₁-C₆)-(Cyclo)alkylgruppen, NRₐR_{b}-Gruppen und ORₐ-Gruppen, darstellen, wobei Rₐ und R_{b} unabhängig voneinander H oder eine (C₁-C₆)-Alkylgruppe darstellen,
sowie ihre pharmazeutisch verträglichen Salze,
wobei die Verbindung der Formel (III-4) in Form eines reinen Stereoisomers oder in Form einer Mischung von Enantiomeren und/oder Diastereoisomeren, einschließlich racemischer Mischungen, vorliegt,
zur Verwendung für die Behandlung von Krebserkrankungen.

2. Verbindung für die Verwendung nach Anspruch 1, wobei R₃ H ist.

3. Verbindung für die Verwendung nach Anspruch 1, wobei R₂ und R₃ H sind.

4. Verbindung für die Verwendung nach einem der Ansprüche 1 bis 3, wobei die Verbindung ausgewählt ist aus:

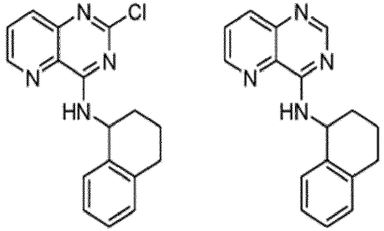

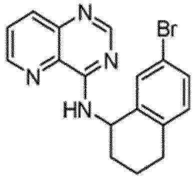

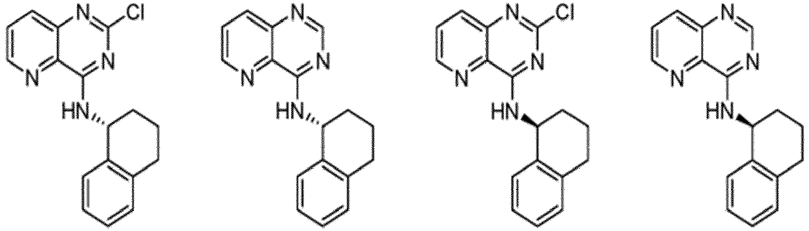

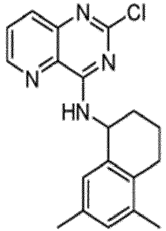

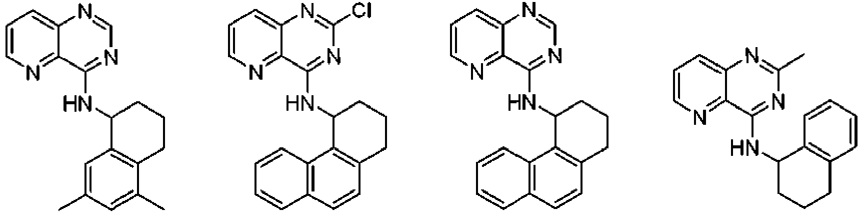

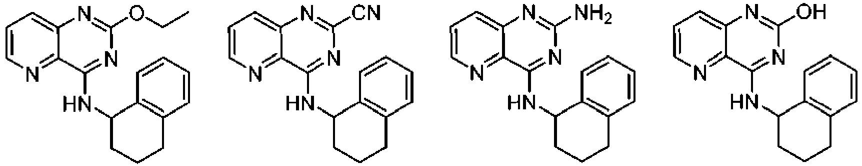

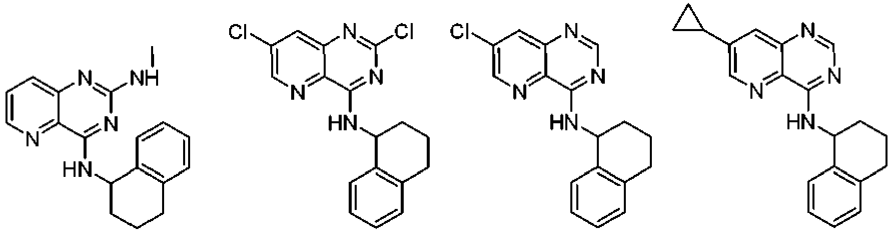

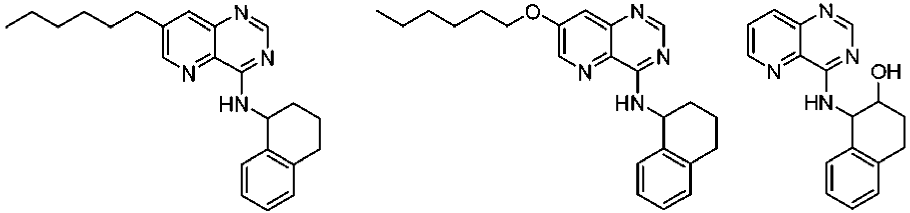

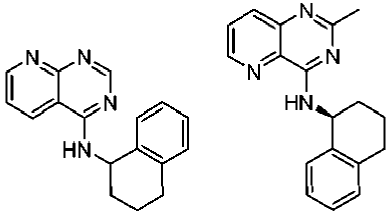

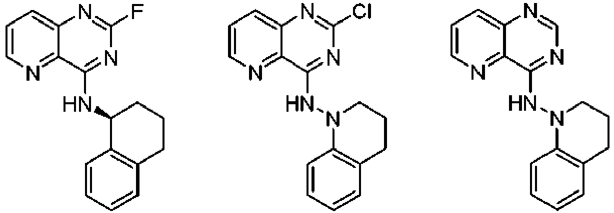

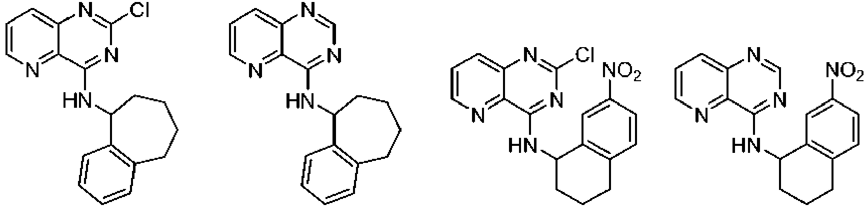

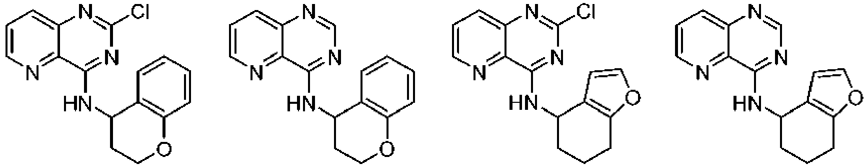

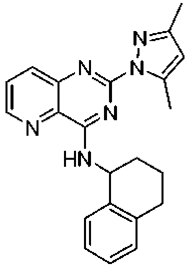

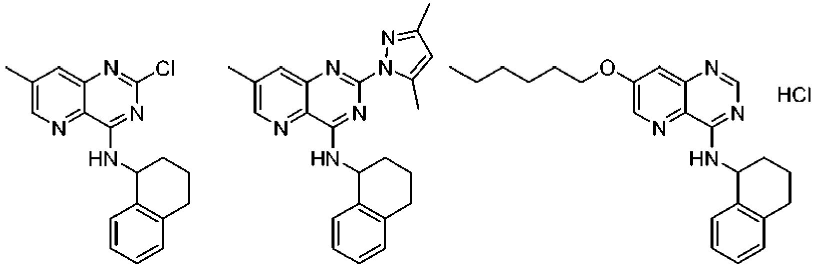

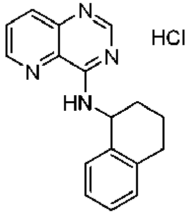

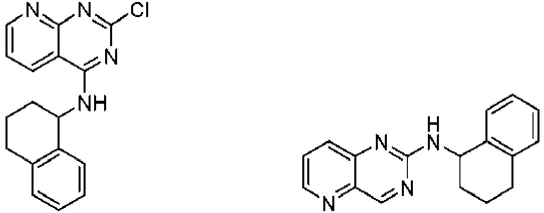

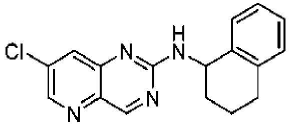

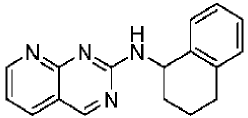

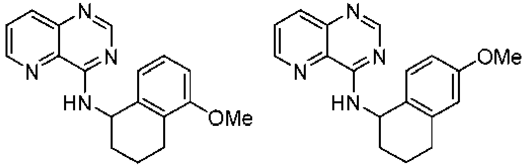

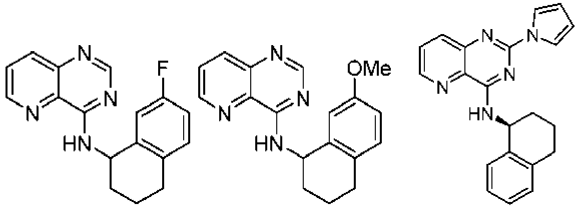

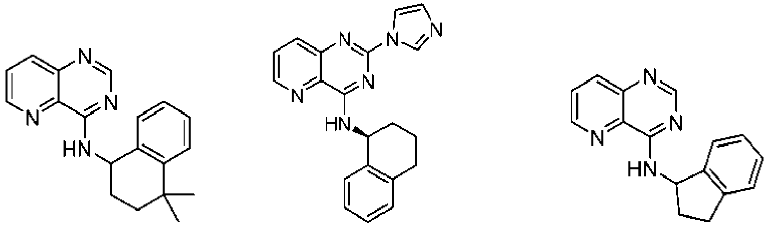

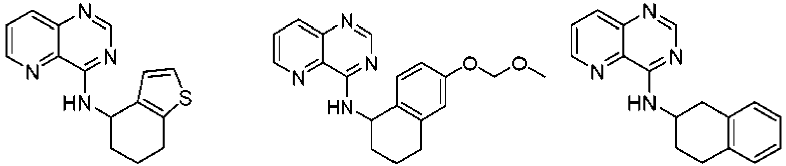

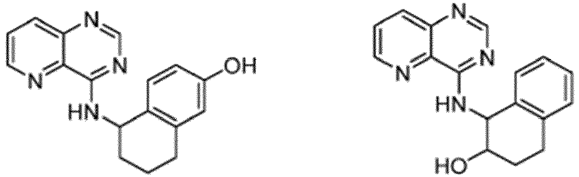

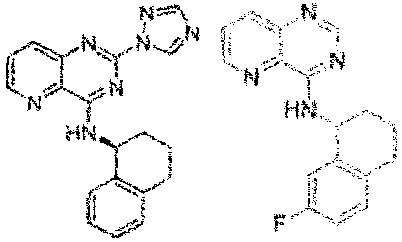

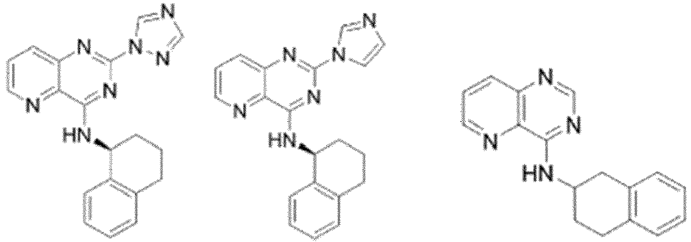

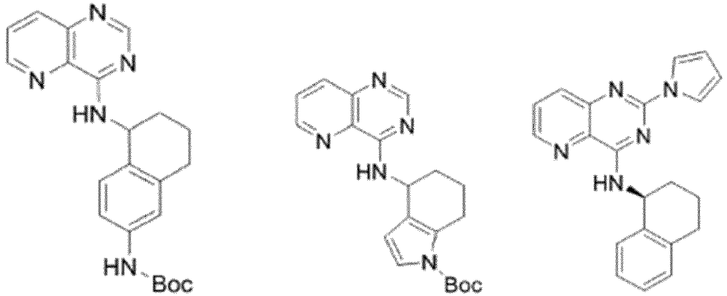

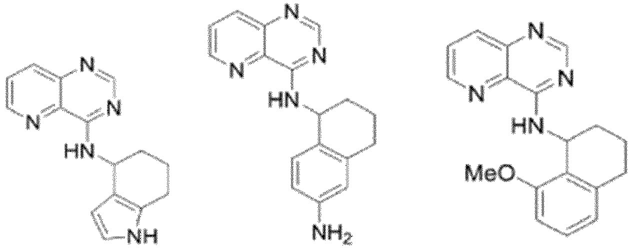

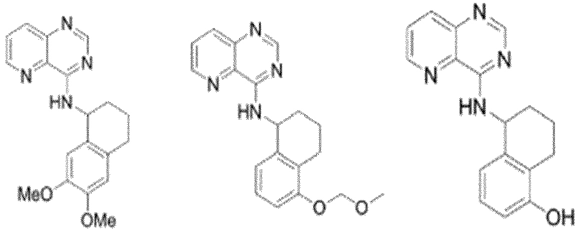

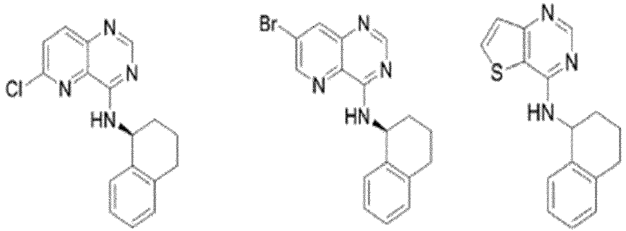

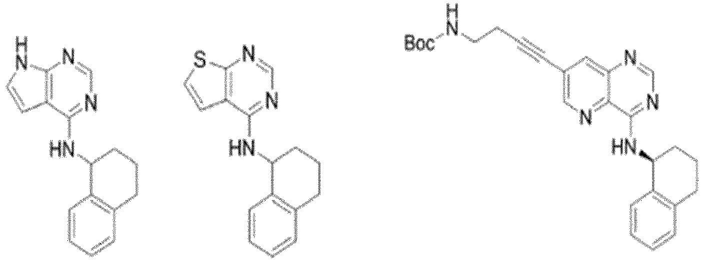

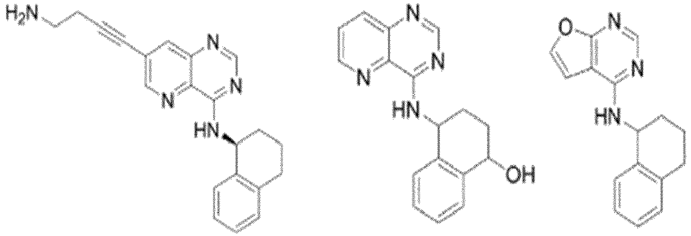

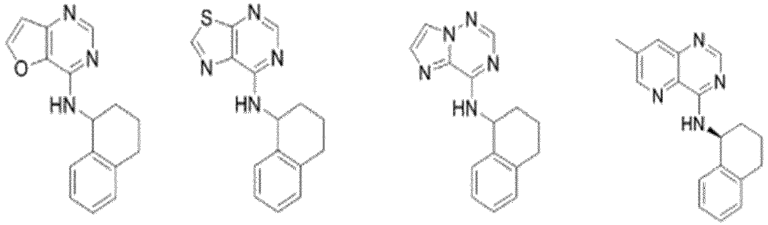

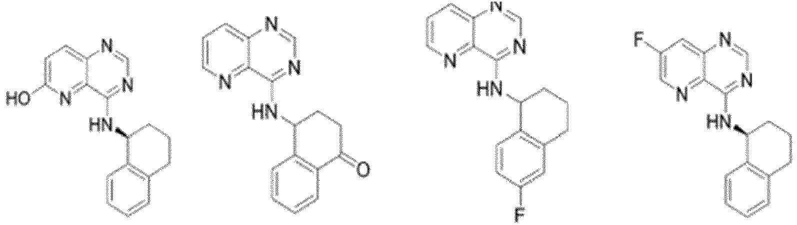

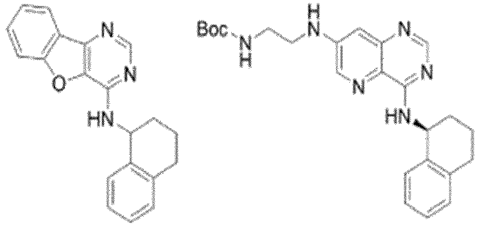

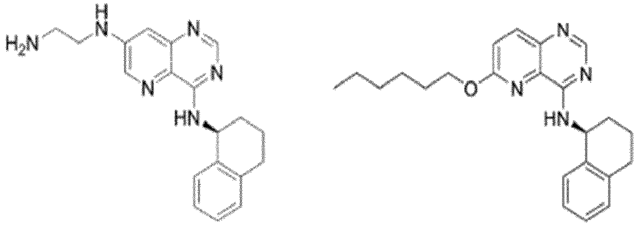

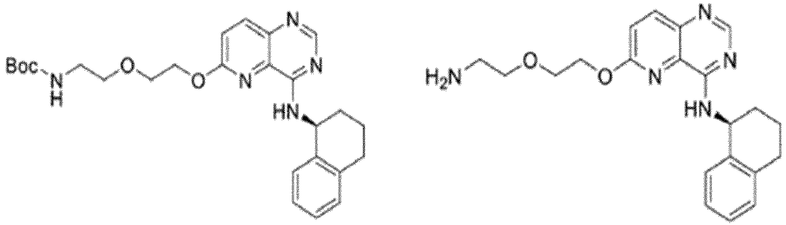

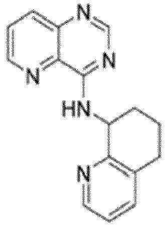

5. Verbindung zur Verwendung nach einem der Ansprüche 1 bis 4 für die Behandlung von Brustkrebs.

6. Verbindung der Formel (III-4): 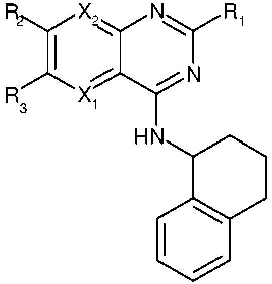 wobei:
- entweder X₁ ein Stickstoffatom darstellt und X₂ eine C(R₄)-Gruppe darstellt,
- oder X₁ eine C(R₅)-Gruppe darstellt und X₂ ein Stickstoffatom darstellt,
- R₁ H, eine (C₁-C₆)-Alkylgruppe oder ein Radikal darstellt, die/das aus der Gruppe ausgewählt ist, bestehend aus: -ORₐ, -NHRₐ, -NRₐR_{b}, -NH-C(=O)Rₐ, -C(=O)Rₐ, -C(=O)ORₐ, -NH-CN, -C(=O)NRₐR_{b}, Cl, F, CN und Stickstoff- und/oder Sauerstoffheterocyclen, wobei Rₐ und R_{b} unabhängig voneinander H, eine (C₁-C₆)-Alkylgruppe oder eine (C₃-C₆)-Cycloalkylgruppe darstellen;
- R₂, R₃, R₄ und R₅ unabhängig voneinander H oder einen Substituenten, der aus der Gruppe ausgewählt ist, bestehend aus Halogenatomen, (C₁-C₆)-(Cyclo)alkylgruppen, NRₐR_{b}-Gruppen und ORₐ-Gruppen, darstellen, wobei Rₐ und R_{b} unabhängig voneinander H oder eine (C₁-C₆)-Alkylgruppe darstellen,
sowie ihre pharmazeutisch verträglichen Salze,
wobei die Verbindung der Formel (III-4) in Form eines reinen Stereoisomers oder in Form einer Mischung von Enantiomeren und/oder Diastereoisomeren, einschließlich racemischer Mischungen, vorliegt.

7. Verbindung der Formel (VI-1): 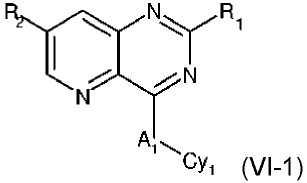 wobei:
- R₁ H, eine (C₁-C₆)-Alkylgruppe oder ein Radikal darstellt, die/das aus der Gruppe ausgewählt ist, bestehend aus: -ORₐ, -NHRₐ, -NRₐR_{b}, -NH-C(=O)Rₐ, -C(=O)Rₐ, -C(=O)ORₐ, -NH-CN, -C(=O)NRₐR_{b}, Cl, F, CN und Stickstoff- und/oder Sauerstoffheterocyclen, wobei Rₐ und R_{b} unabhängig voneinander H, eine (C₁-C₆)-Alkylgruppe oder eine (C₃-C₆)-Cycloalkylgruppe darstellen;
- A₁ ein -NH-Radikal darstellt;
- Cy₁ einen aliphatischen Ring, umfassend mindestens 5 Kohlenstoffatome, optional substituiert, und mit einem (Hetero)arylring, umfassend 2 bis 6 Kohlenstoffatome, zusammengeschmolzen ist, oder einen heteroaliphatischen Ring, umfassend mindestens 4 Kohlenstoffatome, optional substituiert, und optional mit einem (Hetero)arylring, umfassend 2 bis 6 Kohlenstoffatome, zusammengeschmolzen ist, darstellt;
wobei die (hetero)aliphatischen Ringe optional durch mindestens einen Substituenten substituiert sein können, der aus den Gruppen Rₐ, ORₐ, OCH₂OCH₃, C(=O)Rₐ, C(=O)ORₐ, NRₐR_{b} oder F ausgewählt ist, wobei Rₐ und R_{b} wie oben definiert sind,
- R₂ H oder einen Substituenten darstellt, der aus der Gruppe ausgewählt ist, bestehend aus Halogenatomen, (C₁-C₆)-(Cyclo)alkylgruppen, NRₐR_{b}-Gruppen und ORₐ-Gruppen, wobei Rₐ und R_{b} unabhängig voneinander H oder eine (C₁-C₆)-Alkylgruppe darstellen.

8. Verbindung der Formel (III-4) nach Anspruch 6, wobei:
- R₃ H ist; und
- X₁ eine CH-Gruppe darstellt und X₂ ein Stickstoffatom darstellt.

9. Verbindung nach einem der Ansprüche 6 bis 8 für die Verwendung als Arzneimittel.

## Claims

1. Compound of the following formula (III-4): 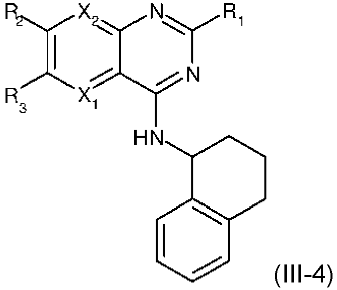 wherein:
- R₁ represents H, a (C₁-C₆) alkyl group or a radical selected from the group consisting of: -ORₐ, -NHRₐ, -NRₐR_{b}, -NH-C(=O)Rₐ, -C(=O)Rₐ, -C(=O)ORₐ, -NH-CN, -C(=O)NRₐR_{b}, Cl, F, CN and nitrogenous and/or oxygenated heterocycles, Rₐ and R_{b} each independently representing H, a (C₁-C₆) alkyl group or a (C₃-C₆) cycloalkyl group;
- either X₁ represents a nitrogen atom and X₂ represents a C(R4) group,
- or X₁ represents a C(R₅) group and X₂ represents a nitrogen atom,
- or X₁ and X₂ represent a nitrogen atom,
- R₄ and R₅ each independently represent H or a substituent selected from the group consisting of halogen atoms, (C₁-C₆) (cyclo)alkyl groups, NRₐR_{b} groups and ORₐ groups, Rₐ and R_{b} each independently representing H or a (C₁-C₆) alkyl group,
- R₂ and R₃ each independently represent H or a substituent selected from the group consisting of halogen atoms, (C₁-C₆) (cyclo)alkyl groups, NRₐR_{b} groups and ORₐ groups, Rₐ and R_{b} each independently representing H or a (C₁-C₆) alkyl group,
as well as pharmaceutically acceptable salts thereof,
said compound of formula (III-4) being in the form of a pure stereoisomer or in the form of a mixture of enantiomers and/or diastereoisomers, including racemic mixtures,
for use in the treatment of cancers.

2. Compound for use according to claim 1, wherein R₃ is H.

3. Compound for use according to claim 1, wherein R₂ and R₃ are H.

4. Compound for use according to any of claims 1 to 3, said compound being selected from:

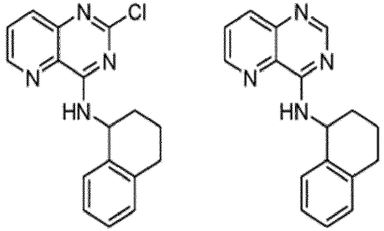

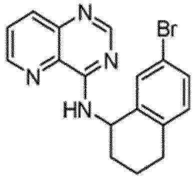

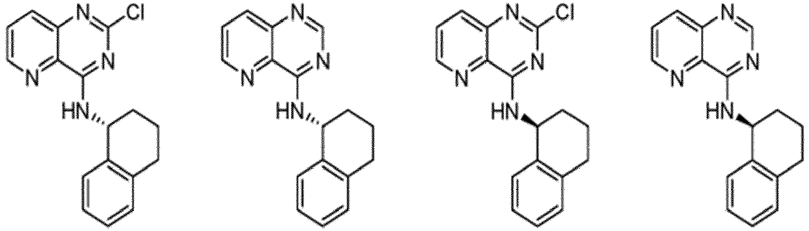

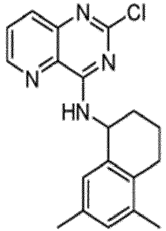

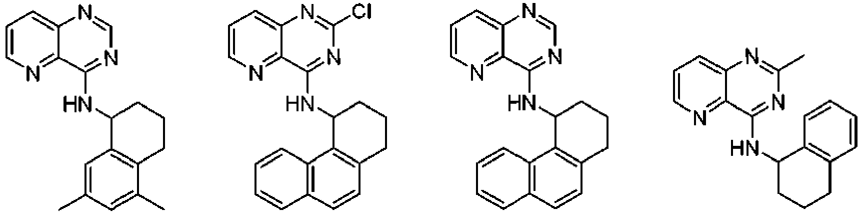

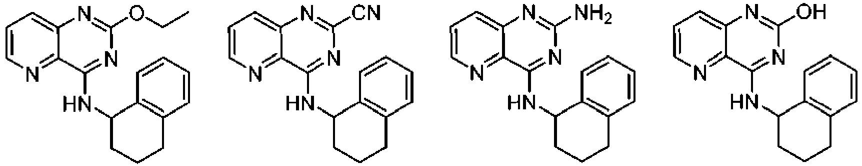

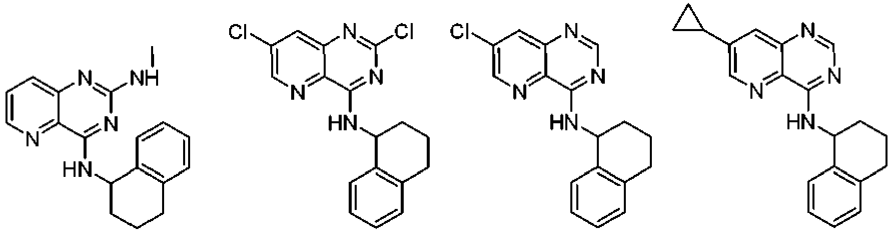

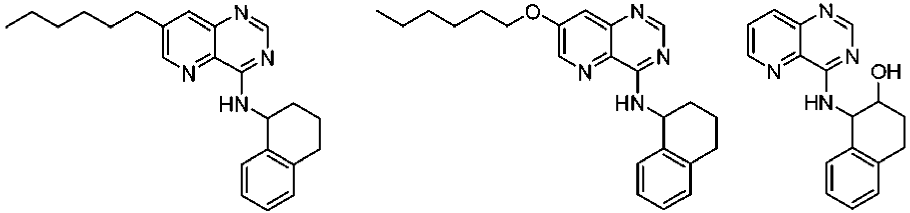

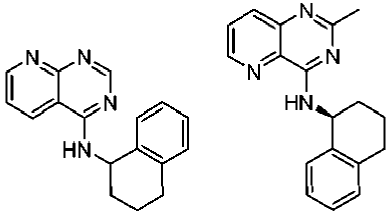

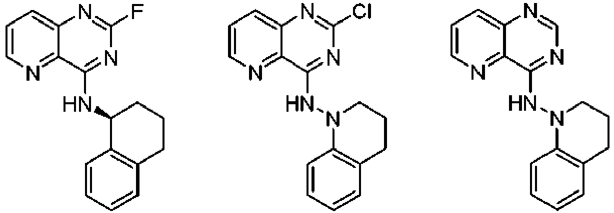

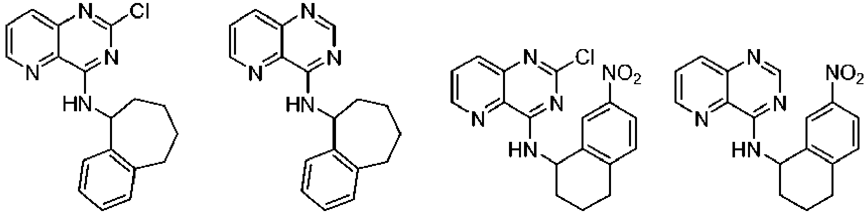

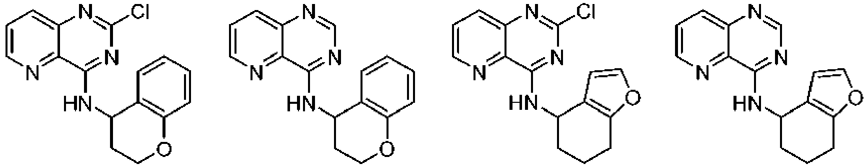

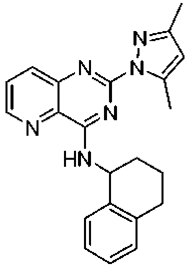

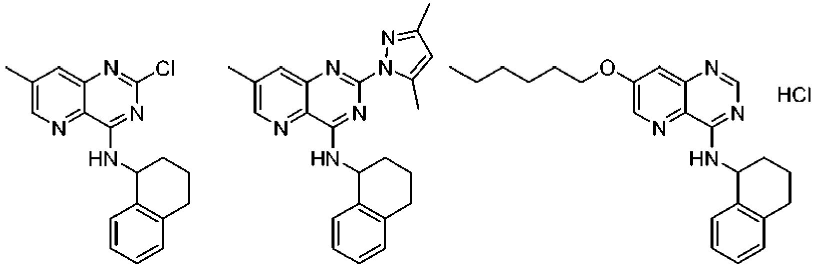

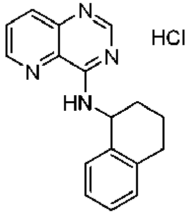

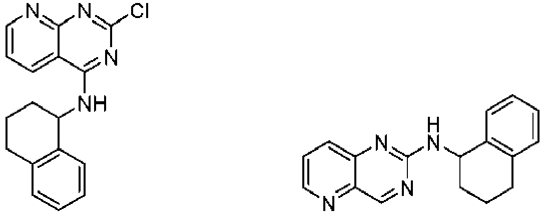

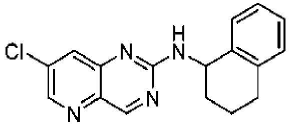

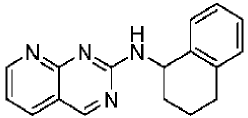

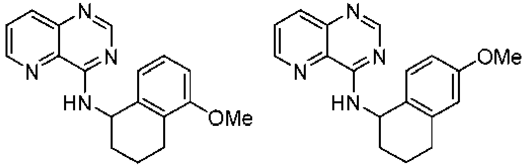

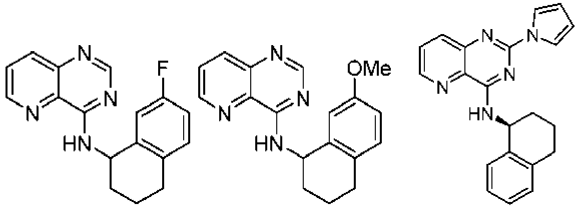

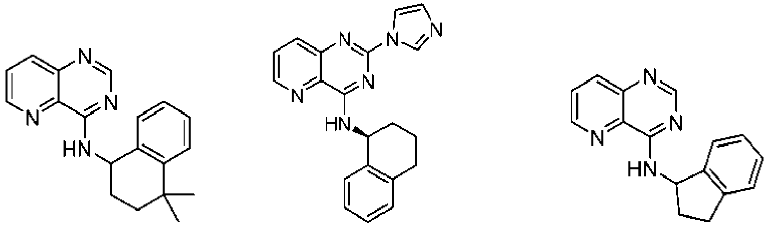

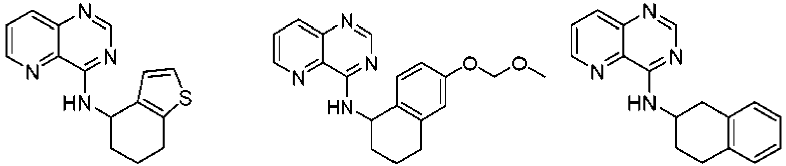

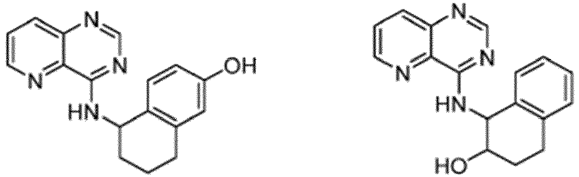

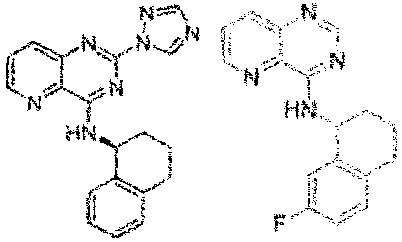

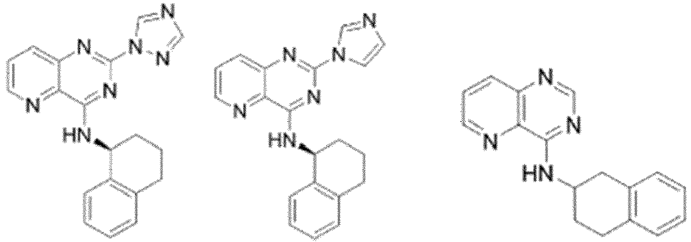

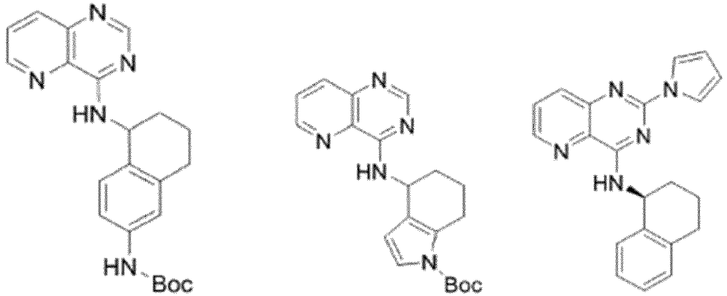

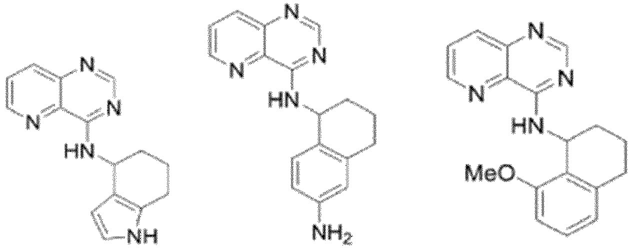

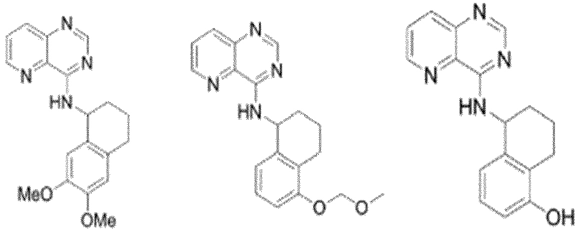

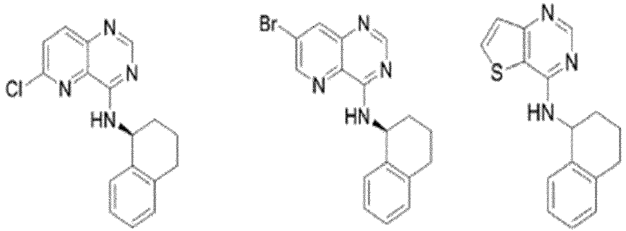

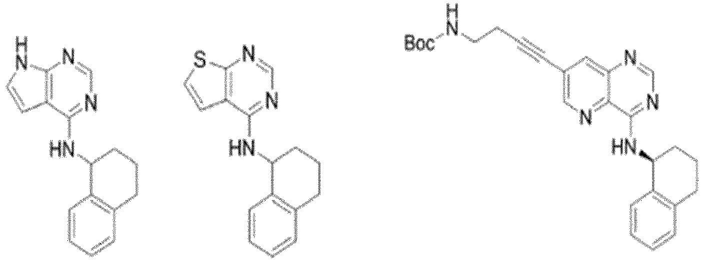

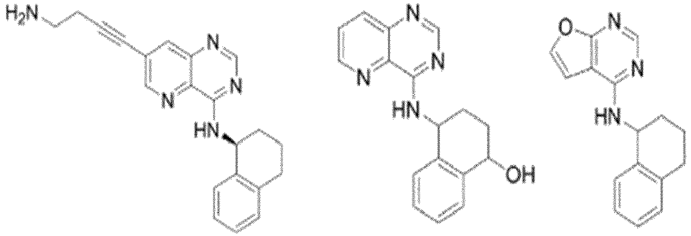

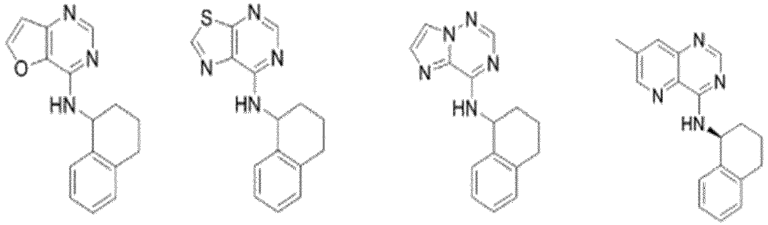

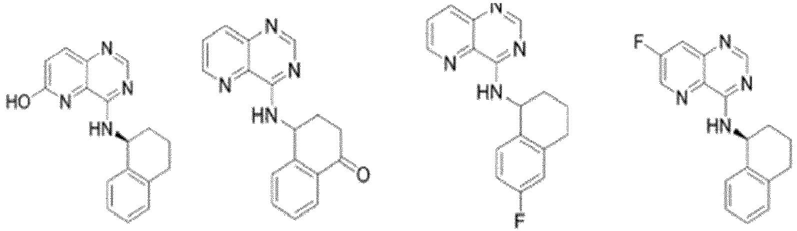

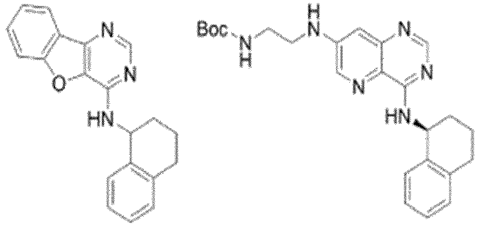

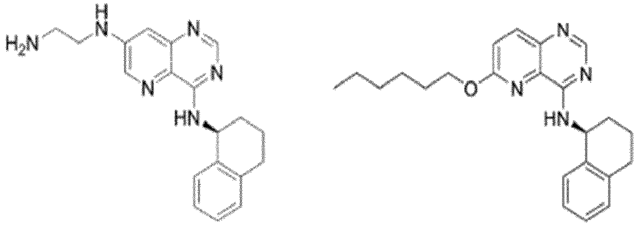

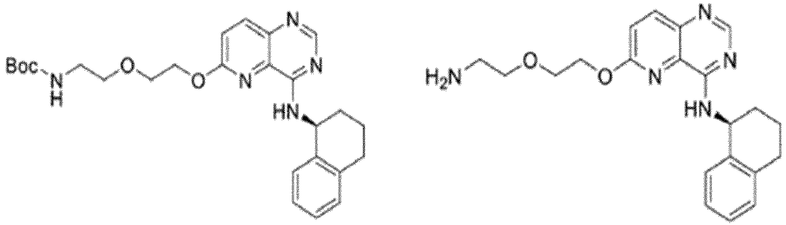

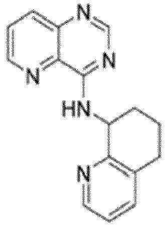

5. Compound for use according to any of claims 1 to 4, for the treatment of breast cancer.

6. Compound of formula (III-4): 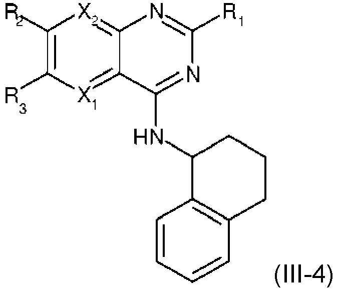 wherein:
- either X₁ represents a nitrogen atom and X₂ represents a C(R₄) group,
- or X₁ represents a C(R₅) group and X₂ represents a nitrogen atom,
- R₁ represents H, a (C₁-C₆) alkyl group or a radical selected from the group consisting of: -ORₐ, -NHRₐ, -NRₐR_{b}, -NH-C(=O)Rₐ, -C(=O)Rₐ, -C(=O)ORₐ, -NH-CN, -C(=O)NRₐR_{b}, Cl, F, CN and nitrogenous and/or oxygenated heterocycles, Rₐ and R_{b} each independently representing H, a (C₁-C₆) alkyl group or a (C₃-C₆) cycloalkyl group;
- R₂, R₃, R₄ and R₅ each independently represent H or a substituent selected from the group consisting of halogen atoms, (C₁-C₆) (cyclo)alkyl groups, NRₐR_{b} groups and ORₐ groups, Rₐ and R_{b} each independently representing H or a (C₁-C₆) alkyl group,
as well as pharmaceutically acceptable salts thereof,
said compound of formula (III-4) being in the form of a pure stereoisomer or in the form of a mixture of enantiomers and/or diastereoisomers, including racemic mixtures.

7. Compound of formula (VI-1): 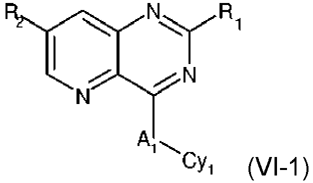 wherein:
- R₁ represents H, a (C₁-C₆) alkyl group or a radical selected from the group consisting of: -ORₐ, -NHRₐ, -NRₐR_{b}, -NH-C(=O)Rₐ, -C(=O)Rₐ, -C(=O)ORₐ, -NH-CN, -C(=O)NRₐR_{b}, Cl, F, CN and nitrogenous and/or oxygenated heterocycles, Rₐ and R_{b} each independently representing H, a (C₁-C₆) alkyl group or a (C₃-C₆) cycloalkyl group;
- A₁ represents an -NH- radical;
- Cy₁ represents an aliphatic ring comprising at least 5 carbon atoms optionally substituted and fused to a (hetero)aryl ring having 2 to 6 carbon atoms, or a heteroaliphatic ring having at least 4 carbon atoms optionally substituted and optionally fused to a (hetero)aryl ring having 2 to 6 carbon atoms;
said (hetero)aliphatic rings optionally being substituted by at least one substituent selected from the groups Rₐ, ORₐ, OCH₂OCH₃, C(=O)Rₐ, C(=O)ORₐ, NRₐR_{b} or F, Rₐand R_{b} being such as defined above,
- R₂ represents H or a substituent selected from the group consisting of halogen atoms, (C₁-C₆) (cyclo)alkyl groups, NRₐR_{b} groups and ORₐ groups, Rₐ and R_{b} each independently representing H or a (C₁-C₆) alkyl group.

8. Compound of formula (III-4) according to claim 6, wherein:
- R₃ is H; and
- X₁ represents a CH group and X₂ represents a nitrogen atom.

9. Compound according to any of claims 6 to 8, for use as a drug.
